# EUROPEAN PATENT APPLICATION

(11) **EP 4 015 537 A1**
(43) Date of publication of application: **22.06.2022**
(21) Application number: 22153762.4
(22) Date of filing: 01.12.2016
(51) Int. Cl.: C07K 16/28, A61K 47/68

(54) **COMBINATION TREATMENTS AND USES AND METHODS THEREOF**

(30) Priority: 01.12.2015 US 201562261481 P
(62) Divisional of application: 16810068.3
(71) Applicant: GlaxoSmithKline Intellectual Property Development Limited, Brentford, Middlesex TW8 9GS (GB)
(72) Inventor: MAYES, Patrick A., Collegeville, PA 19426 (US); SEESTALLER-WEHR, Laura M., Collegeville, PA 19426 (US); TSVETKOV, Lyuben, Collegeville, PA 19426 (US); YANAMANDRA, Niranjan, Collegeville, PA 19426 (US); YANG, Jingsong, Collegeville, PA 19426 (US)
(74) Representative: Lee, Alison Lesley

(57) **Abstract**

Disclosed herein are combinations of an antigen binding protein that binds BCMA with a antigen binding protein that bind to an immunomodulatory agents, such as PD-1 or OX40, pharmaceutical compositions thereof, uses thereof, and methods of treatment comprising administering said combinations, including uses in cancer.

SEQUENCE LISTING

<110> GLAXOSMITHKLINE INTELLECTUAL PROPERTY DEVELOPMENT LIMITED

<120> COMBINATION TREATMENTS AND USES AND METHODS THEREOF

<130> PB65987 WO

<140>
<141>

<150> 62/261,481
<151> 2015-12-01

<160> 244

<170> PatentIn version 3.5

<210> 1
<211> 5
<212> PRT
<213> Mus musculus

<400> 1
Asn Tyr Trp Met His
1               5

<210> 2
<211> 17
<212> PRT
<213> Mus musculus

<400> 2
Ala Thr Tyr Arg Gly His Ser Asp Thr Tyr Tyr Asn Gln Lys Phe Lys
1               5                   10                  15

Gly

<210> 3
<211> 12
<212> PRT
<213> Mus musculus

<400> 3
Gly Ala Ile Tyr Asn Gly Tyr Asp Val Leu Asp Asn
1               5                   10

<210> 4
<211> 11
<212> PRT
<213> Mus musculus

<400> 4
Ser Ala Ser Gln Asp Ile Ser Asn Tyr Leu Asn
1               5                   10

<210> 5

<211> 7
<212> PRT
<213> Mus musculus

<400> 5
Tyr Thr Ser Asn Leu His Ser
1               5


<210> 6
<211> 9
<212> PRT
<213> Mus musculus

<400> 6
Gln Gln Tyr Arg Lys Leu Pro Trp Thr
1               5


<210> 7
<211> 121
<212> PRT
<213> Mus musculus

<400> 7
Glu Val Gln Leu Gln Gln Ser Gly Ala Val Leu Ala Arg Pro Gly Ala
1               5                   10                  15

Ser Val Lys Met Ser Cys Lys Gly Ser Gly Tyr Thr Phe Thr Asn Tyr
            20                  25                  30

Trp Met His Trp Val Lys Gln Arg Pro Gly Gln Gly Leu Glu Trp Ile
        35                  40                  45

Gly Ala Thr Tyr Arg Gly His Ser Asp Thr Tyr Tyr Asn Gln Lys Phe
    50                  55                  60

Lys Gly Lys Ala Lys Leu Thr Ala Val Thr Ser Thr Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Gly Ala Ile Tyr Asn Gly Tyr Asp Val Leu Asp Asn Trp Gly
            100                 105                 110

Gln Gly Thr Leu Val Thr Val Ser Ser
        115                 120


<210> 8
<211> 363
<212> DNA
<213> Mus musculus

<400> 8

gaggtgcagc tgcagcagag cggcgccgtg ctggccaggc ccggagctag cgtgaagatg 60

agctgcaagg gcagcggcta caccttcacc aactactgga tgcactgggt gaaacagagg 120

cccggccagg gactggagtg gatcggcgcc acctacaggg gccacagcga cacctactac 180

aaccagaagt tcaagggcaa ggccaagctg accgccgtga cctcaaccag caccgcctac 240

atggaactga gcagcctgac caacgaggac agcgccgtct attactgcac caggggcgcc 300

atctacaacg gctacgacgt gctggacaat tggggccagg gaacactagt gaccgtgtcc 360

agc 363

<210> 9
<211> 108
<212> PRT
<213> Mus musculus

<400> 9
Asp Ile Gln Leu Thr Gln Thr Thr Ser Ser Leu Ser Ala Ser Leu Gly
1               5                   10                  15

Asp Arg Val Thr Ile Ser Cys Ser Ala Ser Gln Asp Ile Ser Asn Tyr
            20                  25                  30

Leu Asn Trp Tyr Gln Gln Lys Pro Asp Gly Thr Val Glu Leu Val Ile
        35                  40                  45

Tyr Tyr Thr Ser Asn Leu His Ser Gly Val Pro Ser Arg Phe Ser Gly
    50                  55                  60

Ser Gly Ser Gly Thr Asp Tyr Ser Leu Thr Ile Gly Tyr Leu Glu Pro
65                  70                  75                  80

Glu Asp Val Ala Thr Tyr Tyr Cys Gln Gln Tyr Arg Lys Leu Pro Trp
                85                  90                  95

Thr Phe Gly Gly Gly Ser Lys Leu Glu Ile Lys Arg
            100                 105

<210> 10
<211> 324
<212> DNA
<213> Mus musculus

<400> 10
gatatccagc tgacccagac cacaagcagc ctgagcgcct ccctgggcga cagggtgacc 60

attagctgca gcgccagcca ggacatcagc aactacctga actggtacca gcagaagccc 120

gacggcaccg tggagctcgt gatctactac acctccaacc tgcacagcgg cgtgcccagc 180

aggttctctg gcagcggcag cggcaccgac tacagcctga ccatcggcta tctggagccc 240

gaggacgtcg ccacctacta ctgccagcag tacaggaagc tgccctggac cttcggcgga 300

ggctctaagc tggagattaa gcgt 324

<210> 11
<211> 121
<212> PRT
<213> Artificial Sequence

<220>
<221> source
<223> /note="Description of Artificial Sequence: Synthetic
      humanized antibody sequence"

<400> 11
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Ile Tyr Asn Gly Tyr Asp Val Leu Asp Asn Trp Gly
            100                 105                 110

Gln Gly Thr Leu Val Thr Val Ser Ser
        115                 120

<210> 12
<211> 363
<212> DNA
<213> Artificial Sequence

<220>
<221> source
<223> /note="Description of Artificial Sequence: Synthetic
      humanized antibody sequence"

<400> 12
caggtgcagc tggtccagag cggcgccgaa gtgaagaagc ccggcagctc cgtgaaagtg 60

agctgcaagg ccagcggcgg caccttcagc aactactgga tgcactgggt gaggcaggcc 120

cccggacagg gcctggagtg gatgggcgcc acctacaggg gccacagcga cacctactac      180

aaccagaagt tcaagggccg ggtgaccatc accgccgaca agagcaccag caccgcctac      240

atggaactga gcagcctcag gagcgaggac accgctgtgt attactgcgc caggggcgcc      300

atctacaacg gctacgacgt gctggacaac tggggccagg gcacactagt gaccgtgtcc      360

agc                                                                    363

<210> 13
<211> 121
<212> PRT
<213> Artificial Sequence

<220>
<221> source
<223> /note="Description of Artificial Sequence: Synthetic
      humanized antibody sequence"

<400> 13
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Ile Tyr Asn Gly Tyr Asp Val Leu Asp Asn Trp Gly
            100                 105                 110

Gln Gly Thr Leu Val Thr Val Ser Ser
        115                 120

<210> 14
<211> 363
<212> DNA
<213> Artificial Sequence

<220>
<221> source
<223> /note="Description of Artificial Sequence: Synthetic
      humanized antibody sequence"

<400> 14
caggtgcagc tggtccagag cggcgccgaa gtgaagaagc ccggcagctc cgtgaaagtg      60

agctgcaagg ccagcggcta caccttcacc aactactgga tgcactgggt gaggcaggcc     120

cccggacagg gcctggagtg gatgggcgcc acctacaggg gccacagcga cacctactac     180

aaccagaagt tcaagggccg ggtgaccatc accgccgaca agagcaccag caccgcctac     240

atggaactga gcagcctcag gagcgaggac accgctgtgt attactgcgc caggggcgcc     300

atctacaacg gctacgacgt gctggacaac tggggccagg gcacactagt gaccgtgtcc     360

agc                                                                   363

<210> 15
<211> 121
<212> PRT
<213> Artificial Sequence

<220>
<221> source
<223> /note="Description of Artificial Sequence: Synthetic
      humanized antibody sequence"

<400> 15
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asn Gly Tyr Asp Val Leu Asp Asn Trp Gly
            100                 105                 110

Gln Gly Thr Leu Val Thr Val Ser Ser
        115                 120

<210> 16
<211> 363
<212> DNA

<213> Artificial Sequence

<220>
<221> source
<223> /note="Description of Artificial Sequence: Synthetic
      humanized antibody sequence"

<400> 16
caggtgcagc tggtccagag cggcgccgaa gtgaagaagc ccggcagctc cgtgaaagtg        60

agctgcaagg ccagcggcta caccttcacc aactactgga tgcactgggt gaggcaggcc       120

cccggacagg gcctggagtg gatgggcgcc acctacaggg gccacagcga cacctactac       180

aaccagaagt tcaagggccg ggtgaccatc accgccgaca agagcaccag caccgcctac       240

atggaactga gcagcctcag gagcgaggac accgctgtgt attactgcac caggggcgcc       300

atctacaacg gctacgacgt gctggacaac tggggccagg gcacactagt gaccgtgtcc       360

agc                                                                     363


<210> 17
<211> 121
<212> PRT
<213> Artificial Sequence

<220>
<221> source
<223> /note="Description of Artificial Sequence: Synthetic
      humanized antibody sequence"

<400> 17
Gln Val Gln Leu Val Gln Ser Gly Ala Glu Val Lys Lys Pro Gly Ser
1               5                   10                  15

Ser Val Lys Val Ser Cys Lys Gly Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asn Gly Tyr Asp Val Leu Asp Asn Trp Gly
            100                 105                 110

Gln Gly Thr Leu Val Thr Val Ser Ser

115                 120

<210> 18
<211> 363
<212> DNA
<213> Artificial Sequence

<220>
<221> source
<223> /note="Description of Artificial Sequence: Synthetic
      humanized antibody sequence"

<400> 18
caggtgcagc tggtccagag cggcgccgaa gtgaagaagc ccggcagctc cgtgaaagtg      60

agctgcaagg gcagcggcta caccttcacc aactactgga tgcactgggt gaggcaggcc     120

cccggacagg gcctggagtg gatgggcgcc acctacaggg gccacagcga cacctactac     180

aaccagaagt tcaagggccg ggtgaccatc accgccgaca cgagcaccag caccgcctac     240

atggaactga gcagcctcag gagcgaggac accgctgtgt attactgcac caggggcgcc     300

atctacaacg gctacgacgt gctggacaac tggggccagg gcacactagt gaccgtgtcc     360

agc                                                                   363

<210> 19
<211> 121
<212> PRT
<213> Artificial Sequence

<220>
<221> source
<223> /note="Description of Artificial Sequence: Synthetic
      humanized antibody sequence"

<400> 19
Gln Val Gln Leu Val Gln Ser Gly Ala Glu Val Lys Lys Pro Gly Ser
1               5                   10                  15

Ser Val Lys Val Ser Cys Lys Gly Ser Gly Tyr Thr Phe Thr Asn Tyr
            20                  25                  30

Trp Met His Trp Val Arg Gln Ala Pro Gly Gln Gly Leu Glu Trp Ile
        35                  40                  45

Gly Ala Thr Tyr Arg Gly His Ser Asp Thr Tyr Tyr Asn Gln Lys Phe
    50                  55                  60

Lys Gly Arg Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asn Gly Tyr Asp Val Leu Asp Asn Trp Gly
            100                 105                 110

Gln Gly Thr Leu Val Thr Val Ser Ser
        115                 120

<210> 20
<211> 363
<212> DNA
<213> Artificial Sequence

<220>
<221> source
<223> /note="Description of Artificial Sequence: Synthetic
      humanized antibody sequence"

<400> 20
caggtgcagc tggtccagag cggcgccgaa gtgaagaagc ccggcagctc cgtgaaagtg      60

agctgcaagg gcagcggcta caccttcacc aactactgga tgcactgggt gaggcaggcc     120

cccggacagg gcctggagtg gatcggcgcc acctacaggg gccacagcga cacctactac     180

aaccagaagt tcaagggccg ggcgaccctc accgccgaca cgagcaccag caccgcctac     240

atggaactga gcagcctcag gagcgaggac accgctgtgt attactgcac caggggcgcc     300

atctacaacg gctacgacgt gctggacaac tggggccagg gcacactagt gaccgtgtcc     360

agc                                                                   363

<210> 21
<211> 121
<212> PRT
<213> Artificial Sequence

<220>
<221> source
<223> /note="Description of Artificial Sequence: Synthetic
      humanized antibody sequence"

<400> 21
Gln Val Gln Leu Val Gln Ser Gly Ala Glu Val Lys Lys Pro Gly Ser
1               5                   10                  15

Ser Val Lys Val Ser Cys Lys Gly Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Thr Ser Thr Ser Thr Ala Tyr

65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asp Gly Tyr Asp Val Leu Asp Asn Trp Gly
            100                 105                 110

Gln Gly Thr Leu Val Thr Val Ser Ser
        115                 120

<210> 22
<211> 363
<212> DNA
<213> Artificial Sequence

<220>
<221> source
<223> /note="Description of Artificial Sequence: Synthetic
      humanized antibody sequence"

<400> 22
caggtgcagc tggtccagag cggcgccgaa gtgaagaagc ccggcagctc cgtgaaagtg        60

agctgcaagg gcagcggcta caccttcacc aactactgga tgcactgggt gaggcaggcc       120

cccggacagg gcctggagtg gatgggcgcc acctacaggg gccacagcga cacctactac       180

aaccagaagt tcaagggccg ggtgaccatc accgccgaca cgagcaccag caccgcctac       240

atggaactga gcagcctcag gagcgaggac accgctgtgt attactgcac caggggcgcc       300

atctacgacg gctacgacgt gctggacaac tggggccagg gcacactagt gaccgtgtcc       360

agc                                                                     363

<210> 23
<211> 121
<212> PRT
<213> Artificial Sequence

<220>
<221> source
<223> /note="Description of Artificial Sequence: Synthetic
      humanized antibody sequence"

<400> 23
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Ile Tyr Asp Gly Tyr Asp Val Leu Asp Asn Trp Gly
            100                 105                 110

Gln Gly Thr Leu Val Thr Val Ser Ser
        115                 120

<210> 24
<211> 363
<212> DNA
<213> Artificial Sequence

<220>
<221> source
<223> /note="Description of Artificial Sequence: Synthetic
      humanized antibody sequence"

<400> 24
caggtgcagc tggtccagag cggcgccgaa gtgaagaagc ccggcagctc cgtgaaagtg      60

agctgcaagg ccagcggcgg caccttcagc aactactgga tgcactgggt gaggcaggcc     120

cccggacagg gcctggagtg gatgggcgcc acctacaggg gccacagcga cacctactac     180

aaccagaagt tcaagggccg ggtgaccatc accgccgaca agagcaccag caccgcctac     240

atggaactga gcagcctcag gagcgaggac accgctgtgt attactgcgc caggggcgcc     300

atctacgacg gctacgacgt gctggacaac tggggccagg gcacactagt gaccgtgtcc     360

agc                                                                   363

<210> 25
<211> 121
<212> PRT
<213> Artificial Sequence

<220>
<221> source
<223> /note="Description of Artificial Sequence: Synthetic
      humanized antibody sequence"

<400> 25
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr

20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Ile Tyr Asp Gly Tyr Asp Val Leu Asp Asn Trp Gly
            100                 105                 110

Gln Gly Thr Leu Val Thr Val Ser Ser
        115                 120

<210> 26
<211> 363
<212> DNA
<213> Artificial Sequence

<220>
<221> source
<223> /note="Description of Artificial Sequence: Synthetic
      humanized antibody sequence"

<400> 26
caggtgcagc tggtccagag cggcgccgaa gtgaagaagc ccggcagctc cgtgaaagtg      60

agctgcaagg ccagcggcta caccttcacc aactactgga tgcactgggt gaggcaggcc     120

cccggacagg gcctggagtg gatgggcgcc acctacaggg gccacagcga cacctactac     180

aaccagaagt tcaagggccg ggtgaccatc accgccgaca agagcaccag caccgcctac     240

atggaactga gcagcctcag gagcgaggac accgctgtgt attactgcgc caggggcgcc     300

atctacgacg gctacgacgt gctggacaac tggggccagg gcacactagt gaccgtgtcc     360

agc                                                                   363

<210> 27
<211> 121
<212> PRT
<213> Artificial Sequence

<220>
<221> source
<223> /note="Description of Artificial Sequence: Synthetic
      humanized antibody sequence"

<400> 27
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asp Gly Tyr Asp Val Leu Asp Asn Trp Gly
            100                 105                 110

Gln Gly Thr Leu Val Thr Val Ser Ser
        115                 120

<210> 28
<211> 363
<212> DNA
<213> Artificial Sequence

<220>
<221> source
<223> /note="Description of Artificial Sequence: Synthetic
      humanized antibody sequence"

<400> 28
caggtgcagc tggtccagag cggcgccgaa gtgaagaagc ccggcagctc cgtgaaagtg      60

agctgcaagg ccagcggcta caccttcacc aactactgga tgcactgggt gaggcaggcc     120

cccggacagg gcctggagtg gatgggcgcc acctacaggg gccacagcga cacctactac     180

aaccagaagt tcaagggccg ggtgaccatc accgccgaca agagcaccag caccgcctac     240

atggaactga gcagcctcag gagcgaggac accgctgtgt attactgcac caggggcgcc     300

atctacgacg gctacgacgt gctggacaac tggggccagg gcacactagt gaccgtgtcc     360

agc                                                                   363

<210> 29
<211> 121
<212> PRT
<213> Artificial Sequence

<220>
<221> source
<223> /note="Description of Artificial Sequence: Synthetic
      humanized antibody sequence"

<400> 29
Gln Val Gln Leu Val Gln Ser Gly Ala Glu Val Lys Lys Pro Gly Ser
1               5                   10                  15

Ser Val Lys Val Ser Cys Lys Gly Ser Gly Tyr Thr Phe Thr Asn Tyr
            20                  25                  30

Trp Met His Trp Val Arg Gln Ala Pro Gly Gln Gly Leu Glu Trp Ile
        35                  40                  45

Gly Ala Thr Tyr Arg Gly His Ser Asp Thr Tyr Tyr Asn Gln Lys Phe
    50                  55                  60

Lys Gly Arg Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asp Gly Tyr Asp Val Leu Asp Asn Trp Gly
            100                 105                 110

Gln Gly Thr Leu Val Thr Val Ser Ser
        115                 120


<210> 30
<211> 363
<212> DNA
<213> Artificial Sequence

<220>
<221> source
<223> /note="Description of Artificial Sequence: Synthetic
      humanized antibody sequence"

<400> 30
caggtgcagc tggtccagag cggcgccgaa gtgaagaagc ccggcagctc cgtgaaagtg      60

agctgcaagg gcagcggcta caccttcacc aactactgga tgcactgggt gaggcaggcc     120

cccggacagg gcctggagtg gatcggcgcc acctacaggg gccacagcga cacctactac     180

aaccagaagt tcaagggccg ggcgaccctc accgccgaca cgagcaccag caccgcctac     240

atggaactga gcagcctcag gagcgaggac accgctgtgt attactgcac caggggcgcc     300

atctacgacg gctacgacgt gctggacaac tggggccagg gcacactagt gaccgtgtcc     360

agc                                                                   363

<210> 31
<211> 108
<212> PRT
<213> Artificial Sequence

<220>
<221> source
<223> /note="Description of Artificial Sequence: Synthetic
      humanized antibody sequence"

<400> 31
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Asn Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Arg Lys Leu Pro Trp
                85                  90                  95

Thr Phe Gly Gln Gly Thr Lys Leu Glu Ile Lys Arg
            100                 105

<210> 32
<211> 324
<212> DNA
<213> Artificial Sequence

<220>
<221> source
<223> /note="Description of Artificial Sequence: Synthetic
      humanized antibody sequence"

<400> 32
gacatccaga tgacccagag ccctagctca ctgagcgcca gcgtgggcga cagggtgacc      60

attacctgct ccgccagcca ggacatcagc aactacctga actggtacca gcagaagccc     120

ggcaaggccc ccaagctgct gatctactac acctccaacc tgcactccgg cgtgcccagc     180

aggttcagcg gaagcggcag cggcaccgat ttcaccctga ccatctccag cctgcagccc     240

gaggacttcg ccacctacta ctgccagcag tacaggaagc tcccctggac tttcggccag     300

ggcaccaaac tggagatcaa gcgt                                            324

<210> 33
<211> 108
<212> PRT
<213> Artificial Sequence

<220>
<221> source
<223> /note="Description of Artificial Sequence: Synthetic
      humanized antibody sequence"

<400> 33
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Asn Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Tyr Arg Lys Leu Pro Trp
                85                  90                  95

Thr Phe Gly Gln Gly Thr Lys Leu Glu Ile Lys Arg
            100                 105


<210> 34
<211> 324
<212> DNA
<213> Artificial Sequence

<220>
<221> source
<223> /note="Description of Artificial Sequence: Synthetic
      humanized antibody sequence"

<400> 34
gacatccaga tgacccagag ccctagctca ctgagcgcca gcgtgggcga cagggtgacc      60

attacctgct ccgccagcca ggacatcagc aactacctga actggtacca gcagaagccc     120

ggcaaggccc ccaagctgct gatctactac acctccaacc tgcactccgg cgtgcccagc     180

aggttcagcg gaagcggcag cggcaccgat tacaccctga ccatctccag cctgcagccc     240

gaggacttcg ccacctacta ctgccagcag tacaggaagc tcccctggac tttcggccag     300

ggcaccaaac tggagatcaa gcgt                                            324

<210> 35
<211> 108
<212> PRT
<213> Artificial Sequence

<220>
<221> source
<223> /note="Description of Artificial Sequence: Synthetic
      humanized antibody sequence"

<400> 35
Asp Ile Gln Leu Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Glu Leu Val Ile
        35                  40                  45

Tyr Tyr Thr Ser Asn Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Tyr Arg Lys Leu Pro Trp
                85                  90                  95

Thr Phe Gly Gln Gly Thr Lys Leu Glu Ile Lys Arg
            100                 105


<210> 36
<211> 324
<212> DNA
<213> Artificial Sequence

<220>
<221> source
<223> /note="Description of Artificial Sequence: Synthetic
      humanized antibody sequence"

<400> 36
gacatccagc tgacccagag ccctagctca ctgagcgcca gcgtgggcga cagggtgacc      60

attacctgct ccgccagcca ggacatcagc aactacctga actggtacca gcagaagccc     120

ggcaaggccc ccgagctggt gatctactac acctccaacc tgcactccgg cgtgcccagc     180

aggttcagcg gaagcggcag cggcaccgat tacaccctga ccatctccag cctgcagccc     240

gaggacttcg ccacctacta ctgccagcag tacaggaagc tcccctggac tttcggccag     300

ggcaccaaac tggagatcaa gcgt                                            324

<210> 37
<211> 310
<212> PRT
<213> Homo sapiens

<400> 37
Met Pro Leu Leu Leu Leu Leu Pro Leu Leu Trp Ala Gly Ala Leu Ala
1               5                   10                  15

Met Leu Gln Met Ala Gly Gln Cys Ser Gln Asn Glu Tyr Phe Asp Ser
            20                  25                  30

Leu Leu His Ala Cys Ile Pro Cys Gln Leu Arg Cys Ser Ser Asn Thr
        35                  40                  45

Pro Pro Leu Thr Cys Gln Arg Tyr Cys Asn Ala Ser Val Thr Asn Ser
    50                  55                  60

Val Lys Gly Thr Asn Ser Gly Glu Asn Leu Tyr Phe Gln Gly Asp Pro
65                  70                  75                  80

Lys Ser Cys Asp Lys Thr His Thr Cys Pro Pro Cys Pro Ala Pro Glu
                85                  90                  95

Leu Leu Gly Gly Pro Ser Val Phe Leu Phe Pro Pro Lys Pro Lys Asp
            100                 105                 110

Thr Leu Met Ile Ser Arg Thr Pro Glu Val Thr Cys Val Val Val Asp
        115                 120                 125

Val Ser His Glu Asp Pro Glu Val Lys Phe Asn Trp Tyr Val Asp Gly
    130                 135                 140

Val Glu Val His Asn Ala Lys Thr Lys Pro Arg Glu Glu Gln Tyr Asn
145                 150                 155                 160

Ser Thr Tyr Arg Val Val Ser Val Leu Thr Val Leu His Gln Asp Trp
                165                 170                 175

Leu Asn Gly Lys Glu Tyr Lys Cys Lys Val Ser Asn Lys Ala Leu Pro
            180                 185                 190

Ala Pro Ile Glu Lys Thr Ile Ser Lys Ala Lys Gly Gln Pro Arg Glu
        195                 200                 205

Pro Gln Val Tyr Thr Leu Pro Pro Ser Arg Asp Glu Leu Thr Lys Asn
    210                 215                 220

Gln Val Ser Leu Thr Cys Leu Val Lys Gly Phe Tyr Pro Ser Asp Ile
225                 230                 235                 240

Ala Val Glu Trp Glu Ser Asn Gly Gln Pro Glu Asn Asn Tyr Lys Thr
                245                 250                 255

Thr Pro Pro Val Leu Asp Ser Asp Gly Ser Phe Phe Leu Tyr Ser Lys
            260                 265                 270

Leu Thr Val Asp Lys Ser Arg Trp Gln Gln Gly Asn Val Phe Ser Cys
        275                 280                 285

Ser Val Met His Glu Ala Leu His Asn His Tyr Thr Gln Lys Ser Leu
    290                 295                 300

Ser Leu Ser Pro Gly Lys
305                 310

<210> 38
<211> 930
<212> DNA
<213> Homo sapiens

<400> 38
atgccgctgc tgctactgct gcccctgctg tgggcagggg cgctagctat gctgcagatg      60

gccggccagt gcagccagaa cgagtacttc gacagcctgc tgcacgcctg catcccctgc     120

cagctgagat gcagcagcaa cacacctcct ctgacctgcc agagatactg caacgccagc     180

gtgaccaaca gcgtgaaggg caccaactcc ggagagaacc tgtacttcca aggggatccc     240

aaatcttgtg acaaaactca cacatgccca ccgtgcccag cacctgaact cctgggggga     300

ccgtcagtct tcctcttccc cccaaaaccc aaggacaccc tcatgatctc ccggacccct     360

gaggtcacat gcgtggtggt ggacgtgagc cacgaagacc ctgaggtcaa gttcaactgg     420

tacgtggacg gcgtggaggt gcataatgcc aagacaaagc cgcgggagga gcagtacaac     480

agcacgtacc gtgtggtcag cgtcctcacc gtcctgcacc aggactggct gaatggcaag     540

gagtacaagt gcaaggtctc caacaaagcc ctcccagccc ccatcgagaa aaccatctcc     600

aaagccaaag ggcagccccg agagccacag gtgtacaccc tgcccccatc ccgggatgag     660

ctgaccaaga accaggtcag cctgacctgc ctggtcaaag gcttctatcc cagcgacatc     720

gccgtggagt gggagagcaa tgggcagccg gagaacaact acaagaccac gcctcccgtg     780

ctggactccg acggctcctt cttcctctac agcaagctca ccgtggacaa gagcaggtgg     840

cagcagggga acgtcttctc atgctccgtg atgcatgagg ctctgcacaa ccactacacg     900

cagaagagcc tctccctgtc tccgggtaaa                                      930

<210> 39
<211> 307
<212> PRT
<213> Homo sapiens

<400> 39
Met Pro Leu Leu Leu Leu Leu Pro Leu Leu Trp Ala Gly Ala Leu Ala
1               5                   10                  15

Met Ala Gly Gln Cys Ser Gln Asn Glu Tyr Phe Asp Ser Leu Leu His
            20                  25                  30

Ala Cys Ile Pro Cys Gln Leu Arg Cys Ser Ser Asn Thr Pro Pro Leu
        35                  40                  45

Thr Cys Gln Arg Tyr Cys Asn Ala Ser Val Thr Asn Ser Val Lys Gly
    50                  55                  60

Thr Asn Ser Gly Glu Asn Leu Tyr Phe Gln Gly Asp Pro Lys Ser Cys
65                  70                  75                  80

Asp Lys Thr His Thr Cys Pro Pro Cys Pro Ala Pro Glu Leu Leu Gly
                85                  90                  95

Gly Pro Ser Val Phe Leu Phe Pro Pro Lys Pro Lys Asp Thr Leu Met
            100                 105                 110

Ile Ser Arg Thr Pro Glu Val Thr Cys Val Val Val Asp Val Ser His
        115                 120                 125

Glu Asp Pro Glu Val Lys Phe Asn Trp Tyr Val Asp Gly Val Glu Val
    130                 135                 140

His Asn Ala Lys Thr Lys Pro Arg Glu Glu Gln Tyr Asn Ser Thr Tyr
145                 150                 155                 160

Arg Val Val Ser Val Leu Thr Val Leu His Gln Asp Trp Leu Asn Gly
                165                 170                 175

Lys Glu Tyr Lys Cys Lys Val Ser Asn Lys Ala Leu Pro Ala Pro Ile
            180                 185                 190

Glu Lys Thr Ile Ser Lys Ala Lys Gly Gln Pro Arg Glu Pro Gln Val
        195                 200                 205

Tyr Thr Leu Pro Pro Ser Arg Asp Glu Leu Thr Lys Asn Gln Val Ser
    210                 215                 220

Leu Thr Cys Leu Val Lys Gly Phe Tyr Pro Ser Asp Ile Ala Val Glu
225                 230                 235                 240

Trp Glu Ser Asn Gly Gln Pro Glu Asn Asn Tyr Lys Thr Thr Pro Pro
                245                 250                 255

Val Leu Asp Ser Asp Gly Ser Phe Phe Leu Tyr Ser Lys Leu Thr Val
            260                 265                 270

Asp Lys Ser Arg Trp Gln Gln Gly Asn Val Phe Ser Cys Ser Val Met
        275                 280                 285

His Glu Ala Leu His Asn His Tyr Thr Gln Lys Ser Leu Ser Leu Ser
    290                 295                 300

Pro Gly Lys
305

<210> 40
<211> 921
<212> DNA
<213> Homo sapiens

<400> 40
atgccgctgc tgctactgct gcccctgctg tgggcagggg cgctagctat ggccggccag      60

tgcagccaga acgagtactt cgacagcctg ctgcacgcct gcatcccctg ccagctgaga     120

tgcagcagca acacacctcc tctgacctgc cagagatact gcaacgccag cgtgaccaac     180

agcgtgaagg gcaccaactc cggagagaac ctgtacttcc aaggggatcc caaatcttgt     240

gacaaaactc acacatgccc accgtgccca gcacctgaac tcctgggggg accgtcagtc     300

ttcctcttcc ccccaaaacc caaggacacc ctcatgatct cccggacccc tgaggtcaca     360

tgcgtggtgg tggacgtgag ccacgaagac cctgaggtca agttcaactg gtacgtggac     420

ggcgtggagg tgcataatgc caagacaaag ccgcgggagg agcagtacaa cagcacgtac     480

cgtgtggtca gcgtcctcac cgtcctgcac caggactggc tgaatggcaa ggagtacaag     540

tgcaaggtct ccaacaaagc cctcccagcc cccatcgaga aaaccatctc caaagccaaa     600

gggcagcccc gagagccaca ggtgtacacc ctgcccccat cccgggatga gctgaccaag     660

aaccaggtca gcctgacctg cctggtcaaa ggcttctatc ccagcgacat cgccgtggag     720

tgggagagca atgggcagcc ggagaacaac tacaagacca cgcctcccgt gctggactcc     780

gacggctcct tcttcctcta cagcaagctc accgtggaca agagcaggtg gcagcagggg     840

aacgtcttct catgctccgt gatgcatgag gctctgcaca accactacac gcagaagagc     900

ctctccctgt ctccgggtaa a                                               921

<210> 41
<211> 306
<212> PRT
<213> Macaca fascicularis

<400> 41
Met Pro Leu Leu Leu Leu Leu Pro Leu Leu Trp Ala Gly Ala Leu Ala
1               5                   10                  15

Met Ala Arg Gln Cys Ser Gln Asn Glu Tyr Phe Asp Ser Leu Leu His
            20                  25                  30

Asp Cys Lys Pro Cys Gln Leu Arg Cys Ser Ser Thr Pro Pro Leu Thr
        35                  40                  45

Cys Gln Arg Tyr Cys Asn Ala Ser Met Thr Asn Ser Val Lys Gly Met
    50                  55                  60

Asn Ser Gly Glu Asn Leu Tyr Phe Gln Gly Asp Pro Lys Ser Cys Asp
65                  70                  75                  80

Lys Thr His Thr Cys Pro Pro Cys Pro Ala Pro Glu Leu Leu Gly Gly
                85                  90                  95

Pro Ser Val Phe Leu Phe Pro Pro Lys Pro Lys Asp Thr Leu Met Ile
            100                 105                 110

Ser Arg Thr Pro Glu Val Thr Cys Val Val Val Asp Val Ser His Glu
        115                 120                 125

Asp Pro Glu Val Lys Phe Asn Trp Tyr Val Asp Gly Val Glu Val His
    130                 135                 140

Asn Ala Lys Thr Lys Pro Arg Glu Glu Gln Tyr Asn Ser Thr Tyr Arg
145                 150                 155                 160

Val Val Ser Val Leu Thr Val Leu His Gln Asp Trp Leu Asn Gly Lys
                165                 170                 175

Glu Tyr Lys Cys Lys Val Ser Asn Lys Ala Leu Pro Ala Pro Ile Glu
            180                 185                 190

Lys Thr Ile Ser Lys Ala Lys Gly Gln Pro Arg Glu Pro Gln Val Tyr
        195                 200                 205

Thr Leu Pro Pro Ser Arg Asp Glu Leu Thr Lys Asn Gln Val Ser Leu
    210                 215                 220

Thr Cys Leu Val Lys Gly Phe Tyr Pro Ser Asp Ile Ala Val Glu Trp

225                 230                 235                 240

Glu Ser Asn Gly Gln Pro Glu Asn Asn Tyr Lys Thr Thr Pro Pro Val
                245                 250                 255

Leu Asp Ser Asp Gly Ser Phe Phe Leu Tyr Ser Lys Leu Thr Val Asp
            260                 265                 270

Lys Ser Arg Trp Gln Gln Gly Asn Val Phe Ser Cys Ser Val Met His
        275                 280                 285

Glu Ala Leu His Asn His Tyr Thr Gln Lys Ser Leu Ser Leu Ser Pro
    290                 295                 300

Gly Lys
305

<210> 42
<211> 918
<212> DNA
<213> Macaca fascicularis

<400> 42
atgccgctgc tgctactgct gcccctgctg tgggcagggg cgctagctat ggccagacag      60

tgcagccaga acgagtactt cgacagcctg ctgcacgact gcaagccctg ccagctgaga     120

tgcagcagca cacctcctct gacctgccag agatactgca acgccagcat gaccaacagc     180

gtgaagggca tgaactccgg agagaacctg tacttccaag gggatcccaa atcttgtgac     240

aaaactcaca catgcccacc gtgcccagca cctgaactcc tggggggacc gtcagtcttc     300

ctcttccccc caaaacccaa ggacaccctc atgatctccc ggacccctga ggtcacatgc     360

gtggtggtgg acgtgagcca cgaagaccct gaggtcaagt tcaactggta cgtggacggc     420

gtggaggtgc ataatgccaa gacaaagccg cgggaggagc agtacaacag cacgtaccgt     480

gtggtcagcg tcctcaccgt cctgcaccag gactggctga atggcaagga gtacaagtgc     540

aaggtctcca acaaagccct cccagccccc atcgagaaaa ccatctccaa agccaaaggg     600

cagccccgag agccacaggt gtacaccctg cccccatccc gggatgagct gaccaagaac     660

caggtcagcc tgacctgcct ggtcaaaggc ttctatccca gcgacatcgc cgtggagtgg     720

gagagcaatg ggcagccgga gaacaactac aagaccacgc ctcccgtgct ggactccgac     780

ggctccttct tcctctacag caagctcacc gtggacaaga gcaggtggca gcaggggaac     840

gtcttctcat gctccgtgat gcatgaggct ctgcacaacc actacacgca gaagagcctc     900

tccctgtctc cgggtaaa                                                   918

<210> 43

<211> 451
<212> PRT
<213> Artificial Sequence

<220>
<221> source
<223> /note="Description of Artificial Sequence: Synthetic
      humanized antibody sequence"

<400> 43
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Ile Tyr Asn Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys

210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 44
<211> 1353
<212> DNA
<213> Artificial Sequence

<220>
<221> source
<223> /note="Description of Artificial Sequence: Synthetic
      humanized antibody sequence"

<400> 44
caggtgcagc tggtccagag cggcgccgaa gtgaagaagc ccggcagctc cgtgaaagtg      60

agctgcaagg ccagcggcgg caccttcagc aactactgga tgcactgggt gaggcaggcc     120

cccggacagg gcctggagtg gatgggcgcc acctacaggg gccacagcga cacctactac     180

aaccagaagt tcaagggccg ggtgaccatc accgccgaca agagcaccag caccgcctac     240

atggaactga gcagcctcag gagcgaggac accgctgtgt attactgcgc caggggcgcc     300

atctacaacg gctacgacgt gctggacaac tggggccagg gcacactagt gaccgtgtcc     360

agcgccagca ccaagggccc cagcgtgttc cccctggccc ccagcagcaa gagcaccagc     420

ggcggcacag ccgccctggg ctgcctggtg aaggactact tccccgaacc ggtgaccgtg     480

tcctggaaca gcggagccct gaccagcggc gtgcacacct tccccgccgt gctgcagagc     540

agcggcctgt acagcctgag cagcgtggtg accgtgccca gcagcagcct gggcacccag     600

acctacatct gtaacgtgaa ccacaagccc agcaacacca aggtggacaa gaaggtggag     660

cccaagagct gtgacaagac ccacacctgc ccccctgccc ctgcccccga gctgctggga     720

ggccccagcg tgttcctgtt cccccccaag cctaaggaca ccctgatgat cagcagaacc     780

cccgaggtga cctgtgtggt ggtggatgtg agccacgagg accctgaggt gaagttcaac     840

tggtacgtgg acggcgtgga ggtgcacaat gccaagacca agcccaggga ggagcagtac     900

aacagcacct accgggtggt gtccgtgctg accgtgctgc accaggattg gctgaacggc     960

aaggagtaca agtgtaaggt gtccaacaag gccctgcctg cccctatcga gaaaaccatc    1020

agcaaggcca agggccagcc cagagagccc caggtgtaca ccctgccccc tagcagagat    1080

gagctgacca agaaccaggt gtccctgacc tgcctggtga agggcttcta ccccagcgac    1140

atcgccgtgg agtgggagag caacggccag cccgagaaca actacaagac caccccccct    1200

gtgctggaca gcgatggcag cttcttcctg tacagcaagc tgaccgtgga caagagcaga    1260

tggcagcagg gcaacgtgtt cagctgctcc gtgatgcacg aggccctgca caatcactac    1320

acccagaaga gcctgagcct gtcccctggc aag                                 1353

<210> 45
<211> 451
<212> PRT
<213> Artificial Sequence

<220>
<221> source
<223> /note="Description of Artificial Sequence: Synthetic
      humanized antibody sequence"

<400> 45
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Ile Tyr Asn Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly

225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 46
<211> 1353
<212> DNA
<213> Artificial Sequence

<220>
<221> source
<223> /note="Description of Artificial Sequence: Synthetic
      humanized antibody sequence"

<400> 46
caggtgcagc tggtccagag cggcgccgaa gtgaagaagc ccggcagctc cgtgaaagtg        60

agctgcaagg ccagcggcta caccttcacc aactactgga tgcactgggt gaggcaggcc       120

cccggacagg gcctggagtg gatgggcgcc acctacaggg gccacagcga cacctactac       180

aaccagaagt tcaagggccg ggtgaccatc accgccgaca agagcaccag caccgcctac       240

atggaactga gcagcctcag gagcgaggac accgctgtgt attactgcgc caggggcgcc       300

atctacaacg gctacgacgt gctggacaac tggggccagg gcacactagt gaccgtgtcc       360

agcgccagca ccaagggccc cagcgtgttc cccctggccc ccagcagcaa gagcaccagc       420

ggcggcacag ccgccctggg ctgcctggtg aaggactact tccccgaacc ggtgaccgtg       480

tcctggaaca gcggagccct gaccagcggc gtgcacacct tccccgccgt gctgcagagc       540

agcggcctgt acagcctgag cagcgtggtg accgtgccca gcagcagcct gggcacccag       600

acctacatct gtaacgtgaa ccacaagccc agcaacacca aggtggacaa gaaggtggag       660

cccaagagct gtgacaagac ccacacctgc cccccctgcc ctgcccccga gctgctggga       720

ggccccagcg tgttcctgtt cccccccaag cctaaggaca ccctgatgat cagcagaacc       780

cccgaggtga cctgtgtggt ggtggatgtg agccacgagg accctgaggt gaagttcaac       840

tggtacgtgg acggcgtgga ggtgcacaat gccaagacca agcccaggga ggagcagtac       900

aacagcacct accgggtggt gtccgtgctg accgtgctgc accaggattg gctgaacggc       960

aaggagtaca agtgtaaggt gtccaacaag gccctgcctg cccctatcga gaaaaccatc      1020

agcaaggcca agggccagcc cagagagccc caggtgtaca ccctgccccc tagcagagat      1080

gagctgacca agaaccaggt gtccctgacc tgcctggtga agggcttcta ccccagcgac      1140

atcgccgtgg agtgggagag caacggccag cccgagaaca actacaagac caccccccct      1200

gtgctggaca gcgatggcag cttcttcctg tacagcaagc tgaccgtgga caagagcaga      1260

tggcagcagg gcaacgtgtt cagctgctcc gtgatgcacg aggccctgca caatcactac      1320

acccagaaga gcctgagcct gtcccctggc aag                                   1353


<210> 47
<211> 451
<212> PRT
<213> Artificial Sequence

<220>
<221> source
<223> /note="Description of Artificial Sequence: Synthetic
      humanized antibody sequence"

<400> 47
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asn Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met

245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 48
<211> 1353
<212> DNA
<213> Artificial Sequence

<220>
<221> source
<223> /note="Description of Artificial Sequence: Synthetic

humanized antibody sequence"

<400> 48
caggtgcagc tggtccagag cggcgccgaa gtgaagaagc ccggcagctc cgtgaaagtg 60

agctgcaagg ccagcggcta caccttcacc aactactgga tgcactgggt gaggcaggcc 120

cccggacagg gcctggagtg gatgggcgcc acctacaggg gccacagcga cacctactac 180

aaccagaagt tcaagggccg ggtgaccatc accgccgaca agagcaccag caccgcctac 240

atggaactga gcagcctcag gagcgaggac accgctgtgt attactgcac caggggcgcc 300

atctacaacg gctacgacgt gctggacaac tggggccagg gcacactagt gaccgtgtcc 360

agcgccagca ccaagggccc cagcgtgttc cccctggccc ccagcagcaa gagcaccagc 420

ggcggcacag ccgccctggg ctgcctggtg aaggactact tccccgaacc ggtgaccgtg 480

tcctggaaca gcggagccct gaccagcggc gtgcacacct tccccgccgt gctgcagagc 540

agcggcctgt acagcctgag cagcgtggtg accgtgccca gcagcagcct gggcacccag 600

acctacatct gtaacgtgaa ccacaagccc agcaacacca aggtggacaa gaaggtggag 660

cccaagagct gtgacaagac ccacacctgc cccccctgcc ctgcccccga gctgctggga 720

ggccccagcg tgttcctgtt cccccccaag cctaaggaca ccctgatgat cagcagaacc 780

cccgaggtga cctgtgtggt ggtggatgtg agccacgagg accctgaggt gaagttcaac 840

tggtacgtgg acggcgtgga ggtgcacaat gccaagacca agcccaggga ggagcagtac 900

aacagcacct accgggtggt gtccgtgctg accgtgctgc accaggattg gctgaacggc 960

aaggagtaca agtgtaaggt gtccaacaag gccctgcctg cccctatcga gaaaaccatc 1020

agcaaggcca agggccagcc cagagagccc caggtgtaca ccctgccccc tagcagagat 1080

gagctgacca agaaccaggt gtccctgacc tgcctggtga agggcttcta ccccagcgac 1140

atcgccgtgg agtgggagag caacggccag cccgagaaca actacaagac caccccccct 1200

gtgctggaca gcgatggcag cttcttcctg tacagcaagc tgaccgtgga caagagcaga 1260

tggcagcagg gcaacgtgtt cagctgctcc gtgatgcacg aggccctgca caatcactac 1320

acccagaaga gcctgagcct gtcccctggc aag 1353

<210> 49
<211> 451
<212> PRT
<213> Artificial Sequence

<220>
<221> source
<223> /note="Description of Artificial Sequence: Synthetic
humanized antibody sequence"

<400> 49
Gln Val Gln Leu Val Gln Ser Gly Ala Glu Val Lys Lys Pro Gly Ser
1               5                   10                  15

Ser Val Lys Val Ser Cys Lys Gly Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asn Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His

260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 50
<211> 1353
<212> DNA
<213> Artificial Sequence

<220>
<221> source
<223> /note="Description of Artificial Sequence: Synthetic
      humanized antibody sequence"

<400> 50
caggtgcagc tggtccagag cggcgccgaa gtgaagaagc ccggcagctc cgtgaaagtg        60

agctgcaagg gcagcggcta caccttcacc aactactgga tgcactgggt gaggcaggcc 120

cccggacagg gcctggagtg gatgggcgcc acctacaggg gccacagcga cacctactac 180

aaccagaagt tcaagggccg ggtgaccatc accgccgaca cgagcaccag caccgcctac 240

atggaactga gcagcctcag gagcgaggac accgctgtgt attactgcac caggggcgcc 300

atctacaacg gctacgacgt gctggacaac tggggccagg gcacactagt gaccgtgtcc 360

agcgccagca ccaagggccc cagcgtgttc cccctggccc ccagcagcaa gagcaccagc 420

ggcggcacag ccgccctggg ctgcctggtg aaggactact tccccgaacc ggtgaccgtg 480

tcctggaaca gcggagccct gaccagcggc gtgcacacct tccccgccgt gctgcagagc 540

agcggcctgt acagcctgag cagcgtggtg accgtgccca gcagcagcct gggcacccag 600

acctacatct gtaacgtgaa ccacaagccc agcaacacca aggtggacaa gaaggtggag 660

cccaagagct gtgacaagac ccacacctgc ccccctgccc ctgcccccga gctgctggga 720

ggccccagcg tgttcctgtt cccccccaag cctaaggaca ccctgatgat cagcagaacc 780

cccgaggtga cctgtgtggt ggtggatgtg agccacgagg accctgaggt gaagttcaac 840

tggtacgtgg acggcgtgga ggtgcacaat gccaagacca agcccaggga ggagcagtac 900

aacagcacct accgggtggt gtccgtgctg accgtgctgc accaggattg gctgaacggc 960

aaggagtaca agtgtaaggt gtccaacaag gccctgcctg cccctatcga gaaaaccatc 1020

agcaaggcca agggccagcc cagagagccc caggtgtaca ccctgccccc tagcagagat 1080

gagctgacca agaaccaggt gtccctgacc tgcctggtga agggcttcta ccccagcgac 1140

atcgccgtgg agtgggagag caacggccag cccgagaaca actacaagac caccccccct 1200

gtgctggaca gcgatggcag cttcttcctg tacagcaagc tgaccgtgga caagagcaga 1260

tggcagcagg gcaacgtgtt cagctgctcc gtgatgcacg aggccctgca caatcactac 1320

acccagaaga gcctgagcct gtcccctggc aag 1353

<210> 51
<211> 451
<212> PRT
<213> Artificial Sequence

<220>
<221> source
<223> /note="Description of Artificial Sequence: Synthetic
      humanized antibody sequence"

<400> 51
Gln Val Gln Leu Val Gln Ser Gly Ala Glu Val Lys Lys Pro Gly Ser
1               5                   10                  15

Ser Val Lys Val Ser Cys Lys Gly Ser Gly Tyr Thr Phe Thr Asn Tyr
            20                  25                  30

Trp Met His Trp Val Arg Gln Ala Pro Gly Gln Gly Leu Glu Trp Ile
        35                  40                  45

Gly Ala Thr Tyr Arg Gly His Ser Asp Thr Tyr Tyr Asn Gln Lys Phe
    50                  55                  60

Lys Gly Arg Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asn Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val

275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 52
<211> 1353
<212> DNA
<213> Artificial Sequence

<220>
<221> source
<223> /note="Description of Artificial Sequence: Synthetic
      humanized antibody sequence"

<400> 52
caggtgcagc tggtccagag cggcgccgaa gtgaagaagc ccggcagctc cgtgaaagtg      60

agctgcaagg gcagcggcta caccttcacc aactactgga tgcactgggt gaggcaggcc     120

cccggacagg gcctggagtg gatcggcgcc acctacaggg gccacagcga cacctactac     180

aaccagaagt tcaagggccg ggcgaccctc accgccgaca cgagcaccag caccgcctac 240

atggaactga gcagcctcag gagcgaggac accgctgtgt attactgcac caggggcgcc 300

atctacaacg gctacgacgt gctggacaac tggggccagg gcacactagt gaccgtgtcc 360

agcgccagca ccaagggccc cagcgtgttc cccctggccc ccagcagcaa gagcaccagc 420

ggcggcacag ccgccctggg ctgcctggtg aaggactact tccccgaacc ggtgaccgtg 480

tcctggaaca gcggagccct gaccagcggc gtgcacacct tccccgccgt gctgcagagc 540

agcggcctgt acagcctgag cagcgtggtg accgtgccca gcagcagcct gggcacccag 600

acctacatct gtaacgtgaa ccacaagccc agcaacacca aggtggacaa gaaggtggag 660

cccaagagct gtgacaagac ccacacctgc cccccctgcc ctgcccccga gctgctggga 720

ggccccagcg tgttcctgtt cccccccaag cctaaggaca ccctgatgat cagcagaacc 780

cccgaggtga cctgtgtggt ggtggatgtg agccacgagg accctgaggt gaagttcaac 840

tggtacgtgg acggcgtgga ggtgcacaat gccaagacca agcccaggga ggagcagtac 900

aacagcacct accgggtggt gtccgtgctg accgtgctgc accaggattg gctgaacggc 960

aaggagtaca agtgtaaggt gtccaacaag gccctgcctg cccctatcga gaaaaccatc 1020

agcaaggcca agggccagcc cagagagccc caggtgtaca ccctgccccc tagcagagat 1080

gagctgacca agaaccaggt gtccctgacc tgcctggtga agggcttcta ccccagcgac 1140

atcgccgtgg agtgggagag caacggccag cccgagaaca actacaagac caccccccct 1200

gtgctggaca gcgatggcag cttcttcctg tacagcaagc tgaccgtgga caagagcaga 1260

tggcagcagg gcaacgtgtt cagctgctcc gtgatgcacg aggccctgca caatcactac 1320

acccagaaga gcctgagcct gtcccctggc aag 1353

<210> 53
<211> 451
<212> PRT
<213> Artificial Sequence

<220>
<221> source
<223> /note="Description of Artificial Sequence: Synthetic
      humanized antibody sequence"

<400> 53
Gln Val Gln Leu Val Gln Ser Gly Ala Glu Val Lys Lys Pro Gly Ser
1               5                   10                  15

Ser Val Lys Val Ser Cys Lys Gly Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asp Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr

290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 54
<211> 1353
<212> DNA
<213> Artificial Sequence

<220>
<221> source
<223> /note="Description of Artificial Sequence: Synthetic
      humanized antibody sequence"

<400> 54
caggtgcagc tggtccagag cggcgccgaa gtgaagaagc ccggcagctc cgtgaaagtg      60

agctgcaagg gcagcggcta caccttcacc aactactgga tgcactgggt gaggcaggcc     120

cccggacagg gcctggagtg gatgggcgcc acctacaggg gccacagcga cacctactac     180

aaccagaagt tcaagggccg ggtgaccatc accgccgaca cgagcaccag caccgcctac     240

atggaactga gcagcctcag gagcgaggac accgctgtgt attactgcac caggggcgcc     300

atctacgacg gctacgacgt gctggacaac tggggccagg gcacactagt gaccgtgtcc 360

agcgccagca ccaagggccc cagcgtgttc cccctggccc ccagcagcaa gagcaccagc 420

ggcggcacag ccgccctggg ctgcctggtg aaggactact tccccgaacc ggtgaccgtg 480

tcctggaaca gcggagccct gaccagcggc gtgcacacct tccccgccgt gctgcagagc 540

agcggcctgt acagcctgag cagcgtggtg accgtgccca gcagcagcct gggcacccag 600

acctacatct gtaacgtgaa ccacaagccc agcaacacca aggtggacaa gaaggtggag 660

cccaagagct gtgacaagac ccacacctgc ccccctgccc ctgcccccga gctgctggga 720

ggccccagcg tgttcctgtt cccccccaag cctaaggaca ccctgatgat cagcagaacc 780

cccgaggtga cctgtgtggt ggtggatgtg agccacgagg accctgaggt gaagttcaac 840

tggtacgtgg acggcgtgga ggtgcacaat gccaagacca agcccaggga ggagcagtac 900

aacagcacct accgggtggt gtccgtgctg accgtgctgc accaggattg gctgaacggc 960

aaggagtaca agtgtaaggt gtccaacaag gccctgcctg cccctatcga gaaaaccatc 1020

agcaaggcca agggccagcc cagagagccc caggtgtaca ccctgccccc tagcagagat 1080

gagctgacca agaaccaggt gtccctgacc tgcctggtga agggcttcta ccccagcgac 1140

atcgccgtgg agtgggagag caacggccag cccgagaaca actacaagac caccccccct 1200

gtgctggaca gcgatggcag cttcttcctg tacagcaagc tgaccgtgga caagagcaga 1260

tggcagcagg gcaacgtgtt cagctgctcc gtgatgcacg aggccctgca caatcactac 1320

acccagaaga gcctgagcct gtcccctggc aag 1353

<210> 55
<211> 451
<212> PRT
<213> Artificial Sequence

<220>
<221> source
<223> /note="Description of Artificial Sequence: Synthetic
      humanized antibody sequence"

<400> 55
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Ile Tyr Asp Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly

305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 56
<211> 1353
<212> DNA
<213> Artificial Sequence

<220>
<221> source
<223> /note="Description of Artificial Sequence: Synthetic
      humanized antibody sequence"

<400> 56
caggtgcagc tggtccagag cggcgccgaa gtgaagaagc ccggcagctc cgtgaaagtg      60

agctgcaagg ccagcggcgg caccttcagc aactactgga tgcactgggt gaggcaggcc     120

cccggacagg gcctggagtg gatgggcgcc acctacaggg gccacagcga cacctactac     180

aaccagaagt tcaagggccg ggtgaccatc accgccgaca agagcaccag caccgcctac     240

atggaactga gcagcctcag gagcgaggac accgctgtgt attactgcgc caggggcgcc     300

atctacgacg gctacgacgt gctggacaac tggggccagg gcacactagt gaccgtgtcc     360

agcgccagca ccaagggccc cagcgtgttc cccctggccc ccagcagcaa gagcaccagc     420

ggcggcacag ccgccctggg ctgcctggtg aaggactact tccccgaacc ggtgaccgtg 480

tcctggaaca gcggagccct gaccagcggc gtgcacacct tccccgccgt gctgcagagc 540

agcggcctgt acagcctgag cagcgtggtg accgtgccca gcagcagcct gggcacccag 600

acctacatct gtaacgtgaa ccacaagccc agcaacacca aggtggacaa gaaggtggag 660

cccaagagct gtgacaagac ccacacctgc cccccctgcc ctgcccccga gctgctggga 720

ggccccagcg tgttcctgtt cccccccaag cctaaggaca ccctgatgat cagcagaacc 780

cccgaggtga cctgtgtggt ggtggatgtg agccacgagg accctgaggt gaagttcaac 840

tggtacgtgg acggcgtgga ggtgcacaat gccaagacca agcccaggga ggagcagtac 900

aacagcacct accgggtggt gtccgtgctg accgtgctgc accaggattg gctgaacggc 960

aaggagtaca agtgtaaggt gtccaacaag gccctgcctg cccctatcga gaaaaccatc 1020

agcaaggcca agggccagcc cagagagccc caggtgtaca ccctgccccc tagcagagat 1080

gagctgacca agaaccaggt gtccctgacc tgcctggtga agggcttcta ccccagcgac 1140

atcgccgtgg agtgggagag caacggccag cccgagaaca actacaagac caccccccct 1200

gtgctggaca gcgatggcag cttcttcctg tacagcaagc tgaccgtgga caagagcaga 1260

tggcagcagg gcaacgtgtt cagctgctcc gtgatgcacg aggccctgca caatcactac 1320

acccagaaga gcctgagcct gtcccctggc aag 1353

<210> 57
<211> 451
<212> PRT
<213> Artificial Sequence

<220>
<221> source
<223> /note="Description of Artificial Sequence: Synthetic
      humanized antibody sequence"

<400> 57
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Ala Ile Tyr Asp Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile

325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 58
<211> 1353
<212> DNA
<213> Artificial Sequence

<220>
<221> source
<223> /note="Description of Artificial Sequence: Synthetic
      humanized antibody sequence"

<400> 58
   caggtgcagc tggtccagag cggcgccgaa gtgaagaagc ccggcagctc cgtgaaagtg      60
   agctgcaagg ccagcggcta caccttcacc aactactgga tgcactgggt gaggcaggcc     120
   cccggacagg gcctggagtg gatgggcgcc acctacaggg gccacagcga cacctactac     180
   aaccagaagt tcaagggccg ggtgaccatc accgccgaca agagcaccag caccgcctac     240
   atggaactga gcagcctcag gagcgaggac accgctgtgt attactgcgc caggggcgcc     300
   atctacgacg gctacgacgt gctggacaac tggggccagg gcacactagt gaccgtgtcc     360
   agcgccagca ccaagggccc cagcgtgttc cccctggccc ccagcagcaa gagcaccagc     420
   ggcggcacag ccgccctggg ctgcctggtg aaggactact tccccgaacc ggtgaccgtg     480
   tcctggaaca gcggagccct gaccagcggc gtgcacacct tccccgccgt gctgcagagc     540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag 1353

<210> 59
<211> 451
<212> PRT
<213> Artificial Sequence

<220>
<221> source
<223> /note="Description of Artificial Sequence: Synthetic
      humanized antibody sequence"

<400> 59
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Trp Met His Trp Val Arg Gln Ala Pro Gly Gln Gly Leu Glu Trp Met
        35                  40                  45

Gly Ala Thr Tyr Arg Gly His Ser Asp Thr Tyr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asp Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val

340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 60
<211> 1353
<212> DNA
<213> Artificial Sequence

<220>
<221> source
<223> /note="Description of Artificial Sequence: Synthetic
      humanized antibody sequence"

<400> 60
   caggtgcagc tggtccagag cggcgccgaa gtgaagaagc ccggcagctc cgtgaaagtg      60
   agctgcaagg ccagcggcta caccttcacc aactactgga tgcactgggt gaggcaggcc     120
   cccggacagg gcctggagtg gatgggcgcc acctacaggg gccacagcga cacctactac     180
   aaccagaagt tcaagggccg ggtgaccatc accgccgaca agagcaccag caccgcctac     240
   atggaactga gcagcctcag gagcgaggac accgctgtgt attactgcac caggggcgcc     300
   atctacgacg gctacgacgt gctggacaac tggggccagg gcacactagt gaccgtgtcc     360
   agcgccagca ccaagggccc cagcgtgttc cccctggccc ccagcagcaa gagcaccagc     420
   ggcggcacag ccgccctggg ctgcctggtg aaggactact tccccgaacc ggtgaccgtg     480
   tcctggaaca gcggagccct gaccagcggc gtgcacacct tccccgccgt gctgcagagc     540
   agcggcctgt acagcctgag cagcgtggtg accgtgccca gcagcagcct gggcacccag     600
   acctacatct gtaacgtgaa ccacaagccc agcaacacca aggtggacaa gaaggtggag     660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag 1353

<210> 61
<211> 451
<212> PRT
<213> Artificial Sequence

<220>
<221> source
<223> /note="Description of Artificial Sequence: Synthetic
      humanized antibody sequence"

<400> 61
Gln Val Gln Leu Val Gln Ser Gly Ala Glu Val Lys Lys Pro Gly Ser
1               5                   10                  15

Ser Val Lys Val Ser Cys Lys Gly Ser Gly Tyr Thr Phe Thr Asn Tyr
            20                  25                  30

Trp Met His Trp Val Arg Gln Ala Pro Gly Gln Gly Leu Glu Trp Ile
        35                  40                  45

Gly Ala Thr Tyr Arg Gly His Ser Asp Thr Tyr Tyr Asn Gln Lys Phe
    50                  55                  60

Lys Gly Arg Ala Thr Leu Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Ala Ile Tyr Asp Gly Tyr Asp Val Leu Asp Asn Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser

355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210> 62
<211> 1353
<212> DNA
<213> Artificial Sequence

<220>
<221> source
<223> /note="Description of Artificial Sequence: Synthetic
      humanized antibody sequence"

<400> 62
caggtgcagc tggtccagag cggcgccgaa gtgaagaagc ccggcagctc cgtgaaagtg      60

agctgcaagg gcagcggcta caccttcacc aactactgga tgcactgggt gaggcaggcc     120

cccggacagg gcctggagtg gatcggcgcc acctacaggg gccacagcga cacctactac     180

aaccagaagt tcaagggccg ggcgaccctc accgccgaca cgagcaccag caccgcctac     240

atggaactga gcagcctcag gagcgaggac accgctgtgt attactgcac caggggcgcc     300

atctacgacg gctacgacgt gctggacaac tggggccagg gcacactagt gaccgtgtcc     360

agcgccagca ccaagggccc cagcgtgttc cccctggccc ccagcagcaa gagcaccagc     420

ggcggcacag ccgccctggg ctgcctggtg aaggactact tccccgaacc ggtgaccgtg     480

tcctggaaca gcggagccct gaccagcggc gtgcacacct tccccgccgt gctgcagagc     540

agcggcctgt acagcctgag cagcgtggtg accgtgccca gcagcagcct gggcacccag     600

acctacatct gtaacgtgaa ccacaagccc agcaacacca aggtggacaa gaaggtggag     660

cccaagagct gtgacaagac ccacacctgc cccccctgcc ctgcccccga gctgctggga     720

ggccccagcg tgttcctgtt cccccccaag cctaaggaca ccctgatgat cagcagaacc     780

cccgaggtga cctgtgtggt ggtggatgtg agccacgagg accctgaggt gaagttcaac 840

tggtacgtgg acggcgtgga ggtgcacaat gccaagacca agcccaggga ggagcagtac 900

aacagcacct accgggtggt gtccgtgctg accgtgctgc accaggattg gctgaacggc 960

aaggagtaca agtgtaaggt gtccaacaag gccctgcctg cccctatcga gaaaaccatc 1020

agcaaggcca agggccagcc cagagagccc caggtgtaca ccctgccccc tagcagagat 1080

gagctgacca agaaccaggt gtccctgacc tgcctggtga agggcttcta ccccagcgac 1140

atcgccgtgg agtgggagag caacggccag cccgagaaca actacaagac caccccccct 1200

gtgctggaca gcgatggcag cttcttcctg tacagcaagc tgaccgtgga caagagcaga 1260

tggcagcagg gcaacgtgtt cagctgctcc gtgatgcacg aggccctgca caatcactac 1320

acccagaaga gcctgagcct gtcccctggc aag 1353

<210> 63
<211> 214
<212> PRT
<213> Artificial Sequence

<220>
<221> source
<223> /note="Description of Artificial Sequence: Synthetic
      humanized antibody sequence"

<400> 63
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Asn Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Arg Lys Leu Pro Trp
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 64
<211> 642
<212> DNA
<213> Artificial Sequence

<220>
<221> source
<223> /note="Description of Artificial Sequence: Synthetic
      humanized antibody sequence"

<400> 64
gacatccaga tgacccagag ccctagctca ctgagcgcca gcgtgggcga cagggtgacc      60

attacctgct ccgccagcca ggacatcagc aactacctga actggtacca gcagaagccc     120

ggcaaggccc ccaagctgct gatctactac acctccaacc tgcactccgg cgtgcccagc     180

aggttcagcg gaagcggcag cggcaccgat ttcaccctga ccatctccag cctgcagccc     240

gaggacttcg ccacctacta ctgccagcag tacaggaagc tcccctggac tttcggccag     300

ggcaccaaac tggagatcaa gcgtacggtg gccgccccca gcgtgttcat cttccccccc     360

agcgatgagc agctgaagag cggcaccgcc agcgtggtgt gtctgctgaa caacttctac     420

ccccgggagg ccaaggtgca gtggaaggtg gacaatgccc tgcagagcgg caacagccag     480

gagagcgtga ccgagcagga cagcaaggac tccacctaca gcctgagcag caccctgacc     540

ctgagcaagg ccgactacga gaagcacaag gtgtacgcct gtgaggtgac ccaccagggc     600

ctgtccagcc ccgtgaccaa gagcttcaac cggggcgagt gc                        642

<210> 65
<211> 214
<212> PRT

<213> Artificial Sequence

<220>
<221> source
<223> /note="Description of Artificial Sequence: Synthetic
      humanized antibody sequence"

<400> 65
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Asn Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Tyr Arg Lys Leu Pro Trp
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 66
<211> 642
<212> DNA
<213> Artificial Sequence

<220>
<221> source
<223> /note="Description of Artificial Sequence: Synthetic
      humanized antibody sequence"

<400> 66
gacatccaga tgacccagag ccctagctca ctgagcgcca gcgtgggcga cagggtgacc      60

attacctgct ccgccagcca ggacatcagc aactacctga actggtacca gcagaagccc     120

ggcaaggccc ccaagctgct gatctactac acctccaacc tgcactccgg cgtgcccagc     180

aggttcagcg gaagcggcag cggcaccgat tacaccctga ccatctccag cctgcagccc     240

gaggacttcg ccacctacta ctgccagcag tacaggaagc tcccctggac tttcggccag     300

ggcaccaaac tggagatcaa gcgtacggtg gccgccccca gcgtgttcat cttccccccc     360

agcgatgagc agctgaagag cggcaccgcc agcgtggtgt gtctgctgaa caacttctac     420

ccccgggagg ccaaggtgca gtggaaggtg gacaatgccc tgcagagcgg caacagccag     480

gagagcgtga ccgagcagga cagcaaggac tccacctaca gcctgagcag caccctgacc     540

ctgagcaagg ccgactacga gaagcacaag gtgtacgcct gtgaggtgac ccaccagggc     600

ctgtccagcc ccgtgaccaa gagcttcaac cggggcgagt gc                        642


<210> 67
<211> 214
<212> PRT
<213> Artificial Sequence

<220>
<221> source
<223> /note="Description of Artificial Sequence: Synthetic
      humanized antibody sequence"

<400> 67
Asp Ile Gln Leu Thr Gln Ser Pro Ser Ser Leu Ser Ala Ser Val Gly
1               5                   10                  15


Asp Arg Val Thr Ile Thr Cys Ser Ala Ser Gln Asp Ile Ser Asn Tyr
            20                  25                  30


Leu Asn Trp Tyr Gln Gln Lys Pro Gly Lys Ala Pro Glu Leu Val Ile
        35                  40                  45


Tyr Tyr Thr Ser Asn Leu His Ser Gly Val Pro Ser Arg Phe Ser Gly
    50                  55                  60


Ser Gly Ser Gly Thr Asp Tyr Thr Leu Thr Ile Ser Ser Leu Gln Pro

65                  70                  75                  80

Glu Asp Phe Ala Thr Tyr Tyr Cys Gln Gln Tyr Arg Lys Leu Pro Trp
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 68
<211> 642
<212> DNA
<213> Artificial Sequence

<220>
<221> source
<223> /note="Description of Artificial Sequence: Synthetic
      humanized antibody sequence"

<400> 68
gacatccagc tgacccagag ccctagctca ctgagcgcca gcgtgggcga cagggtgacc      60

attacctgct ccgccagcca ggacatcagc aactacctga actggtacca gcagaagccc     120

ggcaaggccc ccgagctggt gatctactac acctccaacc tgcactccgg cgtgcccagc     180

aggttcagcg gaagcggcag cggcaccgat tacaccctga ccatctccag cctgcagccc     240

gaggacttcg ccacctacta ctgccagcag tacaggaagc tcccctggac tttcggccag     300

ggcaccaaac tggagatcaa gcgtacggtg gccgccccca gcgtgttcat cttccccccc     360

agcgatgagc agctgaagag cggcaccgcc agcgtggtgt gtctgctgaa caacttctac     420

ccccgggagg ccaaggtgca gtggaaggtg gacaatgccc tgcagagcgg caacagccag 480

gagagcgtga ccgagcagga cagcaaggac tccacctaca gcctgagcag caccctgacc 540

ctgagcaagg ccgactacga gaagcacaag gtgtacgcct gtgaggtgac ccaccagggc 600

ctgtccagcc ccgtgaccaa gagcttcaac cggggcgagt gc 642

<210> 69
<211> 115
<212> PRT
<213> Mus musculus

<400> 69
Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Thr Phe Thr Asp Tyr
            20                  25                  30

Tyr Met Lys Trp Val Lys Gln Ser His Gly Lys Ser Leu Glu Trp Ile
        35                  40                  45

Gly Glu Ile Tyr Pro Asn Asn Gly Gly Ile Thr Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ala Asn Gly Tyr Glu Phe Val Tyr Trp Gly Gln Gly Thr Leu Val Thr
            100                 105                 110

Val Ser Ala
        115

<210> 70
<211> 345
<212> DNA
<213> Mus musculus

<400> 70
gaggtccagt tgcaacaatc tggacctgag ctggtgaagc ctggggcttc agtgaagata 60

tcctgtaagg cttctggata cacattcact gactactaca tgaagtgggt gaagcagagc 120

catggaaaga gccttgagtg gattggagag atttatccta ataatggtgg tattacctac 180

aaccagaagt tcaagggcaa ggccacattg actgtagaca agtcctccag cacagcctac 240

atggagctcc gcagcctgac atctgaggac tctgcagtct attactgtgc aaatggttac 300

gagtttgttt actggggcca agggactctg gtcactgtct ctgca 345

<210> 71
<211> 108
<212> PRT
<213> Mus musculus

<400> 71
Asp Ile Gln Met Thr Gln Thr Ala Ser Ser Leu Ser Ala Ser Leu Gly
1               5                   10                  15

Asp Arg Val Thr Ile Ser Cys Ser Ala Ser Gln Gly Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Leu Pro Trp
                85                  90                  95

Thr Phe Gly Gly Gly Thr Lys Leu Glu Ile Lys Arg
            100                 105

<210> 72
<211> 324
<212> DNA
<213> Mus musculus

<400> 72
gatatccaga tgacacagac tgcatcctcc ctgtctgcct ctctgggaga cagagtcacc 60

atcagttgca gtgcaagtca gggcattagc aattatttaa actggtatca gcagaaacca 120

gatggaactg ttaaactcct gatctattac acatcaagtt tacactcagg agtcccatca 180

aggttcagtg gcagtgggtc tgggacagat tattctctca ccatcagcaa cctggaacct 240

gaagatattg ccacttacta ttgtcagcag tatagtaagc ttccgtggac gttcggtgga 300

ggcaccaagc tggaaatcaa acgg 324

<210> 73
<211> 445
<212> PRT
<213> Artificial Sequence

<220>

<221> source
<223> /note="Description of Artificial Sequence: Synthetic
      chimeric antibody sequence"

<400> 73
Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Thr Phe Thr Asp Tyr
            20                  25                  30

Tyr Met Lys Trp Val Lys Gln Ser His Gly Lys Ser Leu Glu Trp Ile
        35                  40                  45

Gly Glu Ile Tyr Pro Asn Asn Gly Gly Ile Thr Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ala Asn Gly Tyr Glu Phe Val Tyr Trp Gly Gln Gly Thr Leu Val Thr
            100                 105                 110

Val Ser Ala Ala Lys Thr Thr Ala Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 74
<211> 1335
<212> DNA
<213> Artificial Sequence

<220>
<221> source
<223> /note="Description of Artificial Sequence: Synthetic
      chimeric antibody sequence"

<400> 74
gaggtccagt tgcaacaatc tggacctgag ctggtgaagc ctggggcttc agtgaagata      60

tcctgtaagg cttctggata cacattcact gactactaca tgaagtgggt gaagcagagc     120

catggaaaga gccttgagtg gattggagag atttatccta ataatggtgg tattacctac     180

aaccagaagt tcaagggcaa ggccacattg actgtagaca agtcctccag cacagcctac     240

atggagctcc gcagcctgac atctgaggac tctgcagtct attactgtgc aaatggttac     300

gagtttgttt actggggcca agggactctg gtcactgtct ctgcagccaa aacaacagcc     360

cccagcgtgt tccccctggc ccccagcagc aagagcacca gcggcggcac agccgccctg     420

ggctgcctgg tgaaggacta cttccccgaa ccggtgaccg tgtcctggaa cagcggagcc     480

ctgaccagcg gcgtgcacac cttccccgcc gtgctgcaga gcagcggcct gtacagcctg     540

agcagcgtgg tgaccgtgcc cagcagcagc ctgggcaccc agacctacat ctgtaacgtg     600

aaccacaagc ccagcaacac caaggtggac aagaaggtgg agcccaagag ctgtgacaag     660

acccacacct gccccccctg ccctgccccc gagctgctgg gaggccccag cgtgttcctg     720

ttccccccca agcctaagga caccctgatg atcagcagaa cccccgaggt gacctgtgtg     780

gtggtggatg tgagccacga ggaccctgag gtgaagttca actggtacgt ggacggcgtg     840

gaggtgcaca atgccaagac caagcccagg gaggagcagt acaacagcac ctaccgggtg     900

gtgtccgtgc tgaccgtgct gcaccaggat tggctgaacg gcaaggagta caagtgtaag     960

gtgtccaaca aggccctgcc tgcccctatc gagaaaacca tcagcaaggc caagggccag    1020

cccagagagc cccaggtgta caccctgccc cctagcagag atgagctgac caagaaccag    1080

gtgtccctga cctgcctggt gaagggcttc taccccagcg acatcgccgt ggagtgggag    1140

agcaacggcc agcccgagaa caactacaag accacccccc ctgtgctgga cagcgatggc    1200

agcttcttcc tgtacagcaa gctgaccgtg gacaagagca gatggcagca gggcaacgtg    1260

ttcagctgct ccgtgatgca cgaggccctg cacaatcact acacccagaa gagcctgagc    1320

ctgtcccctg gcaag                                                     1335


<210> 75
<211> 214
<212> PRT
<213> Artificial Sequence

<220>
<221> source
<223> /note="Description of Artificial Sequence: Synthetic
      chimeric antibody sequence"

<400> 75
Asp Ile Gln Met Thr Gln Thr Ala Ser Ser Leu Ser Ala Ser Leu Gly
1               5                   10                  15

Asp Arg Val Thr Ile Ser Cys Ser Ala Ser Gln Gly Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Leu Pro Trp
                85                  90                  95

Thr Phe Gly Gly Gly Thr Lys Leu Glu Leu Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 76
<211> 642
<212> DNA
<213> Artificial Sequence

<220>
<221> source
<223> /note="Description of Artificial Sequence: Synthetic
      chimeric antibody sequence"

<400> 76
gatatccaga tgacacagac tgcatcctcc ctgtctgcct ctctgggaga cagagtcacc 60

atcagttgca gtgcaagtca gggcattagc aattatttaa actggtatca gcagaaacca 120

gatggaactg ttaaactcct gatctattac acatcaagtt tacactcagg agtcccatca 180

aggttcagtg gcagtgggtc tgggacagat tattctctca ccatcagcaa cctggaacct 240

gaagatattg ccacttacta ttgtcagcag tatagtaagc ttccgtggac gttcggtgga 300

ggcaccaagc tggagctgaa acgtacggtg gccgccccca gcgtgttcat cttccccccc 360

agcgatgagc agctgaagag cggcaccgcc agcgtggtgt gtctgctgaa caacttctac 420

ccccgggagg ccaaggtgca gtggaaggtg gacaatgccc tgcagagcgg caacagccag 480

gagagcgtga ccgagcagga cagcaaggac tccacctaca gcctgagcag caccctgacc 540

ctgagcaagg ccgactacga gaagcacaag gtgtacgcct gtgaggtgac ccaccagggc 600

ctgtccagcc ccgtgaccaa gagcttcaac cggggcgagt gc 642

<210> 77
<211> 115
<212> PRT
<213> Artificial Sequence

<220>
<221> source
<223> /note="Description of Artificial Sequence: Synthetic
      humanized antibody sequence"

<400> 77
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asp Tyr
            20                  25                  30

Tyr Met Lys Trp Val Arg Gln Ala Pro Gly Gln Gly Leu Glu Trp Met
        35                  40                  45

Gly Glu Ile Tyr Pro Asn Asn Gly Gly Ile Thr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Tyr Glu Phe Val Tyr Trp Gly Gln Gly Thr Leu Val Thr
            100                 105                 110

Val Ser Ser
        115

<210> 78
<211> 345
<212> DNA
<213> Artificial Sequence

<220>
<221> source
<223> /note="Description of Artificial Sequence: Synthetic
      humanized antibody sequence"

<400> 78
caggtgcagc tggtgcagag cggcgccgaa gtgaagaagc ccggctccag cgtgaaggtg        60

agctgcaagg ctagcggcgg caccttcagc gactactaca tgaagtgggt gaggcaggcc       120

cccggccagg gactggagtg gatgggcgag atctacccca acaacggggg catcacctac       180

aaccagaagt tcaagggcag ggtgaccatc accgccgaca aaagcaccag caccgcctac       240

atggaactga gcagcctgag gagcgaggac accgccgtgt actactgcgc caggggctac       300

gagttcgtgt attggggcca gggcacacta gtgaccgtgt ccagc                       345

<210> 79
<211> 115
<212> PRT
<213> Artificial Sequence

<220>
<221> source
<223> /note="Description of Artificial Sequence: Synthetic
      humanized antibody sequence"

<400> 79
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Met
        35                  40                  45

Gly Glu Ile Tyr Pro Asn Asn Gly Gly Ile Thr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Tyr Glu Phe Val Tyr Trp Gly Gln Gly Thr Leu Val Thr
            100                 105                 110

Val Ser Ser
        115

<210> 80
<211> 345
<212> DNA
<213> Artificial Sequence

<220>
<221> source
<223> /note="Description of Artificial Sequence: Synthetic
      humanized antibody sequence"

<400> 80
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gatgggcgag atctacccca acaacggggg catcacctac     180

aaccagaagt tcaagggcag ggtgaccatc accgccgaca aaagcaccag caccgcctac     240

atggaactga gcagcctgag gagcgaggac accgccgtgt actactgcgc caggggctac     300

gagttcgtgt attggggcca gggcacacta gtgaccgtgt ccagc                     345

<210> 81
<211> 115
<212> PRT
<213> Artificial Sequence

<220>
<221> source
<223> /note="Description of Artificial Sequence: Synthetic
      humanized antibody sequence"

<400> 81
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Met
        35                  40                  45

Gly Glu Ile Tyr Pro Asn Asn Gly Gly Ile Thr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Asn Gly Tyr Glu Phe Val Tyr Trp Gly Gln Gly Thr Leu Val Thr
            100                 105                 110

Val Ser Ser
        115

<210> 82
<211> 345
<212> DNA
<213> Artificial Sequence

<220>
<221> source
<223> /note="Description of Artificial Sequence: Synthetic
      humanized antibody sequence"

<400> 82
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gatgggcgag atctacccca acaacggggg catcacctac     180

aaccagaagt tcaagggcag ggtgaccatc accgccgaca aaagcaccag caccgcctac     240

atggaactga gcagcctgag gagcgaggac accgccgtgt actactgcgc caacggctac     300

gagttcgtgt attggggcca gggcacacta gtgaccgtgt ccagc                     345

<210> 83
<211> 115
<212> PRT
<213> Artificial Sequence

<220>
<221> source
<223> /note="Description of Artificial Sequence: Synthetic
      humanized antibody sequence"

<400> 83
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Ile
        35                  40                  45

Gly Glu Ile Tyr Pro Asn Asn Gly Gly Ile Thr Tyr Asn Gln Lys Phe
    50                  55                  60

Lys Gly Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Asn Gly Tyr Glu Phe Val Tyr Trp Gly Gln Gly Thr Leu Val Thr
            100                 105                 110

Val Ser Ser
        115

<210> 84
<211> 345
<212> DNA
<213> Artificial Sequence

<220>
<221> source
<223> /note="Description of Artificial Sequence: Synthetic
      humanized antibody sequence"

<400> 84
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gataggcgag atctacccca acaacggggg catcacctac     180

aaccagaagt tcaagggcag ggcgaccctc accgtcgaca aaagcaccag caccgcctac     240

atggaactga gcagcctgag gagcgaggac accgccgtgt actactgcgc caacggctac     300

gagttcgtgt attggggcca gggcacacta gtgaccgtgt ccagc                     345

<210> 85
<211> 115
<212> PRT
<213> Artificial Sequence

<220>
<221> source
<223> /note="Description of Artificial Sequence: Synthetic
      humanized antibody sequence"

<400> 85
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Met
        35                  40                  45

Gly Glu Ile Tyr Pro Asn Asn Gly Gly Ile Thr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Asp Gly Tyr Glu Phe Val Tyr Trp Gly Gln Gly Thr Leu Val Thr
            100                 105                 110

Val Ser Ser
        115

<210> 86
<211> 345
<212> DNA
<213> Artificial Sequence

<220>
<221> source
<223> /note="Description of Artificial Sequence: Synthetic
      humanized antibody sequence"

<400> 86
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gatgggcgag atctacccca acaacggggg catcacctac     180

aaccagaagt tcaagggcag ggtgaccatc accgccgaca aaagcaccag caccgcctac     240

atggaactga gcagcctgag gagcgaggac accgccgtgt actactgcgc cgacggctac     300

gagttcgtgt attggggcca gggcacacta gtgaccgtgt ccagc                     345

<210> 87
<211> 115
<212> PRT
<213> Artificial Sequence

<220>
<221> source
<223> /note="Description of Artificial Sequence: Synthetic
      humanized antibody sequence"

<400> 87
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Ile
        35                  40                  45

Gly Glu Ile Tyr Pro Asn Asn Gly Gly Ile Thr Tyr Asn Gln Lys Phe
    50                  55                  60

Lys Gly Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Asn Gly Tyr Glu Phe Asp Tyr Trp Gly Gln Gly Thr Leu Val Thr
            100                 105                 110

Val Ser Ser
        115

<210> 88
<211> 345
<212> DNA
<213> Artificial Sequence

<220>
<221> source
<223> /note="Description of Artificial Sequence: Synthetic
      humanized antibody sequence"

<400> 88
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gataggcgag atctacccca acaacggggg catcacctac     180

aaccagaagt tcaagggcag ggcgaccctc accgtcgaca aaagcaccag caccgcctac     240

atggaactga gcagcctgag gagcgaggac accgccgtgt actactgcgc caacggctac     300

gagttcgact attggggcca gggcacacta gtgaccgtgt ccagc                     345

<210> 89
<211> 108
<212> PRT
<213> Artificial Sequence

<220>
<221> source
<223> /note="Description of Artificial Sequence: Synthetic
      humanized antibody sequence"

<400> 89
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Gly Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Lys Leu Pro Trp
                85                  90                  95

Thr Phe Gly Gln Gly Thr Lys Leu Glu Ile Lys Arg
            100                 105

<210> 90
<211> 324
<212> DNA
<213> Artificial Sequence

<220>
<221> source
<223> /note="Description of Artificial Sequence: Synthetic
      humanized antibody sequence"

<400> 90
gacatccaga tgacccagag cccctcaagc ctgagcgcca gcgtgggcga cagggtgact      60

atcacctgca gcgcctccca gggcatcagc aactacctga actggtacca gcagaagccc     120

ggcaaggccc ctaagctgct gatctactac accagcagcc tgcacagcgg cgtgcccagc     180

aggttctccg gcagcggcag cggaaccgac ttcaccctga ccattagcag cctccagccc     240

gaggacttcg ccacctacta ctgccagcag tacagcaagc tgccctggac cttcggccag     300

ggcaccaaac tggagatcaa gcgt                                            324

<210> 91
<211> 108
<212> PRT
<213> Artificial Sequence

<220>
<221> source
<223> /note="Description of Artificial Sequence: Synthetic
      humanized antibody sequence"

<400> 91
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Gly Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Tyr Ser Lys Leu Pro Trp
                85                  90                  95

Thr Phe Gly Gln Gly Thr Lys Leu Glu Ile Lys Arg
            100                 105

<210> 92
<211> 324
<212> DNA
<213> Artificial Sequence

<220>
<221> source
<223> /note="Description of Artificial Sequence: Synthetic
      humanized antibody sequence"

<400> 92
gacatccaga tgacccagag cccctcaagc ctgagcgcca gcgtgggcga cagggtgact      60

atcacctgca gcgcctccca gggcatcagc aactacctga actggtacca gcagaagccc     120

ggcaaggccc ctaagctgct gatctactac accagcagcc tgcacagcgg cgtgcccagc     180

aggttctccg gcagcggcag cggaaccgac tacaccctga ccattagcag cctccagccc     240

gaggacttcg ccacctacta ctgccagcag tacagcaagc tgccctggac cttcggccag     300

ggcaccaaac tggagatcaa gcgt                                            324

<210> 93
<211> 5
<212> PRT
<213> Mus musculus

<400> 93
Asp Tyr Tyr Met Lys
1               5

<210> 94
<211> 17
<212> PRT
<213> Mus musculus

<400> 94
Glu Ile Tyr Pro Asn Asn Gly Gly Ile Thr Tyr Asn Gln Lys Phe Lys
1               5                   10                  15

Gly

<210> 95
<211> 6
<212> PRT
<213> Mus musculus

<400> 95
Gly Tyr Glu Phe Val Tyr
1               5

<210> 96
<211> 11
<212> PRT
<213> Mus musculus

<400> 96
Ser Ala Ser Gln Gly Ile Ser Asn Tyr Leu Asn
1               5                   10

<210> 97
<211> 7
<212> PRT
<213> Mus musculus

<400> 97
Tyr Thr Ser Ser Leu His Ser
1               5

<210> 98
<211> 9
<212> PRT
<213> Mus musculus

<400> 98
Gln Gln Tyr Ser Lys Leu Pro Trp Thr
1               5

<210> 99
<211> 6
<212> PRT
<213> Artificial Sequence

<220>
<221> source
<223> /note="Description of Artificial Sequence: Synthetic
      humanized antibody sequence"

<400> 99
Gly Tyr Glu Phe Asp Tyr
1               5

<210> 100
<211> 445
<212> PRT
<213> Artificial Sequence

<220>
<221> source
<223> /note="Description of Artificial Sequence: Synthetic
      humanized antibody sequence"

<400> 100
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asp Tyr
            20                  25                  30

Tyr Met Lys Trp Val Arg Gln Ala Pro Gly Gln Gly Leu Glu Trp Met
        35                  40                  45

Gly Glu Ile Tyr Pro Asn Asn Gly Gly Ile Thr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Tyr Glu Phe Val Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 101
<211> 1335

<212> DNA
<213> Artificial Sequence

<220>
<221> source
<223> /note="Description of Artificial Sequence: Synthetic
      humanized antibody sequence"

<400> 101
caggtgcagc tggtgcagag cggcgccgaa gtgaagaagc ccggctccag cgtgaaggtg      60

agctgcaagg ctagcggcgg caccttcagc gactactaca tgaagtgggt gaggcaggcc     120

cccggccagg gactggagtg gatgggcgag atctacccca acaacggggg catcacctac     180

aaccagaagt tcaagggcag ggtgaccatc accgccgaca aaagcaccag caccgcctac     240

atggaactga gcagcctgag gagcgaggac accgccgtgt actactgcgc caggggctac     300

gagttcgtgt attggggcca gggcacacta gtgaccgtgt ccagcgccag caccaagggc     360

cccagcgtgt tccccctggc ccccagcagc aagagcacca gcggcggcac agccgccctg     420

ggctgcctgg tgaaggacta cttccccgaa ccggtgaccg tgtcctggaa cagcggagcc     480

ctgaccagcg gcgtgcacac cttccccgcc gtgctgcaga gcagcggcct gtacagcctg     540

agcagcgtgg tgaccgtgcc cagcagcagc ctgggcaccc agacctacat ctgtaacgtg     600

aaccacaagc ccagcaacac caaggtggac aagaaggtgg agcccaagag ctgtgacaag     660

acccacacct gccccccctg ccctgccccc gagctgctgg gaggccccag cgtgttcctg     720

ttccccccca agcctaagga caccctgatg atcagcagaa cccccgaggt gacctgtgtg     780

gtggtggatg tgagccacga ggaccctgag gtgaagttca actggtacgt ggacggcgtg     840

gaggtgcaca atgccaagac caagcccagg gaggagcagt acaacagcac ctaccgggtg     900

gtgtccgtgc tgaccgtgct gcaccaggat tggctgaacg gcaaggagta caagtgtaag     960

gtgtccaaca aggccctgcc tgcccctatc gagaaaacca tcagcaaggc caagggccag    1020

cccagagagc cccaggtgta caccctgccc cctagcagag atgagctgac caagaaccag    1080

gtgtccctga cctgcctggt gaagggcttc taccccagcg acatcgccgt ggagtgggag    1140

agcaacggcc agcccgagaa caactacaag accacccccc ctgtgctgga cagcgatggc    1200

agcttcttcc tgtacagcaa gctgaccgtg gacaagagca gatggcagca gggcaacgtg    1260

ttcagctgct ccgtgatgca cgaggccctg cacaatcact acacccagaa gagcctgagc    1320

ctgtcccctg gcaag                                                      1335

<210> 102
<211> 445
<212> PRT
<213> Artificial Sequence

<220>
<221> source

<223> /note="Description of Artificial Sequence: Synthetic
      humanized antibody sequence"

<400> 102
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Met
        35                  40                  45

Gly Glu Ile Tyr Pro Asn Asn Gly Gly Ile Thr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Tyr Glu Phe Val Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 103
<211> 1335
<212> DNA
<213> Artificial Sequence

<220>
<221> source
<223> /note="Description of Artificial Sequence: Synthetic
      humanized antibody sequence"

<400> 103
caggtgcagc tggtgcagag cggcgccgaa gtgaagaagc ccggctccag cgtgaaggtg 60

agctgcaagg ctagcggcta caccttcacc gactactaca tgaagtgggt gaggcaggcc 120

cccggccagg gactggagtg gatgggcgag atctacccca acaacggggg catcacctac 180

aaccagaagt tcaagggcag ggtgaccatc accgccgaca aaagcaccag caccgcctac 240

atggaactga gcagcctgag gagcgaggac accgccgtgt actactgcgc caggggctac 300

gagttcgtgt attggggcca gggcacacta gtgaccgtgt ccagcgccag caccaagggc 360

cccagcgtgt tccccctggc ccccagcagc aagagcacca gcggcggcac agccgccctg 420

ggctgcctgg tgaaggacta cttccccgaa ccggtgaccg tgtcctggaa cagcggagcc 480

ctgaccagcg gcgtgcacac cttccccgcc gtgctgcaga gcagcggcct gtacagcctg 540

agcagcgtgg tgaccgtgcc cagcagcagc ctgggcaccc agacctacat ctgtaacgtg 600

aaccacaagc ccagcaacac caaggtggac aagaaggtgg agcccaagag ctgtgacaag 660

acccacacct gccccccctg ccctgccccc gagctgctgg gaggccccag cgtgttcctg 720

ttccccccca agcctaagga caccctgatg atcagcagaa cccccgaggt gacctgtgtg 780

gtggtggatg tgagccacga ggaccctgag gtgaagttca actggtacgt ggacggcgtg 840

gaggtgcaca atgccaagac caagcccagg gaggagcagt acaacagcac ctaccgggtg 900

gtgtccgtgc tgaccgtgct gcaccaggat tggctgaacg gcaaggagta caagtgtaag 960

gtgtccaaca aggccctgcc tgcccctatc gagaaaacca tcagcaaggc caagggccag 1020

cccagagagc cccaggtgta caccctgccc cctagcagag atgagctgac caagaaccag 1080

gtgtccctga cctgcctggt gaagggcttc taccccagcg acatcgccgt ggagtgggag 1140

agcaacggcc agcccgagaa caactacaag accacccccc ctgtgctgga cagcgatggc 1200

agcttcttcc tgtacagcaa gctgaccgtg gacaagagca gatggcagca gggcaacgtg 1260

ttcagctgct ccgtgatgca cgaggccctg cacaatcact acacccagaa gagcctgagc 1320

ctgtcccctg gcaag 1335

<210> 104
<211> 445
<212> PRT
<213> Artificial Sequence

<220>
<221> source
<223> /note="Description of Artificial Sequence: Synthetic
      humanized antibody sequence"

<400> 104
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Met
        35                  40                  45

Gly Glu Ile Tyr Pro Asn Asn Gly Gly Ile Thr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Asn Gly Tyr Glu Phe Val Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 105
<211> 1335
<212> DNA
<213> Artificial Sequence

<220>
<221> source
<223> /note="Description of Artificial Sequence: Synthetic
      humanized antibody sequence"

<400> 105
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gatgggcgag atctacccca acaacggggg catcacctac     180

aaccagaagt tcaagggcag ggtgaccatc accgccgaca aaagcaccag caccgcctac     240

atggaactga gcagcctgag gagcgaggac accgccgtgt actactgcgc caacggctac 300

gagttcgtgt attggggcca gggcacacta gtgaccgtgt ccagcgccag caccaagggc 360

cccagcgtgt tccccctggc ccccagcagc aagagcacca gcggcggcac agccgccctg 420

ggctgcctgg tgaaggacta cttccccgaa ccggtgaccg tgtcctggaa cagcggagcc 480

ctgaccagcg gcgtgcacac cttccccgcc gtgctgcaga gcagcggcct gtacagcctg 540

agcagcgtgg tgaccgtgcc cagcagcagc ctgggcaccc agacctacat ctgtaacgtg 600

aaccacaagc ccagcaacac caaggtggac aagaaggtgg agcccaagag ctgtgacaag 660

acccacacct gccccccctg ccctgccccc gagctgctgg gaggccccag cgtgttcctg 720

ttccccccca agcctaagga caccctgatg atcagcagaa cccccgaggt gacctgtgtg 780

gtggtggatg tgagccacga ggaccctgag gtgaagttca actggtacgt ggacggcgtg 840

gaggtgcaca atgccaagac caagcccagg gaggagcagt acaacagcac ctaccgggtg 900

gtgtccgtgc tgaccgtgct gcaccaggat tggctgaacg gcaaggagta caagtgtaag 960

gtgtccaaca aggccctgcc tgcccctatc gagaaaacca tcagcaaggc caagggccag 1020

cccagagagc cccaggtgta caccctgccc cctagcagag atgagctgac caagaaccag 1080

gtgtccctga cctgcctggt gaagggcttc taccccagcg acatcgccgt ggagtgggag 1140

agcaacggcc agcccgagaa caactacaag accacccccc ctgtgctgga cagcgatggc 1200

agcttcttcc tgtacagcaa gctgaccgtg gacaagagca gatggcagca gggcaacgtg 1260

ttcagctgct ccgtgatgca cgaggccctg cacaatcact acacccagaa gagcctgagc 1320

ctgtcccctg gcaag 1335

<210> 106
<211> 445
<212> PRT
<213> Artificial Sequence

<220>
<221> source
<223> /note="Description of Artificial Sequence: Synthetic
      humanized antibody sequence"

<400> 106
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Ile
        35                  40                  45

Gly Glu Ile Tyr Pro Asn Asn Gly Gly Ile Thr Tyr Asn Gln Lys Phe
    50                  55                  60

Lys Gly Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Asn Gly Tyr Glu Phe Val Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 107
<211> 1335
<212> DNA
<213> Artificial Sequence

<220>
<221> source
<223> /note="Description of Artificial Sequence: Synthetic
      humanized antibody sequence"

<400> 107
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gataggcgag atctacccca acaacggggg catcacctac     180

aaccagaagt tcaagggcag ggcgaccctc accgtcgaca aaagcaccag caccgcctac     240

atggaactga gcagcctgag gagcgaggac accgccgtgt actactgcgc caacggctac     300

gagttcgtgt attggggcca gggcacacta gtgaccgtgt ccagcgccag caccaagggc     360

cccagcgtgt tccccctggc ccccagcagc aagagcacca gcggcggcac agccgccctg     420

ggctgcctgg tgaaggacta cttccccgaa ccggtgaccg tgtcctggaa cagcggagcc     480

ctgaccagcg gcgtgcacac cttccccgcc gtgctgcaga gcagcggcct gtacagcctg 540

agcagcgtgg tgaccgtgcc cagcagcagc ctgggcaccc agacctacat ctgtaacgtg 600

aaccacaagc ccagcaacac caaggtggac aagaaggtgg agcccaagag ctgtgacaag 660

acccacacct gccccccctg ccctgccccc gagctgctgg gaggccccag cgtgttcctg 720

ttccccccca agcctaagga caccctgatg atcagcagaa cccccgaggt gacctgtgtg 780

gtggtggatg tgagccacga ggaccctgag gtgaagttca actggtacgt ggacggcgtg 840

gaggtgcaca atgccaagac caagcccagg gaggagcagt acaacagcac ctaccgggtg 900

gtgtccgtgc tgaccgtgct gcaccaggat tggctgaacg gcaaggagta caagtgtaag 960

gtgtccaaca aggccctgcc tgcccctatc gagaaaacca tcagcaaggc caagggccag 1020

cccagagagc cccaggtgta caccctgccc cctagcagag atgagctgac caagaaccag 1080

gtgtccctga cctgcctggt gaagggcttc taccccagcg acatcgccgt ggagtgggag 1140

agcaacggcc agcccgagaa caactacaag accacccccc ctgtgctgga cagcgatggc 1200

agcttcttcc tgtacagcaa gctgaccgtg gacaagagca gatggcagca gggcaacgtg 1260

ttcagctgct ccgtgatgca cgaggccctg cacaatcact acacccagaa gagcctgagc 1320

ctgtcccctg gcaag 1335

<210> 108
<211> 445
<212> PRT
<213> Artificial Sequence

<220>
<221> source
<223> /note="Description of Artificial Sequence: Synthetic
      humanized antibody sequence"

<400> 108
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Met
        35                  40                  45

Gly Glu Ile Tyr Pro Asn Asn Gly Gly Ile Thr Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Asp Gly Tyr Glu Phe Val Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 109
<211> 1335
<212> DNA
<213> Artificial Sequence

<220>
<221> source
<223> /note="Description of Artificial Sequence: Synthetic
      humanized antibody sequence"

<400> 109
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gatgggcgag atctacccca acaacggggg catcacctac     180

aaccagaagt tcaagggcag ggtgaccatc accgccgaca aaagcaccag caccgcctac     240

atggaactga gcagcctgag gagcgaggac accgccgtgt actactgcgc cgacggctac     300

gagttcgtgt attggggcca gggcacacta gtgaccgtgt ccagcgccag caccaagggc     360

cccagcgtgt tccccctggc ccccagcagc aagagcacca gcggcggcac agccgccctg     420

ggctgcctgg tgaaggacta cttccccgaa ccggtgaccg tgtcctggaa cagcggagcc     480

ctgaccagcg gcgtgcacac cttccccgcc gtgctgcaga gcagcggcct gtacagcctg     540

agcagcgtgg tgaccgtgcc cagcagcagc ctgggcaccc agacctacat ctgtaacgtg     600

aaccacaagc ccagcaacac caaggtggac aagaaggtgg agcccaagag ctgtgacaag     660

acccacacct gccccccctg ccctgccccc gagctgctgg gaggccccag cgtgttcctg     720

ttccccccca agcctaagga caccctgatg atcagcagaa cccccgaggt gacctgtgtg 780

gtggtggatg tgagccacga ggaccctgag gtgaagttca actggtacgt ggacggcgtg 840

gaggtgcaca atgccaagac caagcccagg gaggagcagt acaacagcac ctaccgggtg 900

gtgtccgtgc tgaccgtgct gcaccaggat tggctgaacg gcaaggagta caagtgtaag 960

gtgtccaaca aggccctgcc tgcccctatc gagaaaacca tcagcaaggc caagggccag 1020

cccagagagc cccaggtgta caccctgccc cctagcagag atgagctgac caagaaccag 1080

gtgtccctga cctgcctggt gaagggcttc taccccagcg acatcgccgt ggagtgggag 1140

agcaacggcc agcccgagaa caactacaag accacccccc ctgtgctgga cagcgatggc 1200

agcttcttcc tgtacagcaa gctgaccgtg gacaagagca gatggcagca gggcaacgtg 1260

ttcagctgct ccgtgatgca cgaggccctg cacaatcact acacccagaa gagcctgagc 1320

ctgtcccctg gcaag 1335

<210> 110
<211> 445
<212> PRT
<213> Artificial Sequence

<220>
<221> source
<223> /note="Description of Artificial Sequence: Synthetic
      humanized antibody sequence"

<400> 110
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Asp Tyr
            20                  25                  30

Tyr Met Lys Trp Val Arg Gln Ala Pro Gly Gln Gly Leu Glu Trp Ile
        35                  40                  45

Gly Glu Ile Tyr Pro Asn Asn Gly Gly Ile Thr Tyr Asn Gln Lys Phe
    50                  55                  60

Lys Gly Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Asn Gly Tyr Glu Phe Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 111
<211> 1335
<212> DNA
<213> Artificial Sequence

<220>
<221> source
<223> /note="Description of Artificial Sequence: Synthetic
      humanized antibody sequence"

<400> 111
caggtgcagc tggtgcagag cggcgccgaa gtgaagaagc ccggctccag cgtgaaggtg      60

agctgcaagg ctagcggcta caccttcacc gactactaca tgaagtgggt gaggcaggcc     120

cccggccagg gactggagtg gataggcgag atctacccca acaacggggg catcacctac     180

aaccagaagt tcaagggcag ggcgaccctc accgtcgaca aaagcaccag caccgcctac     240

atggaactga gcagcctgag gagcgaggac accgccgtgt actactgcgc caacggctac     300

gagttcgact attggggcca gggcacacta gtgaccgtgt ccagcgccag caccaagggc     360

cccagcgtgt tccccctggc ccccagcagc aagagcacca gcggcggcac agccgccctg     420

ggctgcctgg tgaaggacta cttccccgaa ccggtgaccg tgtcctggaa cagcggagcc     480

ctgaccagcg gcgtgcacac cttccccgcc gtgctgcaga gcagcggcct gtacagcctg     540

agcagcgtgg tgaccgtgcc cagcagcagc ctgggcaccc agacctacat ctgtaacgtg     600

aaccacaagc ccagcaacac caaggtggac aagaaggtgg agcccaagag ctgtgacaag     660

acccacacct gccccccctg ccctgccccc gagctgctgg gaggccccag cgtgttcctg     720

ttccccccca agcctaagga caccctgatg atcagcagaa cccccgaggt gacctgtgtg     780

gtggtggatg tgagccacga ggaccctgag gtgaagttca actggtacgt ggacggcgtg     840

gaggtgcaca atgccaagac caagcccagg gaggagcagt acaacagcac ctaccgggtg     900

gtgtccgtgc tgaccgtgct gcaccaggat tggctgaacg gcaaggagta caagtgtaag     960

gtgtccaaca aggccctgcc tgcccctatc gagaaaacca tcagcaaggc caagggccag 1020

cccagagagc cccaggtgta caccctgccc cctagcagag atgagctgac caagaaccag 1080

gtgtccctga cctgcctggt gaagggcttc taccccagcg acatcgccgt ggagtgggag 1140

agcaacggcc agcccgagaa caactacaag accacccccc ctgtgctgga cagcgatggc 1200

agcttcttcc tgtacagcaa gctgaccgtg gacaagagca gatggcagca gggcaacgtg 1260

ttcagctgct ccgtgatgca cgaggccctg cacaatcact acacccagaa gagcctgagc 1320

ctgtcccctg gcaag 1335

<210> 112
<211> 214
<212> PRT
<213> Artificial Sequence

<220>
<221> source
<223> /note="Description of Artificial Sequence: Synthetic
      humanized antibody sequence"

<400> 112
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Gly Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Lys Leu Pro Trp
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 113
<211> 642
<212> DNA
<213> Artificial Sequence

<220>
<221> source
<223> /note="Description of Artificial Sequence: Synthetic
      humanized antibody sequence"

<400> 113
gacatccaga tgacccagag cccctcaagc ctgagcgcca gcgtgggcga cagggtgact      60

atcacctgca gcgcctccca gggcatcagc aactacctga actggtacca gcagaagccc     120

ggcaaggccc ctaagctgct gatctactac accagcagcc tgcacagcgg cgtgcccagc     180

aggttctccg gcagcggcag cggaaccgac ttcaccctga ccattagcag cctccagccc     240

gaggacttcg ccacctacta ctgccagcag tacagcaagc tgccctggac cttcggccag     300

ggcaccaaac tggagatcaa gcgtacggtg gccgccccca gcgtgttcat cttccccccc     360

agcgatgagc agctgaagag cggcaccgcc agcgtggtgt gtctgctgaa caacttctac     420

ccccgggagg ccaaggtgca gtggaaggtg gacaatgccc tgcagagcgg caacagccag     480

gagagcgtga ccgagcagga cagcaaggac tccacctaca gcctgagcag caccctgacc     540

ctgagcaagg ccgactacga gaagcacaag gtgtacgcct gtgaggtgac ccaccagggc     600

ctgtccagcc ccgtgaccaa gagcttcaac cggggcgagt gc                        642

<210> 114
<211> 214
<212> PRT
<213> Artificial Sequence

<220>
<221> source
<223> /note="Description of Artificial Sequence: Synthetic
      humanized antibody sequence"

<400> 114
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Gly Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Tyr Ser Lys Leu Pro Trp
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 115
<211> 642
<212> DNA
<213> Artificial Sequence

<220>
<221> source
<223> /note="Description of Artificial Sequence: Synthetic
      humanized antibody sequence"

<400> 115
gacatccaga tgacccagag cccctcaagc ctgagcgcca gcgtgggcga cagggtgact        60

atcacctgca gcgcctccca gggcatcagc aactacctga actggtacca gcagaagccc       120

ggcaaggccc ctaagctgct gatctactac accagcagcc tgcacagcgg cgtgcccagc       180

aggttctccg gcagcggcag cggaaccgac tacaccctga ccattagcag cctccagccc       240

gaggacttcg ccacctacta ctgccagcag tacagcaagc tgccctggac cttcggccag       300

ggcaccaaac tggagatcaa gcgtacggtg gccgccccca gcgtgttcat cttccccccc       360

agcgatgagc agctgaagag cggcaccgcc agcgtggtgt gtctgctgaa caacttctac       420

ccccgggagg ccaaggtgca gtggaaggtg gacaatgccc tgcagagcgg caacagccag       480

gagagcgtga ccgagcagga cagcaaggac tccacctaca gcctgagcag caccctgacc       540

ctgagcaagg ccgactacga gaagcacaag gtgtacgcct gtgaggtgac ccaccagggc       600

ctgtccagcc ccgtgaccaa gagcttcaac cggggcgagt gc                          642


<210> 116
<211> 118
<212> PRT
<213> Mus musculus

<400> 116
Glu Val Gln Leu Gln Gln Ser Gly Pro Val Leu Val Lys Pro Gly Ala
1               5                   10                  15


Ser Val Lys Met Ser Cys Glu Ala Ser Gly Tyr Thr Phe Thr Asp Tyr
            20                  25                  30


Tyr Met Asn Trp Val Lys Gln Ser His Gly Lys Thr Leu Glu Trp Ile
        35                  40                  45


Gly Val Ile Asn Pro Tyr Asn Gly Gly Thr Asp Tyr Asn Gln Lys Phe
    50                  55                  60


Lys Gly Lys Ala Thr Leu Thr Val Asp Lys Ser Ser Ser Thr Ala Tyr
65                  70                  75                  80


Met Glu Leu Asn Ser Leu Thr Ser Glu Asp Ser Ala Val Tyr Tyr Cys
                85                  90                  95


Ala Arg Ser Val Tyr Asp Tyr Pro Phe Asp Tyr Trp Gly Gln Gly Thr
            100                 105                 110

Leu Val Thr Val Ser Ser
        115

<210> 117
<211> 354
<212> DNA
<213> Mus musculus

<400> 117
gaggtgcagc tgcagcagag cggccccgtg ctggtgaagc ctggagccag cgtgaaaatg      60

agctgcgaag ccagcggcta caccttcacc gactactaca tgaactgggt gaagcagagc     120

cacggcaaga ccctggagtg gatcggcgtg atcaacccct acaacggggg caccgactac     180

aaccagaagt tcaagggcaa ggccactctg accgtggaca agagctccag caccgcctac     240

atggaactga acagcctcac ctctgaggac agcgccgtct attactgcgc caggagcgtg     300

tacgactacc ccttcgacta ctggggccag ggcacactag tgaccgtgtc cagc           354

<210> 118
<211> 448
<212> PRT
<213> Artificial Sequence

<220>
<221> source
<223> /note="Description of Artificial Sequence: Synthetic
      chimeric antibody sequence"

<400> 118
Glu Val Gln Leu Gln Gln Ser Gly Pro Val Leu Val Lys Pro Gly Ala
1               5                   10                  15

Ser Val Lys Met Ser Cys Glu Ala Ser Gly Tyr Thr Phe Thr Asp Tyr
            20                  25                  30

Tyr Met Asn Trp Val Lys Gln Ser His Gly Lys Thr Leu Glu Trp Ile
        35                  40                  45

Gly Val Ile Asn Pro Tyr Asn Gly Gly Thr Asp Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Asn Ser Leu Thr Ser Glu Asp Ser Ala Val Tyr Tyr Cys
                85                  90                  95

Ala Arg Ser Val Tyr Asp Tyr Pro Phe Asp Tyr Trp Gly Gln Gly Thr
            100                 105                 110

Leu Val Thr Val Ser Ser Ala Ser Thr Lys Gly Pro Ser Val Phe Pro

115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210> 119
<211> 1344
<212> DNA
<213> Artificial Sequence

<220>
<221> source
<223> /note="Description of Artificial Sequence: Synthetic
      chimeric antibody sequence"

<400> 119
gaggtgcagc tgcagcagag cggccccgtg ctggtgaagc ctggagccag cgtgaaaatg      60

agctgcgaag ccagcggcta caccttcacc gactactaca tgaactgggt gaagcagagc     120

cacggcaaga ccctggagtg gatcggcgtg atcaacccct acaacggggg caccgactac     180

aaccagaagt tcaagggcaa ggccactctg accgtggaca agagctccag caccgcctac     240

atggaactga acagcctcac ctctgaggac agcgccgtct attactgcgc caggagcgtg     300

tacgactacc ccttcgacta ctggggccag ggcacactag tgaccgtgtc cagcgccagc     360

accaagggcc ccagcgtgtt ccccctggcc cccagcagca agagcaccag cggcggcaca     420

gccgccctgg gctgcctggt gaaggactac ttccccgaac cggtgaccgt gtcctggaac     480

agcggagccc tgaccagcgg cgtgcacacc ttccccgccg tgctgcagag cagcggcctg     540

tacagcctga gcagcgtggt gaccgtgccc agcagcagcc tgggcaccca gacctacatc     600

tgtaacgtga accacaagcc cagcaacacc aaggtggaca agaaggtgga gcccaagagc     660

tgtgacaaga cccacacctg ccccccctgc cctgcccccg agctgctggg aggccccagc     720

gtgttcctgt tcccccccaa gcctaaggac accctgatga tcagcagaac ccccgaggtg      780

acctgtgtgg tggtggatgt gagccacgag gaccctgagg tgaagttcaa ctggtacgtg     840

gacggcgtgg aggtgcacaa tgccaagacc aagcccaggg aggagcagta caacagcacc     900

taccgggtgg tgtccgtgct gaccgtgctg caccaggatt ggctgaacgg caaggagtac     960

aagtgtaagg tgtccaacaa ggccctgcct gcccctatcg agaaaaccat cagcaaggcc 1020

aagggccagc ccagagagcc ccaggtgtac accctgcccc ctagcagaga tgagctgacc 1080

aagaaccagg tgtccctgac ctgcctggtg aagggcttct accccagcga catcgccgtg 1140

gagtgggaga gcaacggcca gcccgagaac aactacaaga ccaccccccc tgtgctggac 1200

agcgatggca gcttcttcct gtacagcaag ctgaccgtgg acaagagcag atggcagcag 1260

ggcaacgtgt tcagctgctc cgtgatgcac gaggccctgc acaatcacta cacccagaag 1320

agcctgagcc tgtcccctgg caag 1344

<210> 120
<211> 111
<212> PRT
<213> Mus musculus

<400> 120
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile His
            20                  25                  30

Gly Thr His Leu Met His Trp Tyr Gln Gln Lys Pro Gly Gln Pro Pro
        35                  40                  45

Lys Leu Leu Ile Tyr Ala Ala Ser Asn Leu Glu Ser Gly Val Pro Ala
    50                  55                  60

Arg Phe Ser Gly Ser Gly Ser Glu Thr Asp Phe Thr Leu Asn Ile His
65                  70                  75                  80

Pro Val Glu Glu Glu Asp Ala Ala Thr Tyr Phe Cys Gln Gln Ser Ile
                85                  90                  95

Glu Asp Pro Arg Thr Phe Gly Gly Gly Thr Lys Leu Glu Ile Lys
            100                 105                 110

<210> 121
<211> 336
<212> DNA
<213> Mus musculus

<400> 121
gacatcgtcc tgacccagag ccccgccagc ctggccgtga gcctgggcca gagggccaca 60

atcagctgca gggcctctga gtccgtgagc atccacggca cccacctgat gcactggtat 120

cagcagaagc ccggccagcc tcccaagctg ctgatctacg ccgccagcaa cctggagagc 180

ggcgtgcccg ctaggttcag cggaagcggc agcgagaccg acttcaccct gaacatccac 240

cccgtggagg aggaagacgc cgccacctac ttctgccagc agagcatcga ggaccccagg 300

accttcggcg ggggcaccaa gctcgagatt aagcgt 336

<210> 122
<211> 237
<212> PRT
<213> Artificial Sequence

<220>
<221> source
<223> /note="Description of Artificial Sequence: Synthetic
      chimeric antibody sequence"

<400> 122
Met Gly Trp Ser Cys Ile Ile Leu Phe Leu Val Ala Thr Ala Thr Gly
1               5                   10                  15

Val His Ser Asp Ile Val Leu Thr Gln Ser Pro Ala Ser Leu Ala Val
            20                  25                  30

Ser Leu Gly Gln Arg Ala Thr Ile Ser Cys Arg Ala Ser Glu Ser Val
        35                  40                  45

Ser Ile His Gly Thr His Leu Met His Trp Tyr Gln Gln Lys Pro Gly
    50                  55                  60

Gln Pro Pro Lys Leu Leu Ile Tyr Ala Ala Ser Asn Leu Glu Ser Gly
65                  70                  75                  80

Val Pro Ala Arg Phe Ser Gly Ser Gly Ser Glu Thr Asp Phe Thr Leu
                85                  90                  95

Asn Ile His Pro Val Glu Glu Glu Asp Ala Ala Thr Tyr Phe Cys Gln
            100                 105                 110

Gln Ser Ile Glu Asp Pro Arg Thr Phe Gly Gly Gly Thr Lys Leu Glu
        115                 120                 125

Ile Lys Arg Thr Val Ala Ala Pro Ser Val Phe Ile Phe Pro Pro Ser
    130                 135                 140

Asp Glu Gln Leu Lys Ser Gly Thr Ala Ser Val Val Cys Leu Leu Asn
145                 150                 155                 160

Asn Phe Tyr Pro Arg Glu Ala Lys Val Gln Trp Lys Val Asp Asn Ala
                165                 170                 175

Leu Gln Ser Gly Asn Ser Gln Glu Ser Val Thr Glu Gln Asp Ser Lys
            180                 185                 190

Asp Ser Thr Tyr Ser Leu Ser Ser Thr Leu Thr Leu Ser Lys Ala Asp
        195                 200                 205

Tyr Glu Lys His Lys Val Tyr Ala Cys Glu Val Thr His Gln Gly Leu
    210                 215                 220

Ser Ser Pro Val Thr Lys Ser Phe Asn Arg Gly Glu Cys
225                 230                 235

<210> 123
<211> 711
<212> DNA
<213> Artificial Sequence

<220>
<221> source
<223> /note="Description of Artificial Sequence: Synthetic
      chimeric antibody sequence"

<400> 123
atgggctggt cctgcatcat cctgtttctg gtggccaccg ccaccggcgt gcacagcgac      60

atcgtcctga cccagagccc cgccagcctg gccgtgagcc tgggccagag ggccacaatc     120

agctgcaggg cctctgagtc cgtgagcatc cacggcaccc acctgatgca ctggtatcag     180

cagaagcccg gccagcctcc caagctgctg atctacgccg ccagcaacct ggagagcggc     240

gtgcccgcta ggttcagcgg aagcggcagc gagaccgact tcaccctgaa catccacccc     300

gtggaggagg aagacgccgc cacctacttc tgccagcaga gcatcgagga ccccaggacc     360

ttcggcgggg gcaccaagct cgagattaag cgtacggtgg ccgcccccag cgtgttcatc     420

ttccccccca gcgatgagca gctgaagagc ggcaccgcca gcgtggtgtg tctgctgaac     480

aacttctacc cccgggaggc caaggtgcag tggaaggtgg acaatgccct gcagagcggc     540

aacagccagg agagcgtgac cgagcaggac agcaaggact ccacctacag cctgagcagc     600

accctgaccc tgagcaaggc cgactacgag aagcacaagg tgtacgcctg tgaggtgacc     660

caccagggcc tgtccagccc cgtgaccaag agcttcaacc ggggcgagtg c              711

<210> 124
<211> 119
<212> PRT
<213> Mus musculus

<400> 124
Glu Val Gln Leu Gln Gln Ser Gly Pro Glu Leu Val Lys Pro Gly Thr
1               5                   10                  15

Ser Val Lys Ile Pro Cys Lys Thr Ser Gly Tyr Ile Phe Thr Asp Tyr
            20                  25                  30

Ser Ile Asp Trp Val Lys Gln Ser His Gly Lys Ser Leu Glu Trp Ile
        35                  40                  45

Gly Asp Ile Asp Pro Asn Tyr Gly Asp Pro Ile Tyr Asn His Lys Phe
    50                  55                  60

Lys Gly Lys Ala Thr Leu Thr Val Asp Arg Ser Ser Ser Thr Ala Tyr
65                  70                  75                  80

Met Glu Leu Arg Ser Leu Thr Ser Glu Asp Thr Ala Val Tyr Phe Cys
                85                  90                  95

Ala Arg Arg Ala Thr Gly Thr Asp Trp Phe Ala Phe Trp Gly Gln Gly
            100                 105                 110

Thr Leu Val Thr Val Ser Ser
        115

<210> 125
<211> 357
<212> DNA
<213> Mus musculus

<400> 125
gaggtgcagc tgcagcagag cggccccgag ctggtgaaac ccggcaccag cgtgaagatc      60

ccctgcaaga cctctggcta catcttcacc gactacagca tcgactgggt gaagcagagc     120

cacggcaagt ctctggagtg gattggggac atcgacccca actacggcga ccccatctac     180

aaccacaagt tcaagggcaa ggccaccctg accgtggaca ggagcagcag caccgcctac     240

atggaactca ggagcctgac cagcgaggac accgccgtgt atttttgcgc caggagggcc     300

accggcactg attggttcgc cttctggggc cagggcacac tagtgaccgt gtccagc       357

<210> 126
<211> 449
<212> PRT
<213> Artificial Sequence

<220>
<221> source
<223> /note="Description of Artificial Sequence: Synthetic
      chimeric antibody sequence"

<400> 126
Glu Val Gln Leu Gln Gln Ser Gly Pro Glu Leu Val Lys Pro Gly Thr
1               5                   10                  15

Ser Val Lys Ile Pro Cys Lys Thr Ser Gly Tyr Ile Phe Thr Asp Tyr
            20                  25                  30

Ser Ile Asp Trp Val Lys Gln Ser His Gly Lys Ser Leu Glu Trp Ile

35                  40                  45

Gly Asp Ile Asp Pro Asn Tyr Gly Asp Pro Ile Tyr Asn His Lys Phe
    50                  55                  60

Lys Gly Lys Ala Thr Leu Thr Val Asp Arg Ser Ser Ser Thr Ala Tyr
65                  70                  75                  80

Met Glu Leu Arg Ser Leu Thr Ser Glu Asp Thr Ala Val Tyr Phe Cys
                85                  90                  95

Ala Arg Arg Ala Thr Gly Thr Asp Trp Phe Ala Phe Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210> 127
<211> 1347
<212> DNA
<213> Artificial Sequence

<220>
<221> source
<223> /note="Description of Artificial Sequence: Synthetic
      chimeric antibody sequence"

<400> 127
gaggtgcagc tgcagcagag cggccccgag ctggtgaaac ccggcaccag cgtgaagatc      60

ccctgcaaga cctctggcta catcttcacc gactacagca tcgactgggt gaagcagagc     120

cacggcaagt ctctggagtg gattggggac atcgacccca actacggcga ccccatctac     180

aaccacaagt tcaagggcaa ggccaccctg accgtggaca ggagcagcag caccgcctac     240

atggaactca ggagcctgac cagcgaggac accgccgtgt atttttgcgc caggagggcc 300

accggcactg attggttcgc cttctggggc cagggcacac tagtgaccgt gtccagcgcc 360

agcaccaagg gccccagcgt gttccccctg gcccccagca gcaagagcac cagcggcggc 420

acagccgccc tgggctgcct ggtgaaggac tacttccccg aaccggtgac cgtgtcctgg 480

aacagcggag ccctgaccag cggcgtgcac accttccccg ccgtgctgca gagcagcggc 540

ctgtacagcc tgagcagcgt ggtgaccgtg cccagcagca gcctgggcac ccagacctac 600

atctgtaacg tgaaccacaa gcccagcaac accaaggtgg acaagaaggt ggagcccaag 660

agctgtgaca agacccacac ctgccccccc tgccctgccc ccgagctgct gggaggcccc 720

agcgtgttcc tgttcccccc caagcctaag gacaccctga tgatcagcag aacccccgag 780

gtgacctgtg tggtggtgga tgtgagccac gaggaccctg aggtgaagtt caactggtac 840

gtggacggcg tggaggtgca caatgccaag accaagccca gggaggagca gtacaacagc 900

acctaccggg tggtgtccgt gctgaccgtg ctgcaccagg attggctgaa cggcaaggag 960

tacaagtgta aggtgtccaa caaggccctg cctgccccta tcgagaaaac catcagcaag 1020

gccaagggcc agcccagaga gccccaggtg tacaccctgc cccctagcag agatgagctg 1080

accaagaacc aggtgtccct gacctgcctg gtgaagggct tctaccccag cgacatcgcc 1140

gtggagtggg agagcaacgg ccagcccgag aacaactaca agaccacccc ccctgtgctg 1200

gacagcgatg gcagcttctt cctgtacagc aagctgaccg tggacaagag cagatggcag 1260

cagggcaacg tgttcagctg ctccgtgatg cacgaggccc tgcacaatca ctacacccag 1320

aagagcctga gcctgtcccc tggcaag 1347

<210> 128
<211> 108
<212> PRT
<213> Mus musculus

<400> 128
Asp Ile Gln Met Thr Gln Ser Pro Ala Ser Leu Ser Val Ser Val Gly
1               5                   10                  15

Glu Thr Val Thr Ile Thr Cys Arg Ala Ser Glu Asn Ile Tyr Asn Asn
            20                  25                  30

Leu Ala Trp Tyr Gln Gln Lys Gln Gly Lys Ser Pro Gln Leu Leu Val
        35                  40                  45

Tyr Ala Ala Thr Ile Leu Ala Asp Gly Val Pro Ser Arg Phe Ser Gly
    50                  55                  60

Ser Gly Ser Gly Thr Gln Tyr Ser Leu Lys Ile Asn Ser Leu Gln Ser
65                  70                  75                  80

Gly Asp Phe Gly Thr Tyr Tyr Cys Gln His Phe Trp Gly Thr Pro Leu
                85                  90                  95

Thr Phe Gly Ala Gly Thr Lys Leu Glu Leu Lys Arg
            100                 105

<210> 129
<211> 324
<212> DNA
<213> Mus musculus

<400> 129
gacatccaga tgacccagag ccccgctagc ctcagcgtgt ccgtcggcga gaccgtgacc      60

atcacctgca gggccagcga gaacatctac aacaacctgg cctggtatca gcagaagcag     120

ggcaaaagcc cccagctgct ggtgtacgcc gccaccattc tggccgacgg cgtgcccagc     180

aggttctctg gaagcggcag cggcacccag tacagcctga agatcaacag cctgcagagc     240

ggggacttcg gcacctacta ctgccagcac ttctggggca ctcccctgac cttcggagcc     300

ggcaccaagc tggagctgaa gcgt                                            324

<210> 130
<211> 214
<212> PRT
<213> Artificial Sequence

<220>
<221> source
<223> /note="Description of Artificial Sequence: Synthetic
      chimeric antibody sequence"

<400> 130
Asp Ile Gln Met Thr Gln Ser Pro Ala Ser Leu Ser Val Ser Val Gly
1               5                   10                  15

Glu Thr Val Thr Ile Thr Cys Arg Ala Ser Glu Asn Ile Tyr Asn Asn
            20                  25                  30

Leu Ala Trp Tyr Gln Gln Lys Gln Gly Lys Ser Pro Gln Leu Leu Val
        35                  40                  45

Tyr Ala Ala Thr Ile Leu Ala Asp Gly Val Pro Ser Arg Phe Ser Gly
    50                  55                  60

Ser Gly Ser Gly Thr Gln Tyr Ser Leu Lys Ile Asn Ser Leu Gln Ser
65                  70                  75                  80

Gly Asp Phe Gly Thr Tyr Tyr Cys Gln His Phe Trp Gly Thr Pro Leu
                85                  90                  95

Thr Phe Gly Ala Gly Thr Lys Leu Glu Leu Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 131
<211> 642
<212> DNA
<213> Artificial Sequence

<220>
<221> source
<223> /note="Description of Artificial Sequence: Synthetic
      chimeric antibody sequence"

<400> 131
gacatccaga tgacccagag ccccgctagc ctcagcgtgt ccgtcggcga gaccgtgacc      60

atcacctgca gggccagcga gaacatctac aacaacctgg cctggtatca gcagaagcag     120

ggcaaaagcc cccagctgct ggtgtacgcc gccaccattc tggccgacgg cgtgcccagc     180

aggttctctg gaagcggcag cggcacccag tacagcctga agatcaacag cctgcagagc     240

ggggacttcg gcacctacta ctgccagcac ttctggggca ctcccctgac cttcggagcc     300

ggcaccaagc tggagctgaa gcgtacggtg gccgccccca gcgtgttcat cttccccccc     360

agcgatgagc agctgaagag cggcaccgcc agcgtggtgt gtctgctgaa caacttctac     420

ccccgggagg ccaaggtgca gtggaaggtg gacaatgccc tgcagagcgg caacagccag     480

gagagcgtga ccgagcagga cagcaaggac tccacctaca gcctgagcag caccctgacc     540

ctgagcaagg ccgactacga gaagcacaag gtgtacgcct gtgaggtgac ccaccagggc     600

ctgtccagcc ccgtgaccaa gagcttcaac cggggcgagt gc 642

<210> 132
<211> 118
<212> PRT
<213> Mus musculus

<400> 132
Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Asn Tyr
            20                  25                  30

Trp Met His Trp Val Lys Gln Arg Pro Gly Gln Gly Leu Glu Trp Ile
        35                  40                  45

Gly Ile Ile His Pro Asn Ser Gly Ser Thr Asn Tyr Asn Glu Lys Phe
    50                  55                  60

Lys Ser Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Gly Ile Tyr Asp Tyr Pro Phe Ala Tyr Trp Gly Gln Gly Thr
            100                 105                 110

Leu Val Thr Val Ser Ser
        115

<210> 133
<211> 354
<212> DNA
<213> Mus musculus

<400> 133
caggtgcagc tccagcagcc cggagccgaa ctggtgaagc ccggagccag cgtcaaactg 60

tcctgcaagg ccagcggcta caccttcacc aactactgga tgcactgggt gaagcagagg 120

cccggccagg gcctggagtg gatcggcatc atccacccca acagcgggag caccaactac 180

aacgagaagt tcaagagcaa ggccaccctg accgtggaca agagcagcag cactgcctac 240

atgcagctga gcagcctgac cagcgaggac agcgctgtgt actactgcgc caggggcatc 300

tacgactacc ccttcgccta ttggggccag ggcacactag tgaccgtgtc cagc 354

<210> 134
<211> 448

<212> PRT
<213> Artificial Sequence

<220>
<221> source
<223> /note="Description of Artificial Sequence: Synthetic
      chimeric antibody sequence"

<400> 134
Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Asn Tyr
            20                  25                  30

Trp Met His Trp Val Lys Gln Arg Pro Gly Gln Gly Leu Glu Trp Ile
        35                  40                  45

Gly Ile Ile His Pro Asn Ser Gly Ser Thr Asn Tyr Asn Glu Lys Phe
    50                  55                  60

Lys Ser Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Gly Ile Tyr Asp Tyr Pro Phe Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210> 135
<211> 1344
<212> DNA
<213> Artificial Sequence

<220>
<221> source
<223> /note="Description of Artificial Sequence: Synthetic
      chimeric antibody sequence"

<400> 135
caggtgcagc tccagcagcc cggagccgaa ctggtgaagc ccggagccag cgtcaaactg        60

tcctgcaagg ccagcggcta caccttcacc aactactgga tgcactgggt gaagcagagg       120

cccggccagg gcctggagtg gatcggcatc atccacccca acagcgggag caccaactac       180

aacgagaagt tcaagagcaa ggccaccctg accgtggaca agagcagcag cactgcctac       240

atgcagctga gcagcctgac cagcgaggac agcgctgtgt actactgcgc caggggcatc       300

tacgactacc ccttcgccta ttggggccag ggcacactag tgaccgtgtc cagcgccagc       360

accaagggcc ccagcgtgtt ccccctggcc cccagcagca agagcaccag cggcggcaca       420

gccgccctgg gctgcctggt gaaggactac ttccccgaac cggtgaccgt gtcctggaac       480

agcggagccc tgaccagcgg cgtgcacacc ttccccgccg tgctgcagag cagcggcctg       540

tacagcctga gcagcgtggt gaccgtgccc agcagcagcc tgggcaccca gacctacatc       600

tgtaacgtga accacaagcc cagcaacacc aaggtggaca agaaggtgga gcccaagagc       660

tgtgacaaga cccacacctg ccccccctgc cctgcccccg agctgctggg aggccccagc       720

gtgttcctgt tcccccccaa gcctaaggac accctgatga tcagcagaac ccccgaggtg       780

acctgtgtgg tggtggatgt gagccacgag gaccctgagg tgaagttcaa ctggtacgtg       840

gacggcgtgg aggtgcacaa tgccaagacc aagcccaggg aggagcagta caacagcacc       900

taccgggtgg tgtccgtgct gaccgtgctg caccaggatt ggctgaacgg caaggagtac       960

aagtgtaagg tgtccaacaa ggccctgcct gcccctatcg agaaaaccat cagcaaggcc      1020

aagggccagc ccagagagcc ccaggtgtac accctgcccc ctagcagaga tgagctgacc      1080

aagaaccagg tgtccctgac ctgcctggtg aagggcttct accccagcga catcgccgtg      1140

gagtgggaga gcaacggcca gcccgagaac aactacaaga ccaccccccc tgtgctggac      1200

agcgatggca gcttcttcct gtacagcaag ctgaccgtgg acaagagcag atggcagcag      1260

ggcaacgtgt tcagctgctc cgtgatgcac gaggccctgc acaatcacta cacccagaag      1320

agcctgagcc tgtcccctgg caag                                             1344


<210> 136
<211> 112
<212> PRT
<213> Mus musculus

<400> 136
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile His
            20                  25                  30

Gly Thr His Leu Met His Trp Tyr Gln Gln Lys Pro Gly Gln Pro Pro
        35                  40                  45

Lys Leu Leu Ile Tyr Ala Ala Ser Asn Leu Glu Ser Gly Val Pro Ala
    50                  55                  60

Arg Phe Ser Gly Ser Gly Ser Glu Thr Asp Phe Thr Leu Asn Ile His
65                  70                  75                  80

Pro Val Glu Glu Glu Asp Ala Ala Thr Tyr Phe Cys Gln Gln Ser Ile
                85                  90                  95

Glu Asp Pro Tyr Thr Phe Gly Gly Gly Thr Lys Leu Glu Ile Lys Arg
            100                 105                 110

<210> 137
<211> 336
<212> DNA
<213> Mus musculus

<400> 137
gacatcgtgc tgacccagtc tcccgctagc ctggccgtgt ctctgggcca gagggccaca      60

atcagctgca gggccagcga gagcgtcagc attcacggca cccacctgat gcactggtac     120

cagcagaagc ccggccagcc tcccaagctc ctgatctacg ccgccagcaa cctggaaagc     180

ggagtgcccg ccaggttcag cggcagcggc tccgagaccg acttcaccct gaacatccac     240

cccgtggagg aggaggacgc cgccacctac ttctgccagc agagcatcga ggacccctac     300

accttcggcg gcggcaccaa gctggagatc aagcgt                               336

<210> 138
<211> 218
<212> PRT
<213> Artificial Sequence

<220>
<221> source
<223> /note="Description of Artificial Sequence: Synthetic
      chimeric antibody sequence"

<400> 138
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile His
            20                  25                  30

Gly Thr His Leu Met His Trp Tyr Gln Gln Lys Pro Gly Gln Pro Pro

35                  40                  45

Lys Leu Leu Ile Tyr Ala Ala Ser Asn Leu Glu Ser Gly Val Pro Ala
    50                  55                  60

Arg Phe Ser Gly Ser Gly Ser Glu Thr Asp Phe Thr Leu Asn Ile His
65                  70                  75                  80

Pro Val Glu Glu Glu Asp Ala Ala Thr Tyr Phe Cys Gln Gln Ser Ile
                85                  90                  95

Glu Asp Pro Tyr Thr Phe Gly Gly Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210> 139
<211> 654
<212> DNA
<213> Artificial Sequence

<220>
<221> source
<223> /note="Description of Artificial Sequence: Synthetic
      chimeric antibody sequence"

<400> 139
gacatcgtgc tgacccagtc tcccgctagc ctggccgtgt ctctgggcca gagggccaca        60

atcagctgca gggccagcga gagcgtcagc attcacggca cccacctgat gcactggtac       120

cagcagaagc ccggccagcc tcccaagctc ctgatctacg ccgccagcaa cctggaaagc       180

ggagtgcccg ccaggttcag cggcagcggc tccgagaccg acttcaccct gaacatccac 240

cccgtggagg aggaggacgc cgccacctac ttctgccagc agagcatcga ggacccctac 300

accttcggcg gcggcaccaa gctggagatc aagcgtacgg tggccgcccc cagcgtgttc 360

atcttccccc ccagcgatga gcagctgaag agcggcaccg ccagcgtggt gtgtctgctg 420

aacaacttct acccccggga ggccaaggtg cagtggaagg tggacaatgc cctgcagagc 480

ggcaacagcc aggagagcgt gaccgagcag gacagcaagg actccaccta cagcctgagc 540

agcaccctga ccctgagcaa ggccgactac gagaagcaca aggtgtacgc ctgtgaggtg 600

acccaccagg gcctgtccag ccccgtgacc aagagcttca accggggcga gtgc 654

<210> 140
<211> 120
<212> PRT
<213> Mus musculus

<400> 140
Glu Val Lys Leu Leu Gln Ser Gly Gly Gly Leu Val Gln Pro Gly Gly
1               5                   10                  15

Ser Leu Lys Leu Ser Cys Ala Ala Ser Gly Ile Asp Phe Ser Arg Tyr
            20                  25                  30

Trp Met Ser Trp Val Arg Arg Ala Pro Gly Lys Gly Leu Glu Trp Ile
        35                  40                  45

Gly Glu Ile Asn Pro Asp Arg Ser Thr Ile Asn Tyr Ala Pro Ser Leu
    50                  55                  60

Lys Asp Lys Phe Ile Ile Ser Arg Asp Asn Ala Lys Asn Thr Leu Tyr
65                  70                  75                  80

Leu Gln Met Ser Lys Val Arg Ser Glu Asp Thr Ala Leu Tyr Tyr Cys
                85                  90                  95

Ala Val Phe Tyr Tyr Asp Tyr Glu Gly Ala Met Asp Tyr Trp Gly Gln
            100                 105                 110

Gly Thr Ser Val Thr Val Ser Ser
        115                 120

<210> 141
<211> 360
<212> DNA
<213> Mus musculus

<400> 141
gaggtgaagc ttctccagtc tggaggtggc ctggtgcagc ctggaggatc cctgaaactc 60

tcctgtgcag cctcaggaat cgattttagt agatactgga tgagttgggt tcggcgggct     120

ccagggaaag gactagaatg gattggagaa attaatccag ataggagtac aatcaactat     180

gcaccatctc taaaggataa attcatcatc tccagagaca acgccaaaaa tacgctgtac     240

ctgcaaatga gcaaagtgag atctgaggac acagcccttt attactgtgc agttttctac     300

tatgattacg agggtgctat ggactactgg ggtcaaggaa cctcagtcac cgtctcctca     360

<210> 142
<211> 450
<212> PRT
<213> Artificial Sequence

<220>
<221> source
<223> /note="Description of Artificial Sequence: Synthetic
      chimeric antibody sequence"

<400> 142
Glu Val Lys Leu Leu Gln Ser Gly Gly Gly Leu Val Gln Pro Gly Gly
1               5                   10                  15

Ser Leu Lys Leu Ser Cys Ala Ala Ser Gly Ile Asp Phe Ser Arg Tyr
            20                  25                  30

Trp Met Ser Trp Val Arg Arg Ala Pro Gly Lys Gly Leu Glu Trp Ile
        35                  40                  45

Gly Glu Ile Asn Pro Asp Arg Ser Thr Ile Asn Tyr Ala Pro Ser Leu
    50                  55                  60

Lys Asp Lys Phe Ile Ile Ser Arg Asp Asn Ala Lys Asn Thr Leu Tyr
65                  70                  75                  80

Leu Gln Met Ser Lys Val Arg Ser Glu Asp Thr Ala Leu Tyr Tyr Cys
                85                  90                  95

Ala Val Phe Tyr Tyr Asp Tyr Glu Gly Ala Met Asp Tyr Trp Gly Gln
            100                 105                 110

Gly Thr Ser Val Thr Val Ser Ser Ala Lys Thr Thr Ala Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210> 143
<211> 1350
<212> DNA
<213> Artificial Sequence

<220>
<221> source
<223> /note="Description of Artificial Sequence: Synthetic
      chimeric antibody sequence"

<400> 143
gaggtgaagc ttctccagtc tggaggtggc ctggtgcagc ctggaggatc cctgaaactc      60

tcctgtgcag cctcaggaat cgattttagt agatactgga tgagttgggt tcggcgggct     120

ccagggaaag gactagaatg gattggagaa attaatccag ataggagtac aatcaactat     180

gcaccatctc taaaggataa attcatcatc tccagagaca acgccaaaaa tacgctgtac     240

ctgcaaatga gcaaagtgag atctgaggac acagcccttt attactgtgc agttttctac     300

tatgattacg agggtgctat ggactactgg ggtcaaggaa cctcagtcac cgtctcctca     360

gccaaaacaa cagcccccag cgtgttcccc ctggccccca gcagcaagag caccagcggc     420

ggcacagccg ccctgggctg cctggtgaag gactacttcc ccgaaccggt gaccgtgtcc     480

tggaacagcg gagccctgac cagcggcgtg cacaccttcc ccgccgtgct gcagagcagc     540

ggcctgtaca gcctgagcag cgtggtgacc gtgcccagca gcagcctggg cacccagacc     600

tacatctgta acgtgaacca caagcccagc aacaccaagg tggacaagaa ggtggagccc     660

aagagctgtg acaagaccca cacctgcccc ccctgccctg cccccgagct gctgggaggc     720

cccagcgtgt tcctgttccc ccccaagcct aaggacaccc tgatgatcag cagaaccccc     780

gaggtgacct gtgtggtggt ggatgtgagc cacgaggacc ctgaggtgaa gttcaactgg     840

tacgtggacg gcgtggaggt gcacaatgcc aagaccaagc ccagggagga gcagtacaac     900

agcacctacc gggtggtgtc cgtgctgacc gtgctgcacc aggattggct gaacggcaag     960

gagtacaagt gtaaggtgtc caacaaggcc ctgcctgccc ctatcgagaa aaccatcagc    1020

aaggccaagg gccagcccag agagccccag gtgtacaccc tgccccctag cagagatgag    1080

ctgaccaaga accaggtgtc cctgacctgc ctggtgaagg gcttctaccc cagcgacatc    1140

gccgtggagt gggagagcaa cggccagccc gagaacaact acaagaccac cccccctgtg    1200

ctggacagcg atggcagctt cttcctgtac agcaagctga ccgtggacaa gagcagatgg 1260

cagcagggca acgtgttcag ctgctccgtg atgcacgagg ccctgcacaa tcactacacc 1320

cagaagagcc tgagcctgtc ccctggcaag 1350

<210> 144
<211> 108
<212> PRT
<213> Mus musculus

<400> 144
Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Asp Thr Asn
            20                  25                  30

Val Ala Trp Tyr Gln Gln Lys Pro Gly Gln Ser Pro Lys Ala Leu Ile
        35                  40                  45

Tyr Ser Ala Ser Tyr Arg Phe Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Glu Tyr Phe Cys Gln Gln Tyr Asn Ser Phe Pro Phe
                85                  90                  95

Thr Phe Gly Ser Gly Thr Lys Leu Glu Ile Lys Arg
            100                 105

<210> 145
<211> 324
<212> DNA
<213> Mus musculus

<400> 145
gacattgtga tgacccagtc tcaaaaattc atgtccacat cagtaggaga cagggtcagc 60

gtcacctgca aggccagtca gaatgtggat actaatgtag cctggtatca acaaaaacca 120

gggcaatctc ctaaagcact gatttactcg gcatcctacc ggttcagtgg agtccctgat 180

cgcttcacag gcagtggatc tgggacagat ttcactctca ccatcagcaa tgtgcagtct 240

gaagacttgg cagagtattt ctgtcagcaa tataacagct ttccattcac gttcggctcg 300

gggacaaagt tggaaataaa acgt 324

<210> 146
<211> 214

<212> PRT
<213> Artificial Sequence

<220>
<221> source
<223> /note="Description of Artificial Sequence: Synthetic
      chimeric antibody sequence"

<400> 146
Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Asp Thr Asn
            20                  25                  30

Val Ala Trp Tyr Gln Gln Lys Pro Gly Gln Ser Pro Lys Ala Leu Ile
        35                  40                  45

Tyr Ser Ala Ser Tyr Arg Phe Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Glu Tyr Phe Cys Gln Gln Tyr Asn Ser Phe Pro Phe
                85                  90                  95

Thr Phe Gly Ser Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 147
<211> 642
<212> DNA
<213> Artificial Sequence

<220>
<221> source
<223> /note="Description of Artificial Sequence: Synthetic
      chimeric antibody sequence"

<400> 147
gacattgtga tgacccagtc tcaaaaattc atgtccacat cagtaggaga cagggtcagc      60

gtcacctgca aggccagtca gaatgtggat actaatgtag cctggtatca acaaaaacca     120

gggcaatctc ctaaagcact gatttactcg gcatcctacc ggttcagtgg agtccctgat     180

cgcttcacag gcagtggatc tgggacagat ttcactctca ccatcagcaa tgtgcagtct     240

gaagacttgg cagagtattt ctgtcagcaa tataacagct ttccattcac gttcggctcg     300

gggacaaagt tggaaataaa acgtacggtg gccgccccca gcgtgttcat cttccccccc     360

agcgatgagc agctgaagag cggcaccgcc agcgtggtgt gtctgctgaa caacttctac     420

ccccgggagg ccaaggtgca gtggaaggtg gacaatgccc tgcagagcgg caacagccag     480

gagagcgtga ccgagcagga cagcaaggac tccacctaca gcctgagcag caccctgacc     540

ctgagcaagg ccgactacga gaagcacaag gtgtacgcct gtgaggtgac ccaccagggc     600

ctgtccagcc ccgtgaccaa gagcttcaac cggggcgagt gc                        642


<210> 148
<211> 118
<212> PRT
<213> Mus musculus

<400> 148
Pro Val Gln Leu Gln Gln Pro Gly Thr Glu Leu Val Arg Pro Gly Thr
1               5                   10                  15

Ser Val Lys Leu Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Val Ile Asp Pro Ser Asp Ser Tyr Thr Asn Tyr Asn Gln Lys Phe
    50                  55                  60

Lys Gly Lys Ala Thr Leu Thr Val Asp Thr Ser Ser Ser Thr Ala Tyr
65                  70                  75                  80

Met Gln Leu Ser Ser Leu Thr Ser Glu Asp Ser Ala Val Tyr Tyr Cys

85                  90                  95

Ala Arg Gln Val Phe Asp Tyr Pro Met Asp Tyr Trp Gly Gln Gly Thr
            100                 105                 110

Ser Val Thr Val Ser Ser
        115

<210> 149
<211> 354
<212> DNA
<213> Mus musculus

<400> 149
ccggtccaac tgcagcagcc tgggactgag ctggtgaggc ctgggacttc agtgaagttg      60

tcctgcaagg cttctggcta caccttcacc agctactgga tgcactgggt aaagcagagg     120

cctggacaag gccttgagtg gatcggagtg attgatcctt ctgatagtta tactaactac     180

aatcaaaagt tcaagggcaa ggccacattg actgtagaca catcctccag cacagcctac     240

atgcagctca gcagcctgac atctgaggac tctgcggtct attactgtgc aagacaggtg     300

tttgactatc ctatggacta ctggggtcaa ggaacctcag tcaccgtctc ctca           354

<210> 150
<211> 448
<212> PRT
<213> Artificial Sequence

<220>
<221> source
<223> /note="Description of Artificial Sequence: Synthetic
      chimeric antibody sequence"

<400> 150
Pro Val Gln Leu Gln Gln Pro Gly Thr Glu Leu Val Arg Pro Gly Thr
1               5                   10                  15

Ser Val Lys Leu Ser Cys Lys Ala Ser Gly Tyr Thr Phe Thr Ser His
            20                  25                  30

Trp Met His Trp Val Lys Gln Arg Pro Gly Gln Gly Leu Glu Trp Ile
        35                  40                  45

Gly Val Ile Asp Pro Ser Asp Ser Tyr Thr Asn Tyr Asn Gln Lys Phe
    50                  55                  60

Lys Gly Lys Ala Thr Leu Thr Val Asp Thr Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Gln Val Phe Asp Tyr Pro Met Asp Tyr Trp Gly Gln Gly Thr
        100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu

340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210> 151
<211> 1344
<212> DNA
<213> Artificial Sequence

<220>
<221> source
<223> /note="Description of Artificial Sequence: Synthetic
      chimeric antibody sequence"

<400> 151
ccggtccaac tgcagcagcc tgggactgag ctggtgaggc ctgggacttc agtgaagttg      60

tcctgcaagg cttctggcta caccttcacc agccactgga tgcactgggt aaagcagagg     120

cctggacaag gccttgagtg gatcggagtg attgatcctt ctgatagtta tactaactac     180

aatcaaaagt tcaagggcaa ggccacattg actgtagaca catcctccag cacagcctac     240

atgcagctca gcagcctgac atctgaggac tctgcggtct attactgtgc aagacaggtg     300

tttgactatc ctatggacta ctggggtcaa ggaacactag tgaccgtgtc cagcgccagc     360

accaagggcc ccagcgtgtt ccccctggcc cccagcagca agagcaccag cggcggcaca     420

gccgccctgg gctgcctggt gaaggactac ttccccgaac cggtgaccgt gtcctggaac     480

agcggagccc tgaccagcgg cgtgcacacc ttccccgccg tgctgcagag cagcggcctg     540

tacagcctga gcagcgtggt gaccgtgccc agcagcagcc tgggcaccca gacctacatc     600

tgtaacgtga accacaagcc cagcaacacc aaggtggaca agaaggtgga gcccaagagc     660

tgtgacaaga cccacacctg ccccccctgc cctgcccccg agctgctggg aggccccagc     720

gtgttcctgt tcccccccaa gcctaaggac accctgatga tcagcagaac ccccgaggtg     780

acctgtgtgg tggtggatgt gagccacgag gaccctgagg tgaagttcaa ctggtacgtg 840

gacggcgtgg aggtgcacaa tgccaagacc aagcccaggg aggagcagta caacagcacc 900

taccgggtgg tgtccgtgct gaccgtgctg caccaggatt ggctgaacgg caaggagtac 960

aagtgtaagg tgtccaacaa ggccctgcct gcccctatcg agaaaaccat cagcaaggcc 1020

aagggccagc ccagagagcc ccaggtgtac accctgcccc ctagcagaga tgagctgacc 1080

aagaaccagg tgtccctgac ctgcctggtg aagggcttct accccagcga catcgccgtg 1140

gagtgggaga gcaacggcca gcccgagaac aactacaaga ccaccccccc tgtgctggac 1200

agcgatggca gcttcttcct gtacagcaag ctgaccgtgg acaagagcag atggcagcag 1260

ggcaacgtgt tcagctgctc cgtgatgcac gaggccctgc acaatcacta cacccagaag 1320

agcctgagcc tgtcccctgg caag 1344

<210> 152
<211> 112
<212> PRT
<213> Mus musculus

<400> 152
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile His
            20                  25                  30

Gly Thr His Leu Met His Trp Tyr Gln Gln Lys Pro Gly Gln Pro Pro
        35                  40                  45

Lys Leu Leu Ile Tyr Ala Ala Ser Asn Leu Glu Ser Gly Val Pro Ala
    50                  55                  60

Arg Phe Ser Gly Ser Gly Ser Glu Thr Asp Phe Thr Leu Asn Ile His
65                  70                  75                  80

Pro Val Glu Glu Glu Asp Ala Ala Thr Tyr Phe Cys Gln Gln Ser Ile
                85                  90                  95

Glu Asp Pro Trp Thr Phe Gly Gly Gly Thr Lys Leu Glu Ile Lys Arg
            100                 105                 110

<210> 153
<211> 336
<212> DNA
<213> Mus musculus

<400> 153
gacattgtgc tgacccaatc tccagcttct ttggctgtgt ctctagggca gagggccacc 60

atctcctgca gagccagtga aagtgtcagt attcatggta ctcatttaat gcactggtac 120

caacagaaac caggacagcc acccaaactc ctcatctatg ctgcatccaa cctagaatct 180

ggagtccctg ccaggttcag tggcagtggg tctgagacag acttcaccct caacatccat 240

cctgtggagg aggaggatgc tgcaacctat ttctgtcagc aaagtattga ggatccgtgg 300

acgttcggtg gaggcaccaa gctggaaatc aaacgt 336

<210> 154
<211> 218
<212> PRT
<213> Artificial Sequence

<220>
<221> source
<223> /note="Description of Artificial Sequence: Synthetic
      chimeric antibody sequence"

<400> 154
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile His
            20                  25                  30

Gly Thr His Leu Met His Trp Tyr Gln Gln Lys Pro Gly Gln Pro Pro
        35                  40                  45

Lys Leu Leu Ile Tyr Ala Ala Ser Asn Leu Glu Ser Gly Val Pro Ala
    50                  55                  60

Arg Phe Ser Gly Ser Gly Ser Glu Thr Asp Phe Thr Leu Asn Ile His
65                  70                  75                  80

Pro Val Glu Glu Glu Asp Ala Ala Thr Tyr Phe Cys Gln Gln Ser Ile
                85                  90                  95

Glu Asp Pro Trp Thr Phe Gly Gly Gly Thr Lys Leu Glu Ile Asn Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210> 155
<211> 654
<212> DNA
<213> Artificial Sequence

<220>
<221> source
<223> /note="Description of Artificial Sequence: Synthetic
      chimeric antibody sequence"

<400> 155
gacattgtgc tgacccaatc tccagcttct ttggctgtgt ctctagggca gagggccacc      60

atctcctgca gagccagtga aagtgtcagt attcatggta ctcatttaat gcactggtac     120

caacagaaac caggacagcc acccaaactc ctcatctatg ctgcatccaa cctagaatct     180

ggagtccctg ccaggttcag tggcagtggg tctgagacag acttcaccct caacatccat     240

cctgtggagg aggaggatgc tgcaacctat ttctgtcagc aaagtattga ggatccgtgg     300

acgttcggtg gaggcaccaa gctggaaatc aatcgtacgg tggccgcccc cagcgtgttc     360

atcttccccc ccagcgatga gcagctgaag agcggcaccg ccagcgtggt gtgtctgctg     420

aacaacttct acccccggga ggccaaggtg cagtggaagg tggacaatgc cctgcagagc     480

ggcaacagcc aggagagcgt gaccgagcag gacagcaagg actccaccta cagcctgagc     540

agcaccctga ccctgagcaa ggccgactac gagaagcaca aggtgtacgc ctgtgaggtg     600

acccaccagg gcctgtccag ccccgtgacc aagagcttca accggggcga gtgc            654

<210> 156
<211> 118
<212> PRT
<213> Mus musculus

<400> 156
Gln Val Gln Leu Gln Gln Ser Gly Ala Glu Leu Val Arg Pro Gly Ala
1               5                   10                  15

Ser Val Thr Leu Ser Cys Lys Ala Ser Gly Tyr Thr Phe Thr Asp Tyr
            20                  25                  30

Glu Met His Trp Val Lys Gln Thr Pro Val His Gly Leu Glu Trp Ile
        35                  40                  45

Gly Ala Ile Asp Pro Glu Thr Gly Gly Thr Ala Tyr Asn Gln Lys Phe
    50                  55                  60

Lys Gly Lys Ala Ile Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Ser Ile Tyr Asp Tyr Tyr Phe Asp Tyr Trp Gly Gln Gly Thr
            100                 105                 110

Thr Leu Thr Val Ser Ser
        115

<210> 157
<211> 354
<212> DNA
<213> Mus musculus

<400> 157
caggttcaac tgcagcagtc tggggctgag ctggtgaggc ctggggcttc agtgacgctg      60

tcctgcaagg cttcgggcta cacatttact gactatgaaa tgcactgggt gaagcagaca     120

cctgtgcatg gcctggaatg gattggagct attgatcctg aaactggtgg tactgcctac     180

aatcagaagt tcaagggcaa ggccatactg actgcagaca aatcctccag cacagcctac     240

atggagctcc gcagcctgac atctgaggac tctgccgtct attactgtac aagatcgatt     300

tatgattact actttgacta ctggggccaa ggcaccactc tcacagtctc ctca           354

<210> 158
<211> 448
<212> PRT
<213> Artificial Sequence

<220>
<221> source
<223> /note="Description of Artificial Sequence: Synthetic
      chimeric antibody sequence"

<400> 158
Gln Val Gln Leu Gln Gln Ser Gly Ala Glu Leu Val Arg Pro Gly Ala
1               5                   10                  15

Ser Val Thr Leu Ser Cys Lys Ala Ser Gly Tyr Thr Phe Thr Asp Tyr
            20                  25                  30

Glu Met His Trp Val Lys Gln Thr Pro Val His Gly Leu Glu Trp Ile
        35                  40                  45

Gly Ala Ile Asp Pro Glu Thr Gly Gly Thr Ala Tyr Asn Gln Lys Phe
    50                  55                  60

Lys Gly Lys Ala Ile Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Ser Ile Tyr Asp Tyr Tyr Phe Asp Tyr Trp Gly Gln Gly Thr
            100                 105                 110

Thr Leu Thr Val Ser Ser Ala Lys Thr Thr Pro Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210> 159
<211> 1344
<212> DNA
<213> Artificial Sequence

<220>
<221> source
<223> /note="Description of Artificial Sequence: Synthetic
      chimeric antibody sequence"

<400> 159
caggttcaac tgcagcagtc tggggctgag ctggtgaggc ctggggcttc agtgacgctg      60

tcctgcaagg cttcgggcta cacatttact gactatgaaa tgcactgggt gaagcagaca     120

cctgtgcatg gcctggaatg gattggagct attgatcctg aaactggtgg tactgcctac     180

aatcagaagt tcaagggcaa ggccatactg actgcagaca aatcctccag cacagcctac     240

atggagctcc gcagcctgac atctgaggac tctgccgtct attactgtac aagatcgatt     300

tatgattact actttgacta ctggggccaa ggcaccactc tcacagtctc ctcagccaaa     360

acgacacccc ccagcgtgtt ccccctggcc cccagcagca agagcaccag cggcggcaca 420

gccgccctgg gctgcctggt gaaggactac ttccccgaac cggtgaccgt gtcctggaac 480

agcggagccc tgaccagcgg cgtgcacacc ttccccgccg tgctgcagag cagcggcctg 540

tacagcctga gcagcgtggt gaccgtgccc agcagcagcc tgggcaccca gacctacatc 600

tgtaacgtga accacaagcc cagcaacacc aaggtggaca agaaggtgga gcccaagagc 660

tgtgacaaga cccacacctg ccccccctgc cctgcccccg agctgctggg aggccccagc 720

gtgttcctgt tcccccccaa gcctaaggac accctgatga tcagcagaac ccccgaggtg 780

acctgtgtgg tggtggatgt gagccacgag gaccctgagg tgaagttcaa ctggtacgtg 840

gacggcgtgg aggtgcacaa tgccaagacc aagcccaggg aggagcagta caacagcacc 900

taccgggtgg tgtccgtgct gaccgtgctg caccaggatt ggctgaacgg caaggagtac 960

aagtgtaagg tgtccaacaa ggccctgcct gcccctatcg agaaaaccat cagcaaggcc 1020

aagggccagc ccagagagcc ccaggtgtac accctgcccc ctagcagaga tgagctgacc 1080

aagaaccagg tgtccctgac ctgcctggtg aagggcttct accccagcga catcgccgtg 1140

gagtgggaga gcaacggcca gcccgagaac aactacaaga ccaccccccc tgtgctggac 1200

agcgatggca gcttcttcct gtacagcaag ctgaccgtgg acaagagcag atggcagcag 1260

ggcaacgtgt tcagctgctc cgtgatgcac gaggccctgc acaatcacta cacccagaag 1320

agcctgagcc tgtcccctgg caag 1344

<210> 160
<211> 112
<212> PRT
<213> Mus musculus

<400> 160
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile His
            20                  25                  30

Gly Thr His Leu Met His Trp Tyr Gln Gln Lys Pro Gly Gln Pro Pro
        35                  40                  45

Lys Leu Leu Ile Tyr Ala Ala Ser Asn Leu Glu Ser Gly Val Pro Ala
    50                  55                  60

Arg Phe Ser Gly Gly Gly Ser Glu Thr Asp Phe Thr Leu Asn Ile His
65                  70                  75                  80

Pro Val Glu Glu Glu Asp Gly Ala Thr Tyr Phe Cys Gln Gln Ser Ile

85                  90                  95

Glu Tyr Pro Arg Thr Phe Gly Gly Gly Thr Lys Leu Glu Ile Asn Arg
            100                 105                 110

<210> 161
<211> 336
<212> DNA
<213> Mus musculus

<400> 161
gacattgtgc tgacccaatc tccagcttct ttggctgtgt ctctagggca gagggccacc      60

atctcctgca gagccagtga aagtgtcagt attcatggta ctcatttaat gcactggtac     120

caacagaaac caggacagcc acccaaactc ctcatctatg ctgcatccaa cctagaatct     180

ggagtccctg ccaggttcag tggcggtggg tctgagacag acttcaccct caacatccat     240

cctgtggagg aggaggatgg tgcaacctat ttctgtcagc aaagtattga gtatcctcgg     300

acgttcggtg gaggcaccaa gctggaaatc aatcgt                               336

<210> 162
<211> 218
<212> PRT
<213> Artificial Sequence

<220>
<221> source
<223> /note="Description of Artificial Sequence: Synthetic
      chimeric antibody sequence"

<400> 162
Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Ser Ile His
            20                  25                  30

Gly Thr His Leu Met His Trp Tyr Gln Gln Lys Pro Gly Gln Pro Pro
        35                  40                  45

Lys Leu Leu Ile Tyr Ala Ala Ser Asn Leu Glu Ser Gly Val Pro Ala
    50                  55                  60

Arg Phe Ser Gly Gly Gly Ser Glu Thr Asp Phe Thr Leu Asn Ile His
65                  70                  75                  80

Pro Val Glu Glu Glu Asp Gly Ala Thr Tyr Phe Cys Gln Gln Ser Ile
                85                  90                  95

Glu Tyr Pro Arg Thr Phe Gly Gly Gly Thr Lys Leu Glu Ile Asn Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210> 163
<211> 654
<212> DNA
<213> Artificial Sequence

<220>
<221> source
<223> /note="Description of Artificial Sequence: Synthetic
      chimeric antibody sequence"

<400> 163
gacattgtgc tgacccaatc tccagcttct ttggctgtgt ctctagggca gagggccacc      60

atctcctgca gagccagtga aagtgtcagt attcatggta ctcatttaat gcactggtac     120

caacagaaac caggacagcc acccaaactc ctcatctatg ctgcatccaa cctagaatct     180

ggagtccctg ccaggttcag tggcggtggg tctgagacag acttcaccct caacatccat     240

cctgtggagg aggaggatgg tgcaacctat ttctgtcagc aaagtattga gtatcctcgg     300

acgttcggtg gaggcaccaa gctggaaatc aatcgtacgg tggccgcccc cagcgtgttc     360

atcttccccc ccagcgatga gcagctgaag agcggcaccg ccagcgtggt gtgtctgctg     420

aacaacttct acccccggga ggccaaggtg cagtggaagg tggacaatgc cctgcagagc     480

ggcaacagcc aggagagcgt gaccgagcag gacagcaagg actccaccta cagcctgagc     540

agcaccctga ccctgagcaa ggccgactac gagaagcaca aggtgtacgc ctgtgaggtg     600

acccaccagg gcctgtccag ccccgtgacc aagagcttca accggggcga gtgc           654

<210> 164
<211> 5
<212> PRT
<213> Mus musculus

<400> 164
Asp Tyr Tyr Asn Met
1               5


<210> 165
<211> 16
<212> PRT
<213> Mus musculus

<400> 165
Val Ile Asn Pro Tyr Asn Gly Gly Thr Asp Tyr Asn Gln Lys Phe Gly
1               5                   10                  15


<210> 166
<211> 9
<212> PRT
<213> Mus musculus

<400> 166
Ser Val Tyr Asp Tyr Pro Phe Asp Tyr
1               5


<210> 167
<211> 15
<212> PRT
<213> Mus musculus

<400> 167
Arg Ala Ser Glu Ser Val Ser Ile His Gly Thr His Leu Met His
1               5                   10                  15


<210> 168
<211> 7
<212> PRT
<213> Mus musculus

<400> 168
Ala Ala Ser Asn Leu Glu Ser
1               5


<210> 169
<211> 9
<212> PRT
<213> Mus musculus

<400> 169
Gln Gln Ser Ile Glu Asp Pro Arg Thr
1               5


<210> 170
<211> 5

<212> PRT
<213> Mus musculus

<400> 170
Asp Tyr Ser Ile Asp
1               5


<210> 171
<211> 17
<212> PRT
<213> Mus musculus

<400> 171
Asp Ile Asp Pro Asn Tyr Gly Asp Pro Ile Tyr Asn His Lys Phe Lys
1               5                   10                  15


Gly




<210> 172
<211> 10
<212> PRT
<213> Mus musculus

<400> 172
Arg Ala Thr Gly Thr Asp Trp Phe Ala Phe
1               5                   10


<210> 173
<211> 11
<212> PRT
<213> Mus musculus

<400> 173
Arg Ala Ser Glu Asn Ile Tyr Asn Asn Leu Ala
1               5                   10


<210> 174
<211> 7
<212> PRT
<213> Mus musculus

<400> 174
Ala Ala Thr Ile Leu Ala Asp
1               5


<210> 175
<211> 9
<212> PRT
<213> Mus musculus

<400> 175
Gln His Phe Trp Gly Thr Pro Leu Thr
1               5


<210> 176

<211> 5
<212> PRT
<213> Mus musculus

<400> 176
Asn Tyr Trp Met His
1               5


<210> 177
<211> 17
<212> PRT
<213> Mus musculus

<400> 177
Ile Ile His Pro Asn Ser Gly Ser Thr Asn Tyr Asn Glu Lys Phe Lys
1               5                   10                  15


Ser


<210> 178
<211> 9
<212> PRT
<213> Mus musculus

<400> 178
Gly Ile Tyr Asp Tyr Pro Phe Ala Tyr
1               5


<210> 179
<211> 15
<212> PRT
<213> Mus musculus

<400> 179
Arg Ala Ser Glu Ser Val Ser Ile His Gly Thr His Leu Met His
1               5                   10                  15


<210> 180
<211> 7
<212> PRT
<213> Mus musculus

<400> 180
Ala Ala Ser Asn Leu Glu Ser
1               5


<210> 181
<211> 9
<212> PRT
<213> Mus musculus

<400> 181
Gln Gln Ser Ile Glu Asp Pro Tyr Thr
1               5

<210> 182
<211> 5
<212> PRT
<213> Mus musculus

<400> 182
Arg Tyr Trp Met Ser
1               5


<210> 183
<211> 17
<212> PRT
<213> Mus musculus

<400> 183
Glu Ile Asn Pro Asp Arg Ser Thr Ile Asn Tyr Ala Pro Ser Leu Lys
1               5                   10                  15


Asp


<210> 184
<211> 11
<212> PRT
<213> Mus musculus

<400> 184
Phe Tyr Tyr Asp Tyr Glu Gly Ala Met Asp Tyr
1               5                   10


<210> 185
<211> 11
<212> PRT
<213> Mus musculus

<400> 185
Lys Ala Ser Gln Asn Val Asp Thr Asn Val Ala
1               5                   10


<210> 186
<211> 7
<212> PRT
<213> Mus musculus

<400> 186
Ser Ala Ser Tyr Arg Phe Ser
1               5


<210> 187
<211> 9
<212> PRT
<213> Mus musculus

<400> 187
Gln Gln Tyr Asn Ser Phe Pro Phe Thr
1               5

<210> 188
<211> 5
<212> PRT
<213> Mus musculus

<400> 188
Ser Tyr Trp Met His
1               5


<210> 189
<211> 17
<212> PRT
<213> Mus musculus

<400> 189
Val Ile Asp Pro Ser Asp Ser Tyr Thr Asn Tyr Asn Gln Lys Phe Lys
1               5                   10                  15


Gly


<210> 190
<211> 9
<212> PRT
<213> Mus musculus

<400> 190
Gln Val Phe Asp Tyr Pro Met Asp Tyr
1               5


<210> 191
<211> 15
<212> PRT
<213> Mus musculus

<400> 191
Arg Ala Ser Glu Ser Val Ser Ile His Gly Thr His Leu Met His
1               5                   10                  15


<210> 192
<211> 7
<212> PRT
<213> Mus musculus

<400> 192
Ala Ala Ser Asn Leu Glu Ser
1               5


<210> 193
<211> 9
<212> PRT
<213> Mus musculus

<400> 193
Gln Gln Ser Ile Glu Asp Pro Trp Thr
1               5

<210> 194
<211> 5
<212> PRT
<213> Mus musculus

<400> 194
Asp Tyr Glu Met His
1               5


<210> 195
<211> 17
<212> PRT
<213> Mus musculus

<400> 195
Ala Ile Asp Pro Glu Thr Gly Gly Thr Ala Tyr Asn Gln Lys Phe Lys
1               5                   10                  15


Gly


<210> 196
<211> 9
<212> PRT
<213> Mus musculus

<400> 196
Ser Ile Tyr Asp Tyr Tyr Phe Asp Tyr
1               5


<210> 197
<211> 15
<212> PRT
<213> Mus musculus

<400> 197
Arg Ala Ser Glu Ser Val Ser Ile His Gly Thr His Leu Met His
1               5                   10                  15


<210> 198
<211> 7
<212> PRT
<213> Mus musculus

<400> 198
Ala Ala Ser Asn Leu Glu Ser
1               5


<210> 199
<211> 9
<212> PRT
<213> Mus musculus

<400> 199
Gln Gln Ser Ile Glu Tyr Pro Arg Thr

1                   5


<210> 200
<211> 12
<212> PRT
<213> Artificial Sequence

<220>
<221> source
<223> /note="Description of Artificial Sequence: Synthetic
      humanized antibody sequence"

<400> 200
Gly Ala Ile Tyr Asp Gly Tyr Asp Val Leu Asp Asn
1               5                   10


<210> 201
<211> 5
<212> PRT
<213> Artificial Sequence

<220>
<221> source
<223> /note="Description of Artificial Sequence: Synthetic
      peptide"

<400> 201
Asn Tyr Tyr Met Tyr
1               5


<210> 202
<211> 17
<212> PRT
<213> Artificial Sequence

<220>
<221> source
<223> /note="Description of Artificial Sequence: Synthetic
      peptide"

<400> 202
Gly Ile Asn Pro Ser Asn Gly Gly Thr Asn Phe Asn Glu Lys Phe Lys
1               5                   10                  15


Asn




<210> 203
<211> 11
<212> PRT
<213> Artificial Sequence

<220>
<221> source
<223> /note="Description of Artificial Sequence: Synthetic
      peptide"

<400> 203

Arg Asp Tyr Arg Phe Asp Met Gly Phe Asp Tyr
1               5                   10


<210> 204
<211> 15
<212> PRT
<213> Artificial Sequence

<220>
<221> source
<223> /note="Description of Artificial Sequence: Synthetic
      peptide"

<400> 204
Arg Ala Ser Lys Gly Val Ser Thr Ser Gly Tyr Ser Tyr Leu His
1               5                   10                  15


<210> 205
<211> 7
<212> PRT
<213> Artificial Sequence

<220>
<221> source
<223> /note="Description of Artificial Sequence: Synthetic
      peptide"

<400> 205
Leu Ala Ser Tyr Leu Glu Ser
1               5


<210> 206
<211> 9
<212> PRT
<213> Artificial Sequence

<220>
<221> source
<223> /note="Description of Artificial Sequence: Synthetic
      peptide"

<400> 206
Gln His Ser Arg Asp Leu Pro Leu Thr
1               5


<210> 207
<211> 120
<212> PRT
<213> Artificial Sequence

<220>
<221> source
<223> /note="Description of Artificial Sequence: Synthetic
      polypeptide"

<400> 207

<210> 208
<211> 111
<212> PRT
<213> Artificial Sequence

<220>
<221> source
<223> /note="Description of Artificial Sequence: Synthetic
polypeptide"

<400> 208

<210> 209
<211> 446
<212> PRT
<213> Artificial Sequence

<220>
<221> source
<223> /note="Description of Artificial Sequence: Synthetic
      polypeptide"

<400> 209
Gln Val Gln Leu Val Gln Ser Gly Val Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Met Tyr Trp Val Arg Gln Ala Pro Gly Gln Gly Leu Glu Trp Met
        35                  40                  45

Gly Gly Ile Asn Pro Ser Asn Gly Gly Thr Asn Phe Asn Glu Lys Phe
    50                  55                  60

Lys Asn Arg Val Thr Leu Thr Thr Asp Ser Ser Thr Thr Thr Ala Tyr
65                  70                  75                  80

Met Glu Leu Lys Ser Leu Gln Phe Asp Asp Thr Ala Val Tyr Tyr Cys
                85                  90                  95

Ala Arg Arg Asp Tyr Arg Phe Asp Met Gly Phe Asp Tyr Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Lys Ser Leu Ser Leu Ser Leu Gly Lys
        435                 440                 445

<210> 210
<211> 218
<212> PRT
<213> Artificial Sequence

<220>
<221> source
<223> /note="Description of Artificial Sequence: Synthetic
      polypeptide"

<400> 210
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Gly Val Ser Thr Ser
            20                  25                  30

Gly Tyr Ser Tyr Leu His Trp Tyr Gln Gln Lys Pro Gly Gln Ala Pro
        35                  40                  45

Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln His Ser Arg
                85                  90                  95

Asp Leu Pro Leu Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210> 211
<211> 5
<212> PRT
<213> Artificial Sequence

<220>
<221> source
<223> /note="Description of Artificial Sequence: Synthetic
      peptide"

<400> 211
Asn Ser Gly Met His
1               5

<210> 212
<211> 17
<212> PRT
<213> Artificial Sequence

<220>
<221> source
<223> /note="Description of Artificial Sequence: Synthetic
      peptide"

<400> 212
Val Ile Trp Tyr Asp Gly Ser Lys Arg Tyr Tyr Ala Asp Ser Val Lys
1               5                   10                  15

Gly

<210> 213
<211> 4
<212> PRT
<213> Artificial Sequence

<220>
<221> source
<223> /note="Description of Artificial Sequence: Synthetic
      peptide"

<400> 213
Asn Asp Asp Tyr
1

<210> 214
<211> 11
<212> PRT
<213> Artificial Sequence

<220>
<221> source
<223> /note="Description of Artificial Sequence: Synthetic

peptide"

<400> 214
Arg Ala Ser Gln Ser Val Ser Ser Tyr Leu Ala
1               5                   10

<210> 215
<211> 7
<212> PRT
<213> Artificial Sequence

<220>
<221> source
<223> /note="Description of Artificial Sequence: Synthetic
      peptide"

<400> 215
Asp Ala Ser Asn Arg Ala Thr
1               5

<210> 216
<211> 9
<212> PRT
<213> Artificial Sequence

<220>
<221> source
<223> /note="Description of Artificial Sequence: Synthetic
      peptide"

<400> 216
Gln Gln Ser Ser Asn Trp Pro Arg Thr
1               5

<210> 217
<211> 113
<212> PRT
<213> Artificial Sequence

<220>
<221> source
<223> /note="Description of Artificial Sequence: Synthetic
      polypeptide"

<400> 217

<210> 218
<211> 107
<212> PRT
<213> Artificial Sequence

<220>
<221> source
<223> /note="Description of Artificial Sequence: Synthetic
      polypeptide"

<400> 218

<210> 219
<211> 5
<212> PRT
<213> Mus musculus

<400> 219
Asp Tyr Ser Met His

1               5


<210> 220
<211> 17
<212> PRT
<213> Mus musculus

<400> 220
Trp Ile Asn Thr Glu Thr Gly Glu Pro Thr Tyr Ala Asp Asp Phe Lys
1               5                   10                  15


Gly




<210> 221
<211> 13
<212> PRT
<213> Mus musculus

<400> 221
Pro Tyr Tyr Asp Tyr Val Ser Tyr Tyr Ala Met Asp Tyr
1               5                   10


<210> 222
<211> 11
<212> PRT
<213> Mus musculus

<400> 222
Lys Ala Ser Gln Asp Val Ser Thr Ala Val Ala
1               5                   10


<210> 223
<211> 7
<212> PRT
<213> Mus musculus

<400> 223
Ser Ala Ser Tyr Leu Tyr Thr
1               5


<210> 224
<211> 9
<212> PRT
<213> Mus musculus

<400> 224
Gln Gln His Tyr Ser Thr Pro Arg Thr
1               5


<210> 225
<211> 122
<212> PRT
<213> Artificial Sequence

<220>

<221> source
<223> /note="Description of Artificial Sequence: Synthetic
      polypeptide"

<400> 225
Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asp Tyr
            20                  25                  30

Ser Met His Trp Val Lys Gln Ala Pro Gly Lys Gly Leu Lys Trp Met
        35                  40                  45

Gly Trp Ile Asn Thr Glu Thr Gly Glu Pro Thr Tyr Ala Asp Asp Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Asn Pro Tyr Tyr Asp Tyr Val Ser Tyr Tyr Ala Met Asp Tyr Trp
            100                 105                 110

Gly His Gly Thr Ser Val Thr Val Ser Ser
        115                 120


<210> 226
<211> 455
<212> DNA
<213> Artificial Sequence

<220>
<221> source
<223> /note="Description of Artificial Sequence: Synthetic
      polynucleotide"

<400> 226
actagtacca ccatggcttg ggtgtggacc ttgctattcc tgatggcagc tgcccaaagt      60

atccaagcac aggttcagtt ggtgcagtct ggatctgagc tgaagaagcc tggagcctca     120

gtcaaggttt ctgcaaggct tctggttata ccttcacaga ctattcaatg cactgggtgc     180

gacaggctcc aggacaaggt ttaaagtgga tgggctggat aaacactgag actggtgagc     240

caacatatgc agatgactta gggacggttt gtcttctctt tggacacctc tgtcagcact     300

gcctatttgc agatcagcag cctcaaagct gaggacacgg ctgtgtatta ctgtgctaat     360

ccctactatg attacgtctc ttactatgct atggactact ggggtcaggg aaccacggtc     420

accgtctcct caggtaagaa tggcctctca agctt                                455

<210> 227
<211> 107
<212> PRT
<213> Artificial Sequence

<220>
<221> source
<223> /note="Description of Artificial Sequence: Synthetic
      polypeptide"

<400> 227
Asp Ile Val Met Thr Gln Ser His Lys Phe Met Ser Thr Ser Val Arg
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Ser Ala Ser Tyr Leu Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Thr Pro Arg
                85                  90                  95

Thr Phe Gly Gly Gly Thr Lys Leu Glu Ile Lys
            100                 105


<210> 228
<211> 415
<212> DNA
<213> Artificial Sequence

<220>
<221> source
<223> /note="Description of Artificial Sequence: Synthetic
      polynucleotide"

<400> 228
gctagcacca ccatggagtc acagattcag gtctttgtat tcgtgtttct ctggttgtct      60

ggtgttgacg gagacattca gatgacccag tctccatcct ccctgtccgc atcagtggga     120

gacagggtca ccatcacctg caaggccagt caggatgtga gtactgctgt agcctggtat     180

caacagaaac caggaaaagc cctaaactac tgatttactc ggcatcctac ctctacactg     240

gagtcccttc acgcttcagt ggcagtggat ctgggacgga tttcactttc accatcagca     300

gtctgcagcc tgaagacatt gcaacatatt actgtcagca acattatagt actcctcgga     360

cgttcggtca gggcaccaag ctggaaatca aacgtaagta gaatccaaag aattc 415

<210> 229
<211> 122
<212> PRT
<213> Artificial Sequence

<220>
<221> source
<223> /note="Description of Artificial Sequence: Synthetic
      polypeptide"

<400> 229
Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asp Tyr
            20                  25                  30

Ser Met His Trp Val Arg Gln Ala Pro Gly Gln Gly Leu Lys Trp Met
        35                  40                  45

Gly Trp Ile Asn Thr Glu Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Asn Pro Tyr Tyr Asp Tyr Val Ser Tyr Tyr Ala Met Asp Tyr Trp
            100                 105                 110

Gly Gln Gly Thr Thr Val Thr Val Ser Ser
        115                 120

<210> 230
<211> 107
<212> PRT
<213> Artificial Sequence

<220>
<221> source
<223> /note="Description of Artificial Sequence: Synthetic
      polypeptide"

<400> 230
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Thr Ala

20                  25                  30

Val Ala Trp Tyr Gln Gln Lys Pro Gly Lys Ala Pro Lys Leu Leu Ile
        35                  40                  45

Tyr Ser Ala Ser Tyr Leu Tyr Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln His Tyr Ser Thr Pro Arg
                85                  90                  95

Thr Phe Gly Gln Gly Thr Lys Leu Glu Ile Lys
            100                 105

<210> 231
<211> 5
<212> PRT
<213> Mus musculus

<400> 231
Ser His Asp Met Ser
1               5

<210> 232
<211> 16
<212> PRT
<213> Mus musculus

<400> 232
Ala Ile Asn Ser Asp Gly Gly Ser Thr Tyr Tyr Pro Asp Thr Met Glu
1               5                   10                  15

<210> 233
<211> 11
<212> PRT
<213> Mus musculus

<400> 233
His Tyr Asp Asp Tyr Tyr Ala Trp Phe Ala Tyr
1               5                   10

<210> 234
<211> 15
<212> PRT
<213> Mus musculus

<400> 234
Arg Ala Ser Lys Ser Val Ser Thr Ser Gly Tyr Ser Tyr Met His
1               5                   10                  15

<210> 235
<211> 7
<212> PRT
<213> Mus musculus

<400> 235
Leu Ala Ser Asn Leu Glu Ser
1               5


<210> 236
<211> 9
<212> PRT
<213> Mus musculus

<400> 236
Gln His Ser Arg Glu Leu Pro Leu Thr
1               5


<210> 237
<211> 120
<212> PRT
<213> Artificial Sequence

<220>
<221> source
<223> /note="Description of Artificial Sequence: Synthetic
      polypeptide"

<400> 237
Glu Val Gln Leu Val Glu Ser Gly Gly Gly Leu Val Gln Pro Gly Glu
1               5                   10                  15


Ser Leu Lys Leu Ser Cys Glu Ser Asn Glu Tyr Glu Phe Pro Ser His
            20                  25                  30


Asp Met Ser Trp Val Arg Lys Thr Pro Glu Lys Arg Leu Glu Leu Val
        35                  40                  45


Ala Ala Ile Asn Ser Asp Gly Gly Ser Thr Tyr Tyr Pro Asp Thr Met
    50                  55                  60


Glu Arg Arg Phe Ile Ile Ser Arg Asp Asn Thr Lys Lys Thr Leu Tyr
65                  70                  75                  80


Leu Gln Met Ser Ser Leu Arg Ser Glu Asp Thr Ala Leu Tyr Tyr Cys
                85                  90                  95


Ala Arg His Tyr Asp Asp Tyr Tyr Ala Trp Phe Ala Tyr Trp Gly Gln
            100                 105                 110


Gly Thr Leu Val Thr Val Ser Ala
        115                 120

<210> 238
<211> 450
<212> DNA
<213> Artificial Sequence

<220>
<221> source
<223> /note="Description of Artificial Sequence: Synthetic
      polynucleotide"

<400> 238
actagtacca ccatggactt cgggctcagc ttggttttcc ttgtccttat tttaaaaagt      60

gtacagtgtg aggtgcagct ggtggagtct gggggaggct tagtgcagcc tggagggtcc     120

ctgagactct ctgtgcagcc tctgaatacg agttcccttc ccatgacatg tcttgggtcc     180

gccaggctcc ggggaagggg ctggagttgg tcgcagccat taatagtgat ggtggtagca     240

cctactatcc agacaccatg gagagacgat tcaccatctc cagagacaat gccaagaact     300

cactgtacct gcaaatgaac agtctgaggg ccgaggacac agccgtgtat tactgtgcaa     360

gacactatga tgattactac gcctggtttg cttactgggg ccaagggact atggtcactg     420

tctcttcagg tgagtcctaa cttcaagctt                                      450


<210> 239
<211> 111
<212> PRT
<213> Artificial Sequence

<220>
<221> source
<223> /note="Description of Artificial Sequence: Synthetic
      polypeptide"

<400> 239
Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Ser Val Ser Thr Ser
            20                  25                  30

Gly Tyr Ser Tyr Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln His Ser Arg
                85                  90                  95

Glu Leu Pro Leu Thr Phe Gly Ala Gly Thr Lys Leu Glu Leu Lys
            100                 105                 110

<210> 240
<211> 428
<212> DNA
<213> Artificial Sequence

<220>
<221> source
<223> /note="Description of Artificial Sequence: Synthetic
      polynucleotide"

<400> 240
gctagcacca ccatggagac agacacactc ctgttatggg tactgctgct ctgggttcca      60

ggttccactg gtgaaattgt gctgacacag tctcctgcta ccttatcttt gtctccaggg     120

gaaagggcca ccctctcatg cagggccagc aaaagtgtca gtacatctgg ctatagttat     180

atgcactggt accaacagaa accaggacag gctcccagac tcctcatcta tcttgcatcc     240

aacctagaat ctggggtccc tgccaggttc agtggcagtg ggtctgggac agacttcacc     300

ctcaccatca gcagcctaga gcctgaggat tttgcagttt attactgtca gcacagtagg     360

gagcttccgc tcacgttcgg cggagggacc aaggtcgaga tcaaacgtaa gtacactttt     420

ctgaattc                                                              428

<210> 241
<211> 120
<212> PRT
<213> Artificial Sequence

<220>
<221> source
<223> /note="Description of Artificial Sequence: Synthetic
      polypeptide"

<400> 241
Glu Val Gln Leu Val Glu Ser Gly Gly Gly Leu Val Gln Pro Gly Gly
1               5                   10                  15

Ser Leu Arg Leu Ser Cys Ala Ala Ser Glu Tyr Glu Phe Pro Ser His
            20                  25                  30

Asp Met Ser Trp Val Arg Gln Ala Pro Gly Lys Gly Leu Glu Leu Val
        35                  40                  45

Ala Ala Ile Asn Ser Asp Gly Gly Ser Thr Tyr Tyr Pro Asp Thr Met
    50                  55                  60

Glu Arg Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg His Tyr Asp Asp Tyr Tyr Ala Trp Phe Ala Tyr Trp Gly Gln
            100                 105                 110

Gly Thr Met Val Thr Val Ser Ser
        115                 120

<210> 242
<211> 111
<212> PRT
<213> Artificial Sequence

<220>
<221> source
<223> /note="Description of Artificial Sequence: Synthetic
      polypeptide"

<400> 242
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Ser Val Ser Thr Ser
            20                  25                  30

Gly Tyr Ser Tyr Met His Trp Tyr Gln Gln Lys Pro Gly Gln Ala Pro
        35                  40                  45

Arg Leu Leu Ile Tyr Leu Ala Ser Asn Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln His Ser Arg
                85                  90                  95

Glu Leu Pro Leu Thr Phe Gly Gly Gly Thr Lys Val Glu Ile Lys
            100                 105                 110

<210> 243
<211> 452
<212> PRT
<213> Artificial Sequence

<220>
<221> source
<223> /note="Description of Artificial Sequence: Synthetic
      polypeptide"

<400> 243
Gln Val Gln Leu Val Gln Ser Gly Ser Glu Leu Lys Lys Pro Gly Ala

1               5                   10                  15

Ser Val Lys Val Ser Cys Lys Ala Ser Gly Tyr Thr Phe Thr Asp Tyr
            20                  25                  30

Ser Met His Trp Val Arg Gln Ala Pro Gly Gln Gly Leu Lys Trp Met
        35                  40                  45

Gly Trp Ile Asn Thr Glu Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Asn Pro Tyr Tyr Asp Tyr Val Ser Tyr Tyr Ala Met Asp Tyr Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210> 244
<211> 214
<212> PRT
<213> Artificial Sequence

<220>
<221> source
<223> /note="Description of Artificial Sequence: Synthetic
polypeptide"

<400> 244

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Tyr Leu Tyr Thr Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln His Tyr Ser Thr Pro Arg
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

## Description

### CROSS-REFERENCE TO RELATED APPLICATIONS

This application claims priority to U.S. Application Serial No. 62/261,481, filed on December 1, 2015, the disclosure of which is herein incorporated by reference.

### SEQUENCE LISTING

The instant application contains a Sequence Listing which has been submitted electronically in ASCII format and is hereby incorporated by reference in its entirety. Said ASCII copy, created on November 30, 2016, is named PB65987WO_SL.txt and is 293,882bytes in size.

### FIELD OF THE INVENTION

The present invention relates to combinations comprising anti-BCMA antigen binding proteins, including monoclonal antibodies to human BCMA in combination with immunomodulatory agents such as anti-PD-1 antigen binding agents and/or anti-OX40 antigen binding proteins. Further the invention relates to the use of the combinations in treating cancer in a mammal such as a human.

### BACKGROUND OF THE INVENTION

Effective treatment of hyperproliferative disorders including cancer is a continuing goal in the oncology field. Generally, cancer results from the deregulation of the normal processes that control cell division, differentiation and apoptotic cell death and is characterized by the proliferation of malignant cells which have the potential for unlimited growth, local expansion and systemic metastasis. Deregulation of normal processes include abnormalities in signal transduction pathways and response to factors which differ from those found in normal cells.

Immunotherapies are one approach to treat hyperproliferative disorders. A major hurdle that scientists and clinicians have encountered in the development of various types of cancer immunotherapies has been to break tolerance to self antigen (cancer) in order to mount a robust anti-tumor response leading to tumor regression. Unlike traditional development of small and large molecule agents that target the tumor, cancer immunotherapies target cells of the immune system that have the potential to generate a memory pool of effector cells to induce more durable effects and minimize recurrences.

BCMA (CD269 or TNFRSF17) is a member of the TNF receptor superfamily. It is a non-glycosylated integral membrane receptor for the ligands BAFF and APRIL. BCMA's ligands can also bind additional receptors: TACI (Transmembrane Activator and Calcium modulator and cyclophilin ligand Interactor), which binds APRIL and BAFF; as well as BAFF-R (BAFF Receptor or BR3), which shows restricted but high affinity for BAFF. Together, these receptors and their corresponding ligands regulate different embodiments of humoral immunity, B-cell development and homeostasis.

BCMA's expression is typically restricted to the B-cell lineage and is reported to increase in terminal B-cell differentiation. BCMA is expressed by human plasma blasts, plasma cells from tonsils, spleen and bone marrow, but also by tonsillar memory B cells and by germinal centre B cells, which have a TACI-BAFFR low phenotype (Darce et al, 2007). BCMA is virtually absent on naïve and memory B-cells (Novak et al., 2004a and b). The BCMA antigen is expressed on the cell surface so is accessible to the antibody, but is also expressed in the golgi. As suggested by its expression profile, BCMA signalling, typically linked with B-cell survival and proliferation, is important in the late stages of B-cell differentiation, as well as the survival of long lived bone marrow plasma cells (O'Connor et al., 2004) and plasmablasts (Avery et al., 2003). Furthermore, as BCMA binds APRIL with high affinity, the BCMA-APRIL signalling axis is suggested to predominate at the later stages of B-cell differentiation, perhaps being the most physiologically relevant interaction.

Antigen binding proteins and antibodies that bind BCMA and modulate signalling are known in the art and are disclosed as immunotherapy, for example for cancer.

Binding of the PD-1 ligands, PD-L1 and PD-L2, to the PD-1 receptor found on T cells, inhibits T cell proliferation and cytokine production. Upregulation of PD-1 ligands occurs in some tumors and signaling through this pathway can contribute to inhibition of active T-cell immune surveillance of tumors. Antigen binding proteins and antibodies that bind to the PD-1 receptor and block its interaction with PD-L1 and PD-L2 may release PD-1 pathway-mediated inhibition of the immune response, including the anti-tumor immune response.

Enhancing anti-tumor T cell function and inducing T cell proliferation is a powerful and new approach for cancer treatment. Three immune-oncology antibodies (e.g., immuno-modulators) are presently marketed. Anti-CTLA-4 (YERVOY^{®}/ipilimumab) is thought to augment immune responses at the point of T cell priming and anti-PD-1 antibodies (OPDIVO^{®}/nivolumab and KEYTRUDA^{®}/pembrolizumab) are thought to act in the local tumor microenvironment, by relieving an inhibitory checkpoint in tumor specific T cells that have already been primed and activated.

OX40 (e.g. human OX40 (hOX40) or hOX40R) is a tumor necrosis factor receptor family member that is expressed, among other cells, on activated CD4 and CD8 T cells. One of its functions is in the differentiation and long-term survival of these cells. The ligand for OX40 (OX40L) is expressed by activated antigen-presenting cells.

Though there have been many recent advances in the treatment of cancer, there remains a need for more effective and/or enhanced treatment of an individual suffering the effects of cancer. The combinations and methods herein that relate to combining therapeutic approaches for enhancing anti-tumor immunity address this need.

### SUMMARY OF THE INVENTION

The present invention provides combinations comprising a therapeutically effective amount of an antigen binding protein that binds BCMA and a therapeutically effective amount of an antigen binding protein that binds an immunomodulatory target. Examples of immunomodulatory targets include PD-1 and OX40.

In another embodiment, the antigen binding protein that binds BCMA is conjugated to a cytotoxic agent as an immunoconjugate (e.g. an antibody-drug conjugate (ADC)). The cytotoxic agent may include MMAE or MMAF and the cytotoxic agent may be conjugated to the antigen binding protein that binds BCMA via a linker such as citruline-valine or maleimidocaproyl.

In one embodiment, the antigen binding protein that binds BCMA is an antagonist. In another embodiment, the antigen binding protein that binds BCMA is an IgG1 monoclonal antibody.

In one embodiment, the antigen binding protein that binds BCMA is an antibody that comprises CDRH3 of SEQ. ID. NO: 3, CDRH3 variant N99D of SEQ. ID. NO: 200, or variants thereof. In another embodiment, the antigen binding protein that binds BCMA is an antibody that comprises CDR H1 of SEQ. ID. NO: 1, CDRH2 of SEQ. ID. NO: 2, CDRH3 of SEQ. ID. NO: 3 or CDRH3 variant N99D of SEQ. ID. NO: 200, CDRL1 of SEQ. ID. NO: 4, CDRL2 of SEQ. ID. NO: 5, CDRL3 of SEQ. ID. NO: 6 and variants thereof. In yet another embodiment, the antigen binding protein that binds BCMA is an antibody that comprises a heavy chain variable region of SEQ ID NO: 23 and a light chain variable region of SEQ. ID. NO: 31.

The present invention provides combinations comprising a therapeutically effective amount of an antigen binding protein that binds BCMA and a therapeutically effective amount of an antigen binding protein that binds PD-1.

In one embodiment, the antigen binding protein that binds PD-1 is an antagonist. In another embodiment, the antigen binding protein that binds PD-1 is an IgG4 monoclonal antibody.

In one embodiment, the antigen binding protein that binds PD-1 comprises CDR H1 of SEQ. ID. NO: 201, CDRH2 of SEQ. ID. NO: 202, CDRH3 of SEQ. ID. NO: 203, CDRL1 of SEQ. ID. NO: 204, CDRL2 of SEQ. ID. NO: 205, CDRL3 of SEQ. ID. NO: 206, and variants thereof. In yet another embodiment, the antigen binding protein that binds PD-1 comprises a heavy chain variable region of SEQ. ID. NO: 207 and light chain variable region of SEQ. ID. NO: 208.

In yet another embodiment, the antigen binding protein that binds PD-1 is pembrolizumab, nivolumab, or an antibody comprising 90%, 91%, 92%, 93%, 94%, 95%, 96%, 97%, 98%, 99% or 100% sequence homology to either pembrolizumab or nivolumab.

The present invention provides combinations comprising a therapeutically effective amount of an antigen binding protein that binds BCMA and a therapeutically effective amount of an antigen binding protein that binds OX40.

In one embodiment, the antigen binding protein that binds OX40 is an agonist. In another embodiment, the antigen binding protein that binds OX40 is an IgG1 monoclonal antibody.

In one embodiment, the antigen binding protein that binds OX40 comprises CDR H1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, and variants thereof. In yet another embodiment, the antigen binding protein that binds OX40 comprises a heavy chain variable region of SEQ. ID. NO: 229 and a light chain variable region of SEQ. ID. NO: 230.

Also provided are pharmaceutical compositions comprising the combinations of the present invention.

Also provided are methods of treating cancer in a mammal (such as a human) in need thereof comprising administering a therapeutically effective amount of a combination comprising a therapeutically effective amount of an antigen binding protein that binds BCMA and a therapeutically effective amount of at least one antigen binding protein that binds an immunomodulatory target. In one embodiment, the immunomodulatory target is PD1 or OX40. In one embodiment, the cancer is multiple myeloma (MM) or non-Hodgkin's lymphoma B-cell leukemia (NHL). In one embodiment, the antigen binding protein that binds BCMA and the antigen binding that binds PD-1 or OX40 are administered at the same time or sequentially. Methods provide for systemic (e.g. intravenous) or intratumoral administration of the combinations.

Use of the combinations described herein in the treatment of cancer is provided herein.

Use of the combinations described herein in the manufacture of a medicament for the treatment of cancer is contemplated.

Suitably, kits are provided comprising the pharmaceutical compositions of the invention together with one or more pharmaceutically acceptable carriers.

### BRIEF DESCRIPTION OF THE DRAWINGS

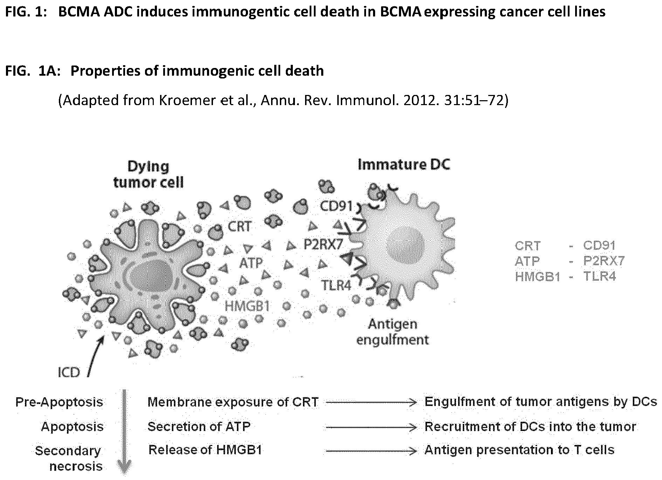

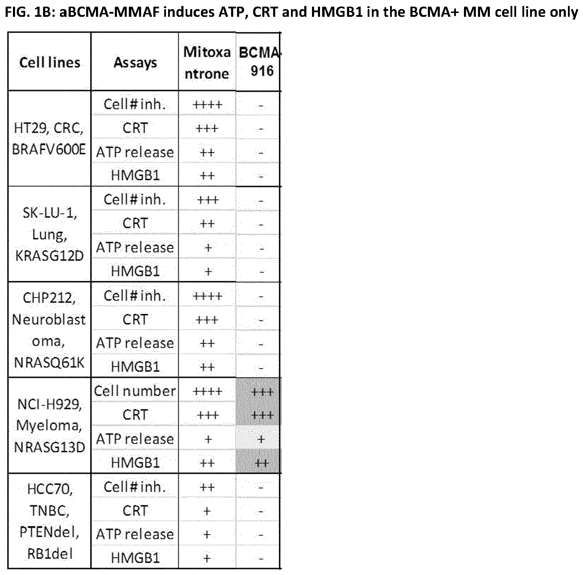

**FIG. 1****. BCMA ADC induces immunogentic cell death in BCMA expressing cancer cell lines**
**FIG. 1A****) Schematic to show Immunogenic cell death (ICD) in BCMA expressing cancer cell lines.** ICD is a special type of apoptosis often associated with the cellular release of ATP and HMGB1, and exposure of CRT at the cellular membrane. ICD induces an immune response through engagement of the antigen presentation process by dendritic cells (DCs).
**FIG. 1B****) aBCMA-MMAF induces ATP, CRT and HMGB1 in the BCMA+ MM cell line**
   Tested cell lines were treated with Anti-BCMA-MMAF antibody drug conjugate or Mitoxantrone for 48 hours and then assessed for: 1) loss in cell numbers by automated flow cytometry, 2) calreticulin (CRT) exposure was marked with a polyclonal anti-CRT antibody and evaluated by flow cytometry, 3) HMGB1 content in the cellular supernatant was evaluated by ELISA and subsequent absorbance assessment, and 4) ATP in the cellular supernatant was evaluated by subsequent luminescence assessment. Anti-BCMA-MMAF induced ATP release, HMGB1 release and CRT exposure only in NCI-H929, a BCMA positive MM cell line.
**FIG. 2****: Induction of dendritic cell maturation activation via induction of immunogenetic cell death**
**FIG. 2A****)** CD83 cell surface expression increased on HLA-DR+ Dendritic cells from three healthy donors following 24 hour co-culture with BCMA ADC treated NCI-H929 Multiple Myeloma cells.

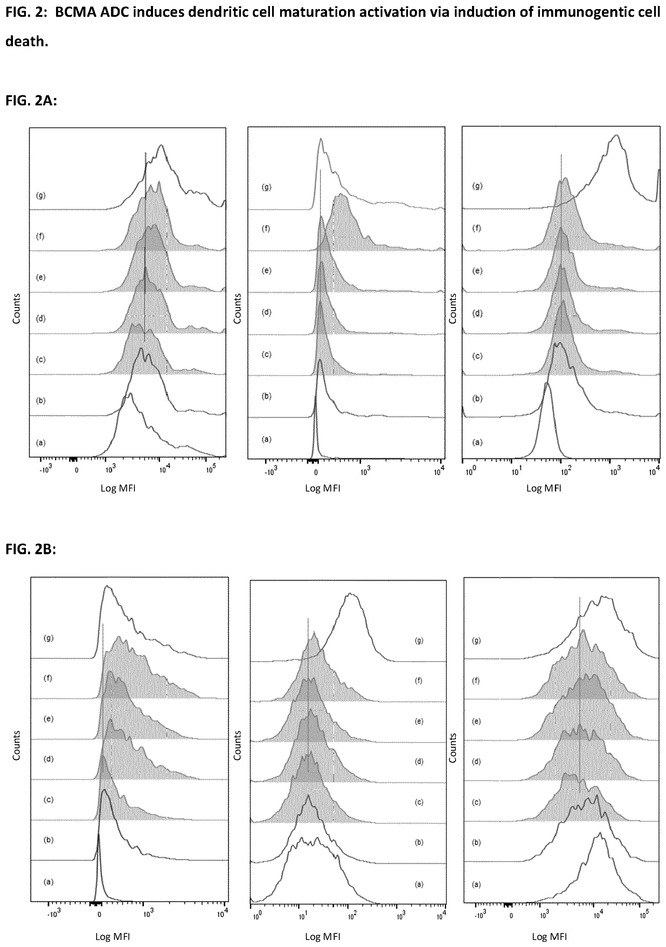

**FIG. 2B****)** CD40 cell surface expression increased on CD11c+ Dendritic cells from three healthy donors following 24 hour co-culture with BCMA ADC treated NCI-H929 Multiple Myeloma cells. Marker expression was measured in (a) untreated (no BCMA ADC) Dendritic cells alone and (b - f) Dendritic cells that were co-cultured with (b) untreated (no BCMA ADC), (c) IgG Control (10 µg/mL), (d) 0.1, (e) 1 and (f) 10 µg/mL BCMA ADC treated (48 hours) NCI-H929 cells. (g) 3µg/mL Lipopolysaccharide (LPS) treatment of Dendritic cells was included as a positive control. X-axis: Log Mean Fluorescence Intensity (MFI). The vertical line represents the MFI of the IgG control.
**FIG. 3****. Suppression of IL-10 production from both NCI-H929 cells and the co-culture system with BCMA ADC treated NCI-H929 cells and immature dendritic cells**
**FIG. 3A and 3B****.** IL-10 decreased in the supernatants from co-cultured Dendritic cells from two healthy human donors and BCMA ADC treated NCI-H929 Multiple Myeloma cells. IL-10 was measured in (a) untreated (no BCMA ADC) Dendritic cells alone and (b - f) Dendritic cells that were co-cultured with (b) untreated (no BCMA ADC), (c) IgG Control (10 µg/mL), (d) 0.1, (e) 1 and (f) 10 µg/mL BCMA ADC treated (48 hours) NCI-H929 cells. (g) 3 µg/mL Lipopolysaccharide (LPS) treatment of Dendritic cells is included as a positive control.
**FIG. 3C****.** IL-10 decreased with BCMA ADC treatment in supernatants from NCI-H929 Multiple Myeloma cells cultured alone. IL-10 was measured in Untreated (a), IgG Control (b), 0.1 (c), 1 (d), and 10 µg/mL BCMA ADC (e) treated (48 hours) NCI-H929 Multiple Myeloma cells.
**FIG. 4****: Effect of BCMA ADC on human T cells**
**FIG. 4A****)** The average % difference (Avg) and the coefficient of variation (CV) for the percentage (%) and MFI of markers in CD4 cells in PBMC after anti-CD3/anti-CD28 stimulation in the presence of BCMA ADC for 24 and 72 hrs;
**FIG. 4B****)** The average % difference (Avg) and the coefficient of variation (CV) for the percentage (%) and MFI of markers in CD8 cells in PBMC after anti-CD3/anti-CD28 stimulation in the presence of BCMA ADC for 24 and 72 hrs;
**FIG. 4C****)** The average % difference (Avg) and the coefficient of variation (CV) for the percentage (%) CD4 and CD8 cells expressing IFNy and IL-4 in PBMC with anti-CD3/ anti-CD28 stimulation in the presence of BCMA ADC for 48 and 72 hrs;
**FIG. 4D****)** Effect of BCMA ADC on proliferation of CD4+ and CD8+ T cells. CD4+ and CD8+ T cells stimulated with anti-CD3 and anti-CD28 antibodies in the presence or absence of various concentrations of BCMA ADC. After 96 hours cell proliferation was analyzed by flow cytometry. Data represent mean ± SD from 3 different donors.
   BCMA-ADC does not appear to have direct effects on human T cells.
**FIG. 5****:** Graphs demonstrating effect of the combination of an anti-BCMA antibody and anti-OX40 antibody on tumor volume (FIG. 5A) and survival rate (FIG. 5B) in EL4-Luc2-hBCMA mice.
**FIG. 6****:** Graphs demonstrating effect of the combination of an anti-BCMA antibody conjugated to MMAF and anti-PD-1 antibody on tumor volume in EL4-Luc2-hBCMA mice.
**FIG. 7****:** Graphs demonstrating effect of the combination of an anti-BCMA antibody conjugated to MMAF and anti-PD-1 antibody on tumor volume in EL4-Luc2-hBCMA mice.

### DETAILED DESCRIPTION OF THE INVENTION

### COMBINATIONS

In one embodiment of the invention there is provided a combination comprising a therapeutically effective amount of an antigen binding protein or fragment thereof that binds BCMA and a therapeutically effective amount of an antigen binding protein or fragment thereof that binds an immunomodulatory target.

In one embodiment of the invention there is provided a combination comprising a therapeutically effective amount of an antigen binding protein or fragment thereof that binds BCMA and a therapeutically effective amount of an antigen binding protein of fragment thereof that binds PD-1.

In one embodiment of the invention there is provided a combination comprising a therapeutically effective amount of an antigen binding protein or fragment thereof that binds BCMA and a therapeutically effective amount of an antigen binding protein or fragment thereof that binds OX40.

In one embodiment, there is provided a combination comprising a therapeutically effective amount of an antigen binding protein or fragment thereof that binds BCMA, a therapeutically effective amount of an antigen binding protein of fragment thereof that binds PD-1, and a therapeutically effective amount of an antigen binding protein or fragment thereof that binds OX40.

### ANTIGEN BINDING PROTEINS THAT BIND TO BCMA

In one embodiment there is provided antigen binding proteins (e.g. an antibody) or fragments thereof which specifically bind to BCMA, for example which specifically bind human BCMA (hBCMA). In another embodiment, the antigen binding protein that binds BCMA inhibits the binding of BAFF and/or APRIL to the BCMA receptor.

In a further embodiment the antigen binding proteins or fragments thereof have the ability to bind to FcγRIIIA and mediate FcgRIIIA mediated effector functions, or have enhanced FcγRIIIA mediated effector function. In one embodiment of the invention as herein provided the antigen binding proteins are capable of internalization. In one specific embodiment of the invention as herein provided the antigen binding proteins as herein described are capable of internalization at a rapid rate. For example the antigen binding proteins internalize in less than 12 hours, or in less than 6 hours, or in less than 120 minutes. In one embodiment the antigen binding proteins internalize in less than 30 minutes for example in 15 minutes. Internalization of the antigen binding proteins can be measured using techniques known in the art for example by confocal microscopy to visualize the BCMA bound to its receptor and colocalised with intracellular vesicles (endosomes and lysosomes) or present in the cytoplasm or alternatively by flow cytometry to detect the variation over time of the presence of BCMA on the cell surface with disappearance of BCMA indicating internalization.

In a further embodiment the antigen binding proteins of the present invention have effector function for example antibody dependant cellular cytoxicity (ADCC) for example the antigen binding protein has enhanced ADCC effector function.

In a further embodiment the antigen binding proteins are conjugated to a drug which is a cytotoxic agent to form an immunoconjugate (e.g. an antibody-drug conjugate (ADC)). In one such embodiment the cytotoxic agent is an auristatin. In yet a further embodiment the cytotoxic agent is monomethyl auristatin E (MMAE) or monomethyl auristatitin F (MMAF). In one embodiment the immunoconjugate is also ADCC enhanced.

In one embodiment the cytotoxic agent is conjugated to the antigen binding protein that binds BCMA via a linker, such as valine-citruline (VC) or maleimidocaproyl (mc).

In one such embodiment the immunoconjugate is able to cause immunogenic cell death. In one embodiment of the invention there is provided an antigen binding protein according to the invention as herein described which binds to non-membrane bound BCMA, for example to serum BCMA.

In another example, the antigen binding protein that binds to BCMA is an antagonist that blocks binding of BCMA with a BCMA ligand such as BAFF or APRIL.

In one embodiment, the antigen binding protein that binds BCMA contains an immunoglobulin-like domain or fragment thereof. In another embodiment, the antigen binding protein that binds BCMA is a monoclonal antibody, for example IgG, IgM, IgA, IgD or IgE, subclasses thereof, or modified variants thereof. In yet another embodiment, the antigen binding protein that binds BCMA is an IgG antibody.

In one embodiment of the invention there is provided an antigen binding protein as herein described wherein the antigen binding protein comprises CDRH3 of SEQ. ID. NO: 3, CDRH3 variant N99D of SEQ. ID. NO: 200, or variants thereof. In another embodiment, the antigen binding protein comprises a CDRH3 region comprising an amino acid sequence with at least 90%, 91%, 92%, 93%, 94%, 95%, 96%, 97%, 98%, 99% or 100% sequence identity to the amino acid sequence as set forth in SEQ. ID. NO: 3 or SEQ. ID. NO: 200.

In a further embodiment of the invention there is provided an antigen binding protein as herein described wherein the antigen binding protein further comprises one or more of: CDRH1 of SEQ. ID. NO: 1, CDRH2 of SEQ. ID. NO: 2, CDRL1 of SEQ. ID. NO: 4, CDRL2 of SEQ. ID. NO: 5 and/or CDRL3 of SEQ. ID. NO: 6 and or variants thereof.

In one embodiment of the invention there is provided an antigen binding protein as herein described wherein the antigen binding protein comprises CDRH3 of SEQ. ID. NO: 184 or a variant of SEQ. ID. NO: 184.

In a further embodiment of the invention there is provided an antigen binding protein as herein described wherein the antigen binding protein further comprises one or more of: CDRH1 of SEQ. ID. NO: 182, CDRH2 of SEQ. ID. NO: 183, CDRL1 of SEQ. ID. NO: 185, CDRL2 of SEQ. ID. NO: 186 and/or CDRL3 of SEQ. ID. NO: 187 and or variants thereof.

In yet a further embodiment the antigen binding protein comprises CDRH1 of SEQ. ID. NO: 1, CDRH2 of SEQ. ID. NO: 2, CDRH3 variant N99D of SEQ. ID. NO: 200, CDRL1 of SEQ. ID. NO: 4, CDRL2 of SEQ. ID. NO: 5 and CDRL3 of SEQ. ID. NO: 6. In another embodiment, the antigen binding protein comprises CDR regions comprising amino acid sequences with at least 90%, 91%, 92%, 93%, 94%, 95%, 96%, 97%, 98%, 99% or 100% sequence identity to the amino acid sequence as set forth in SEQ. ID. NO: 1, SEQ. ID. NO: 2, SEQ. ID. NO: 200, SEQ. ID. NO: 4, SEQ. ID. NO: 5 and SEQ. ID. NO: 6.

In a further embodiment, the antigen binding protein comprises CDRH1 of SEQ. ID. NO: 1, CDRH2 of SEQ. ID. NO: 2, CDRH3 of SEQ. ID. NO: 3, CDRL1 of SEQ. ID. NO: 4, CDRL2 of SEQ. ID. NO: 5, and CDRL3 of SEQ. ID. NO: 6. In another embodiment, the antigen binding protein comprises CDR regions comprising amino acid sequences with at least 90%, 91%, 92%, 93%, 94%, 95%, 96%, 97%, 98%, 99% or 100% sequence identity to the amino acid sequence as set forth in SEQ. ID. NO: 1, SEQ. ID. NO: 2, SEQ. ID. NO: 3, SEQ. ID. NO: 4, SEQ. ID. NO: 5 and SEQ. ID. NO: 6.

In yet a further embodiment the antigen binding protein comprises CDRH3 of SEQ. ID. NO: 184, CDRH2 of SEQ. ID. NO: 183, CDRH1 of SEQ. ID. NO: 182, CDRL1 of SEQ. ID. NO: 185, CDRL2 of SEQ. ID. NO: 186 and CDRL3 of SEQ. ID. NO: 187.

The antigen binding proteins of the present invention are derived from the murine antibody having the variable regions as described in SEQ. ID. NO: 7 and SEQ. ID. NO: 9 or non-murine equivalents thereof, such as rat, human, chimeric or humanised variants thereof, for example they are derived from the antibody having the variable heavy chain sequences as described in SEQ. ID. NO: 11, SEQ. ID. NO: 13, SEQ. ID. NO: 15, SEQ. ID. NO: 17, SEQ. ID. NO: 19, SEQ. ID. NO: 21, SEQ. ID. NO: 23, SEQ. ID. NO: 25, SEQ. ID. NO: 27 and SEQ. ID. NO: 29 and/or the variable light chain sequences as described in SEQ. ID. NO: 31, SEQ. ID. NO: 33 and/or SEQ. ID. NO: 35.

In another embodiment the antigen binding proteins of the present invention are derived from an antibody having the heavy chain variable region of SEQ ID NO:116 or SEQ ID NO:118 and/or the variable light chain sequences as described in SEQ ID NO:120, or SEQ ID NO:122. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 116 or SEQ ID NO: 118 and/or a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 120, or SEQ. ID. NO: 122.

In another embodiment the antigen binding proteins of the present invention are derived from an antibody having the heavy chain variable region sequences of SEQ. ID. NO: 140 and/or the light chain variable region sequences of SEQ. ID. NO: 144. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 140 and/or a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 144.

In one embodiment of the invention there is provided an antigen binding protein comprising an isolated heavy chain variable region selected from any one of the following: SEQ. ID. NO: 11, SEQ. ID. NO: 13, SEQ. ID. NO: 15, SEQ. ID. NO: 17, SEQ. ID. NO: 19, SEQ. ID. NO: 21, SEQ. ID. NO: 23, SEQ. ID. NO: 25, SEQ. ID. NO: 27, SEQ. ID. NO: 29, SEQ. ID. NO: 116 or SEQ. ID. NO: 118.

In another embodiment of the invention there is provided an antigen binding protein comprising an isolated light chain variable region selected from any one of the following: SEQ. ID. NO: 31, SEQ. ID. NO: 33 or SEQ. ID. NO: 35, SEQ. ID. NO: 120 or SEQ. ID. NO: 122.

In a further embodiment of the invention there is provided an antigen binding protein comprising an isolated heavy chain variable region selected from any one of the following: SEQ. ID. NO: 11, SEQ. ID. NO: 13, SEQ. ID. NO: 15, SEQ. ID. NO: 17, SEQ. ID. NO: 19, SEQ. ID. NO: 21, SEQ. ID. NO: 23, SEQ. ID. NO: 25, SEQ. ID. NO: 27 and SEQ. ID. NO: 29 and an isolated light chain variable region selected from any one of the following: SEQ. ID. NO: 31, SEQ. ID. NO: 33 and/or SEQ. ID. NO: 35.

In one embodiment the antigen binding protein of the present invention comprises a heavy chain variable region of SEQ. ID. NO: 23 and a light chain variable region of SEQ. ID. NO: 31. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 23 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 31.

In one embodiment the antigen binding protein of the present invention comprises a heavy chain variable region of SEQ. ID. NO: 27 and a light chain variable region of SEQ. ID. NO: 31. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 27 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 31.

In one embodiment the antigen binding protein of the present invention comprises a heavy chain variable region of SEQ. ID. NO: 29 and a light chain variable region of SEQ. ID. NO: 31. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 29 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 31.

In one embodiment the antigen binding protein of the present invention comprises a heavy chain variable region of SEQ. ID. NO: 116 and a light chain variable region of SEQ. ID. NO: 120. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 116 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 120.

In one embodiment the antigen binding protein of the present invention comprises a heavy chain variable region of SEQ. ID. NO: 118 and a light chain variable region of SEQ. ID. NO: 122. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 118 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 122.

In one embodiment, the immunoconjugate is GSK2857916. Tai, Blood, 123(2):3128-38 (2014). GSK2857916 comprises an anti-BCMA antibody conjugated to monomethyl auristatitin F (MMAF) via a maleimidocaproyl (mc) linker. GSK2857916 is derived from clone J6M0 and comprises the amino acid sequences of SEQ. ID. NO: 55, SEQ. ID. NO: 63, SEQ. ID. NO: 23, SEQ. ID. NO: 31, SEQ. ID. NO: 1, SEQ. ID. NO: 2, SEQ. ID. NO: 200, SEQ. ID. NO: 4, SEQ. ID. NO: 5, and SEQ. ID. NO: 6.

In one embodiment there is provided a polynucleotide encoding an isolated heavy chain variable region of SEQ. ID. NO: 12, or SEQ. ID. NO: 14, or SEQ. ID. NO: 16, or SEQ. ID. NO: 18, or SEQ. ID. NO: 20, or SEQ. ID. NO: 22, or SEQ. ID. NO: 24, or SEQ. ID. NO: 26, or SEQ. ID. NO: 28, or SEQ. ID. NO: 30 or SEQ. ID. NO: 117 or SEQ. ID. NO: 119 or SEQ. ID. NO: 141.

In one embodiment there is provided a polynucleotide encoding an isolated light chain variable region of SEQ. ID. NO: 32, or SEQ. ID. NO: 34, or SEQ. ID. NO: 36 or SEQ. ID. NO: 121 or SEQ. ID. NO: 123 or SEQ. ID. NO: 145.

In a further embodiment there is provided a polynucleotide encoding an isolated heavy chain variable region of SEQ. ID. NO: 24, or SEQ. ID. NO: 28 or SEQ. ID. NO: 30 and a polynucleotide encoding an isolated light chain variable region of SEQ. ID. NO: 32, or SEQ. ID. NO: 34.

In yet a further embodiment there is provided a polynucleotide encoding an isolated heavy chain variable region of SEQ. ID. NO: 24 and a polynucleotide encoding an isolated light chain variable region of SEQ. ID. NO: 32.

In yet a further embodiment there is provided a polynucleotide encoding an isolated heavy chain variable region of SEQ. ID. NO: 117 and a polynucleotide encoding an isolated light chain variable region of SEQ. ID. NO: 121.

In yet a further embodiment there is provided a polynucleotide encoding an isolated heavy chain variable region of SEQ. ID. NO: 119 and a polynucleotide encoding an isolated light chain variable region of SEQ. ID. NO: 123.

In yet a further embodiment there is provided a polynucleotide encoding an isolated heavy chain variable region of SEQ. ID. NO: 141 and a polynucleotide encoding an isolated light chain variable region of SEQ. ID. NO: 145.

In a further embodiment the antigen binding protein may comprise any one of the heavy chain variable regions as described herein in combination with any one of the light chain variable regions as described herein. In some embodiments, the antigen binding protein can bind (e.g., and antagonize) BCMA, e.g., human BCMA.

In one embodiment the antigen binding protein is an antibody or antigen binding fragment thereof comprising one or more CDR's according to the invention described herein, or one or both of the heavy chain variable region or light chain variable region according to the invention described herein. In one embodiment the antigen binding protein binds primate BCMA. In one such embodiment the antigen binding protein additionally binds non-human primate BCMA, for example cynomolgus macaque monkey BCMA.

In another embodiment the antigen binding protein is selected from the group consisting of a dAb, Fab, Fab', F(ab')2, Fv, diabody, triabody, tetrabody, miniantibody, and a minibody.

In one embodiment of the present invention the antigen binding protein is a humanised or chimeric antibody, in a further embodiment the antibody is humanised.

In one embodiment the antibody is a monoclonal antibody.

In one embodiment of the present invention there is provided an antibody with the heavy chain sequence as set forth in SEQ. ID. NO: 55 or SEQ. ID. NO: 59 or SEQ. ID. NO: 61.

In one embodiment of the present invention there is provided an antibody with the light chain sequence as set forth in SEQ. ID. NO: 63 or SEQ. ID. NO: 65.

In a further embodiment of the invention there is provided an antibody with the heavy chain sequence of SEQ. ID. NO: 55 and a light chain sequence as set forth in SEQ. ID. NO: 63. In another embodiment, the antigen binding protein comprises a heavy chain region comprising amino acid sequences with at least 90%, 91%, 92%, 93%, 94%, 95%, 96%, 97%, 98%, 99% or 100% sequence identity to the amino acid sequence as set forth in SEQ. ID. NO: 55 and a light chain region comprising amino acid sequences with at least 90%, 91%, 92%, 93%, 94%, 95%, 96%, 97%, 98%, 99% or 100% sequence identity to the amino acid sequence as set forth in SEQ. ID. NO: 63.

In one embodiment there is provided an antigen binding protein or fragment thereof which competes with an antigen binding protein of the invention as herein described. In one such embodiment there is therefore provided an antigen binding protein which competes with an antigen binding protein which comprises the heavy chain variable sequence of SEQ. ID. NO: 23 and the light chain variable region of SEQ. ID. NO: 31.

In a further embodiment there is therefore provided an antigen binding protein which competes with an antigen binding protein which comprises a heavy chain variable sequence selected from one of SEQ. ID. NO: 27, SEQ. ID. NO: 29, SEQ. ID. NO: 116, SEQ. ID. NO: 118 and SEQ. ID. NO: 140 and a light chain variable region selected from one of SEQ. ID. NO: 31, SEQ. ID. NO: 120, SEQ. ID. NO: 122 and SEQ. ID. NO: 144.

In one embodiment of the invention the antigen binding protein is a chimeric antigen receptor (CAR). In a further embodiment the CAR comprises a binding domain, a transmembrane domain and an intracellular effector domain.

In one embodiment, the transmembrane domain can be derived either from a natural or from a synthetic source. In one embodiment, the transmembrane domain can be derived from any membrane-bound or transmembrane protein. Alternatively the transmembrane domain can be synthetic and can comprise predominantly hydrophobic residues such as leucine and valine. For example, the transmembrane domain can be the transmembrane domain of CD proteins, such as CD4, CD8, CD3 or CD28, a subunit of the T cell receptor, such as α, β, γ or δ, a subunit of the IL-2 receptor (a chain), a submit of the Low-Affinity Nerve Growth Factor Receptor (LNGFR or p75) (β chain or γ chain), or a subunit chain of Fc receptors. In one embodiment, the transmembrane domain comprises the transmembrane domain of CD4, CD8 or CD28. In a further embodiment, the transmembrane domain comprises the transmembrane domain of CD4 or CD8 (e.g. the CD8 alpha chain, as described in NCBI Reference Sequence: NP_001139345.1, incorporated herein by reference). In a yet further embodiment, the transmembrane domain comprises the transmembrane domain of CD4.

The intracellular effector domain or "signalling domain" is responsible for intracellular signalling following the binding of the target binding domain to the target. The intracellular effector domain is responsible for the activation of at least one of the normal effector functions of the immune cell in which the CAR is expressed. For example, the effector function of a T cell can be a cytolytic activity or helper activity including the secretion of cytokines. Preferred examples of the effector domain for use in a CAR scaffold can be the cytoplasmic sequences of the natural T cell receptor and co-receptors that act in concert to initiate signal transduction following antigen binding, as well as any derivate or variant of these sequences and any synthetic sequence that has the same functional capability. Effector domains can be separated into two classes: those that initiate antigen-dependent primary activation, and those that act in an antigen-independent manner to provide a secondary or costimulatory signal. Primary activation effector domains can comprise signalling motifs which are known as immunoreceptor tyrosine-based activation motifs (ITAMs). ITAMs are well defined signalling motifs, commonly found in the intracytoplasmic tail of a variety of receptors, and serve as binding sites for syk/zap70 class tyrosine kinases. Examples of ITAMs used in the invention can include, as non-limiting examples, those derived from CD3zeta, FcRgamma, FcRbeta, FcRepsilon, CD3gamma, CD3delta, CD3epsilon, CD5, CD22, CD79a, CD79b and CD66d. In one embodiment, the intracellular effector domain comprises a CD3zeta signalling domain (also known as CD247). Natural TCRs contain a CD3zeta signalling molecule, therefore the use of this effector domain is closest to the TCR construct which occurs in nature.

In one embodiment of the invention the intracellular signalling domain is a CD3 zeta effector domain.

Effector domains may also provide a secondary or costimulatory signal. T cells additionally comprise costimulatory molecules which bind to cognate costimulatory ligands on antigen presenting cells in order to enhance the T cell response, for example by increasing proliferation activation, differentiation and the like. Therefore, in one embodiment, the intracellular effector domain additionally comprises a costimulatory domain. In a further embodiment, the costimulatory domain comprises the intracellular domain of a costimulatory molecule, selected from CD28, CD27, 4-1BB (CD137), OX40 (CD134), ICOS (CD278), CD30, CD40, PD-1 (CD279), CD2, CD7, NKG2C (CD94), B7-H3 (CD276) or any combination thereof. In a yet further embodiment, the costimulatory domain comprises the intracellular domain of a costimulatory molecule, selected from CD28, CD27, 4-1BB, OX40, ICOS or any combination thereof.

Competition between an antigen binding protein of the present invention and a reference antibody may be determined by competition ELISA, FMAT or Biacore. In one embodiment, the competition assay is carried out by Biacore. There are several possible reasons for this competition: the two proteins may bind to the same or overlapping epitopes, there may be steric inhibition of binding, or binding of the first protein may induce a conformational change in the antigen that prevents or reduces binding of the second protein.

In another embodiment the antigen binding protein binds to human BCMA with high affinity for example when measured by Biacore the antigen binding protein binds to human BCMA with an affinity of 20nM or less or an affinity of 15nM or less or an affinity of 5nM or less or an affinity of 1000 pM or less or an affinity of 500pM or less or an affinity of 400pM or less, or 300pM or less or for example about 120pM. In a further embodiment the antigen binding protein binds to human BCMA when measured by Biacore of between about 100pM and about 500pM or between about 100pM and about 400pM, or between about 100pM and about 300pM. In one embodiment of the present invention the antigen binding protein binds BCMA with an affinity of less than 150pm. In one such embodiment, this is measured by Biacore, for example as set out in Example 4 of WO2012163805 as herein incorporated by reference.

In another embodiment the antigen binding protein binds to human BCMA and neutralizes the binding of the ligands BAFF and/or APRIL to the BCMA receptor in a cell neutralization assay wherein the antigen binding protein has an IC50 of between about 1nM and about 500nM, or between about 1nM and about 100nM, or between about 1nM and about 50nM, or between about 1nM and about 25nM, or between about 5nM and about 15nM. In a further embodiment of the present invention the antigen binding protein binds BCMA and neutralizes BCMA in a cell neutralization assay wherein the antigen binding protein has an IC50 of about 10nM.

In one such embodiment, this is measured by a cell neutralization assay, for example as set out in Example 4.6 of WO2012163805 as herein incorporated by reference.

### ANTIGEN BINDING PROTEINS THAT BIND TO PD-1

In one embodiment of the invention, as herein described, the combination comprises an antigen binding protein (e.g. an antibody) which specifically binds to BCMA and an antigen binding protein (e.g. an antibody) or fragment thereof which specifically binds to PD-1. In one example, the antigen binding protein that binds to PD-1 specifically binds human PD-1 (hPD-1). In another example, the antigen binding protein that binds to PD-1 is an antagonist that blocks binding of PD-1 with a PD-1 ligand such as PD-L1 or PD-L2.

In one embodiment, the antigen binding protein that binds PD-1 contains an immunoglobulin-like domain or fragment thereof. In another embodiment, the antigen binding protein that binds PD-1 is a monoclonal antibody, for example IgG, IgM, IgA, IgD or IgE, subclasses thereof, or modified variants thereof. In yet another embodiment, the antigen binding protein that binds PD-1 is an IgG antibody. In another embodiment, the antigen binding protein that binds PD-1 is an IgG4 antibody.

In one embodiment of the invention there is provided an antigen binding protein as herein described wherein the antigen binding protein comprises CDRH3 of SEQ. ID .NO: 203 or a variant thereof. In another embodiment, the antigen binding protein comprises a CDRH3 region comprising an amino acid sequence with at least 90%, 91%, 92%, 93%, 94%, 95%, 96%, 97%, 98%, 99% or 100% sequence identity to the amino acid sequence as set forth in SEQ. ID. NO: 203.

In a further embodiment of the invention there is provided an antigen binding protein as herein described wherein the antigen binding protein comprises CDRH1 of SEQ. ID. NO: 201, CDRH2 of SEQ. ID. NO: 202, CDRH3 of SEQ. ID. NO: 203, CDRL1 of SEQ. ID. NO: 204, CDRL2 of SEQ. ID. NO: 205, CDRL3: SEQ. ID. NO: 206, and or variants thereof. In another embodiment, the antigen binding protein comprises CDR regions comprising amino acid sequences with at least 90%, 91%, 92%, 93%, 94%, 95%, 96%, 97%, 98%, 99% or 100% sequence identity to the amino acid sequences as set forth in SEQ. ID. NO: 201, SEQ. ID. NO: 202, SEQ. ID. NO: 203, SEQ. ID. NO: 204, SEQ. ID. NO: 205 and SEQ. ID. NO: 206.

In one embodiment of the invention there is provided an antigen binding protein as herein described wherein the antigen binding protein comprises CDRH3 of SEQ. ID. NO: 213 or a variant thereof.

In a further embodiment of the invention there is provided an antigen binding protein as herein described wherein the antigen binding protein further comprises one or more of: CDRH1 of SEQ. ID. NO: 211, CDRH2 of SEQ. ID. NO: 212, CDRL1 of SEQ. ID. NO: 214, CDRL2 of SEQ. ID. NO: 215 and/or CDRL3 of SEQ. ID. NO: 216 or variants thereof.

In yet a further embodiment the antigen binding protein comprises CDRH3 of SEQ. ID. NO: 203, CDRH2 of SEQ. ID. NO: 202, CDRH1 of SEQ. ID. NO: 201, CDRL1 of SEQ. ID. NO: 204, CDRL2 of SEQ. ID. NO: 205 and CDRL3 of SEQ. ID. NO: 206.

In yet a further embodiment the antigen binding protein comprises CDRH3 of SEQ. ID. NO: 213, CDRH2 of SEQ. ID. NO: 211, CDRH1 of SEQ. ID. NO: 212, CDRL1 of SEQ. ID. NO: 214, CDRL2 of SEQ. ID. NO: 215 and CDRL3 of SEQ. ID. NO: 216.

In one embodiment of the invention there is provided an antigen binding protein comprising an isolated heavy chain variable domain selected from SEQ. ID. NO: 207 or SEQ. ID. NO: 217. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 207 or SEQ. ID. NO: 217.

In another embodiment of the invention there is provided an antigen binding protein comprising an isolated light chain variable region selected from SEQ. ID. NO: 208 or SEQ. ID. NO: 218. In another embodiment, the antigen binding protein comprises a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 208 or SEQ. ID. NO: 218.

In a further embodiment of the invention there is provided an antigen binding protein comprising an isolated heavy chain variable region selected from SEQ. ID. NO: 207 or SEQ. ID. NO: 217 and an isolated light chain variable domain selected from SEQ. ID. NO: 208 or SEQ. ID. NO: 218. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 207 or SEQ. ID. NO: 217 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 208 or SEQ. ID. NO: 218.

In one embodiment the antigen binding protein of the present invention comprises a heavy chain variable region encoded by SEQ. ID. NO: 207 and a light chain variable region encoded by SEQ. ID. NO: 208. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 207 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 208.

In one embodiment the antigen binding protein of the present invention comprises a heavy chain variable region encoded by SEQ. ID. NO: 217 and a light chain variable region encoded by SEQ. ID. NO: 218. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 217 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 218.

In one combination contemplated, the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ ID.NO: 200, CDRL1 of SEQ. ID. NO: 4, CDRL2 of SEQ. ID. NO: 5, CDRL3 of SEQ. ID. NO: 6 or variants thereof conjugated to MMAF; and the antigen binding protein that binds PD-1 comprises CDRH1 of SEQ. ID. NO: 201, CDRH2 of SEQ. ID. NO: 202, CDRH3 of SEQ ID NO: 203, CDRL1 of SEQ. ID. NO: 204, CDRL2 of SEQ. ID. NO: 205, CDRL3 of SEQ. ID. NO: 206, or variants thereof.

In one embodiment there is provided a polynucleotide encoding an isolated heavy chain variable region of SEQ. ID. NO: 207. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 207.

In one embodiment there is provided a polynucleotide encoding the CDR regions of SEQ. ID. NO: 201, SEQ. ID. NO: 202, SEQ. ID. NO: 203, SEQ. ID. NO: 204, SEQ. ID. NO: 205 and SEQ. ID. NO: 206. In another embodiment, the antigen binding protein comprises CDR regions comprising amino acid sequences with at least 90%, 91%, 92%, 93%, 94%, 95%, 96%, 97%, 98%, 99% or 100% sequence identity to the amino acid sequence as set forth in SEQ. ID. NO: 201, SEQ. ID. NO: 202, SEQ. ID. NO: 203, SEQ. ID. NO: 204, SEQ. ID. NO: 205 and SEQ. ID. NO: 206.

In one embodiment the antigen binding protein is an antibody or antigen binding fragment thereof comprising one or more CDR's according to the invention described herein, or one or both of the heavy chain variable regions or light chain variable regions according to the invention described herein.

In another embodiment the antigen binding protein is selected from the group consisting of a dAb, Fab, Fab', F(ab')₂, Fv, diabody, triabody, tetrabody, miniantibody, and a minibody,.
In one embodiment the antigen binding proteins of the present invention are humanised or chimeric antibodies, in a further embodiment the antibody is humanised.

In one embodiment the antibody is a monoclonal antibody.

In one embodiment of the present invention there is provided an antibody with the heavy chain sequence as set forth in SEQ. ID. NO: 209.

In one embodiment of the present invention there is provided an antibody with the light chain sequence as set forth in SEQ. ID. NO: 210.

In a further embodiment of the invention there is provided an antibody with the heavy chain sequence of SEQ. ID. NO: 209 and a light chain sequence as set forth in SEQ ID. NO: 210.

In one embodiment there is provided an antigen binding protein or fragment thereof which competes with an antigen binding protein of the invention as herein described. In one such embodiment there is therefore provided an antigen binding protein which competes with an antigen binding protein which comprises the heavy chain variable sequence of SEQ. ID. NO: 207 and the light chain variable region of SEQ. ID. NO: 208.

The isolated antibodies as described herein bind to human PD-1, and may bind to human PD-1 encoded by the gene Pdcd1, or genes or cDNA sequences having 90 percent homology or 90 percent identity thereto. The complete hPD-1 mRNA sequence can be found under GenBank Accession No. U64863. The protein sequence for human PD-1 can be found at GenBank Accession No. AAC51773.

In another embodiment the antigen binding protein binds to human PD-1 with high affinity for example when measured by Biacore the antigen binding protein binds to human PD-1 with an affinity of 1nM or less. In one embodiment of the present invention the antigen binding protein binds PD-1 with an affinity of less than 100pm.

The binding affinity of pembrolizumab for cynomolgus PD-1 was evaluated by ELISA, cellular ELISA and by bio-light interferometry. In these studies, the binding affinity of pembrolizumab to cynomolgus and human PD-1 was found to be in the same range, albeit slightly lower for cynomolgus PD-1. By kinetic analysis, KD was 29 pM for human PD-1 and 118 pM for cynomolgus PD-1. Functionally, pembrolizumab blocked the binding of human PD-1 ligands to cells expressing human or cynomolgus PD-1 with a comparable potency. A skilled person will appreciate that the smaller the KD numerical value, the stronger the binding. The reciprocal of KD (i.e. 1/KD) is the equilibrium association constant (KA) having units M-1. A skilled person will appreciate that the larger the KA numerical value, the stronger the binding.

The dissociation rate constant (kd) or "off-rate" describes the stability of the complex i.e. the fraction of complexes that decay per second. For example, a kd of 0.01 s-1 equates to 1% of the complexes decaying per second. In an embodiment, the dissociation rate constant (kd) is 1×10-3 s-1 or less, 1×10-4 s-1 or less, 1×10-5 s-1 or less, or 1×10-6 s-1 or less. The kd may be between 1×10-5 s-1 and 1×10-4 s-1; or between 1×10-4 s-1 and 1×10-3 s-1.

Competition between an antigen binding protein of the present invention and a reference antibody may be determined by competition ELISA, FMAT or Biacore. In one embodiment, the competition assay is carried out by Biacore. There are several possible reasons for this competition: the two proteins may bind to the same or overlapping epitopes, there may be steric inhibition of binding, or binding of the first protein may induce a conformational change in the antigen that prevents or reduces binding of the second protein.

In one embodiment of the invention the PD-1 antigen binding protein is Pembrolizumab also known as KEYTRUDA^{®} or MK3475 and as lambrolizumab. Pembrolizumab is a monoclonal antibody that binds to the PD-1 receptor and blocks its interaction with PD-L1 and PD-L2, releasing PD-1 pathway-mediated inhibition of the immune response, including the anti-tumor immune response.

In syngeneic mouse tumor models, blocking PD-1 activity resulted in decreased tumor growth. Pembrolizumab is an IgG4 kappa immunoglobulin with an approximate molecular weight of 149 kDa.

Pembrolizumab (KEYTRUDA^{®}) is a human programmed death receptor-1 (PD-1)-blocking antibody indicated for the treatment of patients with unresectable or metastatic melanoma and disease progression following ipilimumab and, if BRAF V600 mutation positive, a BRAF inhibitor. The recommended dose of pembrolizumab is 2 mg/kg administered as an intravenous infusion over 30 minutes every 3 weeks until disease progression or unacceptable toxicity.

Pembrolizumab is a sterile, preservative-free, white to off-white lyophilized powder in single-use vials. Each vial is reconstituted and diluted for intravenous infusion. Each 2 mL of reconstituted solution contains 50 mg of pembrolizumab and is formulated in L-histidine (3.1 mg), polysorbate-80 (0.4 mg), sucrose (140 mg). May contain hydrochloric acid/sodium hydroxide to adjust pH to 5.5.

Pembrolizumab is described, e.g. in U.S. Patent Nos. 8,354,509 and 8,900,587.

Pembrolizumab has been approved for the treatment of patients with unresectable or metastatic melanoma and disease progression following ipilimumab and, if BRAF V600 mutation positive, a BRAF inhibitor.

In one embodiment, the combination comprises pembrolizumab and GSK2857916.

As another example, an anti-BCMA antibody can be used in combination with nivolumab (OPDIVO^{®}). Nivolumab is a human immunoglobulin G4 (IgG4) monoclonal antibody that binds to the PD-1 receptor and blocks its interaction with PD-L1 and PD-L2, releasing PD-1 pathway-mediated inhibition of the immune response, including the anti-tumor immune response. In syngeneic mouse tumor models, blocking PD-1 activity resulted in decreased tumor growth.

Nivolumab (OPDIVO^{®}) is a programmed death receptor-1 (PD-1) blocking antibody indicated for the treatment of patients with: unresectable or metastatic melanoma and disease progression following ipilimumab and, if BRAF V600 mutation positive, a BRAF inhibitor. This indication is approved under accelerated approval based on tumor response rate and durability of response. Continued approval for this indication may be contingent upon verification and description of clinical benefit in the confirmatory trials and metastatic squamous non-small cell lung cancer with progression on or after platinum-based chemotherapy.

The recommended dose of nivolumab (OPDIVO^{®}) is 3 mg/kg administered as an intravenous infusion over 60 minutes every 2 weeks until disease progression or unacceptable toxicity. U.S. Patent No. 8,008,449 exemplifies seven anti-PD-1 HuMAbs: 17D8, 2D3, 4H1, 5C4 (also referred to herein as nivolumab or BMS-936558), 4A1 1, 7D3 and 5F4. See also U.S. Patent No. 8,779,105. Any one of these antibodies, or the CDRs thereof (or an amino acid sequence with at least 90% (e.g., 90, 91, 92, 93, 94, 95, 96, 97, 98, or 99%) identity to any of these amino acid sequences), can be used in the compositions and methods described herein.

### ANTIGEN BINDING PROTEINS THAT BIND TO OX40

In one embodiment of the invention, as herein described, the combination comprises an antigen binding protein (*e.g.* an antibody) which specifically binds to BCMA and an antigen binding protein (*e.g.* an antibody) or fragment thereof which specifically binds to OX40. In one example, the antigen binding protein that binds to OX40 specifically binds human OX40 (hOX40). In another example, the antigen binding protein that binds to OX40 is an agonist.

In one embodiment, the antigen binding protein that binds OX40 contains an immunoglobulin-like domain or fragment thereof. In another embodiment, the antigen binding protein that binds OX40 is a monoclonal antibody, for example IgG, IgM, IgA, IgD or IgE, subclasses thereof, or modified variants thereof. In yet another embodiment, the antigen binding protein that binds OX40 is an IgG1 antibody.

In one embodiment of the invention there is provided an antigen binding protein as herein described wherein the antigen binding protein comprises CDRH3 of SEQ. ID. NO: 221 or a variant thereof. In another embodiment, the antigen binding protein comprises a CDRH3 region comprising an amino acid sequence with at least 90%, 91%, 92%, 93%, 94%, 95%, 96%, 97%, 98%, 99% or 100% sequence identity to the amino acid sequence as set forth in SEQ. ID. NO: 221.

In a further embodiment of the invention there is provided an antigen binding protein as herein described wherein the antigen binding protein comprises CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223 and CDRL3 of SEQ. ID. NO: 224 or variants thereof. In another embodiment, the antigen binding protein comprises CDR regions comprising amino acid sequences with at least 90%, 91%, 92%, 93%, 94%, 95%, 96%, 97%, 98%, 99% or 100% sequence identity to the amino acid sequence as set forth in SEQ. ID. NO: 219, SEQ. ID. NO: 220, SEQ. ID. NO: 221, SEQ. ID. NO: 222, SEQ. ID. NO: 223 and SEQ. ID. NO: 224

In one embodiment of the invention there is provided an antigen binding protein as herein described wherein the antigen binding protein comprises CDRH3 of SEQ. ID. NO: 233 or a variant thereof.

In a further embodiment of the invention there is provided an antigen binding protein as herein described wherein the antigen binding protein further comprises one or more of: CDRH1 of SEQ. ID. NO: 231, CDRH2 of SEQ. ID. NO: 232, CDRL1 of SEQ. ID. NO: 234, CDRL2 of SEQ. ID. NO: 235 and/or CDRL3 of SEQ. ID. NO: 236 and or variants thereof.

In one embodiment of the invention there is provided an antigen binding protein comprising an isolated heavy chain variable region selected from: SEQ ID NO: 229, SEQ ID NO: 225, or SEQ ID NO: 237.

In another embodiment of the invention there is provided an antigen binding protein comprising an isolated light chain variable region selected from SEQ. ID. NO: 230, SEQ. ID. NO: 227, or SEQ. ID. NO: 239.

In a further embodiment of the invention there is provided an antigen binding protein comprising an isolated heavy chain variable region selected from: SEQ. ID. NO: 225 or SEQ. ID. NO: 237 and an isolated light chain variable region selected from: SEQ. ID. NO: 227 or SEQ. ID. NO: 239.

In one embodiment the antigen binding protein of the present invention comprises a heavy chain variable region of SEQ. ID. NO: 225 and a light chain variable region of SEQ. ID. NO: 227. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 225 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 227.

In one embodiment the antigen binding protein of the present invention comprises a heavy chain variable region of SEQ. ID. NO: 237 and a light chain variable region of SEQ. ID. NO: 239. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 237 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 239.

In a further embodiment of the invention there is provided an antigen binding protein comprising an isolated heavy chain variable region of SEQ. ID. NO: 229 and an isolated light chain variable region of SEQ. ID. NO: 230. In another embodiment, the antigen binding protein comprises a heavy chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 229 and a light chain variable region comprising amino acid sequences with at least 90%, 91%, 92%, 93%, 94%, 95%, 96%, 97%, 98%, 99% or 100% sequence identity to the amino acid sequence as set forth in SEQ. ID. NO: 230.

In one embodiment of the invention there is provided an antigen binding protein comprising an isolated heavy chain region of SEQ. ID. NO: 243 and an isolated light chain region of SEQ. ID. NO: 244. In another embodiment, the antigen binding protein comprises a heavy chain region comprising amino acid sequences with at least 90%, 91%, 92%, 93%, 94%, 95%, 96%, 97%, 98%, 99% or 100% sequence identity to the amino acid sequence as set forth in SEQ. ID. NO: 243 and a light chain region comprising amino acid sequences with at least 90%, 91%, 92%, 93%, 94%, 95%, 96%, 97%, 98%, 99% or 100% sequence identity to the amino acid sequence as set forth in SEQ. ID. NO: 244.

In one combination contemplated, the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ ID. NO: 200, CDRL1 of SEQ. ID. NO: 4, CDRL2 of SEQ. ID. NO: 5, CDRL3 of SEQ. ID. NO: 6 or variants thereof conjugated to MMAF; and the antigen binding protein that binds OX40 comprises CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223 and CDRL3 of SEQ. ID. NO: 224 or variants thereof.

In one embodiment there is provided a polynucleotide encoding an isolated heavy chain variable region of SEQ. ID. NO: 229, SEQ. ID. NO: 226, or SEQ. ID. NO: 238.

In one embodiment there is provided a polynucleotide encoding an isolated light chain variable region comprising SEQ. ID. NO: 230, SEQ. ID. NO: 228, or SEQ. ID. NO: 240.

In a further embodiment there is provided a polynucleotide encoding an isolated heavy chain variable region of SEQ. ID. NO: 229, SEQ. ID. NO: 226, or SEQ. ID. NO: 238 and a polynucleotide encoding an isolated light chain variable region of SEQ. ID. NO: 230, SEQ. ID. NO: 228, or SEQ. ID. NO: 240.

In one embodiment, the antigen binding protein of the invention binds its target (e.g. OX40) with high affinity. For example, when measured by Biacore, the antibody binds to OX40, preferably human OX40, with an affinity of 1-1000nM or 500nM or less or an affinity of 200nM or less or an affinity of 100nM or less or an affinity of 50 nM or less or an affinity of 500pM or less or an affinity of 400pM or less, or 300pM or less. In a further embodiment the antibody binds to OX40, preferably human OX40, when measured by Biacore of between about 50nM and about 200nM or between about 50nM and about 150nM. In one embodiment of the present invention the antibody binds OX40, preferably human OX40, with an affinity of less than 100nM.

Competition between an antigen binding protein of the present invention and a reference antibody may be determined by competition ELISA, FMAT or Biacore. In one embodiment, the competition assay is carried out by Biacore. There are several possible reasons for this competition: the two proteins may bind to the same or overlapping epitopes, there may be steric inhibition of binding, or binding of the first protein may induce a conformational change in the antigen that prevents or reduces binding of the second protein.

In an embodiment, the equilibrium dissociation constant (KD) of the antigen binding protein of a combination of the invention, or a method or use thereof, and OX40, preferably human OX40, interaction is 100 nM or less, 10 nM or less, 2 nM or less or 1 nM or less. Alternatively the KD may be between 5 and 10 nM; or between 1 and 2 nM. The KD may be between 1 pM and 500 pM; or between 500 pM and 1 nM. A skilled person will appreciate that the smaller the KD numerical value, the stronger the binding.

In a further embodiment the antigen binding protein may comprise any one of the variable heavy chains as described herein in combination with any one of the light chains as described in Table A herein.

In one embodiment, the antigen binding protein is a monoclonal antibody that binds to OX40 and is administered at a dose of about 0.1 mg/kg to about 10 mg/kg.

In one embodiment, the antigen binding protein is a monoclonal antibody that binds to OX40 and is administered at a dose selected from the group consisting of: about 0.003 mg/kg, about 0.01 mg/kg, about 0.03 mg/kg, about 0.1 mg/kg, about 0.3 mg/kg, about 1 mg/kg, about 3 mg/kg, and about 10mg/kg.

In another embodiment, the monoclonal antibody that binds to OX40 is administered at a frequency selected from the group consisting of: once daily, once weekly, once every two weeks (Q2W), and once every three weeks (Q3W).

In yet another embodiment, the monoclonal antibody that binds to human PD-1 is administered at a dose of about 200 mg.

In one embodiment, the monoclonal antibody that binds to OX40 is administered at a dose of about 0.1 mg/kg to about 10 mg/kg. The monoclonal antibody that binds to OX40 can be administered at a frequency selected from: once daily, once weekly, once every two weeks (Q2W) and once every three weeks (Q3W). Suitably, the antibody that binds to OX40 is administered once every three weeks. In one aspect, the pembrolizumab is administered at a dose of 200 mg Q3W.

### PHARMACEUTICAL COMPOSITIONS AND METHODS OF TREATMENT

The invention further provides pharmaceutical compositions, which include the combinations described herein, and one or more pharmaceutically acceptable carriers, diluents, or excipients. The combination of the invention may comprise two pharmaceutical compositions, one comprising an anti-BCMA antigen binding proteins or fragments, and the other comprising an anti-PD-1 or OX40 antigen binding protein or fragments thereof, each of which may have the same or different carriers, diluents or excipients. The carrier(s), diluent(s) or excipient(s) must be acceptable in the sense of being compatible with the other ingredients of the formulation, capable of pharmaceutical formulation, and not deleterious to the recipient thereof.

The components of the combination of the invention, and pharmaceutical compositions comprising such components may be administered sequentially in any order, and in different routes; the components and pharmaceutical compositions comprising the same may be administered simultaneously.

Each of the components of the combinations described herein can be manufactured into the same vial/container or into different vials/containers. For example, in one embodiment, an antigen binding protein that binds BCMA is manufactured into the same vial/container as an antigen binding protein that binds to PD-1 or an antigen binding protein that binds to OX40 and all components of the combination are administered simultaneously. In another exemplary embodiment, an antigen binding protein that binds BCMA is manufactured into a different vial/container as an antigen binding protein that binds to PD-1 or an antigen binding protein that binds to OX40 and each of the components of the combination can be administered simultaneously, sequentially, and with the same or different routes of administration.

In accordance with another embodiment of the invention there is also provided a process for the preparation of a pharmaceutical composition including admixing a component of the combination of the invention and one or more pharmaceutically acceptable carriers, diluents or excipients.

The components of the invention may be administered by any appropriate route. For some components, suitable routes include oral, rectal, nasal, topical (including buccal and sublingual), vaginal, and parenteral (including subcutaneous, intramuscular, intravenous, intradermal, intrathecal, and epidural). It will be appreciated that the preferred route may vary with, for example, the condition of the recipient of the combination and the cancer to be treated. It will also be appreciated that each of the agents administered may be administered by the same or different routes and that the components may be compounded together or in separate pharmaceutical compositions.

In one embodiment, one or more components of a combination of the invention are administered intravenously. In another embodiment, one or more components of a combination of the invention are administered intratumorally. In another embodiment, one or more components of a combination of the invention are administered systemically, e.g. intravenously, and one or more other components of a combination of the invention are administered intratumorally. In another embodiment, all of the components of a combination of the invention are administered systemically, e.g. intravenously. In an alternative embodiment, all of the components of the combination of the invention are administered intratumorally. In any of the embodiments, e.g. in this paragraph, the components of the invention are administered as one or more pharmaceutical compositions.

The administration of a therapeutically effective amount of the combinations of the invention (or therapeutically effective amounts of each of the components of the combination) are advantageous over the individual component compounds in that the combinations provide one or more of the following improved properties when compared to the individual administration of a therapeutically effective amount of a component compound: i) a greater anticancer effect than the most active single agent, ii) synergistic or highly synergistic anticancer activity, iii) a dosing protocol that provides enhanced anticancer activity with reduced side effect profile, iv) a reduction in the toxic effect profile, v) an increase in the therapeutic window, or vi) an increase in the bioavailability of one or both of the component compounds.

In one embodiment, the present invention provides methods of treating cancer such as in treatment of B cell disorders in a mammal (*e.g.* a human) in need thereof comprising administering a therapeutically effective amount of the combination as herein described. In one embodiment the cancer is multiple myeloma. In another embodiment the cancer is non-Hodgkin's lymphoma.

In one embodiment, methods are provided for treating cancer comprising administering any one of the antigen binding proteins that bind BCMA of the present invention or antibody drug conjugates thereof; and pembrolizumab, or an antibody comprising 90%, 91%, 92%, 93%, 94%, 95%, 96%, 97%, 98%, 99% or 100% sequence homology thereto.

In one embodiment, methods are provided for treating cancer comprising administering any one of the antigen binding proteins that bind BCMA or immunoconjugates thereof; and nivolumab, or an antibody comprising 90%, 91%, 92%, 93%, 94%, 95%, 96%, 97%, 98%, 99% or 100% sequence homology thereto

In one embodiment, methods are provided for treating cancer in a human in need thereof comprising administering a therapeutically effective amount of a combination comprising:
i) a therapeutically effective amount an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount an antigen binding protein that binds PD-1 comprising CDRH1 of SEQ. ID. NO: 201, CDRH2 of SEQ. ID. NO: 202, CDRH3 of SEQ. ID. NO: 203, CDRL1 of SEQ. ID. NO: 204, CDRL2 of SEQ. ID. NO: 205, CDRL3 of SEQ. ID. NO: 206, or variants thereof.

In one embodiment, methods are provided for treating cancer in a human in need thereof comprising administering a therapeutically effective amount of a combination comprising:
i) a therapeutically effective amount an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of pembrolizumab or nivolumab.

In one embodiment, methods are provided for treating cancer in a human in need thereof comprising administering a therapeutically effective amount of a combination comprising:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ ID NO: 221, CDRL1 of SEQ. ID. NO: 222, CDRL2 of SEQ. ID. NO: 223, CDRL3 of SEQ. ID. NO: 224, or variants thereof.

In one embodiment, methods are provided for treating multiple myeloma in a human in need thereof comprising administering a therapeutically effective amount of a combination comprising:
i) a therapeutically effective amount an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount an antigen binding protein that binds PD-1 comprising CDRH1 of SEQ. ID. NO: 201, CDRH2 of SEQ. ID. NO: 202, CDRH3 of SEQ. ID NO: 203, CDRL1 of SEQ. ID. NO: 204, CDRL2 of SEQ. ID. NO: 205, CDRL3 of SEQ. ID. NO: 206, or variants thereof.

In one embodiment, methods are provided for treating non-Hodgkins lymphoma in a human in need thereof comprising administering a therapeutically effective amount of a combination comprising:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds PD-1 comprising CDRH1 of SEQ. ID. NO: 201, CDRH2 of SEQ. ID. NO: 202, CDRH3 of SEQ. ID NO: 203, CDRL1 of SEQ. ID. NO: 204, CDRL2 of SEQ. ID. NO: 205, CDRL3 of SEQ. ID. NO: 206, or variants thereof.

In one embodiment, methods are provided for treating multiple myeloma in a human in need thereof comprising administering a therapeutically effective amount of a combination comprising:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

In one embodiment, methods are provided for treating non-Hodgkins lymphoma in a human in need thereof comprising administering a therapeutically effective amount of a combination comprising:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

Methods are provided wherein the tumor size of the cancer in said mammal is reduced by more than an additive amount compared with treatment with the antigen binding protein to BCMA and the antigen binding protein that binds PD-1 or the antigen binding protein that binds OX40 as used as monotherapy. Suitably the combination may be synergistic.

In one embodiment, the mammal has increased survival when treated with the combination as herein described compared with a mammal who received the antigen binding protein to BCMA or the antigen binding protein to PD-1 or OX40 as a monotherapy. In one embodiment, the methods further comprise administering at least one anti-neoplastic agent to the mammal in need thereof.

Use in the treatment of cancer, in particular in the treatment of B cell disorders in a mammal (e.g. a human) in need thereof, is contemplated. In one embodiment the cancer is multiple myeloma. In another embodiment the cancer is non-Hodgkin's lymphoma.

In one embodiment, use of the combinations described herein is provided for treating cancer wherein the combination comprises any one of the antigen binding proteins that bind BCMA of the present invention or antibody drug conjugates thereof; and pembrolizumab, or an antibody comprising 90%, 91%, 92%, 93%, 94%, 95%, 96%, 97%, 98%, 99% or 100% sequence homology thereto.

In one embodiment, use of the combinations described herein is provided for treating cancer wherein the combination comprises any one of the antigen binding proteins that bind BCMA or immunoconjugates thereof; and nivolumab, or an antibody comprising 90%, 91%, 92%, 93%, 94%, 95%, 96%, 97%, 98%, 99% or 100% sequence homology thereto

In one embodiment, use of combinations is provided for treating cancer in a human in need thereof wherein the combination comprises:
i) a therapeutically effective amount an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount an antigen binding protein that binds PD-1 comprising CDRH1 of SEQ. ID. NO: 201, CDRH2 of SEQ. ID. NO: 202, CDRH3 of SEQ. ID. NO: 203, CDRL1 of SEQ. ID. NO: 204, CDRL2 of SEQ. ID. NO: 205, CDRL3 of SEQ. ID. NO: 206, or variants thereof.

In one embodiment, use of combinations is provided for treating cancer in a human in need thereof wherein the combination comprises:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

In one embodiment, use of combinations is provided for treating multiple myeloma in a human in need thereof wherein the combination comprises:
i) a therapeutically effective amount an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount an antigen binding protein that binds PD-1 comprising CDRH1 of SEQ. ID. NO: 201, CDRH2 of SEQ. ID. NO: 202, CDRH3 of SEQ ID. NO: 203, CDRL1 of SEQ. ID. NO: 204, CDRL2 of SEQ. ID. NO: 205, CDRL3 of SEQ. ID. NO: 206, or variants thereof.

In one embodiment, use of combinations is provided for treating non-Hodgkin's lymphoma in a human in need thereof wherein the combination comprises:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds PD-1 comprising CDRH1 of SEQ. ID. NO: 201, CDRH2 of SEQ. ID. NO: 202, CDRH3 of SEQ. ID. NO: 203, CDRL1 of SEQ. ID. NO: 204, CDRL2 of SEQ. ID. NO: 205, CDRL3 of SEQ. ID. NO: 206, or variants thereof.

In one embodiment, use of combinations is provided for treating multiple myeloma in a human in need thereof wherein the combination comprises:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

In one embodiment, use of combinations is provided for treating non-Hodgkin's lymphoma in a human in need thereof wherein the combination comprises:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ ID.NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

Also provided in the present invention is use of a combination or pharmaceutical compositions of this invention in the manufacture of a medicament for the treatment of cancer such as in particular in the treatment of B cell disorders. In one embodiment the cancer is multiple myeloma. In another embodiment the cancer is non-Hodgkin's lymphoma.

In one embodiment, use of a combination or pharmaceutical compositions of this invention in the manufacture of a medicament for the treatment of cancer includes combinations comprising any one of the antigen binding proteins that bind BCMA of the present invention or antibody drug conjugates thereof; and pembrolizumab, or an antibody comprising 90%, 91%, 92%, 93%, 94%, 95%, 96%, 97%, 98%, 99% or 100% sequence homology thereto.

In one embodiment, use of a combination or pharmaceutical compositions of this invention in the manufacture of a medicament for the treatment of cancer includes combinations comprising any one of the antigen binding proteins that bind BCMA or immunoconjugates thereof; and nivolumab, or an antibody comprising 90%, 91%, 92%, 93%, 94%, 95%, 96%, 97%, 98%, 99% or 100% sequence homology thereto

In one embodiment, use of combinations in the manufacture of a medicament for the treatment of cancer is contemplated, wherein the combination comprises:
i) a therapeutically effective amount an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount an antigen binding protein that binds PD-1 comprising CDRH1 of SEQ. ID. NO: 201, CDRH2 of SEQ. ID. NO: 202, CDRH3 of SEQ. ID. NO: 203, CDRL1 of SEQ. ID. NO: 204, CDRL2 of SEQ. ID. NO: 205, CDRL3 of SEQ. ID. NO: 206, or variants thereof.

In one embodiment, use of combinations in the manufacture of a medicament for the treatment of cancer is contemplated, wherein the combination comprises:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ ID NO: 221, CDRL1 of SEQ. ID. NO: 222, CDRL2 of SEQ. ID. NO: 223, CDRL3 of SEQ. ID. NO: 224, or variants thereof.

In one embodiment, use of combinations in the manufacture of a medicament for the treatment of multiple myeloma is contemplated, wherein the combination comprises:
i) a therapeutically effective amount an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount an antigen binding protein that binds PD-1 comprising CDRH1 of SEQ. ID. NO: 201, CDRH2 of SEQ. ID. NO: 202, CDRH3 of SEQ. ID. NO: 203, CDRL1 of SEQ. ID. NO: 204, CDRL2 of SEQ. ID. NO: 205, CDRL3 of SEQ. ID. NO: 206, or variants thereof.

In one embodiment, use of combinations in the manufacture of a medicament for the treatment of non-Hodgkin's lymphoma is contemplated, wherein the combination comprises:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds PD-1 comprising CDRH1 of SEQ. ID. NO: 201, CDRH2 of SEQ. ID. NO: 202, CDRH3 of SEQ. ID. NO: 203, CDRL1 of SEQ. ID. NO: 204, CDRL2 of SEQ. ID. NO: 205, CDRL3 of SEQ. ID. NO: 206, or variants thereof.

In one embodiment, use of combinations in the manufacture of a medicament for the treatment of multiple myeloma is contemplated, wherein the combination comprises:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

In one embodiment, use of combinations in the manufacture of a medicament for the treatment of non-Hodgkin's lymphoma is contemplated, wherein the combination comprises:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

Also provided are pharmaceutical compositions comprising the combination of the present invention for treating cancer. The present invention also provides combination kit comprising pharmaceutical compositions of the invention together with one or more pharmaceutically acceptable carriers. The kit may optionally include instructions for use.

B-cell disorders can be divided into defects of B-cell development/immunoglobulin production (immunodeficiencies) and excessive/uncontrolled proliferation (lymphomas, leukemias). As used herein, B-cell disorder refers to both types of diseases, and methods are provided for treating B-cell disorders with an antigen binding protein.

Examples of cancers and in particular B-cell mediated or plasma cell mediated diseases or antibody mediated diseases or disorders include Multiple Myeloma (MM), chronic lymphocytic leukemia (CLL), Follicular Lymphoma (FL), Diffuse Large B-Cell Lymphoma (DLBCL), Non-secretory multiple myeloma, Smoldering multiple myeloma, Monoclonal gammopathy of undetermined significance (MGUS), Solitary plasmacytoma (Bone, Extramedullary), Lymphoplasmacytic lymphoma (LPL), Waldenström's Macroglobulinemia, Plasma cell leukemia,, Primary Amyloidosis (AL), Heavy chain disease, Systemic lupus erythematosus (SLE), POEMS syndrome / osteosclerotic myeloma, Type I and II cryoglobulinemia, Light chain deposition disease, Goodpasture's syndrome, Idiopathic thrombocytopenic purpura (ITP), Acute glomerulonephritis, Pemphigus and Pemphigoid disorders, and Epidermolysis bullosa acquisita; or any Non-Hodgkin's Lymphoma B-cell leukemia (NHL) or Hodgkin's lymphoma (HL).

In a particular embodiment, the disease or disorder is selected from the group consisting of Multiple Myeloma (MM),, Non-Hodgkin's Lymphoma B-cell leukemia (NHL), Follicular Lymphoma (FL), and Diffuse Large B-Cell Lymphoma (DLBCL).

In one embodiment of the present invention the disease is Multiple Myeloma, or Non-Hodgkin's Lymphoma B-cell leukemia (NHL).

In one embodiment of the present invention the disease is Multiple Myeloma.

Suitably, the present invention relates to a method for treating or lessening the severity of a cancer as herein described.

The combination of the invention may be used alone or in combination with one or more other therapeutic agents.

When a pharmaceutical composition or combination of the present invention is administered for the treatment of cancer, the term "administering" and derivatives thereof as used herein is meant either simultaneous administration or any manner of separate sequential administration of a combination, as described herein, and a further active ingredient or ingredients, known to be useful in the treatment of cancer, including chemotherapy (e.g., and anti-neoplastic agent), radiation treatment, and surgery. The term further active ingredient or ingredients, as used herein, includes any compound or therapeutic agent known to or that demonstrates advantageous properties when administered to a patient in need of treatment for cancer. Preferably, if the administration is not simultaneous, the compounds are administered in a close time proximity to each other. Furthermore, it does not matter if the compounds are administered in the same dosage form, e.g. one compound may be administered intravenously and another compound may be administered orally.

Typically, any anti-neoplastic agent that has activity versus a susceptible tumor being treated may be administered with the combinations described herein in the treatment of cancer in the present invention. Examples of such agents can be found in Cancer Principles and Practice f Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Typical anti-neoplastic agents useful in the present invention include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and anti-folate compounds; topoisomerase I inhibitors such as camptothecins; hormones and hormonal analogues; signal transduction pathway inhibitors; non-receptor tyrosine kinase angiogenesis inhibitors; immunotherapeutic agents; proapoptotic agents; and cell cycle signaling inhibitors. A non-limiting list of anti-neoplastic agents are provided herein.

Examples of a further active ingredient or ingredients for administration with the combinations described herein are chemotherapeutic agents.

Anti-microtubule or anti-mitotic agents are phase specific agents active against the microtubules of tumor cells during M or the mitosis phase of the cell cycle. Examples of anti-microtubule agents include, but are not limited to, diterpenoids and vinca alkaloids.

Diterpenoids, which are derived from natural sources, are phase specific anti -cancer agents that operate at the G₂/M phases of the cell cycle. It is believed that the diterpenoids stabilize the β-tubulin subunit of the microtubules, by binding with this protein. Disassembly of the protein appears then to be inhibited with mitosis being arrested and cell death following. Examples of diterpenoids include, but are not limited to, paclitaxel and its analog docetaxel.

Paclitaxel, 5β,20-epoxy-1,2α,4,7β,10β,13α-hexa-hydroxytax-11-en-9-one 4,10-diacetate 2-benzoate 13-ester with (2R,3S)-N-benzoyl-3-phenylisoserine; is a natural diterpene product isolated from the Pacific yew tree *Taxus brevifolia* and is commercially available as an injectable solution TAXOL^{®}. It is a member of the taxane family of terpenes. It was first isolated in 1971 by Wani et al. J. Am. Chem, Soc., 93:2325. 1971), who characterized its structure by chemical and X-ray crystallographic methods. One mechanism for its activity relates to paclitaxel's capacity to bind tubulin, thereby inhibiting cancer cell growth. Schiff et al., Proc. Natl, Acad, Sci. USA, 77:1561-1565 (1980); Schiff et al., Nature, 277:665-667 (1979); Kumar, J. Biol, Chem, 256: 10435-10441 (1981). For a review of synthesis and anticancer activity of some paclitaxel derivatives see: D. G. I. Kingston et al., Studies in Organic Chemistry vol. 26, entitled "New trends in Natural Products Chemistry 1986", Attaur-Rahman, P.W. Le Quesne, Eds. (Elsevier, Amsterdam, 1986) pp 219-235.

Paclitaxel has been approved for clinical use in the treatment of refractory ovarian cancer in the United States (Markman et al., Yale Journal of Biology and Medicine, 64:583, 1991; McGuire et al., Ann. Intem, Med., 111:273,1989) and for the treatment of breast cancer (Holmes et al., J. Nat. Cancer Inst., 83:1797,1991.) It is a potential candidate for treatment of neoplasms in the skin (Einzig et. al., Proc. Am. Soc. Clin. Oncol., 20:46) and head and neck carcinomas (Forastire et. al., Sem. Oncol., 20:56, 1990). The compound also shows potential for the treatment of polycystic kidney disease (Woo et. al., Nature, 368:750. 1994), lung cancer and malaria. Treatment of patients with paclitaxel results in bone marrow suppression (multiple cell lineages, Ignoff, R.J. et. al, Cancer Chemotherapy Pocket Guide, 1998) related to the duration of dosing above a threshold concentration (50nM) (Kearns, C.M. et. al., Seminars in Oncology, 3(6) p.16-23, 1995).

Docetaxel, (2R,3S)- N-carboxy-3-phenylisoserine, N-*tert*-butyl ester, 13-ester with 5β-20-epoxy-1,2α,4,7β,10β,13α-hexahydroxytax-11-en-9-one 4-acetate 2-benzoate, trihydrate; is commercially available as an injectable solution as TAXOTERE^{®}. Docetaxel is indicated for the treatment of breast cancer. Docetaxel is a semisynthetic derivative of paclitaxel *q.v.,* prepared using a natural precursor, 10-deacetyl-baccatin III, extracted from the needle of the European Yew tree. The dose limiting toxicity of docetaxel is neutropenia.

Vinca alkaloids are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include, but are not limited to, vinblastine, vincristine, and vinorelbine.

Vinblastine, vincaleukoblastine sulfate, is commercially available as VELBAN^{®} as an injectable solution. Although, it has possible indication as a second line therapy of various solid tumors, it is primarily indicated in the treatment of testicular cancer and various lymphomas including Hodgkin's Disease; and lymphocytic and histiocytic lymphomas. Myelosuppression is the dose limiting side effect of vinblastine.

Vincristine, vincaleukoblastine, 22-oxo-, sulfate, is commercially available as ONCOVIN^{®} as an injectable solution. Vincristine is indicated for the treatment of acute leukemias and has also found use in treatment regimens for Hodgkin's and non-Hodgkin's malignant lymphomas. Alopecia and neurologic effects are the most common side effect of vincristine and to a lesser extent myelosupression and gastrointestinal mucositis effects occur.

Vinorelbine, 3',4'-didehydro -4'-deoxy-C'-norvincaleukoblastine [R-(R^{∗},R^{∗})-2,3-dihydroxybutanedioate (1:2)(salt)], commercially available as an injectable solution of vinorelbine tartrate (NAVELBINE^{®}), is a semisynthetic vinca alkaloid. Vinorelbine is indicated as a single agent or in combination with other chemotherapeutic agents, such as cisplatin, in the treatment of various solid tumors, particularly non-small cell lung, advanced breast, and hormone refractory prostate cancers. Myelosuppression is the most common dose limiting side effect of vinorelbine.

Platinum coordination complexes are non-phase specific anti-cancer agents, which are interactive with DNA. The platinum complexes enter tumor cells, undergo, aquation and form intra- and interstrand crosslinks with DNA causing adverse biological effects to the tumor. Examples of platinum coordination complexes include, but are not limited to, cisplatin and carboplatin.

Cisplatin, cis-diamminedichloroplatinum, is commercially available as PLATINOL^{®} as an injectable solution. Cisplatin is primarily indicated in the treatment of metastatic testicular and ovarian cancer and advanced bladder cancer. The primary dose limiting side effects of cisplatin are nephrotoxicity, which may be controlled by hydration and diuresis, and ototoxicity.

Carboplatin, platinum, diammine [1,1-cyclobutane-dicarboxylate(2-)-O,O'], is commercially available as PARAPLATIN^{®} as an injectable solution. Carboplatin is primarily indicated in the first and second line treatment of advanced ovarian carcinoma. Bone marrow suppression is the dose limiting toxicity of carboplatin.

Alkylating agents are non-phase anti-cancer specific agents and strong electrophiles. Typically, alkylating agents form covalent linkages, by alkylation, to DNA through nucleophilic moieties of the DNA molecule such as phosphate, amino, sulfhydryl, hydroxyl, carboxyl, and imidazole groups. Such alkylation disrupts nucleic acid function leading to cell death. Examples of alkylating agents include, but are not limited to, nitrogen mustards such as cyclophosphamide, melphalan, and chlorambucil; alkyl sulfonates such as busulfan; nitrosoureas such as carmustine; and triazenes such as dacarbazine.

Cyclophosphamide, 2-[bis(2-chloroethyl)amino]tetrahydro-2H-1,3,2-oxazaphosphorine 2-oxide monohydrate, is commercially available as an injectable solution or tablets as CYTOXAN^{®}. Cyclophosphamide is indicated as a single agent or in combination with other chemotherapeutic agents, in the treatment of malignant lymphomas, multiple myeloma, and leukemias. Alopecia, nausea, vomiting and leukopenia are the most common dose limiting side effects of cyclophosphamide.

Melphalan, 4-[bis(2-chloroethyl)amino]-L-phenylalanine, is commercially available as an injectable solution or tablets as ALKERAN^{®}. Melphalan is indicated for the palliative treatment of multiple myeloma and non-resectable epithelial carcinoma of the ovary. Bone marrow suppression is the most common dose limiting side effect of melphalan.

Chlorambucil, 4-[bis(2-chloroethyl)amino]benzenebutanoic acid, is commercially available as LEUKERAN^{®} tablets. Chlorambucil is indicated for the palliative treatment of chronic lymphatic leukemia, and malignant lymphomas such as lymphosarcoma, giant follicular lymphoma, and Hodgkin's disease. Bone marrow suppression is the most common dose limiting side effect of chlorambucil.

Busulfan, 1,4-butanediol dimethanesulfonate, is commercially available as MYLERAN^{®} TABLETS. Busulfan is indicated for the palliative treatment of chronic myelogenous leukemia. Bone marrow suppression is the most common dose limiting side effects of busulfan.

Carmustine, 1,3-[bis(2-chloroethyl)-1-nitrosourea, is commercially available as single vials of lyophilized material as BiCNU^{®}. Carmustine is indicated for the palliative treatment as a single agent or in combination with other agents for brain tumors, multiple myeloma, Hodgkin's disease, and non-Hodgkin's lymphomas. Delayed myelosuppression is the most common dose limiting side effects of carmustine.

Dacarbazine, 5-(3,3-dimethyl-1-triazeno)-imidazole-4-carboxamide, is commercially available as single vials of material as DTIC-Dome^{®}. Dacarbazine is indicated for the treatment of metastatic malignant melanoma and in combination with other agents for the second line treatment of Hodgkin's Disease. Nausea, vomiting, and anorexia are the most common dose limiting side effects of dacarbazine.

Antibiotic anti-neoplastics are non-phase specific agents, which bind or intercalate with DNA. Typically, such action results in stable DNA complexes or strand breakage, which disrupts ordinary function of the nucleic acids leading to cell death. Examples of antibiotic anti-neoplastic agents include, but are not limited to, actinomycins such as dactinomycin, anthrocyclins such as daunorubicin and doxorubicin; and bleomycins.

Dactinomycin, also known as Actinomycin D, is commercially available in injectable form as COSMEGEN^{®}. Dactinomycin is indicated for the treatment of Wilm's tumor and rhabdomyosarcoma. Nausea, vomiting, and anorexia are the most common dose limiting side effects of dactinomycin.

Daunorubicin, (8S-cis-)-8-acetyl-10-[(3-amino-2,3,6-trideoxy-α-L-lyxo-hexopyranosyl)oxy]-7,8,9,10-tetrahydro-6,8,11-trihydroxy-1-methoxy-5,12 naphthacenedione hydrochloride, is commercially available as a liposomal injectable form as DAUNOXOME^{®} or as an injectable as CERUBIDINE^{®}. Daunorubicin is indicated for remission induction in the treatment of acute nonlymphocytic leukemia and advanced HIV associated Kaposi's sarcoma. Myelosuppression is the most common dose limiting side effect of daunorubicin.

Doxorubicin, (8S, 10S)-10-[(3-amino-2,3,6-trideoxy-α-L-lyxo-hexopyranosyl)oxy]-8-glycoloyl, 7,8,9,10-tetrahydro-6,8,11-trihydroxy-1-methoxy-5,12 naphthacenedione hydrochloride, is commercially available as an injectable form as RUBEX^{®} or ADRIAMYCIN RDF^{®}. Doxorubicin is primarily indicated for the treatment of acute lymphoblastic leukemia and acute myeloblastic leukemia, but is also a useful component in the treatment of some solid tumors and lymphomas. Myelosuppression is the most common dose limiting side effect of doxorubicin.

Bleomycin, a mixture of cytotoxic glycopeptide antibiotics isolated from a strain of *Streptomyces verticillus,* is commercially available as BLENOXANE^{®}. Bleomycin is indicated as a palliative treatment, as a single agent or in combination with other agents, of squamous cell carcinoma, lymphomas, and testicular carcinomas. Pulmonary and cutaneous toxicities are the most common dose limiting side effects of bleomycin.

Topoisomerase II inhibitors include, but are not limited to, epipodophyllotoxins.

Epipodophyllotoxins are phase specific anti-neoplastic agents derived from the mandrake plant. Epipodophyllotoxins typically affect cells in the S and G₂ phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9[4,6-0-(R)-ethylidene-β-D-glucopyranoside], is commercially available as an injectable solution or capsules as VePESID^{®} and is commonly known as VP-16. Etoposide is indicated as a single agent or in combination with other chemotherapy agents in the treatment of testicular and non-small cell lung cancers. Myelosuppression is the most common side effect of etoposide. The incidence of leucopenia tends to be more severe than thrombocytopenia.

Teniposide, 4'-demethyl-epipodophyllotoxin 9[4,6-0-(R)-thenylidene-β-D-glucopyranoside], is commercially available as an injectable solution as VUMON^{®} and is commonly known as VM-26. Teniposide is indicated as a single agent or in combination with other chemotherapy agents in the treatment of acute leukemia in children. Myelosuppression is the most common dose limiting side effect of teniposide. Teniposide can induce both leucopenia and thrombocytopenia.

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to, fluorouracil, methotrexate, cytarabine, mecaptopurine, thioguanine, and gemcitabine.

5-fluorouracil, 5-fluoro-2,4- (1H,3H) pyrimidinedione, is commercially available as fluorouracil. Administration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Myelosuppression and mucositis are dose limiting side effects of 5-fluorouracil. Other fluoropyrimidine analogs include 5-fluoro deoxyuridine (floxuridine) and 5-fluorodeoxyuridine monophosphate.

Cytarabine, 4-amino-1-β-D-arabinofuranosyl-2 (1H)-pyrimidinone, is commercially available as CYTOSAR-U^{®} and is commonly known as Ara-C. It is believed that cytarabine exhibits cell phase specificity at S-phase by inhibiting DNA chain elongation by terminal incorporation of cytarabine into the growing DNA chain. Cytarabine is indicated as a single agent or in combination with other chemotherapy agents in the treatment of acute leukemia. Other cytidine analogs include 5-azacytidine and 2',2'-difluorodeoxycytidine (gemcitabine). Cytarabine induces leucopenia, thrombocytopenia, and mucositis.

Mercaptopurine, 1,7-dihydro-6H-purine-6-thione monohydrate, is commercially available as PURINETHOL^{®}. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. Myelosuppression and gastrointestinal mucositis are expected side effects of mercaptopurine at high doses. A useful mercaptopurine analog is azathioprine.

Thioguanine, 2-amino-1,7-dihydro-6H-purine-6-thione, is commercially available as TABLOID^{®}. Thioguanine exhibits cell phase specificity at S-phase by inhibiting DNA synthesis by an as of yet unspecified mechanism. Thioguanine is indicated as a single agent or in combination with other chemotherapy agents in the treatment of acute leukemia. Myelosuppression, including leucopenia, thrombocytopenia, and anemia, is the most common dose limiting side effect of thioguanine administration. However, gastrointestinal side effects occur and can be dose limiting. Other purine analogs include pentostatin, erythrohydroxynonyladenine, fludarabine phosphate, and cladribine.

Gemcitabine, 2'-deoxy-2', 2'-difluorocytidine monohydrochloride (β-isomer), is commercially available as GEMZAR^{®}. Gemcitabine exhibits cell phase specificity at S-phase and by blocking progression of cells through the G1/S boundary. Gemcitabine is indicated in combination with cisplatin in the treatment of locally advanced non-small cell lung cancer and alone in the treatment of locally advanced pancreatic cancer. Myelosuppression, including leucopenia, thrombocytopenia, and anemia, is the most common dose limiting side effect of gemcitabine administration.

Methotrexate, N-[4[[(2,4-diamino-6-pteridinyl) methyl]methylamino] benzoyl]-L-glutamic acid, is commercially available as methotrexate sodium. Methotrexate exhibits cell phase effects specifically at S-phase by inhibiting DNA synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarcinoma, meningeal leukemia, non-Hodgkin's lymphoma, and carcinomas of the breast, head, neck, ovary and bladder. Myelosuppression (leucopenia, thrombocytopenia, and anemia) and mucositis are expected side effect of methotrexate administration.

Camptothecins, including, camptothecin and camptothecin derivatives are available or under development as Topoisomerase I inhibitors. Camptothecins cytotoxic activity is believed to be related to its Topoisomerase I inhibitory activity. Examples of camptothecins include, but are not limited to irinotecan, topotecan, and the various optical forms of 7-(4-methylpiperazino-methylene)-10,11-ethylenedioxy-20-camptothecin described below.

Irinotecan HCl, (4S)-4,11-diethyl-4-hydroxy-9-[(4-piperidinopiperidino) carbonyloxy]-1H-pyrano[3',4',6,7]indolizino[1,2-b]quinoline-3,14(4H,12H)-dione hydrochloride, is commercially available as the injectable solution CAMPTOSAR^{®}.

Irinotecan is a derivative of camptothecin which binds, along with its active metabolite SN-38, to the topoisomerase I - DNA complex. It is believed that cytotoxicity occurs as a result of irreparable double strand breaks caused by interaction of the topoisomerase I: DNA: irintecan or SN-38 ternary complex with replication enzymes. Irinotecan is indicated for treatment of metastatic cancer of the colon or rectum. The dose limiting side effects of irinotecan HCl are myelosuppression, including neutropenia, and GI effects, including diarrhea.

Topotecan HCl, (S)-10-[(dimethylamino)methyl]-4-ethyl-4,9-dihydroxy-1H-pyrano[3',4',6,7]indolizino[1,2-b]quinoline-3,14-(4H,12H)-dione monohydrochloride, is commercially available as the injectable solution HYCAMTIN^{®}. Topotecan is a derivative of camptothecin which binds to the topoisomerase I - DNA complex and prevents religation of singles strand breaks caused by Topoisomerase I in response to torsional strain of the DNA molecule. Topotecan is indicated for second line treatment of metastatic carcinoma of the ovary and small cell lung cancer. The dose limiting side effect of topotecan HCl is myelosuppression, primarily neutropenia.

Also of interest, is the camptothecin derivative of formula A following, currently under development, including the racemic mixture (R,S) form as well as the R and S enantiomers: 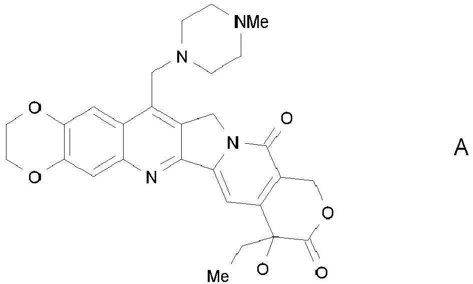 known by the chemical name "7-(4-methylpiperazino-methylene)-10,11-ethylenedioxy-20(R,S)-camptothecin (racemic mixture) or "7-(4-methylpiperazino-methylene)-10,11-ethylenedioxy-20(R)-camptothecin (R enantiomer) or "7-(4-methylpiperazino-methylene)-10,11-ethylenedioxy-20(S)-camptothecin (S enantiomer). Such compound as well as related compounds are described, including methods of making, in U.S. Patent Nos. 6,063,923; 5,342,947; 5,559,235; 5,491,237 and pending U.S. patent Application No. 08/977,217 filed November 24, 1997.

Hormones and hormonal analogues are useful compounds for treating cancers in which there is a relationship between the hormone(s) and growth and/or lack of growth of the cancer. Examples of hormones and hormonal analogues useful in cancer treatment include, but are not limited to, adrenocorticosteroids such as prednisone and prednisolone which are useful in the treatment of malignant lymphoma and acute leukemia in children; aminoglutethimide and other aromatase inhibitors such as anastrozole, letrazole, vorazole, and exemestane useful in the treatment of adrenocortical carcinoma and hormone dependent breast carcinoma containing estrogen receptors; progestrins such as megestrol acetate useful in the treatment of hormone dependent breast cancer and endometrial carcinoma; estrogens, androgens, and anti-androgens such as flutamide, nilutamide, bicalutamide, cyproterone acetate and 5α-reductases such as finasteride and dutasteride, useful in the treatment of prostatic carcinoma and benign prostatic hypertrophy; anti-estrogens such as tamoxifen, toremifene, raloxifene, droloxifene, iodoxyfene, as well as selective estrogen receptor modulators (SERMS) such those described in U.S. Patent Nos. 5,681,835, 5,877,219, and 6,207,716, useful in the treatment of hormone dependent breast carcinoma and other susceptible cancers; and gonadotropin-releasing hormone (GnRH) and analogues thereof which stimulate the release of leutinizing hormone (LH) and/or follicle stimulating hormone (FSH) for the treatment prostatic carcinoma, for instance, LHRH agonists and antagagonists such as goserelin acetate and luprolide.

Letrozole (trade name Femara) is an oral non-steroidal aromatase inhibitor for the treatment of hormonally-responsive breast cancer after surgery. Estrogens are produced by the conversion of androgens through the activity of the aromatase enzyme. Estrogens then bind to an estrogen receptor, which causes cells to divide. Letrozole prevents the aromatase from producing estrogens by competitive, reversible binding to the heme of its cytochrome P450 unit. The action is specific, and letrozole does not reduce production of mineralo- or corticosteroids.

Signal transduction pathway inhibitors are those inhibitors, which block or inhibit a chemical process which evokes an intracellular change. As used herein this change is cell proliferation or differentiation. Signal tranduction inhibitors useful in the present invention include inhibitors of receptor tyrosine kinases, non-receptor tyrosine kinases, SH2/SH3domain blockers, serine/threonine kinases, phosphotidyl inositol-3 kinases, myo-inositol signaling, and Ras oncogenes.

Several protein tyrosine kinases catalyse the phosphorylation of specific tyrosyl residues in various proteins involved in the regulation of cell growth. Such protein tyrosine kinases can be broadly classified as receptor or non-receptor kinases.

Receptor tyrosine kinases are transmembrane proteins having an extracellular ligand binding domain, a transmembrane domain, and a tyrosine kinase domain. Receptor tyrosine kinases are involved in the regulation of cell growth and are generally termed growth factor receptors. Inappropriate or uncontrolled activation of many of these kinases, i.e. aberrant kinase growth factor receptor activity, for example by over-expression or mutation, has been shown to result in uncontrolled cell growth. Accordingly, the aberrant activity of such kinases has been linked to malignant tissue growth. Consequently, inhibitors of such kinases could provide cancer treatment methods. Growth factor receptors include, for example, epidermal growth factor receptor (EGFr), platelet derived growth factor receptor (PDGFr), erbB2, erbB4, vascular endothelial growth factor receptor (VEGFr), tyrosine kinase with immunoglobulin-like and epidermal growth factor homology domains (TIE-2), insulin growth factor -I (IGFI) receptor, macrophage colony stimulating factor (cfms), BTK, ckit, cmet, fibroblast growth factor (FGF) receptors, Trk receptors (TrkA, TrkB, and TrkC), ephrin (eph) receptors, and the RET protooncogene. Several inhibitors of growth receptors are under development and include ligand antagonists, antibodies, tyrosine kinase inhibitors and anti-sense oligonucleotides. Growth factor receptors and agents that inhibit growth factor receptor function are described, for instance, in Kath, John C., Exp. Opin. Ther. Patents (2000) 10(6):803-818; Shawver et al DDT Vol 2, No. 2 February 1997; and Lofts, F. J. et al, "Growth factor receptors as targets", New Molecular Targets for Cancer Chemotherapy, ed. Workman, Paul and Kerr, David, CRC press 1994, London.

Tyrosine kinases, which are not growth factor receptor kinases are termed non-receptor tyrosine kinases. Non-receptor tyrosine kinases useful in the present invention, which are targets or potential targets of anti-cancer drugs, include cSrc, Lck, Fyn, Yes, Jak, cAbl, FAK (Focal adhesion kinase), Brutons tyrosine kinase, and Bcr-Abl. Such non-receptor kinases and agents which inhibit non-receptor tyrosine kinase function are described in Sinh, S. and Corey, S.J., (1999) Journal of Hematotherapy and Stem Cell Research 8 (5): 465 - 80; and Bolen, J.B., Brugge, J.S., (1997) Annual review of Immunology. 15: 371-404.

SH2/SH3 domain blockers are agents that disrupt SH2 or SH3 domain binding in a variety of enzymes or adaptor proteins including, PI3-K p85 subunit, Src family kinases, adaptor molecules (Shc, Crk, Nck, Grb2) and Ras-GAP. SH2/SH3 domains as targets for anti-cancer drugs are discussed in Smithgall, T.E. (1995), Journal of Pharmacological and Toxicological Methods. 34(3) 125-32.

Inhibitors of Serine/Threonine Kinases including MAP kinase cascade blockers which include blockers of Raf kinases (rafk), Mitogen or Extracellular Regulated Kinase (MEKs), and Extracellular Regulated Kinases (ERKs); and Protein kinase C family member blockers including blockers of PKCs (alpha, beta, gamma, epsilon, mu, lambda, iota, zeta). IkB kinase family (IKKa, IKKb), PKB family kinases, AKT kinase family members, and TGF beta receptor kinases. Such Serine/Threonine kinases and inhibitors thereof are described in Yamamoto, T., Taya, S., Kaibuchi, K., (1999), Journal of Biochemistry. 126 (5) 799-803; Brodt, P, Samani, A., and Navab, R. (2000), Biochemical Pharmacology, 60. 1101-1107; Massague, J., Weis-Garcia, F. (1996) Cancer Surveys. 27:41-64; Philip, P.A., and Harris, A.L. (1995), Cancer Treatment and Research. 78: 3-27, Lackey, K. et al Bioorganic and Medicinal Chemistry Letters, (10), 2000, 223-226; U.S. Patent No. 6,268,391; and Martinezlacaci, L., et al, Int. J. Cancer (2000), 88(1), 44-52.

Inhibitors of Phosphotidyl inositol-3 Kinase family members including blockers of PI3-kinase, ATM, DNA-PK, and Ku are also useful in the present invention. Such kinases are discussed in Abraham, R.T. (1996), Current Opinion in Immunology. 8 (3) 412-8; Canman, C.E., Lim, D.S. (1998), Oncogene 17 (25) 3301-3308; Jackson, S.P. (1997), International Journal of Biochemistry and Cell Biology. 29 (7):935-8; and Zhong, H. et al, Cancer res, (2000) 60(6), 1541-1545.

Also useful in the present invention are Myo-inositol signaling inhibitors such as phospholipase C blockers and Myoinositol analogues. Such signal inhibitors are described in Powis, G., and Kozikowski A., (1994) New Molecular Targets for Cancer Chemotherapy ed., Paul Workman and David Kerr, CRC press 1994, London.

Another group of signal transduction pathway inhibitors are inhibitors of Ras Oncogene. Such inhibitors include inhibitors of farnesyltransferase, geranyl-geranyl transferase, and CAAX proteases as well as anti-sense oligonucleotides, ribozymes and immunotherapy. Such inhibitors have been shown to block ras activation in cells containing wild type mutant ras, thereby acting as antiproliferation agents. Ras oncogene inhibition is discussed in Scharovsky, O.G., Rozados, V.R., Gervasoni, S.I. Matar, P. (2000), Journal of Biomedical Science. 7(4) 292-8; Ashby, M.N. (1998), Current Opinion in Lipidology. 9 (2) 99 - 102; and Bennett, C.F. and Cowsert, L.M. BioChim. Biophys. Acta, (1999) 1489(1):19-30.

As mentioned above, antibody antagonists to receptor kinase ligand binding may also serve as signal transduction inhibitors. This group of signal transduction pathway inhibitors includes the use of humanized antibodies to the extracellular ligand binding domain of receptor tyrosine kinases. For example Imclone C225 EGFR specific antibody (see Green, M.C. et al, Monoclonal Antibody Therapy for Solid Tumors, Cancer Treat. Rev., (2000), 26(4), 269-286); Herceptin^{®} erbB2 antibody (see Tyrosine Kinase Signalling in Breast cancer:erbB Family Receptor Tyrosine Kniases, Breast cancer Res., 2000, 2(3), 176-183); and 2CB VEGFR2 specific antibody (see Brekken, R.A. et al, Selective Inhibition of VEGFR2 Activity by a monoclonal Anti-VEGF antibody blocks tumor growth in mice, Cancer Res. (2000) 60, 5117-5124).

Non-receptor kinase angiogenesis inhibitors may also find use in the present invention. Inhibitors of angiogenesis related VEGFR and TIE2 are discussed above in regard to signal transduction inhibitors (both receptors are receptor tyrosine kinases). Angiogenesis in general is linked to erbB2/EGFR signaling since inhibitors of erbB2 and EGFR have been shown to inhibit angiogenesis, primarily VEGF expression. Thus, the combination of an erbB2/EGFR inhibitor with an inhibitor of angiogenesis makes sense. Accordingly, non-receptor tyrosine kinase inhibitors may be used in combination with the EGFR/erbB2 inhibitors of the present invention. For example, anti-VEGF antibodies, which do not recognize VEGFR (the receptor tyrosine kinase), but bind to the ligand; small molecule inhibitors of integrin (alphaᵥ beta₃) that will inhibit angiogenesis; endostatin and angiostatin (non-RTK) may also prove useful in combination with the disclosed erb family inhibitors. (See Bruns CJ et al (2000), Cancer Res., 60: 2926-2935; Schreiber AB, Winkler ME, and Derynck R. (1986), Science, 232: 1250-1253; Yen L et al. (2000), Oncogene 19: 3460-3469).

Pazopanib which commercially available as VOTRIENT^{®} is a tyrosine kinase inhibitor (TKI). Pazopanib is presented as the hydrochloride salt, with the chemical name 5-[[4-[(2,3-dimethyl-2H-indazol-6-yl)methylamino]-2-pyrimidinyl]amino]-2-methylbenzenesulfonamide monohydrochloride. Pazoponib is approved for treatment of patients with advanced renal cell carcinoma.

Bevacisumab which is commercially available as AVASTIN^{®} is a humanized monoclonal antibody that blocks VEGF-A. AVASTIN^{®} is approved form the treatment of various cancers including colorectal, lung, breast, kidney, and glioblastomas.

Rituximab is a chimeric monoclonal antibody which is sold as RITUXAN^{®} and MABTHERA^{®}. Rituximab binds to CD20 on B cells and causes cell apoptosis. Rituximab is administered intravenously and is approved for treatment of rheumatoid arthritis and B-cell non-Hodgkin's lymphoma.

Ofatumumab is a fully human monoclonal antibody which is sold as ARZERRA^{®}. Ofatumumab binds to CD20 on B cells and is used to treat chronic lymphocytic leukemia CLL; a type of cancer of the white blood cells) in adults who are refractory to treatment with fludarabine (Fludara) and alemtuzumab Campath).

Trastuzumab (HEREPTIN^{®}) is a humanized monoclonal antibody that binds to the HER2 receptor. It original indication is HER2 positive breast cancer. Trastuzumab emtansine (trade name Kadcyla) is anantibody-drug conjugate consisting of the monoclonal
antibody trastuzumab (Herceptin) linked to the cytotoxic agent DM1. Trastuzumab alone stops growth of cancer cells by binding to the HER2/neu receptor, whereas DM1 enters cells and destroys them by binding to tubulin. Because the monoclonal antibody targets HER2, and HER2 is only over-expressed in cancer cells, the conjugate delivers the toxin specifically to tumor cells.^{[8]} The conjugate is abbreviated T-DM1.

Cetuximab (ERBITUX^{®}) is a chimeric mouse human antibody that inhibits epidermal growth factor receptor (EGFR).

mTOR inhibitors include but are not limited to rapamycin (FK506) and rapalogs, RAD001 or everolimus (Afinitor), CCI-779 or temsirolimus, AP23573, AZD8055, WYE-354, WYE-600, WYE-687 and Pp121.

Everolimus is sold as Afinitor^{®} by Novartis and is the 40-O-(2-hydroxyethyl) derivative of sirolimus and works similarly to sirolimus as an mTOR (mammalian target of rapamycin) inhibitor. It is currently used as an immunosuppressant to prevent rejection of organ transplants and treatment of renal cell cancer. Much research has also been conducted on everolimus and other mTOR inhibitors for use in a number of cancers. It has the following chemical structure (formula II) and chemical name: 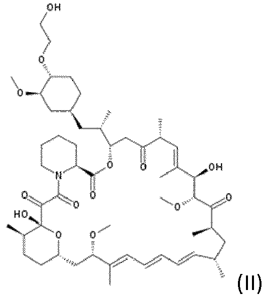 dihydroxy-12-[(2*R*)-1-[(1*S*,3*R*,4*R*)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0^{4,9}]hexatriaconta-16,24,26,28-tetraene-2,3,10,14,20-pentone.

Bexarotene is sold as Targretin^{®} and is a member of a subclass of retinoids that selectively activate retinoid X receptors (RXRs). These retinoid receptors have biologic activity distinct from that of retinoic acid receptors (RARs). The chemical name is 4-[1-(5,6,7,8-tetrahydro-3,5,5,8,8-pentamethyl-2-naphthalenyl) ethenyl] benzoic acid. Bexarotene is used to treat cutaneous T-cell lymphoma (CTCL, a type of skin cancer) in people whose disease could not be treated successfully with at least one other medication.

Sorafenib marketed as Nexavar^{®} is in a class of medications called multikinase inhibitors. Its chemical name is 4-[4-[[4-chloro-3-(trifluoromethyl)phenyl]carbamoylamino] phenoxy]-*N*-methylpyridine-2-carboxamide. Sorafenib is used to treat advanced renal cell carcinoma (a type of cancer that begins in the kidneys). Sorafenib is also used to treat unresectable hepatocellular carcinoma (a type of liver cancer that cannot be treated with surgery).

Agents used in immunotherapeutic regimens may also be useful in combination with the compounds of formula (I). There are a number of immunologic strategies to generate an immune response against erbB2 or EGFR. These strategies are generally in the realm of tumor vaccinations. The efficacy of immunologic approaches may be greatly enhanced through combined inhibition of erbB2/EGFR signaling pathways using a small molecule inhibitor. Discussion of the immunologic/tumor vaccine approach against erbB2/EGFR are found in Reilly RT et al. (2000), Cancer Res. 60: 3569-3576; and Chen Y, Hu D, Eling DJ, Robbins J, and Kipps TJ. (1998), Cancer Res. 58: 1965-1971.

Examples of erbB inhibitors include lapatinib, erlotinib, and gefitinib. Lapatinib, *N*-(3-chloro-4-{[(3-fluorophenyl)methyl]oxy}phenyl)-6-[5-({[2-(methylsulfonyl)ethyl]amino}methyl)-2-furanyl]-4-quinazolinamine (represented by Formula III, as illustrated), is a potent, oral, small-molecule, dual inhibitor of erbB-1 and erbB-2 (EGFR and HER2) tyrosine kinases that is approved in combination with capecitabine for the treatment of HER2-positive metastatic breast cancer. 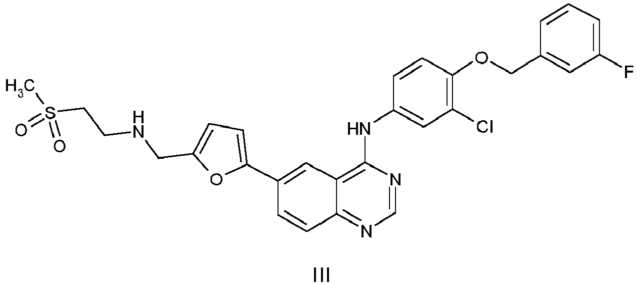 The free base, HCl salts, and ditosylate salts of the compound of formula (III) may be prepared according to the procedures disclosed in WO 99/35146, published July 15, 1999; and WO 02/02552 published January 10, 2002.

Erlotinib, *N*-(3-ethynylphenyl)-6,7-bis{[2-(methyloxy)ethyl]oxy}-4-quinazolinamine Commercially available under the tradename Tarceva) is represented by formula IV, as illustrated: 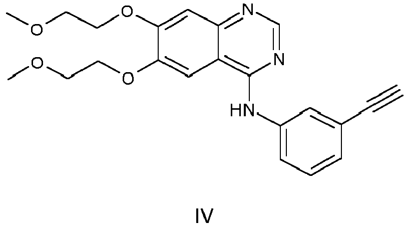 The free base and HCl salt of erlotinib may be prepared, for example, according to U.S. 5,747,498, Example 20.

Gefitinib, 4-quinazolinamine, N-(3-chloro-4-fluorophenyl)-7-methoxy-6-[3-4-morpholin)propoxy] is represented by formula V, as illustrated: 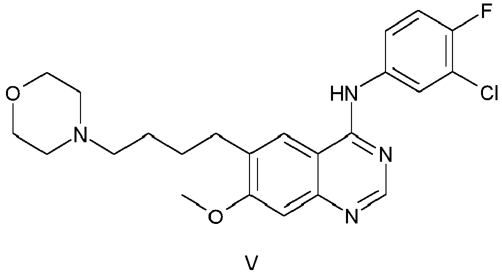 Gefitinib, which is commercially available under the trade name IRESSA^{®} (Astra-Zenenca) is an erbB-1 inhibitor that is indicated as monotherapy for the treatment of patients with locally advanced or metastatic non-small-cell lung cancer after failure of both platinum-based and docetaxel chemotherapies. The free base, HCl salts, and diHCl salts of gefitinib may be prepared according to the procedures of International Patent Application No. PCT/GB96/00961, filed April 23, 1996, and published as WO 96/33980 on October 31, 1996.

Trastuzumab (HEREPTIN^{®}) is a humanized monoclonal antibody that binds to the HER2 receptor. It original indication is HER2 positive breast cancer.

Cetuximab (ERBITUX^{®}) is a chimeric mouse human antibody that inhibits epidermal growth factor receptor (EGFR).

Pertuzumab (also called 2C4, trade name Omnitarg) is a monoclonal antibody. The first of its class in a line of agents called "HER dimerization inhibitors". By binding to HER2, it inhibits the dimerization of HER2 with other HER receptors, which is hypothesized to result in slowed tumor growth. Pertuzumab is described in WO01/00245 published January 4, 2001.

Rituximab is a chimeric monoclonal antibody which is sold as RITUXAN^{®} and MABTHERA^{®}. Rituximab binds to CD20 on B cells and causes cell apoptosis. Rituximab is administered intravenously and is approved for treatment of rheumatoid arthritis and B-cell non-Hodgkin's lymphoma.

Ofatumumab is a fully human monoclonal antibody which is sold as ARZERRA^{®}. Ofatumumab binds to CD20 on B cells and is used to treat chronic lymphocytic leukemia (CLL; a type of cancer of the white blood cells) in adults who are refractory to treatment with fludarabine (Fludara) and alemtuzumab (Campath).

Agents used in proapoptotic regimens (e.g., bcl-2 antisense oligonucleotides) may also be used in the combination of the present invention. Members of the Bcl-2 family of proteins block apoptosis. Upregulation of bcl-2 has therefore been linked to chemoresistance. Studies have shown that the epidermal growth factor (EGF) stimulates anti-apoptotic members of the bcl-2 family (i.e., mcl-1). Therefore, strategies designed to downregulate the expression of bcl-2 in tumors have demonstrated clinical benefit and are now in Phase II/III trials, namely Genta's G3139 bcl-2 antisense oligonucleotide. Such proapoptotic strategies using the antisense oligonucleotide strategy for bcl-2 are discussed in Water JS et al. (2000), J. Clin. Oncol. 18: 1812-1823; and Kitada S et al. (1994), Antisense Res. Dev. 4: 71-79.

Cell cycle signalling inhibitors inhibit molecules involved in the control of the cell cycle. A family of protein kinases called cyclin dependent kinases (CDKs) and their interaction with a family of proteins termed cyclins controls progression through the eukaryotic cell cycle. The coordinate activation and inactivation of different cyclin/CDK complexes is necessary for normal progression through the cell cycle. Several inhibitors of cell cycle signalling are under development. For instance, examples of cyclin dependent kinases, including CDK2, CDK4, and CDK6 and inhibitors for the same are described in, for instance, Rosania et al, Exp. Opin. Ther. Patents (2000) 10(2):215-230.

As used herein "immuno-modulators" refer to any substance including monoclonal antibodies that effects the immune system. The PD-1 and OX40 antigen binding proteins of the present invention can be considered immune-modulators. Immuno-modulators can be used as anti-neoplastic agents for the treatment of cancer. For example, immune-modulators include, but are not limited to, anti-CTLA-4 antibodies such as ipilimumab (YERVOY^{®}) and anti-PD-1 antibodies (Opdivo/nivolumab and Keytruda/pembrolizumab). Other immuno-modulators include, but are not limited to, OX-40 antibodies, PD-L1 antibodies, LAG3 antibodies, TIM-3 antibodies, 41BB antibodies and GITR antibodies.

YERVOY^{®} (ipilimumab) is a fully human CTLA-4 antibody marketed by Bristol Myers Squibb. The protein structure of ipilimumab and methods are using are described in US Patent Nos. 6,984,720 and 7,605,238.

OPDIVO^{®}/nivolumab is a fully human monoclonal antibody marketed by Bristol Myers Squibb directed against the negative immunoregulatory human cell surface receptor PD-1 (programmed death-1 or programmed cell death-1/PCD-1) with immunopotentiation activity. Nivolumab binds to and blocks the activation of PD-1, an Ig superfamily transmembrane protein, by its ligands PD-L1 and PD-L2, resulting in the activation of T-cells and cell-mediated immune responses against tumor cells or pathogens. Activated PD-1 negatively regulates T-cell activation and effector function through the suppression of P13k/Akt pathway activation. Other names for nivolumab include: BMS-936558, MDX-1106, and ONO-4538. The amino acid sequence for nivolumab and methods of using and making are disclosed in US Patent No. US 8,008,449.

KEYTRUDA^{®}/pembrolizumab is an anti-PD-1 antibodies marketed for the treatment of lung cancer by Merck. The amino acid sequence of pembrolizumab and methods of using are disclosed in US Patent No. 8,168,757.

CD134, also known as OX40, is a member of the TNFR-superfamily of receptors which is not constitutively expressed on resting naive T cells, unlike CD28. OX40 is a secondary costimulatory molecule, expressed after 24 to 72 hours following activation; its ligand, OX40L, is also not expressed on resting antigen presenting cells, but is following their activation. Expression of OX40 is dependent on full activation of the T cell; without CD28, expression of OX40 is delayed and of fourfold lower levels. OX-40 antibodies, OX-40 fusion proteins and methods of using them are disclosed in US Patent Nos: US 7,504,101; US 7,758,852; US 7,858,765; US 7,550,140; US 7,960,515; WO2012027328; WO2013028231.

Antibodies to PD-L1 (also referred to as CD274 or B7-H1) and methods for use are disclosed in US Patent No. 7,943,743; US Patent No. 8,383,796; US20130034559, WO2014055897, US Patent No. 8,168,179; and US Patent No. 7,595,048. PD-L1 antibodies are in development as immuno-modulatory agents for the treatment of cancer.

In another embodiment, methods are provided of treating cancer in a mammal in need thereof comprising: administering to such mammal a therapeutically effective amount of
a) a combination of the present invention; and
b) at least one anti-neoplastic agent.

In another embodiment, methods are provided of treating cancer in a mammal in need thereof comprising: administering to such mammal a therapeutically effective amount of
a) a combination of the present invention; and
b) at least one immune-modulator.

In one embodiment methods are provided for treating cancer in a human in need thereof comprising administering a therapeutically effective amount of a combination of the present invention wherein the combination comprises a therapeutically effective amount of an antigen binding protein that binds BCMA and a therapeutically effective amount of an antigen binding protein that binds PD-1 and a therapeutically effective amount of an antigen binding protein that binds OX-40.

In the embodiment, the combination of the invention may be employed with other therapeutic methods of cancer treatment. In particular, in anti-neoplastic therapy, combination therapy with other chemotherapeutic, hormonal, antibody agents as well as surgical and/or radiation treatments other than those mentioned above are envisaged.

In one embodiment, the further anti-cancer therapy is surgical and/or radiotherapy. In one embodiment, the further anti-cancer therapy is at least one additional anti-neoplastic agent.

Any anti-neoplastic agent that has activity versus a susceptible tumor being treated may be utilized in the combination. Typical anti-neoplastic agents useful include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and anti-folate compounds; topoisomerase I inhibitors such as camptothecins; hormones and hormonal analogues; signal transduction pathway inhibitors; non-receptor tyrosine angiogenesis inhibitors; immunotherapeutic agents; proapoptotic agents; and cell cycle signaling inhibitors.

In one embodiment, the combination of the present invention comprises an anti-BCMA antigen binding protein and either a PD-1 or OX40 antigen binding protein and at least one anti-neoplastic agent selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine MEKngiogenesis inhibitors, immunotherapeutic agents, proapoptotic agents, and cell cycle signaling inhibitors.

In one embodiment, the combination of the present invention comprises an anti-BCMA antigen binding protein and a PD-1 or OX40 antigen binding protein and at least one anti-neoplastic agent which is an anti-microtubule agent selected from diterpenoids and vinca alkaloids.

In a further embodiment, the at least one anti-neoplastic agent agent is a diterpenoid.

In a further embodiment, the at least one anti-neoplastic agent is a vinca alkaloid.

In one embodiment, the combination of the present invention comprises an anti-BCMA antigen binding protein and a PD-1 or OX40 antigen binding protein and at least one anti-neoplastic agent, which is a platinum coordination complex.

In a further embodiment, the at least one anti-neoplastic agent is paclitaxel, carboplatin, or vinorelbine.

In a further embodiment, the at least one anti-neoplastic agent is carboplatin.

In a further embodiment, the at least one anti-neoplastic agent is vinorelbine.

In a further embodiment, the at least one anti-neoplastic agent is paclitaxel.

In one embodiment, the combination of the present invention comprises an anti-BCMA antigen binding protein and a PD-1 or OX40 antigen binding protein and at least one anti-neoplastic agent which is a signal transduction pathway inhibitor.

In a further embodiment the signal transduction pathway inhibitor is an inhibitor of a growth factor receptor kinase VEGFR2, TIE2, PDGFR, BTK, erbB2, EGFr, IGFR-1, TrkA, TrkB, TrkC, or c-fms.

In a further embodiment the signal transduction pathway inhibitor is an inhibitor of a serine/threonine kinase rafk, akt, or PKC-zeta.

In a further embodiment the signal transduction pathway inhibitor is an inhibitor of a non-receptor tyrosine kinase selected from the src family of kinases.

In a further embodiment the signal transduction pathway inhibitor is an inhibitor of c-src.

In a further embodiment the signal transduction pathway inhibitor is an inhibitor of Ras oncogene selected from inhibitors of farnesyl transferase and geranylgeranyl transferase.

In a further embodiment the signal transduction pathway inhibitor is an inhibitor of a serine/threonine kinase selected from the group consisting of PI3K.

In a further embodiment the signal transduction pathway inhibitor is a dual EGFr/erbB2 inhibitor, for example N-{3-Chloro-4-[(3-fluorobenzyl) oxy]phenyl}-6-[5-({[2-(methanesulphonyl) ethyl]amino}methyl)-2-furyl]-4-quinazolinamine (structure below):

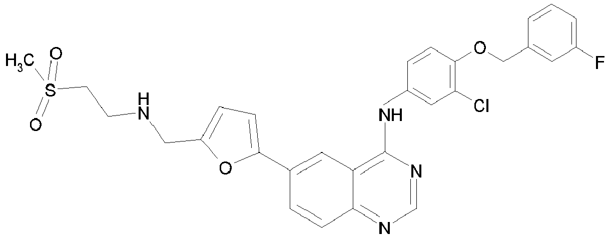

### Definitions

As used herein the term "agonist" refers to an antigen binding protein including but not limited to an antibody, which upon contact with a co-signalling receptor causes one or more of the following (1) stimulates or activates the receptor, (2) enhances, increases or promotes, induces or prolongs an activity, function or presence of the receptor (3) mimics one or more functions of a natural ligand or molecule that interacts with a target or receptor and includes initiating one or more signaling events through the receptor, mimicking one or more functions of a natural ligand, or initiating one or more partial or full conformational changes that are seen in known functioning or signaling through the receptor and/or (4) enhances, increases, promotes or induces the expression of the receptor. Agonist activity can be measured in vitro by various assays known in the art such as, but not limited to, measurement of cell signalling, cell proliferation, immune cell activation markers, cytokine production. Agonist activity can also be measured in vivo by various assays that measure surrogate end points such as, but not limited to the measurement of T cell proliferation or cytokine production.

As used herein the term "antagonist" refers to an antigen binding protein including but not limited to an antibody, which upon contact with a co-signalling receptor causes one or more of the following (1) attenuates, blocks or inactivates the receptor and/or blocks activation of a receptor by its natural ligand, (2) reduces, decreases or shortens the activity, function or presence of the receptor and/or (3) reduces, decrease, abrogates the expression of the receptor. Antagonist activity can be measured in vitro by various assays known in the art such as, but not limited to, measurement of an increase or decrease in cell signalling, cell proliferation, immune cell activation markers, cytokine production. Antagonist activity can also be measured in vivo by various assays that measure surrogate end points such as, but not limited to the measurement of T cell proliferation or cytokine production.

Thus, as used herein the term "combination of the invention" refers to a combination comprising an anti-BCMA antigen binding protein suitably an antagonist anti-BCMA antigen binding protein and either a PD-1 antigen binding protein, suitably an antagonist anti-PD-1 antigen binding protein or an OX40 antigen binding protein, suitably an agonistic OX40 antigen binding protein each of which may be administered separately or simultaneously as described herein.

As used herein, the terms "cancer," "neoplasm," and "tumor," are used interchangeably and in either the singular or plural form, refer to cells that have undergone a malignant transformation or undergone cellular changes that result in aberrant or unregulated growth or hyperproliferation Such changes or malignant transformations usually make such cells pathological to the host organism, thus precancers or precancerous cells that are or could become pathological and require or could benefit from intervention are also intended to be included. Primary cancer cells (that is, cells obtained from near the site of malignant transformation) can be readily distinguished from non-cancerous cells by well-established techniques, particularly histological examination. The definition of a cancer cell, as used herein, includes not only a primary cancer cell, but any cell derived from a cancer cell ancestor. This includes metastasized cancer cells, and in vitro cultures and cell lines derived from cancer cells. When referring to a type of cancer that normally manifests as a solid tumor, a "clinically detectable" tumor is one that is detectable on the basis of tumor mass; e.g., by procedures such as CAT scan, MR imaging, X-ray, ultrasound or palpation, and/or which is detectable because of the expression of one or more cancer-specific antigens in a sample obtainable from a patient. In other words, the terms herein include cells, neoplasms, cancers, and tumors of any stage, including what a clinician refers to as precancer, tumors, in situ growths, as well as late stage metastatic growths, Tumors may be hematopoietic tumor, for example, tumors of blood cells or the like, meaning liquid tumors. Specific examples of clinical conditions based on such a tumor include leukemia such as chronic myelocytic leukemia or acute myelocytic leukemia; myeloma such as multiple myeloma; lymphoma and the like.

As used herein the term "agent" is understood to mean a substance that produces a desired effect in a tissue, system, animal, mammal, human, or other subject. Accordingly, the term "anti-neoplastic agent" is understood to mean a substance producing an anti-neoplastic effect in a tissue, system, animal, mammal, human, or other subject. It is also to be understood that an "agent" may be a single compound or a combination or composition of two or more compounds.

By the term "treating" and derivatives thereof as used herein, is meant therapeutic therapy. In reference to a particular condition, treating means: (1) to ameliorate the condition or one or more of the biological manifestations of the condition; (2) to interfere with (a) one or more points in the biological cascade that leads to or is responsible for the condition or (b) one or more of the biological manifestations of the condition; (3) to alleviate one or more of the symptoms, effects or side effects associated with the condition or one or more of the symptoms, effects or side effects associated with the condition or treatment thereof; (4) to slow the progression of the condition or one or more of the biological manifestations of the condition and/or (5) to cure said condition or one or more of the biological manifestations of the condition by eliminating or reducing to undetectable levels one or more of the biological manifestations of the condition for a period of time considered to be a state of remission for that manifestation without additional treatment over the period of remission. One skilled in the art will understand the duration of time considered to be remission for a particular disease or condition. Prophylactic therapy is also contemplated thereby. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. Prophylactic therapy is appropriate, for example, when a subject is considered at high risk for developing cancer, such as when a subject has a strong family history of cancer or when a subject has been exposed to a carcinogen.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

"Antigen Binding Protein" means a protein that binds an antigen, including antibodies or engineered molecules that function in similar ways to antibodies. Such alternative antibody formats include triabody, tetrabody, miniantibody, and a minibody, Also included are alternative scaffolds in which the one or more CDRs of any molecules in accordance with the disclosure can be arranged onto a suitable non-immunoglobulin protein scaffold or skeleton, such as an affibody, a SpA scaffold, an LDL receptor class A domain, an avimer (see, e.g., U.S. Patent Application Publication Nos. 2005/0053973, 2005/0089932, 2005/0164301) or an EGF domain. An ABP also includes antigen binding fragments of such antibodies or other molecules. Further, an ABP of a combination of the invention, or a method or use thereof, may comprise the VH regions formatted into a full length antibody, a (Fab')2 fragment, a Fab fragment, a bi-specific or biparatopic molecule or equivalent thereof (such as scFV, bi- tri- or tetra-bodies, Tandabs etc.), when paired with an appropriate light chain. The antigen binding protein may comprise an antibody that is an IgG1, IgG2, IgG3, or IgG4; or IgM; IgA, IgE or IgD or a modified variant thereof. The constant domain of the antibody heavy chain may be selected accordingly. The light chain constant domain may be a kappa or lambda constant domain. The ABP may also be a chimeric antibody of the type described in WO86/01533 which comprises an antigen binding region and a non-immunoglobulin region.

An antigen binding protein may also be a chimeric antigen receptor. The term "chimeric antigen receptors" ("CARs") as used herein, refers to an engineered receptor which consists of an extracellular target binding domain (which is usually derived from a monoclonal antibody), a spacer region, a transmembrane region, and one or more intracellular effector domains. CARs have also been referred to as chimeric T cell receptors or chimeric immunoreceptors (CIRs). CARs are genetically introduced into hematopoietic cells, such as T cells, to redirect specificity for a desired cell-surface antigen.

Chimeric antigen receptors (CARs) have been developed as artificial TCRs to generate novel specificities in T cells without the need to bind to MHC-antigenic peptide complexes. These synthetic receptors contain a target binding domain that is associated with one or more signalling domains via a flexible linker in a single fusion molecule. The target binding domain is used to target the T cell to specific targets on the surface of pathologic cells and the signalling domains contain molecular machinery for T cell activation and proliferation. The flexible linker which passes through the T cell membrane (i.e. forming a transmembrane domain) allows for cell membrane display of the target binding domain of the CAR. CARs have successfully allowed T cells to be redirected against antigens expressed at the surface of tumour cells from various malignancies including lymphomas and solid tumours (Jena et al. (2010) Blood, 116(7):1035-44).

The development of CARs has comprised three generations so far. The first generation CARs comprised target binding domains attached to a signalling domain derived from the cytoplasmic region of the CD3zeta or the Fc receptor gamma chains. First generation CARs were shown to successfully redirect T cells to the selected target, however, they failed to provide prolonged expansion and antitumor activity in vivo. The second and third generation CARs have focussed on enhancing modified T cell survival and increasing proliferation by including co-stimulatory molecules, such as CD28, OX-40 (CD134) and 4-1BB (CD137).

T cells bearing CARs could be used to eliminate pathologic cells in a disease setting. One clinical aim would be to transform patient cells with recombinant DNA containing an expression construct for the CAR via a vector (e.g. a lentiviral vector) following aphaeresis and T cell isolation. Following expansion of the T cells they are re-introduced into the patient with the aim of targeting and killing the pathologic target cells.

The term "antibody" as used herein refers to molecules with an antigen binding domain, and optionally an immunoglobulin-like domain or fragment thereof and includes monoclonal (for example IgG, IgM, IgA, IgD or IgE and modified variants thereof), recombinant, polyclonal, chimeric, humanized, biparatopic, bispecific and heteroconjugate antibodies, or a closed conformation multispecific antibody. An "antibody" included xenogeneic, allogeneic, syngeneic, or other modified forms thereof. An antibody may be isolated or purified. An antibody may also be recombinant, i.e. produced by recombinant means; for example, an antibody that is 90% identical to a reference antibody may be generated by mutagenesis of certain residues using recombinant molecular biology techniques known in the art. Thus, the antibodies of the present invention may comprise heavy chain variable regions and light chain variable regions of a combination of the invention, or a method or use thereof, which may be formatted into the structure of a natural antibody or formatted into a full length recombinant antibody, a (Fab')2 fragment, a Fab fragment, a bi-specific or biparatopic molecule or equivalent thereof (such as scFV, bi- tri- or tetra-bodies, Tandabs etc.), when paired with an appropriate light chain. The antibody may be an IgG1, IgG2, IgG3, or IgG4 or a modified variant thereof. The constant domain of the antibody heavy chain may be selected accordingly. The light chain constant domain may be a kappa or lambda constant domain. The antibody may also be a chimeric antibody of the type described in WO86/01533 which comprises an antigen binding region and a non-immunoglobulin region.

One of skill in the art will recognize that the antigen binding proteins of the invention bind to an epitope on their targets. The epitope of an antigen binding protein is the region of its antigen to which the antigen binding protein binds. Two antigen binding proteins bind to the same or overlapping epitope if each competitively inhibits (blocks) binding of the other to the antigen. That is, a 1x, 5x, 10x, 20x or 100x excess of one antibody inhibits binding of the other by at least 50% but preferably 75%, 90% or even 99% as measured in a competitive binding assay compared to a control lacking the competing antibody (see, e.g., Junghans et al., Cancer Res. 50:1495, 1990, which is incorporated herein by reference). Alternatively, two antibodies have the same epitope if essentially all amino acid mutations in the antigen that reduce or eliminate binding of one antibody reduce or eliminate binding of the other. Also the same epitope may include "overlapping epitopes" e.g. if some amino acid mutations that reduce or eliminate binding of one antibody reduce or eliminate binding of the other.

The strength of binding may be important in dosing and administration of an antigen binding protein of the combination, or method or use thereof, of the invention. In one embodiment, the antigen binding protein of the invention binds its target (e.g. BCMA or PD-1 or OX40) with high affinity. Affinity is the strength of binding of one molecule, e.g. an antibody of a combination of the invention, or a method or use thereof, to another, e.g. its target antigen, at a single binding site. The binding affinity of an antibody to its target may be determined by equilibrium methods (e.g. enzyme-linked immunoabsorbent assay (ELISA) or radioimmunoassay (RIA)), or kinetics (e.g. BIACORE analysis). For example, the Biacore methods known in the art may be used to measure binding affinity.

Avidity is the sum total of the strength of binding of two molecules to one another at multiple sites, e.g. taking into account the valency of the interaction.

Functional fragments of the antigen binding proteins of a combination of the invention, or a method or use thereof, are contemplated herein.

Thus, "binding fragments" and "functional fragments" may be an Fab and F(ab')2 fragments which lack the Fc fragment of an intact antibody, clear more rapidly from the circulation, and may have less non-specific tissue binding than an intact antibody (Wahl et al., J. Nuc. Med. 24:316-325 (1983)). Also included are Fv fragments (Hochman, J. et al. (1973) Biochemistry 12:1130-1135; Sharon, J. et al.(1976) Biochemistry 15:1591-1594). These various fragments are produced using conventional techniques such as protease cleavage or chemical cleavage (see, e.g., Rousseaux et al., Meth. Enzymol., 121:663-69 (1986)).

"Functional fragments" as used herein means a portion or fragment of the antigen binding proteins of a combination of the invention, or a method or use thereof, that include the antigen-binding site and are capable of binding the same target as the parent antigen binding protein, e.g. but not limited to binding the same epitope, and that also retain one or more modulating or other functions described herein or known in the art.

As the antigen binding proteins of the present invention may comprise heavy chain variable regions and light chain variable regions of a combination of the invention, or a method or use thereof, which may be formatted into the structure of a natural antibody, a functional fragment is one that retains binding or one or more functions of the full length antigen binding protein as described herein. A binding fragment of an antigen binding protein of a combination of the invention, or a method or use thereof, may therefore comprise the VL or VH regions, a (Fab')2 fragment, a Fab fragment, a fragment of a bi-specific or biparatopic molecule or equivalent thereof (such as scFV, bi- tri- or tetra-bodies, Tandabs etc.), when paired with an appropriate light chain. The term "CDR" as used herein, refers to the complementarity determining region amino acid sequences of an antigen binding protein. These are the hypervariable regions of immunoglobulin heavy and light chains. There are three heavy chain and three light chain CDRs (or CDR regions) in the variable portion of an immunoglobulin.

It will be apparent to those skilled in the art that there are various numbering conventions for CDR sequences; Chothia (Chothia et al. (1989) Nature 342: 877-883), Kabat (Kabat et al., Sequences of Proteins of Immunological Interest, 4th Ed., U.S. Department of Health and Human Services, National Institutes of Health (1987)), AbM (University of Bath) and Contact (University College London). The minimum overlapping region using at least two of the Kabat, Chothia, AbM and contact methods can be determined to provide the "minimum binding unit". The minimum binding unit may be a subportion of a CDR. The structure and protein folding of the antibody may mean that other residues are considered part of the CDR sequence and would be understood to be so by a skilled person. It is noted that some of the CDR definitions may vary depending on the individual publication used.

Unless otherwise stated and/or in absence of a specifically identified sequence, references herein to "CDR", "CDRL1" (or "LC CDR1"), "CDRL2" (or "LC CDR2"), "CDRL3" (or "LC CDR3"), "CDRH1" (or "HC CDR1"), "CDRH2" (or "HC CDR2"), "CDRH3" (or "HC CDR3") refer to amino acid sequences numbered according to any of the known conventions; alternatively, the CDRs are referred to as "CDR1," "CDR2," "CDR3" of the variable light chain and "CDR1," "CDR2," and "CDR3" of the variable heavy chain. In particular embodiments, the numbering convention is the Kabat convention.

The term "variant" as used herein refers to a heavy chain variable region or light chain variable region that has been modified by at least one, for example 1, 2 or 3, amino acid substitution(s), deletion(s) or addition(s), wherein the modified antigen binding protein comprising the heavy chain or light chain variant substantially retains the biological characteristics of the antigen binding protein pre-modification. In one embodiment, the antigen binding protein containing a variant heavy chain variable region or light chain variable region sequence retains 60%, 70%, 80%, 90%, 100% biological characteristics of the antigen binding protein pre-modification. It will be appreciated that each heavy chain variable region or light chain variable region may be modified alone or in combination with another heavy chain variable region or light chain variable region. The antigen binding proteins of the disclosure include heavy chain variable region amino acid sequences that are 90%, 91%, 92%, 93%, 94%, 95%, 96%, 97%, 98%, or 99% homologous to the heavy chain variable region amino acid sequences described herein. The antigen binding proteins of the disclosure include light chain variable region amino acid sequence that are 90%, 91%, 92%, 93%, 94%, 95%, 96%, 97%, 98%, or 99% homologous to the light chain variable region amino acid sequences described herein.

The percent homology can be over the entire heavy chain variable region and/or entire light chain variable region or the percent homology can be confined to the framework regions, while the sequences that correspond to CDRs have 100% identity to the CDRs disclosed herein within the heavy chain variable region and/or light chain variable region.

The term "CDR variant" as used herein, refers to a CDR that has been modified by at least one, for example 1, 2 or 3, amino acid substitution(s), deletion(s) or addition(s), wherein the modified antigen binding protein comprising the CDR variant substantially retains the biological characteristics of the antigen binding protein pre-modification. In one embodiment, the antigen binding protein containing a variant CDR retains 60%, 70%, 80%, 90%, 100% biological characteristics of the antigen binding protein pre-modification. It will be appreciated that each CDR that can be modified may be modified alone or in combination with another CDR. In one embodiment, the modification is a substitution, particularly a conservative substitution, for example as shown in Table 1.

**Table 1**

| Side chain | Members |
|---|---|
| Hydrophobic | Met, Ala, Val, Leu, Ile |
| Neutral hydrophilic | Cys, Ser, Thr |
| Acidic | Asp, Glu |
| Basic | Asn, Gln, His, Lys, Arg |
| Residues that influence chain orientation | Gly, Pro |
| Aromatic | Trp, Tyr, Phe |

For example, in one CDR variant, the amino acid residues of the minimum binding unit may remain the same, but the flanking residues that comprise the CDR as part of the Kabat or Chothia definition(s) may be substituted with a conservative amino acid residue.

Such antigen binding proteins comprising modified CDRs or minimum binding units as described above may be referred to herein as "functional CDR variants" or "functional binding unit variants".

The antibody may be of any species, or modified to be suitable to administer to a cross species. For example the CDRs from a mouse antibody may be humanized for administration to humans. In any embodiment, the antigen binding protein is optionally a humanized antibody.

A "humanized antibody" refers to a type of engineered antibody having its CDRs derived from a non-human donor immunoglobulin, the remaining immunoglobulin-derived parts of the molecule being derived from one (or more) human immunoglobulin(s). In addition, framework support residues may be altered to preserve binding affinity (see, e.g., Queen et al., Proc. Natl Acad Sci USA, 86:10029-10032 (1989), Hodgson et al., Bio/Technology, 9:421 (1991)). A suitable human acceptor antibody may be one selected from a conventional database, e.g., the KABAT^{®} database, Los Alamos database, and Swiss Protein database, by homology to the nucleotide and amino acid sequences of the donor antibody. A human antibody characterized by a homology to the framework regions of the donor antibody (on an amino acid basis) may be suitable to provide a heavy chain constant region and/or a heavy chain variable framework region for insertion of the donor CDRs. A suitable acceptor antibody capable of donating light chain constant or variable framework regions may be selected in a similar manner. It should be noted that the acceptor antibody heavy and light chains are not required to originate from the same acceptor antibody. The prior art describes several ways of producing such humanised antibodies - see for example EP-A-0239400 and EP-A-054951.

In yet a further embodiment, the humanized antibody has a human antibody constant region that is an IgG. In another embodiment, the IgG is a sequence as disclosed in any of the above references or patent publications.

"Enhanced FcγRIIIA mediated effector function" as used herein denotes that the usual effector function of the antigen binding protein has been deliberately increased compared to its usual levels. This may be carried out by any means known in the art for example by mutations which increase affinity for FcYRIIIA binding or by alteration of the glycosylation of the antigen binding protein (for example knock out a fucosyltransferase)

For nucleotide and amino acid sequences, the term "identical" or "identity" indicates the degree of identity between two nucleic acid or two amino acid sequences when optimally aligned and compared with appropriate insertions or deletions.

The percent sequence identity between two sequences is a function of the number of identical positions shared by the sequences (i.e., % identity = number of identical positions/total number of positions multiplied by 100), taking into account the number of gaps, and the length of each gap, which need to be introduced for optimal alignment of the two sequences. The comparison of sequences and determination of percent identity between two sequences can be accomplished using a mathematical algorithm, as described below.

Percent identity between a query nucleic acid sequence and a subject nucleic acid sequence is the "Identities" value, expressed as a percentage, which is calculated by the BLASTN algorithm when a subject nucleic acid sequence has 100% query coverage with a query nucleic acid sequence after a pair-wise BLASTN alignment is performed. Such pair-wise BLASTN alignments between a query nucleic acid sequence and a subject nucleic acid sequence are performed by using the default settings of the BLASTN algorithm available on the National Center for Biotechnology Institute's website with the filter for low complexity regions turned off. Importantly, a query nucleic acid sequence may be described by a nucleic acid sequence identified in one or more claims herein.

Percent identity between a query amino acid sequence and a subject amino acid sequence is the "Identities" value, expressed as a percentage, which is calculated by the BLASTP algorithm when a subject amino acid sequence has 100% query coverage with a query amino acid sequence after a pair-wise BLASTP alignment is performed. Such pair-wise BLASTP alignments between a query amino acid sequence and a subject amino acid sequence are performed by using the default settings of the BLASTP algorithm available on the National Center for Biotechnology Institute's website with the filter for low complexity regions turned off. Importantly, a query amino acid sequence may be described by an amino acid sequence identified in one or more claims herein.

In one embodiment of the invention as herein described the antigen binding proteins have an amino acid sequence with at least 90%, 91%, 92%, 93%, 94%, 95%, 96%, 97%, 98%, 99% or 100% sequence identity to the amino acid sequences as set forth in in the sequence listing. In a particular embodiment the antigen binding proteins have at least 98% for example 99% sequence identity to those found in the sequence listing.

Any references or publications described herein are hereby incorporated by reference.

### Examples

The following examples illustrate various non-limiting aspects of this invention.

### Example 1. Production of BCMA antigen binding proteins.

Production of antigen binding proteins according to the invention as herein described and conjugation to a toxin and the respective binding affinities of such antigen binding proteins can be found in WO2012163805 as herein incorporated by reference.

### Example 2 - Immunogenic cell death (ICD)

The process of immunogenic cell death can induce the production of danger molecules that lead to the activation of dendritic cells (see FIG. 1A). BCMA ADC (anti-BCMA antibody conjugated to MMAF: GSK285791) treated NCI-H929 cells produced three danger molecules (ATP, HMGB1 and CRT) upon cell killing (FIG. 1B). To investigate the effect of NCI-H929 cell killing by BCMA ADC on the activation of dendritic cells, co-culture experiments were carried out with BCMA ADC treated NCI-H929 cells and immature dendritic cells (iDCs) differentiated from human monocytes in vitro with GM-CSF/IL-4 treatment. A number of dendritic cell maturation markers as well as IL-10 and IL-12p70, two major cytokines secreted by dendritic cells upon activation, were monitored in this process to assess the effect of BCMA ADC induced cell death on dendritic cell activation.

Fresh, whole Human blood was obtained from three healthy donors from the Upper Providence Blood Donation Unit with syringes coated in liquid sodium heparin (Sagent 10 IU/mL final concentration). Monocytes were isolated from fresh, whole human blood using the RosetteSep Monocyte Enrichment kit (Cat.# 15068) and were assessed for expression of cell surface CD14 (BD Bioscience Cat.# 562698) using flow cytometry. Isolated Monocytes (1.5x106 cells/well) were cultured in 2mLs of X-Vivo-15 Media supplemented with 1% autologous plasma, 50 ng/mL recombinant human GM-CSF (R&D, 215-GM-050) and 100ng/mL recombinant human IL-4 (R&D, 204-IL-050) for 7 Days at 37C°/5%CO2. The cultures received a half media change at Day 3 or 4 while maintaining stimulation factor concentrations. NCI-H929 Multiple Myeloma cells were passaged for ≥ 2 passages from thaw at 37°C, 5% CO₂, 90% humid air in RPMI-1640 supplemented with 10% FBS. Cells were plated at a density of 7.5×10⁵ cells/ml in 2 mLs on 12 well plates. A dose response of J6M0 ADC enhanced antigen binding protein was added to the media and the cells were incubated at 37°C/5% CO₂ for 48 hours.

Day 7 iDCs were co-cultured with J6M0 ADC enhanced antigen binding protein treated NCI-H929 cells at 1:1 ratio for 24 hours on 96 well plates. All cells were counted at the initiation of the co-culture and diluted with X-Vivo-15 media to a concentration of 7.5×10⁵ cells/mL. 7.5×10⁴ cells/well (100 µl) each of iDCS and pre-treated NCI-H929 cells were combined in each well on a 96 well plate. Co-cultured cells were incubated 37°C/5% CO₂ for 24 hours. A flow cytometry panel consisting of CD1a, CD11c, CD40, CD80, CD83, CD86, HLA-DR, & CD14 was used to assess fresh, FcR blocked Dendritic Cell differentiation and maturation on the FACS Canto II. Additionally, cell supernatants were collected, frozen at -20°C, and assayed for IL-10 and IL-12p70 using MSD kits.

Data analysis was performed in Flow Jo v7.6.5 for the flow cytometry panel and used either CD11c+ or HLA-DR+ cell gating to distinguish the dendritic Cells from the tumor cells. NCI-H929 cells are negative for HLA-DR and have much lower CD11c expression than dendritic cells allowing the two cell populations to be distinctly gated. Data for the secreted IL-10 and IL-12p70 from supernatants in the co-culture assays w analyzed in MSD Discovery Workbench v4.0.12.

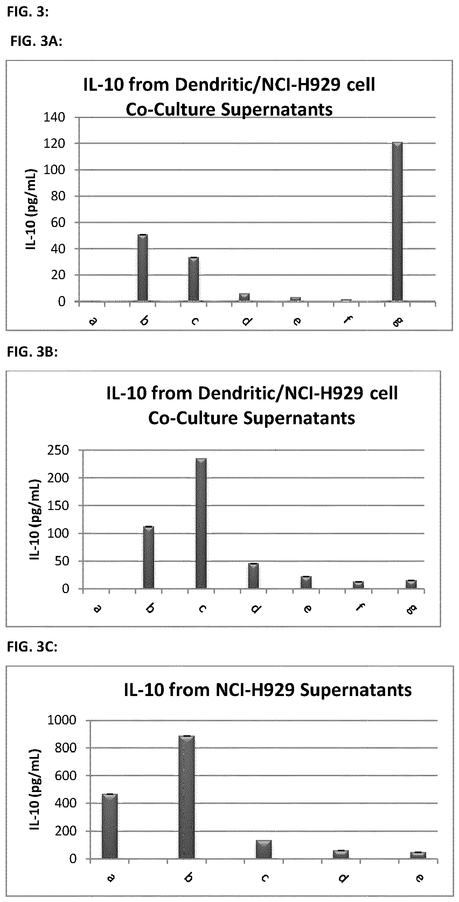

These results suggest that the killing of NCI-H929 cells with BCMA ADC has a stimulatory effect on dendritic cells and can lead to dendritic cell activation. When IL-12p70 and IL-10 were monitored, IL-12p70 was below the detection limit under all conditions. IL-10 could be detected and there was a significant inhibitory effect of BCMA ADC on Il-10 production. However, this effect may not be related to the dendritic cell activation by the immunogenic cell death of NCI-H929 cells induced by BCMA ADC since the inhibitory effect on IL-10 was observed with NCI-H929 cells alone treated with BCMA ADC. The inhibitory effect on IL-10 production is beneficial in stimulating the immune response. (See FIG. 2 and FIG. 3)

### Example 3

T cells can be activated through the engagement of the T cell receptor (TCR) and co-stimulatory molecules expressed on the cell surface. Upon T cell activation, a number of additional surface markers are upregulated. In vitro effect of BCMA ADC (anti-BCMA antibody conjugated to MMAF: GSK285791) on T cell activation and function was characterized by monitoring a number of these T cell activation associated markers. Furthermore, upon activation, T cells produce cytokines such as IFNy and IL-4. The effect of BCMA ADC on IFNy and IL-4 productions in stimulated T cells was also studied. These experiments can provide data on the effect of BCMA ADC on T cells activation and function. In addition, proliferation of human CD4+ and CD8+ T cells upon stimulation in the presence of BCMA ADC was also evaluated. Human blood was obtained from the blood donor procurement program at GlaxoSmithKline. Peripheral blood mononuclear cells (PBMCs) were isolated from human whole blood by Ficoll density gradient centrifugation (GE Healthcare). Antihuman CD3 antibody (eBioscience, catalog#16-0037-85, clone OKT3) diluted with coating buffer (Biolegend, catalog# 421701) was coated on 96 well flat-bottom plate overnight.

### 3.1 Effect of BCMA ADC on T cell activation by anti-CD3/anti-CD28 stimulation in PBMC

BCMA ADC (anti-BCMA antibody conjugated to MMAF: GSK285791) was tested in the PBMC T cell activation assay. In this study, BCMA ADC treatment and PBMC activation occurred concurrently and the effects were monitored at 24 and 72 hrs. This study was repeated two times (n=2) with blood from two different donors. In this study, 100 µL PBMCs (2×10^6 cells/mL) in RPMI-1640 with 10% FBS (Hyclone; catalog# SH30071.03) were added into anti-CD3 antibody coated wells with or without soluble 0.5 µg/mL anti-CD28 antibody (eBioscience, catalog# 16-0289, clone CD28.2). A stock solution of 9.6 mg/mL BCMA ADC or_4.6 mg/mL BCMA Ab IgG control was first diluted in RPMI-1640 media to give antibody concentrations of 1 mg/mL which were further diluted in equal volume of RPMI-1640 media to give antibody concentrations of 60, 6 and 0.6 µg/mL. 100 µL each of the final diluted antibody solutions was added to 100 µL of the PBMC in RPMI-1640/10% FBS to give final antibody concentrations of 30, 3 and 0.3 µg/mL. Three technical replicates were included for each assay condition. PBMCs were cultured at 37°C and 5% CO2 for various times as indicated above. Cells were transferred into 96-deep well plate and washed twice with 1 mL staining buffer (BD Biosciences, catalog# 554656), stained with fluorescent-conjugated antibodies or isotype controls (see section 3.3. Drugs and Materials), and incubated for 30 min on ice. Immunofluorescence analysis was performed on a FACS CANTO II flow cytometer (BD Biosciences) and analyzed with DIVA software (BD Biosciences). Flow cytometry was used to monitor the percentage of CD4 or CD8 cells that express a given marker, and the mean fluorescence intensity (MFI) of this maker in CD4 or CD8 cells.

### 3.2 Effect of BCMA ADC on IFNγ and IL-4 production by T cells upon anti-CD3/anti-CD28 stimulation in PBMC

This study was repeated two times (n=2) with blood from two different donors. 100 µL PBMCs (1×10^6 cells/mL) in RPMI-1640 with 10% FBS were added into anti-CD3 antibody coated wells with or without soluble 0.5 µg/mL anti-CD28 antibody. 100 µL each of the diluted BCMA ADC (anti-BCMA antibody conjugated to MMAF: GSK285791) and IgG control solutions (see section 3.1 for antibody dilution protocol) was then added and PBMCs were cultured at 37°C and 5% CO2 for 48 hours and 72 hours. Three technical replicates were included for each assay condition. At the end of the study, cells were transferred, washed, stained and analyzed by flow cytometry as described in section 3.1. For intracellular staining, cells were fixed by cytofix buffer (BD Biosciences, Catalog# 554655) at room temperature for 20 minutes and permeablized by perm/wash buffer (BD Biosciences, catalog# 554723) at room temperature for 30 minutes before staining with antibodies or isotype controls.

### 3.3 Effect of BCMA ADC on T cell proliferation upon anti-CD3/anti-CD28 stimulation

Fresh normal peripheral blood isolated CD4+ or CD8+ T cells were received and counted using a Beckman Coulter ViCell then centrifuged at 330g for 7 minutes. Cells were then stained with Molecular Probes Cell Trace CFSE (cat # C34554) proliferation dye (5-10 µM) in PBS/0.5% BSA. Cells were incubated for 5 minutes on ice prior to a further incubation for 5 minutes on ice in cold RPMI 1640/10% FBS. Any free dye was removed by two further cell centrifugations at 300g for 5 minutes. Cells were resuspended in complete media RPMI 1640/10% FBS/IL-2 (2.8 ng/mL). Cells were then seeded (10⁵ cells/100 µL volume/well) in a 96 well tissue culture plates pre-coated with anti-CD3 (1µg/mL) and anti-CD28 (1µg/mL). BCMA ADC (anti-BCMA antibody conjugated to MMAF: GSK285791) was added to cells immediately after seeding plate and incubated at 37°C for 96 hours.

After the 4 day incubation, cell supernatant was harvested and frozen at minus 80°C and the cells were collected for flow cytometry staining. Analyzed using a CANTO II flow cytometer with 488 nm excitation and emission filters appropriate for fluorescein (FITC) for Cell Trace CFSE.

The antibodies and isotypes for the markers monitored by flow cytometry analysis are listed in Table 2 below.

**Table 2.**

| **Marker** | **Fluorescence** | **Clone** | **Isotype** | **Vendor** | **Catalog No.** |
|---|---|---|---|---|---|
| CD4 | V450 | SK3 | mouse IgG1 | BD Biosciences | 651850 |
| CD8 | PerCP-Cy5.5 | RPA-T8 | mouse IgG1 | BioLegend | 301032 |
| CD25 | PE | BC96 | mouse IgG1 | eBioscience | 12-0259-42 |
| CD69 | PE-Cy7 | FN50 | mouse IgG1 | BioLegend | 310912 |
| PD-1 | APC | EH12.2H7 | mouse IgG1 | BioLegend | 329908 |
| OX-40 | FITC | ACT-35 | mouse IgG1 | eBioscience | 11-1347-42 |
| CTLA-4 | PE | L3D10 | mouse IgG1 | BioLegend | 349906 |
| CD39 | APC | A1 | mouse IgG1 | eBioscience | 17-0399-42 |
| CD73 | PE-Cy7 | AD2 | mouse IgG1 | BD Biosciences | 561258 |
| ICOS | FITC | Isa3 | mouse IgG1 | eBioscience | 11-9948-42 |
| CD137 | PE | 4B4-1 | mouse IgG1 | BD Biosciences | 555956 |
| LAG-3 | APC | 3DS223H | mouse IgG1 | eBioscience | 17-2239-42 |
| TIM-3 | FITC | F38-2E2 | mouse IgG1 | eBioscience | 11-3109-42 |
| CD4 | PE-Cy5 | OKT4 | mouse IgG2b | BioLegend | 317412 |
| IL-4 | PE | 8D4-8 | mouse IgG1 | BioLegend | 500704 |
| IFNγ | FITC | B27 | mouse IgG1 | BD Biosciences | 554700 |

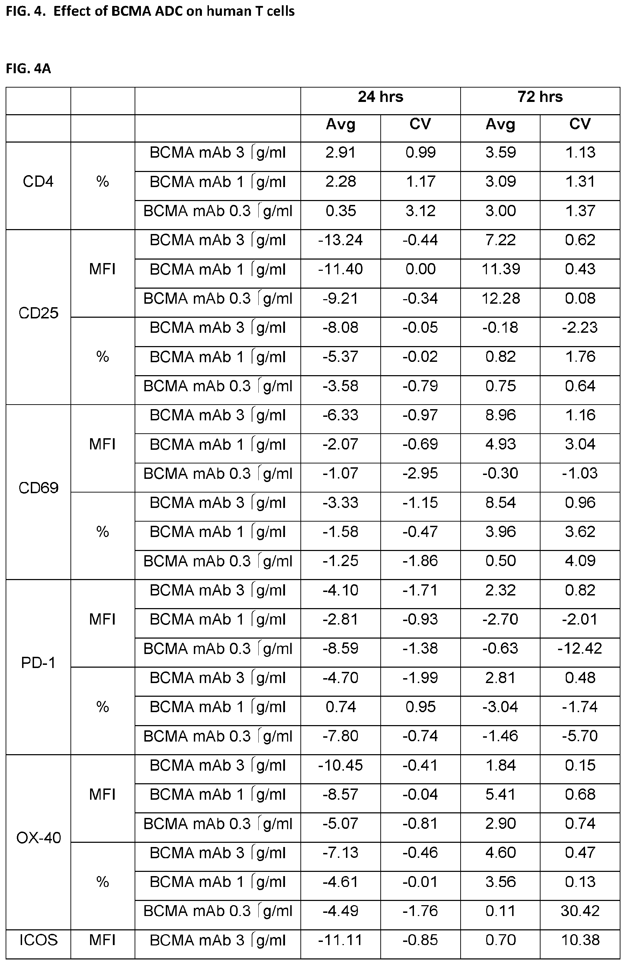

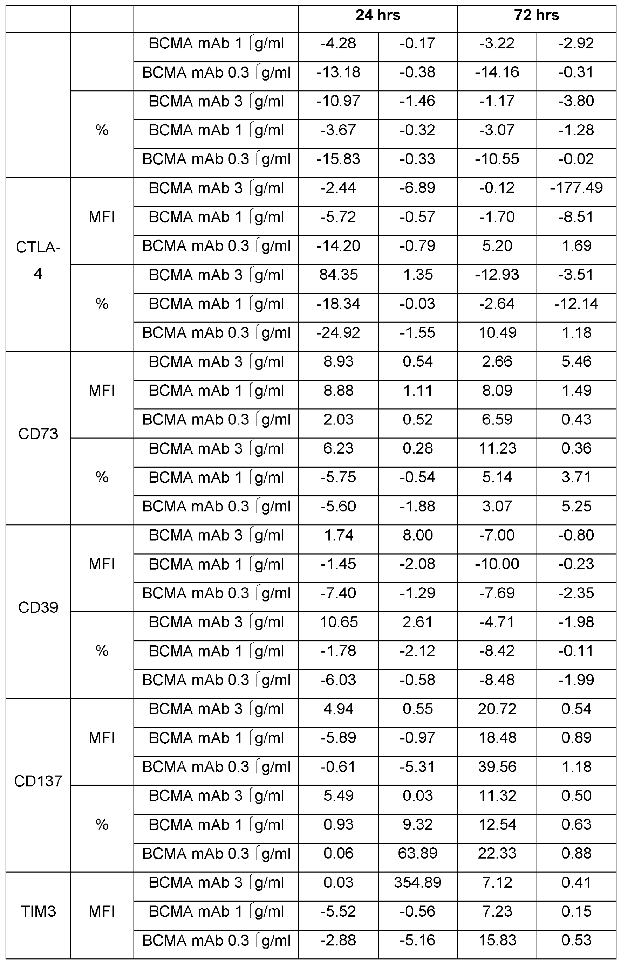

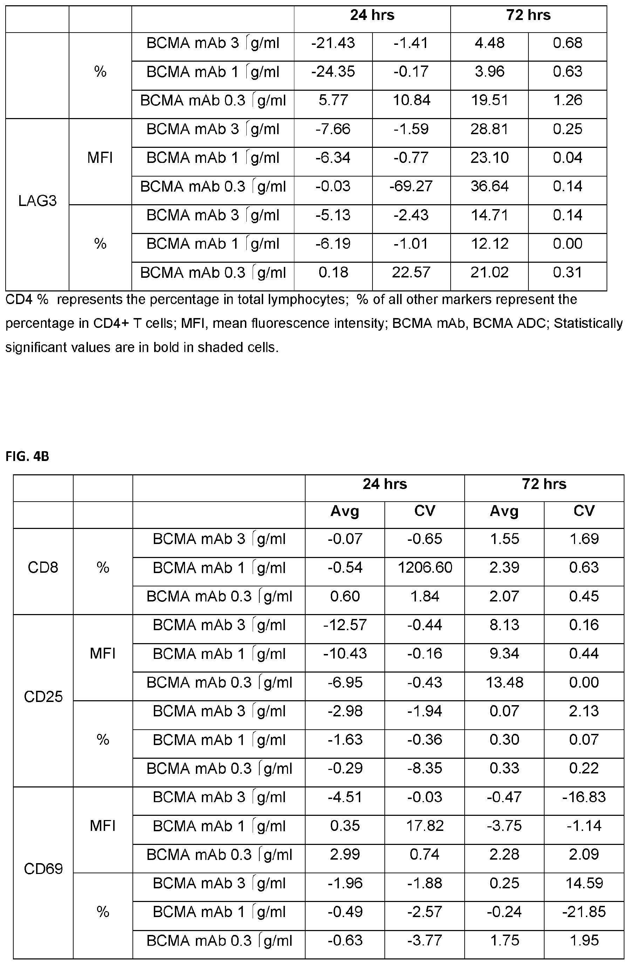

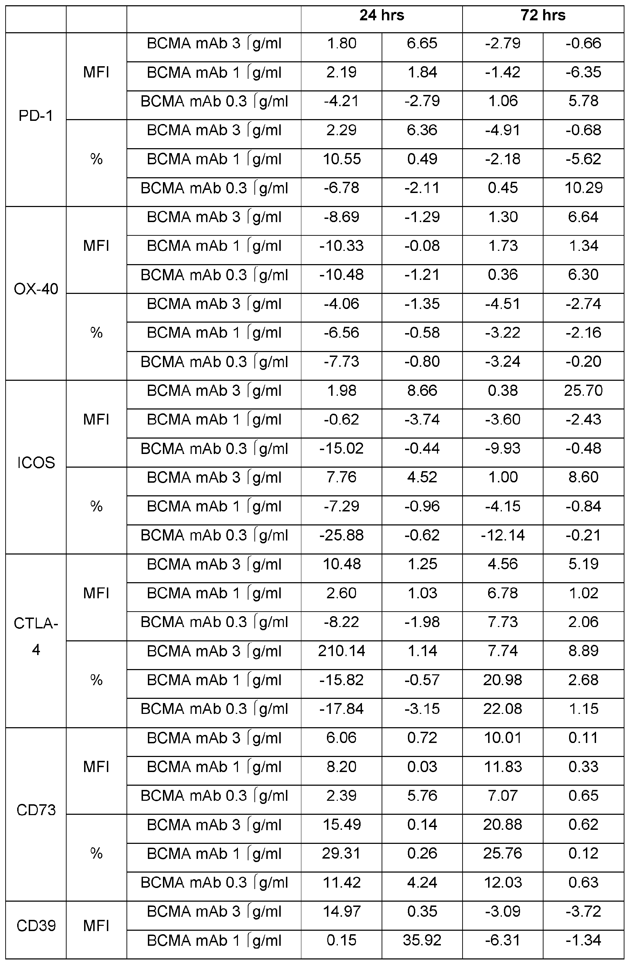

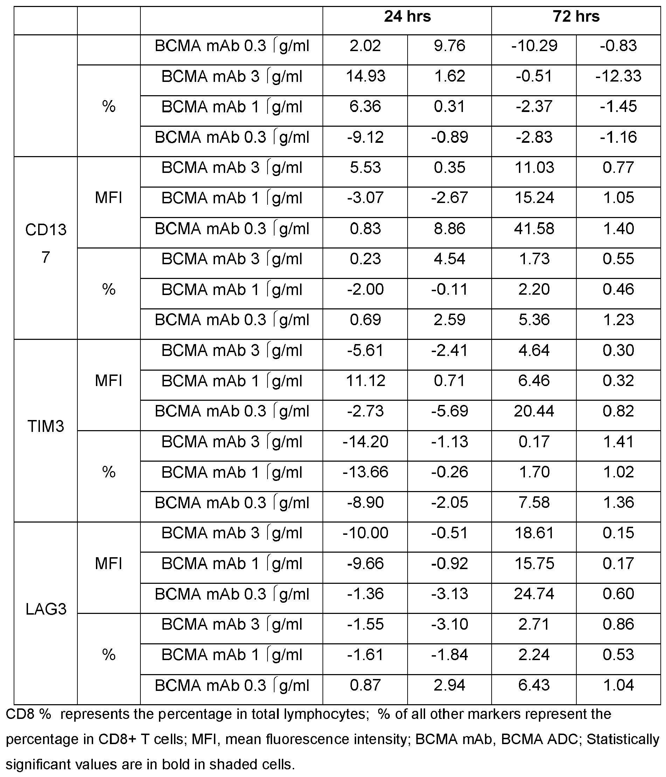

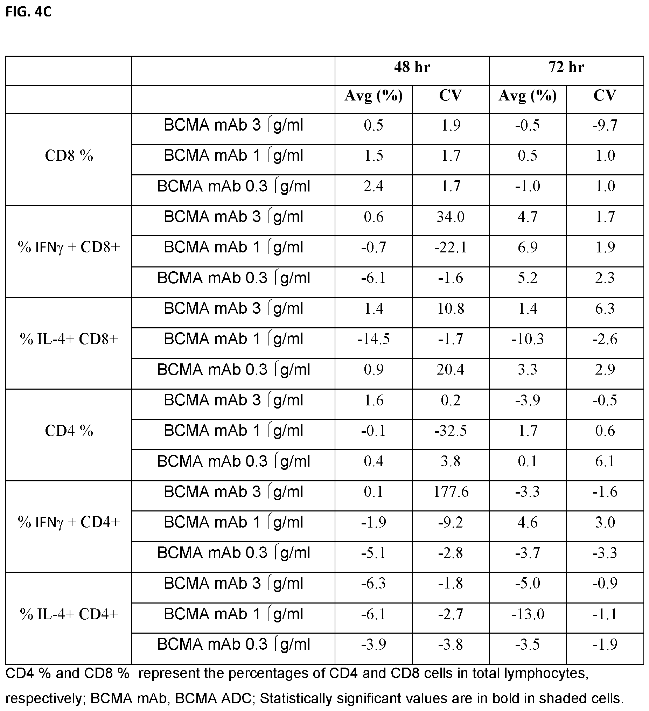

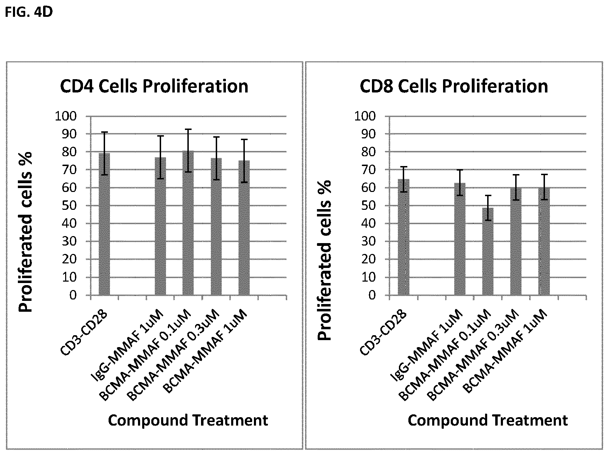

The raw percentage and MFI data for each marker at each concentration of BCMA ADC was compared to the corresponding IgG control in the statistical analysis. To account for the variability of the different blood donors, a linear mixed effect model was used to analyze the data. Briefly, donor and the interaction between donor and group (BCMA ADC treatment or IgG control) were treated as random effects, and group was treated as a fixed effect in the model. Following the mixed effect model analysis, each BCMA ADC treatment group was then compared with the IgG control group. Due to multiple comparisons, Dunnett's method was used to control the overall Type-1 error rate. The adjusted p-values ≤ 0.05 by Dunnett's method were declared to be significant for the percentage or MFI value at a specific BCMA ADC concentration. BCMA ADC was considered to significantly change the expression of a marker if at least 2 out of the 3 concentrations of BCMA ADC induced a statistically significant (p-values ≤ 0.05) change of the percentage or MFI value of the marker. For data reporting, the average percentage or average MFI value among three technical replicates was generated at each BCMA ADC concentration and the % difference values were calculated as: % difference = (Avg 916 - Avg IgG)^{∗}100/Avg IgG, where Avg 916 and Avg IgG represent the average values of the BCMA ADC treatment group and the IgG control group, respectively. The % difference values with different donors for a given marker at a given BCMA ADC concentration and time point were used to calculate the average % difference value and CV (coefficient of variation) which were reported in FIG. 4A-C. Positive and negative % difference values represent upregulation and downregulation of a marker, respectively.

CD4 % and CD8 % were measured independently in three groups for each experiment, with three technical replicates in each group. Each group was analyzed statistically as described above. Changes in CD4 % and CD8 % values were considered significant if at least 2 out of the 3 groups had significant changes (p-values ≤ 0.05). For data reporting, a pooled CV was generated by taking the average of CVs for 3 groups.

In this study, in vitro effect of BCMA ADC on T cell activation and function was characterized by monitoring the effect of compound on a number of T cell activation associated markers. Majority of these markers are upregulated upon T cell activation. These markers include T cell activation markers CD25 and CD69; co-inhibitory markers PD-1, CTLA-4; co-stimulatory markers ICOS, OX-40 and CD137; and T cell exhaustion markers TIM3 and LAG3. CD73 and CD39 are surface proteins involved in the adenosine pathway activation and are considered as co-inhibitory molecules in T cell activation. The correlation between their expression level and T cell activation is less well understood. Overall, the data indicate that BCMA ADC has minimal effect on anti-CD3/anti-CD28 stimulated CD4 and CD8 T cell activation in PBMC. BCMA ADC has no significant effect on IFNy and IL-4 production in both CD4 and CD8 cells in PBMC after anti-CD3/anti-CD28 stimulation. These data are consistent with the lack of BCMA expression on human T cells.

### Example 4: In vivo efficacy of BCMA ADC in combination with anti-OX40 antibody

All procedures on animals were reviewed and approved by the GSK Institutional Animal Care and Use Committee prior to initiation of the studies.

### 4.1 Syngeneic EL4-Luc2-hBCMA Mouse Model

The purpose of these experiments was to evaluate the combinations described herein in mouse syngeneic tumorgenesis models. EL4-Luc2 (Bioware Ultra EL4-luc2 # 58230C40) was transfected with a plasmid encoding human BCMA. EL4 is a mouse lymphoma cell induced in a C57BL mouse by DBMA (ATCC TIB-39). At day -13, C57BL/6 female mice (n=10) were weighed and inoculated into the right flank of with 1 × 10⁵ of the transduced EL4- Luc2-hBCMA cells and allowed to grow until tumor volume reached ^{~} 700mm³. Tumor growth was measured using a Fowler "ProMax" digital caliper. Length (L) and width (W) of tumors were measured in order to determine tumor volume using the formula: Tumor Volume = 0.52 × L × W².

### 4.2 Dosing Regimen

At Day 0 when target tumor volume was achieved, the mice were randomized into 12 treatment groups. Treatments were admisistered at Day 4, Day 7, Day 11, and Day 15. Tumor volume and body weight was measured starting at Day 0 through Day 27 at 3 times per week. Animals were euthanized when a tumor reached a volume of 2000 mm³. The dosing regimen is summarized in Table 3.

**Table 3:**

| | | | **Treatment Days** | | | |
|---|---|---|---|---|---|---|
| **Group** | **Treatment** | **[mg/kg]** | **Day 4** | **Day 7** | **Day 11** | **Day 15** |
| **1** | Saline | | | | | |
| | | | | | | |
| **2** | Rat IgG1 | 5 | | | | |
| | | | | | | |
| **3** | MMAF-human IgG1 | 15 | | | | |
| | | | | | | |
| **4** | anti-mouse OX40 mAb (BioCell #BE0031) | 1 | | | | |
| **4** | MMAF-human IgG1 | 15 | | | | |
| | | | | | | |
| | anti-mouse OX40 mAb | | | | | |
| **5** | (BioCell #BE0031) | 5 | | | | |
| **5** | MMAF-human IgG1 | 15 | | | | |
| | | | | | | |
| **6** | anti-mouse OX40 mAb (BioCell #BE0031) | 0.25 | | | | |
| **6** | MMAF-human IgG1 | 15 | | | | |
| | | | | | | |
| **7** | Rat IgG1 | 5 | | | | |
| **7** | MMAF-human IgG1 | 15 | | | | |
| | | | | | | |
| **8** | GSK2857916 | 15 | | | | |
| | | | | | | |
| **9** | Rat IgG1 | 5 | | | | |
| **9** | GSK2857916 | 15 | | | | |
| | | | | | | |
| | anti-mouse OX40 mAb | | | | | |
| **10** | (BioCell #BE0031) | 5 | | | | |
| **10** | GSK2857916 | 15 | | | | |
| | | | | | | |
| **11** | anti-mouse OX40 mAb (BioCell #BE0031) | 1 | | | | |
| **11** | GSK2857916 | 15 | | | | |
| | | | | | | |
| **12** | anti-mouse OX40 mAb (BioCell #BE0031) | 0.25 | | | | |
| **12** | GSK2857916 | 15 | | | | |

### 4.3 Results

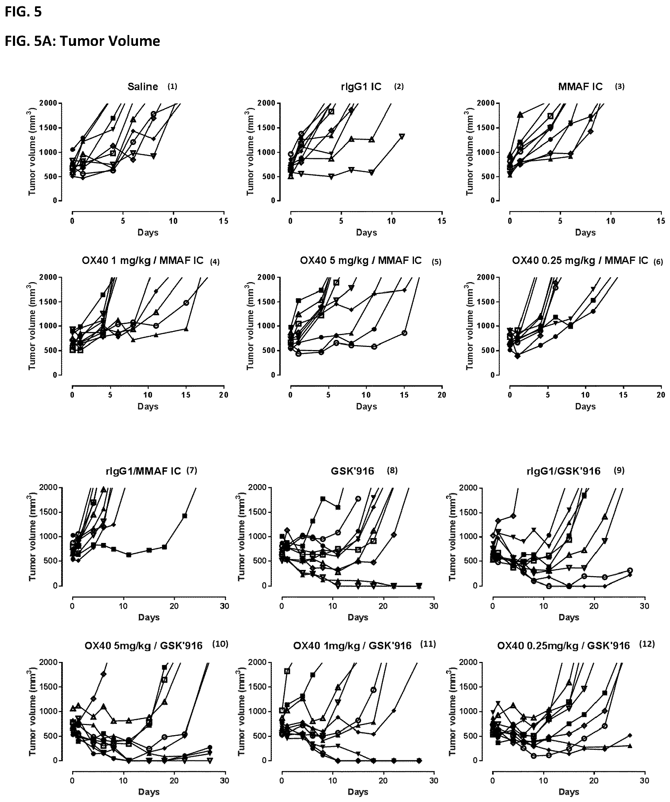

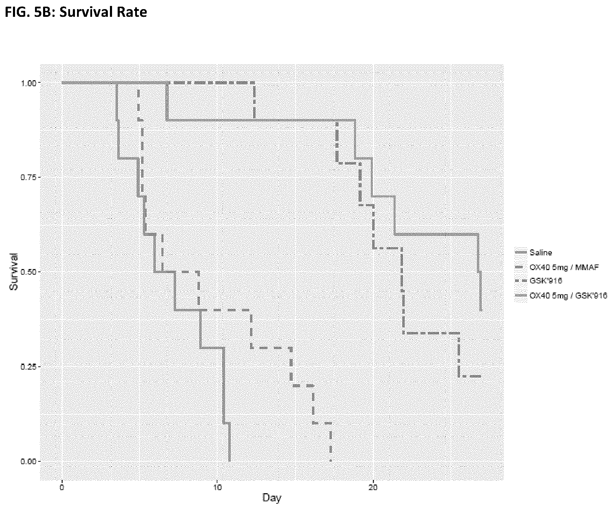

The results of Example 4 are reproduced in FIG. 5. FIG. 5A represents tumor volume. The X axis represents the number of days in the study and the Y axis represents tumor volume (mm³). Each line in a single graph in FIG. 5A represents a single mouse (n=10 mice for each treatment group). FIG. 5B represents the overall survival rate. These results demonstrate that tumor volume shrinkage is modestly enhanced when a BCMA ACD (anti-BCMA antibody conjugated to MMAF: GSK285791) is combined with an anti-OX40 antibody.

### Example 5: In vivo Efficacy of anti-BCMA Antibody in Combination with anti-PD-1 Antibody

All procedures on animals were reviewed and approved by the GSK Institutional Animal Care and Use Committee prior to initiation of the studies.

### 5.1 Syngeneic EL4-Luc2-hBCMA Mouse Model

The same EL4-Luc2-hBCMA mouse model was used as described in Example 4.

### 5.2 Dosing Regimen

At Day 0 when tumor volume reached an average of 200mm³, the mice were randomized into 13 treatment groups. Treatments were administered on Day 0, Day 4, Day 8, Day 11, Day 15, and Day 17. Treatments days and dosing schedule is summarized in Table 4. Tumor volume and body weight was measured starting at Day 0 through Day 57. Animals were euthanized when a tumor reached a volume of 2000 mm³. The dosing regimen is summarized in Table 4.

**Table 4:**

| | | | **Treatment Days** | | | | | |
|---|---|---|---|---|---|---|---|---|
| **Group** | **Treatment** | **[mg/kg]** | **Day 0** | **Day 4** | **Day 8** | **Day 11** | **Day 15** | **Day 17** |
| **1** | Saline | | | | | | | |
| | | | | | | | | |
| **2** | Rat anti-mouse IgG2a | 10 | | | | | | |
| | | | | | | | | |
| **3** | IgG-MMAF | 15 | | | | | | |
| | | | | | | | | |
| **4** | IgG-MMAF | 15 | | | | | | |
| **4** | Rat anti-mouse IgG2a | 10 | | | | | | |
| | | | | | | | | |
| **5** | Rat anti-mouse PD-1 (BioCel #BE0146 | 10 | | | | | | |
| | | | | | | | | |
| **6** | GSK2857916 | 10 | | | | | | |
| | | | | | | | | |
| **7** | GSK2857916 | 10 | | | | | | |
| **7** | Rat anti-mouse PD-1 (BioCel #BE0146) | 10 | | | | | | |
| | | | | | | | | |
| **8** | GSK2857916 | 15 | | | | | | |
| | | | | | | | | |
| **9** | GSK2857916 | 15 | | | | | | |
| **9** | Rat anti-mouse PD-1 (BioCel #8E0146) | 10 | | | | | | |
| | | | | | | | | |
| **10** | GSK2857916 | 15 | | | | | | |
| **10** | Rat anti-mouse PD-1 (BioCel #BE0146) | 10 | | | | | | |
| | | | | | | | | |
| **11** | GSK2857916 | 15 | | | | | | |
| **11** | Rat anti-mouse PD-1 (BioCel #BE0146) | 10 | | | | | | |
| | | | | | | | | |
| **12** | GSK2857916 | 15 | | | | | | |
| **12** | Rat anti-mouse PD-1 (BioCel #BE0146) | 10 | | | | | | |
| | | | | | | | | |
| **13** | GSK2857916 | 15 | | | | | | |
| **13** | Rat anti-mouse PD-1 (BioCel #BE0146) | 10 | | | | | | |

### 5.3 Results

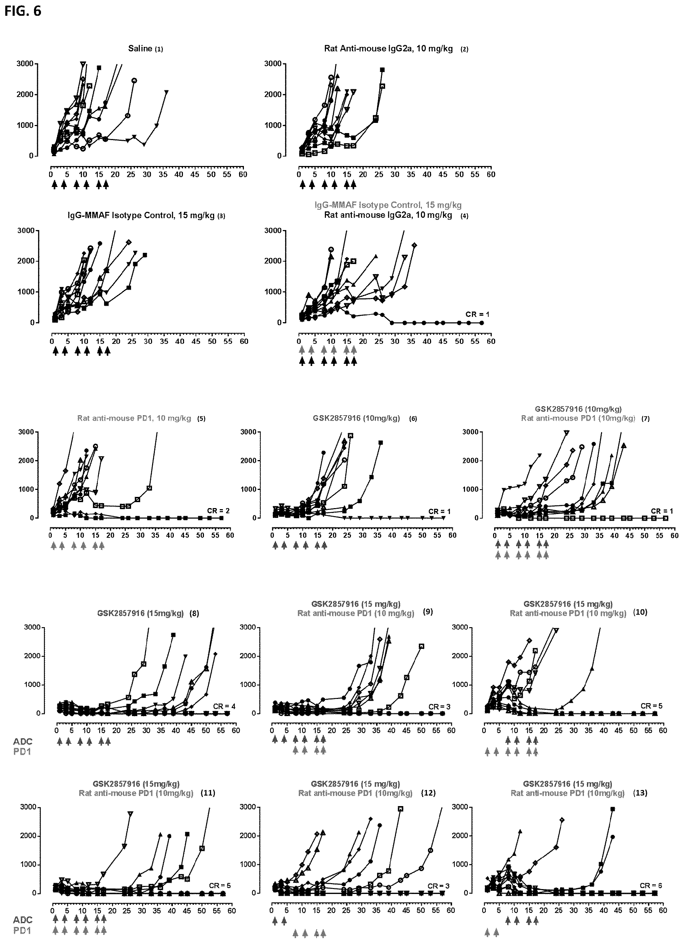

The resulting tumor volumes for Example 5 are represented in FIG. 6. The X axis represents the number of days in the study and the Y axis represents tumor volume (mm³). Each line in a single graph in FIG. 6 represents a single mouse (n=10 mice for each treatment group).

### Example 6: In vivo Efficacy of Combinations

All procedures on animals were reviewed and approved by the GSK Institutional Animal Care and Use Committee prior to initiation of the studies.

### 6.1 Syngeneic EL4-Luc2-hBCMA Mouse Model

The same EL4-Luc2-hBCMA mouse model was used as described in Example 4.

### 6.2 Dosing Regimen

At Day 0 when target tumor volume was achieved, the mice were randomized into 14 treatment groups. Treatments were admisistered at Day 0, Day 3, Day 7, Day 10, Day 14, and Day 17. Tumor volume and body weight was measured starting at Day 0 through Day 102. Animals were euthanized when a tumor reached a volume of 2000 mm³. The dosing regimen is summarized in Table 4. The dosing regimen is summarized in Table 5.

**Table 5:**

| | | | **Treatment Days** | | | | | |
|---|---|---|---|---|---|---|---|---|
| **Group** | **Treatment** | **(mg/kg)** | 0 | 3 | 7 | 10 | 14 | 17 |
| 1 | Rat anti-mouse IgG2a | 10 | | | | | | |
| | | | | | | | | |
| 2 | Rat anti-mouse PD-1 (BioCel #BE0146) | 10 | | | | | | |
| | | | | | | | | |
| 3 | MMAF human IgG1 | 15 | | | | | | |
| | | | | | | | | |
| 4 | MMAF human IgG1 | 15 | | | | | | |
| 4 | Rat anti-mouse IgG2a | 10 | | | | | | |
| | | | | | | | | |
| 5 | MMAF human IgG1 | 15 | | | | | | |
| 5 | Rat anti-mouse PD-1 (BioCel #BE0146) | 10 | | | | | | |
| | | | | | | | | |
| 6 | GSK2857916 | 15 | | | | | | |
| | | | | | | | | |
| 7 | GSK2857916 | 15 | | | | | | |
| 7 | Rat anti-mouse IgG2a | 10 | | | | | | |
| | | | | | | | | |
| 8 | GSK2857916 | 15 | | | | | | |
| 8 | Rat anti-mouse PD-1 (BioCel #BE0146) | 10 | | | | | | |
| | | | | | | | | |
| 9 | MMAF human IgG1 | 15 | | | | | | |
| | | | | | | | | |
| 10 | MMAF human IgG1 | 15 | | | | | | |
| 10 | Rat anti-mouse IgG2a | 10 | | | | | | |
| | | | | | | | | |
| 11 | MMAF human IgG1 | 15 | | | | | | |
| 11 | Rat anti-mouse PD-1 (BioCel #BE0146) | 10 | | | | | | |
| | | | | | | | | |
| 12 | GSK2857916 | 15 | | | | | | |
| | | | | | | | | |
| 13 | GSK2857916 | 15 | | | | | | |
| 13 | Rat anti-mouse IgG2a | 10 | | | | | | |
| | | | | | | | | |
| 14 | GSK2857916 | 15 | | | | | | |
| 14 | Rat anti-mouse PD-1 (BioCel #BE0146) | 10 | | | | | | |

### 6.3 Results

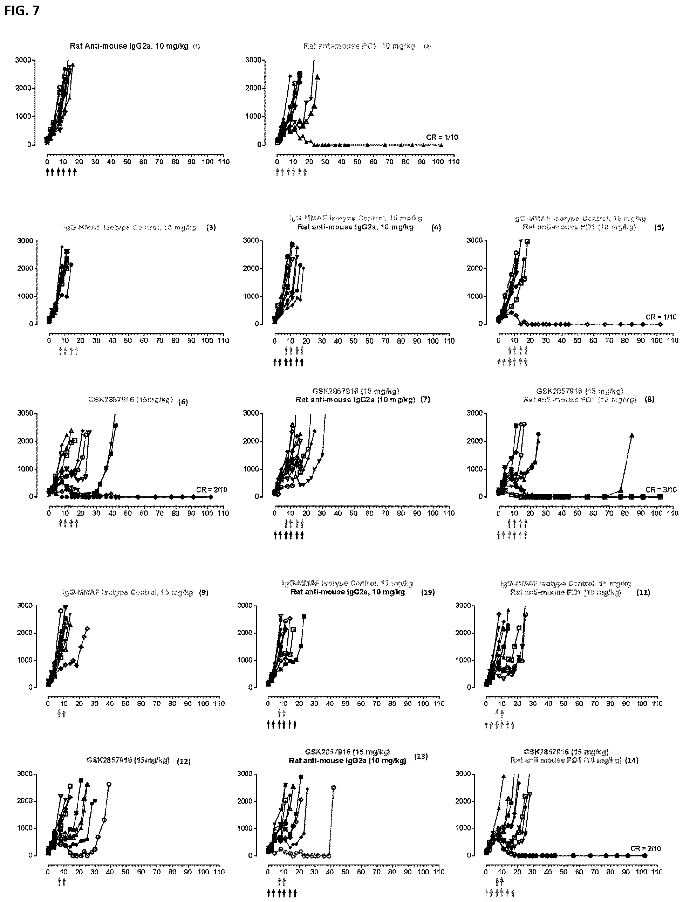

The resulting tumor volumes for Example 6 are represented by the graphs in FIG. 7. The X axis represents the number of days in the study and the Y axis represents tumor volume (mm³). Each line in a single graph in FIG. 7 represents a single mouse (n=10 mice for each treatment group). The results demonstrate that treatment with a combination of a BCMA ADC (anti-BCMA antibody conjugated to MMAF: GSK285791) and an anti-PD-1 antibody results in a moderate and consistent reduction in tumor volume compared to treatment with a BCMA ADC (anti-BCMA antibody conjugated to MMAF: GSK285791) alone or an anti-PD-1 antibody alone.

**Sequence Summary (Table A)**

| Description | Amino acid sequence | Polynucleotide sequence |
|---|---|---|
| CA8 CDRH1 | SEQ.I.D.NO:1 | n/a |
| CA8 CDRH2 | SEQ.I.D.NO:2 | n/a |
| CA8 CDRH3 | SEQ.I.D.NO:3 | n/a |
| CA8 CDRL1 | SEQ.I.D.NO:4 | n/a |
| CA8 CDRL2 | SEQ.I.D.NO:5 | n/a |
| CA8 CDRL3 | SEQ.I.D.NO:6 | n/a |
| CA8 V_{H} domain (murine) | SEQ.I.D.NO:7 | SEQ.I.D.NO:8 |
| CA8 V_{L} domain (murine) | SEQ.I.D.NO:9 | SEQ.I.D.NO:10 |
| CA8 Humanised V_{H} J0 | SEQ.I.D.NO:11 | SEQ.I.D.NO:12 |
| CA8 Humanised V_{H} J1 | SEQ.I.D.NO:13 | SEQ.I.D.NO:14 |
| CA8 Humanised V_{H} J2 | SEQ.I.D.NO:15 | SEQ.I.D.NO:16 |
| CA8 Humanised V_{H} J3 | SEQ.I.D.NO:17 | SEQ.I.D.NO:18 |
| CA8 Humanised V_{H} J4 | SEQ.I.D.NO:19 | SEQ.I.D.NO:20 |
| CA8 Humanised V_{H} J5 | SEQ.I.D.NO:21 | SEQ.I.D.NO:22 |
| CA8 Humanised V_{H} J6 | SEQ.I.D.NO:23 | SEQ.I.D.NO:24 |
| CA8 Humanised V_{H} J7 | SEQ.I.D.NO:25 | SEQ.I.D.NO:26 |
| CA8 Humanised V_{H} J8 | SEQ.I.D.NO:27 | SEQ.I.D.NO:28 |
| CA8 Humanised V_{H} J9 | SEQ.I.D.NO:29 | SEQ.I.D.NO:30 |
| CA8 Humanised V_{L} M0 | SEQ.I.D. NO:31 | SEQ.I.D.NO:32 |
| CA8 Humanised V_{L} M1 | SEQ.I.D. NO:33 | SEQ.I.D.NO:34 |
| CA8 Humanised V_{L} M2 | SEQ.I.D. NO:35 | SEQ.I.D.NO:36 |
| Human BCMA CD33-hBCMA ECD (1-53) TEV-Fc | SEQ.I.D.NO:37 | SEQ.I.D.NO:38 |
| Human BCMA CD33-hBCMA ECD (4-53) TEV-Fc | SEQ.I.D.NO:39 | SEQ.I.D.NQ:40 |
| Cyno BCMA CD33 cyno BCMA ECD (4-52) TEV-Fc | SEQ.I.D.NO:41 | SEQ.I.D.NO:42 |
| CA8 J0 Humanised heavy chain | SEQ.I.D.NO:43 | SEQ.I.D.NO:44 |
| CA8 J1 Humanised heavy chain | SEQ.I.D.NO:45 | SEQ.I.D.NO:46 |
| CA8 J2 Humanised heavy chain | SEQ.I.D.NO:47 | SEQ.I.D.NO:48 |
| CA8 J3 Humanised heavy chain | SEQ.I.D.NO:49 | SEQ.I.D.NO:50 |
| CA8 J4 Humanised heavy chain | SEQ.I.D.NO:51 | SEQ.I.D.NO:52 |
| CA8 J5 Humanised heavy chain | SEQ.I.D.NO:53 | SEQ.I.D.NO:54 |
| CA8 J6 Humanised heavy chain | SEQ.I.D.NO:55 | SEQ.I.D.NO:56 |
| CA8 J7 Humanised heavy chain | SEQ.I.D.NO:57 | SEQ.I.D.NO:58 |
| CA8 J8 Humanised heavy chain | SEQ.I.D.NO:59 | SEQ.I.D.NO:60 |
| CA8 J9 Humanised heavy chain | SEQ.I.D.NO:61 | SEQ.I.D.NO:62 |
| CA8 M0 Humanised light chain | SEQ.I.D.NO:63 | SEQ.I.D.NO:64 |
| CA8 M1 Humanised light chain | SEQ.I.D.NO:65 | SEQ.I.D.NO:66 |
| CA8 M2 Humanised light chain | SEQ.I.D.NO:67 | SEQ.I.D.NO:68 |
| S307118G03 V_{H} domain (murine) | SEQ.I.D.NO:69 | SEQ.I.D.NO:70 |
| S307118G03 V_{L} domain (murine) | SEQ.I.D.NO:71 | SEQ.I.D.NO:72 |
| S307118G03 heavy chain (chimeric) | SEQ.I.D.NO:73 | SEQ.I.D.NO:74 |
| S307118G03 light chain(chimeric) | SEQ.I.D.NO:75 | SEQ.I.D.NO:76 |
| S307118G03 Humanised V_{H} H0 | SEQ.I.D.NO:77 | SEQ.I.D.NO:78 |
| S307118G03 Humanised V_{H} H1 | SEQ.I.D.NO:79 | SEQ.I.D.NO:80 |
| S307118G03 humanised V_{H} H2 | SEQ.I.D.NO:81 | SEQ.I.D.NO:82 |
| S307118G03 humanised V_{H} H3 | SEQ.I.D.NO:83 | SEQ.I.D.NO:84 |
| S307118G03 humanised V_{H} H4 | SEQ.I.D.NO:85 | SEQ.I.D.NO:86 |
| S307118G03 humanised V_{H} H5 | SEQ.I.D.NO:87 | SEQ.I.D.NO:88 |
| S307118G03 humanised V_{L} L0 | SEQ.I.D.NO:89 | SEQ.I.D.NO:90 |
| S307118G03 humanised V_{L} L1 | SEQ.I.D.NO:91 | SEQ.I.D.NO:92 |
| S307118G03 CDRH1 | SEQ.I.D.NO:93 | n/a |
| S307118G03 CDRH2 | SEQ.I.D.NO:94 | n/a |
| S307118G03 CDRH3 | SEQ.I.D.NO:95 | n/a |
| S307118G03 CDRL1 | SEQ.I.D.NO:96 | n/a |
| S307118G03 CDRL2 | SEQ.I.D.NO:97 | n/a |
| S307118G03 CDRL3 | SEQ.I.D.NO:98 | n/a |
| S307118G03 humanised H5 CDRH3 | SEQ.I.D.NO:99 | n/a |
| S307118G03 H0 Humanised heavy chain | SEQ.I.D.NO:100 | SEQ.I.D.NO:101 |
| S307118G03 H1 humanised heavy chain | SEQ.I.D.NO:102 | SEQ.I.D.NO:103 |
| S307118G03 H2 humanised heavy chain | SEQ.I.D.NO:104 | SEQ.I.D.NO:105 |
| S307118G03 H3 humanised heavy chain | SEQ.I.D.NO:106 | SEQ.I.D.NO:107 |
| S307118G03 H4 humanised heavy chain | SEQ.I.D.NO:108 | SEQ.I.D.NO:109 |
| S307118G03 H5 humanised heavy chain | SEQ.I.D.NO:110 | SEQ.I.D.NO:111 |
| S307118G03 L0 humanised light chain | SEQ.I.D.NO:112 | SEQ.I.D.NO:113 |
| S307118G03 L1 humanised light chain | SEQ.I.D.NO:114 | SEQ.I.D.NO:115 |
| S332121F02 murine variable heavy chain | SEQ.I.D.NO:116 | SEQ.I.D.NO:117 |
| S332121F02 chimeric variable heavy chain | SEQ.I.D.NO:118 | SEQ.I.D.NO:119 |
| S332121F02 murine variable light chain | SEQ.I.D.NO:120 | SEQ.I.D.NO:121 |
| S332121F02 chimeric variable light chain | SEQ.I.D.NO:122 | SEQ.I.D.NO:123 |
| S322110D07 murine variable heavy chain | SEQ.I.D.NO:124 | SEQ.I.D.NO:125 |
| S322110D07 chimeric heavy chain | SEQ.I.D.NO:126 | SEQ.I.D.NO:127 |
| S322110D07 murine variable light chain | SEQ.I.D.NO:128 | SEQ.I.D.NO:129 |
| S322110D07 chimeric light chain | SEQ.I.D.NO:130 | SEQ.I.D.NO:131 |
| S332126E04 murine variable heavy chain | SEQ.I.D.NO:132 | SEQ.I.D.NO:133 |
| S332126E04 Chimeric heavy chain | SEQ.I.D.NO:134 | SEQ.I.D.NO:135 |
| S332126E04 murine variable light chain | SEQ.I.D.NO:136 | SEQ.I.D.NO:137 |
| S332126E04 Chimeric light chain | SEQ.I.D.NO:138 | SEQ.I.D.NO:139 |
| S336105A07 murine variable heavy chain | SEQ.I.D.NO:140 | SEQ.I.D.NO:141 |
| S336105A07 Chimeric heavy chain | SEQ.I.D.NO:142 | SEQ.I.D.NO:143 |
| S336105A07 murine variable light chain | SEQ.I.D.NO:144 | SEQ.I.D.NO:145 |
| S336105A07 chimeric light chain | SEQ.I.D.NO:146 | SEQ.I.D.NO:147 |
| S335115G01 murine variable heavy chain | SEQ.I.D.NO:148 | SEQ.I.D.NO:149 |
| S335115G01 Chimeric heavy chain | SEQ.I.D.NO:150 | SEQ.I.D.NO:151 |
| S335115G01 murine variable light chain | SEQ.I.D.NO:152 | SEQ.I.D.NO:153 |
| S335115G01 Chimeric light chain | SEQ.I.D.NO:154 | SEQ.I.D.NO:155 |
| S335122F05 murine variable heavy chain | SEQ.I.D.NO:156 | SEQ.I.D.NO:158 |
| S335122F05 Chimeric heavy chain | SEQ.I.D.NO:158 | SEQ.I.D.NO:159 |
| S335122F05 murine variable light chain | SEQ.I.D.NO:160 | SEQ.I.D.NO:161 |
| S335122F05 Chimeric light chain | SEQ.I.D.NO:162 | SEQ.I.D.NO:163 |
| S332121F02 CDRH1 | SEQ.I.D.NO: 164 | n/a |
| S332121F02 CDRH2 | SEQ.I.D.NO: 165 | n/a |
| S332121F02 CDRH3 | SEQ.I.D.NO: 166 | n/a |
| S332121F02 CDRL1 | SEQ.I.D.NO: 167 | n/a |
| S332121F02 CDRL2 | SEQ.I.D.NO: 168 | n/a |
| S332121F02 CDRL3 | SEQ.I.D.NO: 169 | n/a |
| S322110D07 CDRH1 | SEQ.I.D.NO: 170 | n/a |
| S322110D07 CDRH2 | SEQ.I.D.NO: 171 | n/a |
| S322110D07 CDRH3 | SEQ.I.D.NO: 172 | n/a |
| S322110D07CDRL1 | SEQ.I.D.NO: 173 | n/a |
| S322110D07 CDRL2 | SEQ.I.D.NO: 174 | n/a |
| S322110D07 CDRL3 | SEQ.I.D.NO: 175 | n/a |
| S332126E04CDRH1 | SEQ.I.D.NO: 176 | n/a |
| S332126E04 CDRH2 | SEQ.I.D.NO: 177 | n/a |
| S332126E04 CDRH3 | SEQ.I.D.NO: 178 | n/a |
| S332126E04 CDRL1 | SEQ.I.D.NO: 179 | n/a |
| S332126E04 CDRL2 | SEQ.I.D.NO: 180 | n/a |
| S332126E04 CDRL3 | SEQ.I.D.NO: 181 | n/a |
| S336105A07 CDRH1 | SEQ.I.D.NO: 182 | n/a |
| S336105A07 CDRH2 | SEQ.I.D.NO: 183 | n/a |
| S336105A07 CDRH3 | SEQ.I.D.NO: 184 | n/a |
| S336105A07 CDRL1 | SEQ.I.D.NO: 185 | n/a |
| S336105A07 CDRL2 | SEQ.I.D.NO: 186 | n/a |
| S336105A07 CDRL3 | SEQ.I.D.NO: 187 | n/a |
| S335115G01 CDRH1 | SEQ.I.D.NO: 188 | n/a |
| S335115G01 CDRH2 | SEQ.I.D.NO: 189 | n/a |
| S335115G01 CDRH3 | SEQ.I.D.NO: 190 | n/a |
| S335115G01 CDRL1 | SEQ.I.D.NO: 191 | n/a |
| S335115G01 CDRL2 | SEQ.I.D.NO: 192 | n/a |
| S335115G01 CDRL3 | SEQ.I.D.NO: 193 | n/a |
| S335122F05 CDRH1 | SEQ.I.D.NO: 194 | n/a |
| S335122F05 CDRH2 | SEQ.I.D.NO: 195 | n/a |
| S335122F05 CDRH3 | SEQ.I.D.NO: 196 | n/a |
| S335122F05 CDRL1 | SEQ.I.D.NO: 197 | n/a |
| S335122F05 CDRL2 | SEQ.I.D.NO: 198 | n/a |
| S335122F05 CDRL3 | SEQ.I.D.NO: 199 | n/a |
| CA8 CDRH3 variant N99D | SEQ.I.D.NO:200 | n/a |
| Pembrolizumab CDRH1 | SEQ.I.D.NO:201 | n/a |
| Pembrolizumab CDRH2 | SEQ.I.D.NO:202 | n/a |
| Pembrolizumab CDRH3 | SEQ.I.D.NO:203 | n/a |
| Pembrolizumab CDRL1 | SEQ.I.D.NO:204 | n/a |
| Pembrolizumab CDRL2 | SEQ.I.D.NO:205 | n/a |
| Pembrolizumab CDRL3 | SEQ.I.D.NO:206 | n/a |
| Pembrolizumab variable heavy chain (VH) | SEQ.I.D.NO:207 | n/a |
| Pembrolizumab variable light chain (VL) | SEQ.I.D.NO:208 | n/a |
| Pembrolizumab Heavy chain | SEQ.I.D.NO:209 | n/a |
| Pembrolizumab Light chain | SEQ.I.D.NO:210 | n/a |
| Nivolumab CDRH1 | SEQ.I.D.NO:211 | n/a |
| Nivolumab CDRH2 | SEQ.I.D.NO:212 | n/a |
| Nivolumab CDRH3 | SEQ.I.D.NO:213 | n/a |
| Nivolumab CDRL1 | SEQ.I.D.NO:214 | n/a |
| Nivolumab CDRL2 | SEQ.I.D.NO:215 | n/a |
| Nivolumab CDRL3 | SEQ.I.D.NO:216 | n/a |
| Nivolumab variable heavy chain (VH) | SEQ.I.D.NO:217 | n/a |
| Nivolumab variable light chain (VL) | SEQ.I.D.NO:218 | n/a |
| 106-222 CDRH1 | SEQ.I.D.NO:219 | n/a |
| 106-222 CDRH2 | SEQ.I.D.NO:220 | n/a |
| 106-222 CDRH3 | SEQ.I.D.NO:221 | n/a |
| 106-222 CDRL1 | SEQ.I.D.NO:222 | n/a |
| 106-222 CDRL2 | SEQ.I.D.NO:223 | n/a |
| 106-222 CDRL3 | SEQ.I.D.NO:224 | n/a |
| 106-222 Variable heavy chain | SEQ.I.D.NO:225 | SEQ.I.D.NO:226 |
| 106-222 Variable light chain | SEQ.I.D.NO:227 | SEQ.I.D.NO:228 |
| 106-222 Variable heavy chain | SEQ.I.D.NO:229 | n/a |
| 106-222 Variable light chain | SEQ.I.D.NO:230 | n/a |
| 119-222 CDRH1 | SEQ.I.D.NO:231 | n/a |
| 1119-222 CDRH2 | SEQ.I.D.NO:232 | n/a |
| 119-222 CDRH3 | SEQ.I.D.NO:233 | n/a |
| 119-222 CDRL1 | SEQ.I.D.NO:234 | n/a |
| 119-222 CDRL2 | SEQ.I.D.NO:235 | n/a |
| 119-222 CDRL3 | SEQ.I.D.NO:236 | n/a |
| 119-222 Variable heavy chain | SEQ.I.D.NO:237 | SEQ.I.D.NO:238 |
| 119-222 Variable light chain | SEQ.I.D.NO:239 | SEQ.I.D.NO:240 |
| 119-222 Variable heavy chain | SEQ.I.D.NO:241 | n/a |
| 119-222 Variable light chain | SEQ.I.D.NO:242 | n/a |
| 106-222 Heavy Chain | SEQ.I.D.NO: 243 | n/a |
| 106-222 Light Chain | SEQ.I.D.NO: 244 | n/a |

### SEQUENCE LISTING

**SEQ ID 1- CA8 CDRH1**
NYWMH

**SEQ ID 2 - CA8 CDRH2**
ATYRGHSDTYYNQKFKG

**SEQ ID 3 - CA8 CDRH3**
GAIYNGYDVLDN

**SEQ ID 4 - CA8 CDRL1**
SASQDISNYLN

**SEQ ID 5 - CA8 CDRL2**
YTSNLHS

**SEQ ID 6 - CA8 CDRL3**
QQYRKLPWT

**SEQ ID 7 - CA8 V_{H} domain (murine)**

EVQLQQSGAVLARPGASVKMSCKGSGYTFTNYWMHWVKQRPGQGLEWIGATYRGHSDTYYNQKFKGKAKLTA

VTSTSTAYMELSSLTNEDSAVYYCTRGAIYNGYDVLDNWGQGTLVTVSS

**SEQ ID 8 - CA8 V_{H} domain (murine) (Polynucleotide)**

GAGGTGCAGCTGCAGCAGAGCGGCGCCGTGCTGGCCAGGCCCGGAGCTAGCGTGAAGATGAGCTGCAAGG
GCAGCGGCTACACCTTCACCAACTACTGGATGCACTGGGTGAAACAGAGGCCCGGCCAGGGACTGGAGTGG
ATCGGCGCCACCTACAGGGGCCACAGCGACACCTACTACAACCAGAAGTTCAAGGGCAAGGCCAAGCTGACC
GCCGTGACCTCAACCAGCACCGCCTACATGGAACTGAGCAGCCTGACCAACGAGGACAGCGCCGTCTATTACT
GCACCAGGGGCGCCATCTACAACGGCTACGACGTGCTGGACAATTGGGGCCAGGGAACACTAGTGACCGTGT
CCAGC

**SEQ ID 9 - CA8 V_{L} domain (murine)**

DIQLTQTTSSLSASLGDRVTISCSASQDISNYLNWYQQKPDGTVELVIYYTSNLHSGVPSRFSGSGSGTDYSLTIGYLE
PEDVATYYCQQYRKLPWTFGGGSKLEIKR

**SEQ ID 10 - CA8 V_{L} domain (murine) (Polynucleotide)**

GATATCCAGCTGACCCAGACCACAAGCAGCCTGAGCGCCTCCCTGGGCGACAGGGTGACCATTAGCTGCAGC
GCCAGCCAGGACATCAGCAACTACCTGAACTGGTACCAGCAGAAGCCCGACGGCACCGTGGAGCTCGTGATC
TACTACACCTCCAACCTGCACAGCGGCGTGCCCAGCAGGTTCTCTGGCAGCGGCAGCGGCACCGACTACAGCC
TGACCATCGGCTATCTGGAGCCCGAGGACGTCGCCACCTACTACTGCCAGCAGTACAGGAAGCTGCCCTGGA
CCTTCGGCGGAGGCTCTAAGCTGGAGATTAAGCGT

**SEQ ID 11 - CA8 Humanised V_{H} J0**

QVQLVQSGAEVKKPGSSVKVSCKASGGTFSNYWMHWVRQAPGQGLEWMGATYRGHSDTYYNQKFKGRVTITA
DKSTSTAYMELSSLRSEDTAVYYCARGAIYNGYDVLDNWGQGTLVTVSS

**SEQ ID 12 - CA8 Humanised V_{H} J0 (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCGGCACCTTCAGCAACTACTGGATGCACTGGGTGAGGCAGGCCCCCGGACAGGGCCTGGAGTGGA
TGGGCGCCACCTACAGGGGCCACAGCGACACCTACTACAACCAGAAGTTCAAGGGCCGGGTGACCATCACCG
CCGACAAGAGCACCAGCACCGCCTACATGGAACTGAGCAGCCTCAGGAGCGAGGACACCGCTGTGTATTACT
GCGCCAGGGGCGCCATCTACAACGGCTACGACGTGCTGGACAACTGGGGCCAGGGCACACTAGTGACCGTG
TCCAGC

**SEQ ID 13 - CA8 Humanised V_{H} J1**

QVQLVQSGAEVKKPGSSVKVSCKASGYTFTNYWMHWVRQAPGQGLEWMGATYRGHSDTYYNQKFKGRVTITA
DKSTSTAYMELSSLRSEDTAVYYCARGAIYNGYDVLDNWGQGTLVTVSS

**SEQ ID 14 - CA8 Humanised V_{H} J1 (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACAAGAGCACCAGCACCGCCTACATGGAACTGAGCAGCCTCAGGAGCGAGGACACCGCTGTGTATTACTG
CGCCAGGGGCGCCATCTACAACGGCTACGACGTGCTGGACAACTGGGGCCAGGGCACACTAGTGACCGTGTC
CAGC

**SEQ ID 15 - CA8 Humanised V_{H} J2**

QVQLVQSGAEVKKPGSSVKVSCKASGYTFTNYWMHWVRQAPGQGLEWMGATYRGHSDTYYNQKFKGRVTITA
DKSTSTAYMELSSLRSEDTAVYYCTRGAIYNGYDVLDNWGQGTLVTVSS

**SEQ ID 16 - CA8 Humanised V_{H} J2 (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACAAGAGCACCAGCACCGCCTACATGGAACTGAGCAGCCTCAGGAGCGAGGACACCGCTGTGTATTACTG
CACCAGGGGCGCCATCTACAACGGCTACGACGTGCTGGACAACTGGGGCCAGGGCACACTAGTGACCGTGTC
CAGC

**SEQ ID 17 - CA8 Humanised V_{H} J3**

QVQLVQSGAEVKKPGSSVKVSCKGSGYTFTNYWMHWVRQAPGQGLEWMGATYRGHSDTYYNQKFKGRVTITA
DTSTSTAYMELSSLRSEDTAVYYCTRGAIYNGYDVLDNWGQGTLVTVSS

**SEQ ID 18 - CA8 Humanised V_{H} J3 (Polynucleotide)**

CAGGTGCAGCTGGTCCAGAGCGGCGCCGAAGTGAAGAAGCCCGGCAGCTCCGTGAAAGTGAGCTGCAAGGG
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACACGAGCACCAGCACCGCCTACATGGAACTGAGCAGCCTCAGGAGCGAGGACACCGCTGTGTATTACTG
CACCAGGGGCGCCATCTACAACGGCTACGACGTGCTGGACAACTGGGGCCAGGGCACACTAGTGACCGTGTC
CAGC

**SEQ ID 19 - CA8 Humanised V_{H} J4**

QVQLVQSGAEVKKPGSSVKVSCKGSGYTFTNYWMHWVRQAPGQGLEWIGATYRGHSDTYYNQKFKGRATLTA
DTSTSTAYMELSSLRSEDTAVYYCTRGAIYNGYDVLDNWGQGTLVTVSS

**SEQ ID 20 - CA8 Humanised V_{H} J4 (Polynucleotide)**

CAGGTGCAGCTGGTCCAGAGCGGCGCCGAAGTGAAGAAGCCCGGCAGCTCCGTGAAAGTGAGCTGCAAGGG
CAGCGGCTACACCTTCACCAACTACTGGATGCACTGGGTGAGGCAGGCCCCCGGACAGGGCCTGGAGTGGAT
CGGCGCCACCTACAGGGGCCACAGCGACACCTACTACAACCAGAAGTTCAAGGGCCGGGCGACCCTCACCGC
CGACACGAGCACCAGCACCGCCTACATGGAACTGAGCAGCCTCAGGAGCGAGGACACCGCTGTGTATTACTG
CACCAGGGGCGCCATCTACAACGGCTACGACGTGCTGGACAACTGGGGCCAGGGCACACTAGTGACCGTGTC
CAGC

**SEQ ID 21 - CA8 Humanised V_{H} J5**

QVQLVQSGAEVKKPGSSVKVSCKGSGYTFTNYWMHWVRQAPGQGLEWMGATYRGHSDTYYNQKFKGRVTITA
DTSTSTAYMELSSLRSEDTAVYYCTRGAIYDGYDVLDNWGQGTLVTVSS

**SEQ ID 22 - CA8 Humanised V_{H} J5 (Polynucleotide)**

CAGGTGCAGCTGGTCCAGAGCGGCGCCGAAGTGAAGAAGCCCGGCAGCTCCGTGAAAGTGAGCTGCAAGGG
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACACGAGCACCAGCACCGCCTACATGGAACTGAGCAGCCTCAGGAGCGAGGACACCGCTGTGTATTACTG
CACCAGGGGCGCCATCTACGACGGCTACGACGTGCTGGACAACTGGGGCCAGGGCACACTAGTGACCGTGTC
CAGC

**SEQ ID 23 - CA8 Humanised V_{H} J6**

QVQLVQSGAEVKKPGSSVKVSCKASGGTFSNYWMHWVRQAPGQGLEWMGATYRGHSDTYYNQKFKGRVTITA
DKSTSTAYMELSSLRSEDTAVYYCARGAIYDGYDVLDNWGQGTLVTVSS

**SEQ ID 24 - CA8 Humanised V_{H} J6 (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCGGCACCTTCAGCAACTACTGGATGCACTGGGTGAGGCAGGCCCCCGGACAGGGCCTGGAGTGGA
TGGGCGCCACCTACAGGGGCCACAGCGACACCTACTACAACCAGAAGTTCAAGGGCCGGGTGACCATCACCG

CCGACAAGAGCACCAGCACCGCCTACATGGAACTGAGCAGCCTCAGGAGCGAGGACACCGCTGTGTATTACT
GCGCCAGGGGCGCCATCTACGACGGCTACGACGTGCTGGACAACTGGGGCCAGGGCACACTAGTGACCGTG
TCCAGC

**SEQ ID 25 - CA8 Humanised V_{H} J7**

QVQLVQSGAEVKKPGSSVKVSCKASGYTFTNYWMHWVRQAPGQGLEWMGATYRGHSDTYYNQKFKGRVTITA
DKSTSTAYMELSSLRSEDTAVYYCARGAIYDGYDVLDNWGQGTLVTVSS

**SEQ ID 26 - CA8 Humanised V_{H} J7 (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACAAGAGCACCAGCACCGCCTACATGGAACTGAGCAGCCTCAGGAGCGAGGACACCGCTGTGTATTACTG
CGCCAGGGGCGCCATCTACGACGGCTACGACGTGCTGGACAACTGGGGCCAGGGCACACTAGTGACCGTGTC
CAGC

**SEQ ID 27 - CA8 Humanised V_{H} J8**

QVQLVQSGAEVKKPGSSVKVSCKASGYTFTNYWMHWVRQAPGQGLEWMGATYRGHSDTYYNQKFKGRVTITA
DKSTSTAYMELSSLRSEDTAVYYCTRGAIYDGYDVLDNWGQGTLVTVSS

**SEQ ID 28 - CA8 Humanised V_{H} J8 (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACAAGAGCACCAGCACCGCCTACATGGAACTGAGCAGCCTCAGGAGCGAGGACACCGCTGTGTATTACTG
CACCAGGGGCGCCATCTACGACGGCTACGACGTGCTGGACAACTGGGGCCAGGGCACACTAGTGACCGTGTC
CAGC

**SEQ ID 29 - CA8 Humanised V_{H} J9**

QVQLVQSGAEVKKPGSSVKVSCKGSGYTFTNYWMHWVRQAPGQGLEWIGATYRGHSDTYYNQKFKGRATLTA
DTSTSTAYMELSSLRSEDTAVYYCTRGAIYDGYDVLDNWGQGTLVTVSS

**SEQ ID 30 - CA8 Humanised V_{H} J9 (Polynucleotide)**

CAGGTGCAGCTGGTCCAGAGCGGCGCCGAAGTGAAGAAGCCCGGCAGCTCCGTGAAAGTGAGCTGCAAGGG
CAGCGGCTACACCTTCACCAACTACTGGATGCACTGGGTGAGGCAGGCCCCCGGACAGGGCCTGGAGTGGAT
CGGCGCCACCTACAGGGGCCACAGCGACACCTACTACAACCAGAAGTTCAAGGGCCGGGCGACCCTCACCGC
CGACACGAGCACCAGCACCGCCTACATGGAACTGAGCAGCCTCAGGAGCGAGGACACCGCTGTGTATTACTG
CACCAGGGGCGCCATCTACGACGGCTACGACGTGCTGGACAACTGGGGCCAGGGCACACTAGTGACCGTGTC
CAGC

**SEQ ID 31 - CA8 Humanised V_{L} M0**

DIQMTQSPSSLSASVGDRVTITCSASQDISNYLNWYQQKPGKAPKLLIYYTSNLHSGVPSRFSGSGSGTDFTLTISSL
QPEDFATYYCQQYRKLPWTFGQGTKLEIKR

**SEQ ID 32 - CA8 Humanised V_{L} M0 (Polynucleotide)**

GACATCCAGATGACCCAGAGCCCTAGCTCACTGAGCGCCAGCGTGGGCGACAGGGTGACCATTACCTGCTCC
GCCAGCCAGGACATCAGCAACTACCTGAACTGGTACCAGCAGAAGCCCGGCAAGGCCCCCAAGCTGCTGATC
TACTACACCTCCAACCTGCACTCCGGCGTGCCCAGCAGGTTCAGCGGAAGCGGCAGCGGCACCGATTTCACCC
TGACCATCTCCAGCCTGCAGCCCGAGGACTTCGCCACCTACTACTGCCAGCAGTACAGGAAGCTCCCCTGGAC
TTTCGGCCAGGGCACCAAACTGGAGATCAAGCGT

**SEQ ID 33 - CA8 Humanised V_{L} M1**

DIQMTQSPSSLSASVGDRVTITCSASQDISNYLNWYQQKPGKAPKLLIYYTSNLHSGVPSRFSGSGSGTDYTLTISSL
QPEDFATYYCQQYRKLPWTFGQGTKLEIKR

**SEQ ID 34 - CA8 Humanised V_{L} M1 (Polynucleotide)**

GACATCCAGATGACCCAGAGCCCTAGCTCACTGAGCGCCAGCGTGGGCGACAGGGTGACCATTACCTGCTCC
GCCAGCCAGGACATCAGCAACTACCTGAACTGGTACCAGCAGAAGCCCGGCAAGGCCCCCAAGCTGCTGATC
TACTACACCTCCAACCTGCACTCCGGCGTGCCCAGCAGGTTCAGCGGAAGCGGCAGCGGCACCGATTACACCC
TGACCATCTCCAGCCTGCAGCCCGAGGACTTCGCCACCTACTACTGCCAGCAGTACAGGAAGCTCCCCTGGAC
TTTCGGCCAGGGCACCAAACTGGAGATCAAGCGT

**SEQ ID 35 - CA8 Humanised V_{L} M2**

DIQLTQSPSSLSASVGDRVTITCSASQDISNYLNWYQQKPGKAPELVIYYTSNLHSGVPSRFSGSGSGTDYTLTISSLQ
PEDFATYYCQQYRKLPWTFGQGTKLEIKR

**SEQ ID 36 - CA8 Humanised V_{L} M2 (Polynucleotide)**

GACATCCAGCTGACCCAGAGCCCTAGCTCACTGAGCGCCAGCGTGGGCGACAGGGTGACCATTACCTGCTCC
GCCAGCCAGGACATCAGCAACTACCTGAACTGGTACCAGCAGAAGCCCGGCAAGGCCCCCGAGCTGGTGATC
TACTACACCTCCAACCTGCACTCCGGCGTGCCCAGCAGGTTCAGCGGAAGCGGCAGCGGCACCGATTACACCC
TGACCATCTCCAGCCTGCAGCCCGAGGACTTCGCCACCTACTACTGCCAGCAGTACAGGAAGCTCCCCTGGAC
TTTCGGCCAGGGCACCAAACTGGAGATCAAGCGT

**SEQ ID 37 - Human BCMA CD33-hBCMA ECD (1-53) TEV-Fc**

MPLLLLLPLLWAGALAMLQMAGQCSQNEYFDSLLHACIPCQLRCSSNTPPLTCQRYCNASVTNSVKGTNSGENLY
FQGDPKSCDKTHTCPPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYTQK
SLSLSPGK

**SEQ ID 38 - Human BCMA CD33-hBCMA ECD (1-53) TEV-Fc (Polynucleotide)**

ATGCCGCTGCTGCTACTGCTGCCCCTGCTGTGGGCAGGGGCGCTAGCTATGCTGCAGATGGCCGGCCAGTGC
AGCCAGAACGAGTACTTCGACAGCCTGCTGCACGCCTGCATCCCCTGCCAGCTGAGATGCAGCAGCAACACAC
CTCCTCTGACCTGCCAGAGATACTGCAACGCCAGCGTGACCAACAGCGTGAAGGGCACCAACTCCGGAGAGA
ACCTGTACTTCCAAGGGGATCCCAAATCTTGTGACAAAACTCACACATGCCCACCGTGCCCAGCACCTGAACTC
CTGGGGGGACCGTCAGTCTTCCTCTTCCCCCCAAAACCCAAGGACACCCTCATGATCTCCCGGACCCCTGAGGT
CACATGCGTGGTGGTGGACGTGAGCCACGAAGACCCTGAGGTCAAGTTCAACTGGTACGTGGACGGCGTGG
AGGTGCATAATGCCAAGACAAAGCCGCGGGAGGAGCAGTACAACAGCACGTACCGTGTGGTCAGCGTCCTCA
CCGTCCTGCACCAGGACTGGCTGAATGGCAAGGAGTACAAGTGCAAGGTCTCCAACAAAGCCCTCCCAGCCC
CCATCGAGAAAACCATCTCCAAAGCCAAAGGGCAGCCCCGAGAGCCACAGGTGTACACCCTGCCCCCATCCCG
GGATGAGCTGACCAAGAACCAGGTCAGCCTGACCTGCCTGGTCAAAGGCTTCTATCCCAGCGACATCGCCGT
GGAGTGGGAGAGCAATGGGCAGCCGGAGAACAACTACAAGACCACGCCTCCCGTGCTGGACTCCGACGGCT
CCTTCTTCCTCTACAGCAAGCTCACCGTGGACAAGAGCAGGTGGCAGCAGGGGAACGTCTTCTCATGCTCCGT
GATGCATGAGGCTCTGCACAACCACTACACGCAGAAGAGCCTCTCCCTGTCTCCGGGTAAA

**SEQ ID 39- Human BCMA CD33-hBCMA ECD (4-53) TEV-Fc**

MPLLLLLPLLWAGALAMAGQCSQNEYFDSLLHACIPCQLRCSSNTPPLTCQRYCNASVTNSVKGTNSGENLYFQG
DPKSCDKTHTCPPCPAPELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSLSL
SPGK

**SEQ ID 40 - Human BCMA CD33-hBCMA ECD (4-53) TEV-Fc (Polynucleotide)**

ATGCCGCTGCTGCTACTGCTGCCCCTGCTGTGGGCAGGGGCGCTAGCTATGGCCGGCCAGTGCAGCCAGAAC
GAGTACTTCGACAGCCTGCTGCACGCCTGCATCCCCTGCCAGCTGAGATGCAGCAGCAACACACCTCCTCTGA
CCTGCCAGAGATACTGCAACGCCAGCGTGACCAACAGCGTGAAGGGCACCAACTCCGGAGAGAACCTGTACT
TCCAAGGGGATCCCAAATCTTGTGACAAAACTCACACATGCCCACCGTGCCCAGCACCTGAACTCCTGGGGGG
ACCGTCAGTCTTCCTCTTCCCCCCAAAACCCAAGGACACCCTCATGATCTCCCGGACCCCTGAGGTCACATGCG
TGGTGGTGGACGTGAGCCACGAAGACCCTGAGGTCAAGTTCAACTGGTACGTGGACGGCGTGGAGGTGCAT
AATGCCAAGACAAAGCCGCGGGAGGAGCAGTACAACAGCACGTACCGTGTGGTCAGCGTCCTCACCGTCCTG
CACCAGGACTGGCTGAATGGCAAGGAGTACAAGTGCAAGGTCTCCAACAAAGCCCTCCCAGCCCCCATCGAG
AAAACCATCTCCAAAGCCAAAGGGCAGCCCCGAGAGCCACAGGTGTACACCCTGCCCCCATCCCGGGATGAG
CTGACCAAGAACCAGGTCAGCCTGACCTGCCTGGTCAAAGGCTTCTATCCCAGCGACATCGCCGTGGAGTGG
GAGAGCAATGGGCAGCCGGAGAACAACTACAAGACCACGCCTCCCGTGCTGGACTCCGACGGCTCCTTCTTC
CTCTACAGCAAGCTCACCGTGGACAAGAGCAGGTGGCAGCAGGGGAACGTCTTCTCATGCTCCGTGATGCAT
GAGGCTCTGCACAACCACTACACGCAGAAGAGCCTCTCCCTGTCTCCGGGTAAA

**SEQ ID 41- Cynomolgous BCMA CD33 cyno BCMA ECD (4-52) TEV-Fc**

MPLLLLLPLLWAGALAMARQCSQNEYFDSLLHDCKPCQLRCSSTPPLTCQRYCNASMTNSVKGMNSGENLYFQG
DPKSCDKTHTCPPCPAPELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEALHNHYTQKSLSL
SPGK

**SEQ ID 42 - Cynomolgous BCMA CD33 cyno BCMA ECD (4-52) TEV-Fc (Polynucleotide)**

ATGCCGCTGCTGCTACTGCTGCCCCTGCTGTGGGCAGGGGCGCTAGCTATGGCCAGACAGTGCAGCCAGAAC
GAGTACTTCGACAGCCTGCTGCACGACTGCAAGCCCTGCCAGCTGAGATGCAGCAGCACCTCCTCTGACCT
GCCAGAGATACTGCAACGCCAGCATGACCAACAGCGTGAAGGGCATGAACTCCGGAGAACCTGTACTTCC
AAGGGGATCCCAAATCTTGTGACAAAACTCACACATGCCCACCGTGCCCAGCACCTGAACTCCTGGGGGGACC
GTCAGTCTTCCTCTTCCCCCCAAAACCCAAGGACACCCTCATGATCTCCCGGACCCCTGAGGTCACATGCGTGG
TGGTGGACGTGAGCCACGAAGACCCTGAGGTCAAGTTCAACTGGTACGTGGACGGCGTGGAGGTGCATAAT
GCCAAGACAAAGCCGCGGGAGGAGCAGTACAACAGCACGTACCGTGTGGTCAGCGTCCTCACCGTCCTGCAC
CAGGACTGGCTGAATGGCAAGGAGTACAAGTGCAAGGTCTCCAACAAAGCCCTCCCAGCCCCCATCGAGAAA
ACCATCTCCAAAGCCAAAGGGCAGCCCCGAGAGCCACAGGTGTACACCCTGCCCCCATCCCGGGATGAGCTG
ACCAAGAACCAGGTCAGCCTGACCTGCCTGGTCAAAGGCTTCTATCCCA
GCGACATCGCCGTGGAGTGGGAGAGCAATGGGCAGCCGGAGAACAACTACAAGACCACGCCTCCCGTGCTG
GACTCCGACGGCTCCTTCTTCCTCTACAGCAAGCTCACCGTGGACAAGAGCAGGTGGCAGCAGGGGAACGTC
TTCTCATGCTCCGTGATGCATGAGGCTCTGCACAACCACTACACGCAGAAGAGCCTCTCCCTGTCTCCGGGTAA
A

**SEQ ID 43- CA8 J0 Humanised heavy chain**

QVQLVQSGAEVKKPGSSVKVSCKASGGTFSNYWMHWVRQAPGQGLEWMGATYRGHSDTYYNQKFKGRVTITA
DKSTSTAYMELSSLRSEDTAVYYCARGAIYNGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 44 - CA8 J0 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCGGCACCTTCAGCAACTACTGGATGCACTGGGTGAGGCAGGCCCCCGGACAGGGCCTGGAGTGGA
TGGGCGCCACCTACAGGGGCCACAGCGACACCTACTACAACCAGAAGTTCAAGGGCCGGGTGACCATCACCG
CCGACAAGAGCACCAGCACCGCCTACATGGAACTGAGCAGCCTCAGGAGCGAGGACACCGCTGTGTATTACT
GCGCCAGGGGCGCCATCTACAACGGCTACGACGTGCTGGACAACTGGGGCCAGGGCACACTAGTGACCGTG

TCCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGCGTGGTGACC
GTGCCCAGCAGCAGCCTGGGCACCCAGACCTACATCTGTAACGTGAACCACAAGCCCAGCAACACCAAGGTG
GACAAGAAGGTGGAGCCCAAGAGCTGTGACAAGACCCACACCTGCCCCCCCTGCCCTGCCCCCGAGCTGCTG
GGAGGCCCCAGCGTGTTCCTGTTCCCCCCCAAGCCTAAGGACACCCTGATGATCAGCAGAACCCCCGAGGTGA
CCTGTGTGGTGGTGGATGTGAGCCACGAGGACCCTGAGGTGAAGTTCAACTGGTACGTGGACGGCGTGGAG
GTGCACAATGCCAAGACCAAGCCCAGGGAGGAGCAGTACAACAGCACCTACCGGGTGGTGTCCGTGCTGACC
GTGCTGCACCAGGATTGGCTGAACGGCAAGGAGTACAAGTGTAAGGTGTCCAACAAGGCCCTGCCTGCCCCT
ATCGAGAAAACCATCAGCAAGGCCAAGGGCCAGCCCAGAGAGCCCCAGGTGTACACCCTGCCCCCTAGCAGA
GATGAGCTGACCAAGAACCAGGTGTCCCTGACCTGCCTGGTGAAGGGCTTCTACCCCAGCGACATCGCCGTG
GAGTGGGAGAGCAACGGCCAGCCCGAGAACAACTACAAGACCACCCCCCCTGTGCTGGACAGCGATGGCAG
CTTCTTCCTGTACAGCAAGCTGACCGTGGACAAGAGCAGATGGCAGCAGGGCAACGTGTTCAGCTGCTCCGT
GATGCACGAGGCCCTGCACAATCACTACACCCAGAAGAGCCTGAGCCTGTCCCCTGGCAAG

**SEQ ID 45- CA8 J1 Humanised heavy chain**

QVQLVQSGAEVKKPGSSVKVSCKASGYTFTNYWMHWVRQAPGQGLEWMGATYRGHSDTYYNQKFKGRVTITA
DKSTSTAYMELSSLRSEDTAVYYCARGAIYNGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 46 - CA8 J1 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACAAGAGCACCAGCACCGCCTACATGGAACTGAGCAGCCTCAGGAGCGAGGACACCGCTGTGTATTACTG
CGCCAGGGGCGCCATCTACAACGGCTACGACGTGCTGGACAACTGGGGCCAGGGCACACTAGTGACCGTGTC
CAGCGCCAGCACCAAGGGCCCCAGCGTGTTCCCCCTGGCCCCCAGCAGCAAGAGCACCAGCGGCGGCACAGC
CGCCCTGGGCTGCCTGGTGAAGGACTACTTCCCCGAACCGGTGACCGTGTCCTGGAACAGCGGAGCCCTGAC
CAGCGGCGTGCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACACCAAGGTGGA
CAAGAAGGTGGAGCCCAAGAGCTGTGACAAGACCCACACCTGCCCCCCCTGCCCTGCCCCCGAGCTGCTGGG
AGGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT

GCACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAG

**SEQ ID 47 - CA8 J2 Humanised heavy chain**

QVQLVQSGAEVKKPGSSVKVSCKASGYTFTNYWMHWVRQAPGQGLEWMGATYRGHSDTYYNQKFKGRVTITA
DKSTSTAYMELSSLRSEDTAVYYCTRGAIYNGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 48 - CA8 J2 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACAAGAGCACCAGCACCGCCTACATGGAACTGAGCAGCCTCAGGAGCGAGGACACCGCTGTGTATTACTG
CACCAGGGGCGCCATCTACAACGGCTACGACGTGCTGGACAACTGGGGCCAGGGCACACTAGTGACCGTGTC
CAGCGCCAGCACCAAGGGCCCCAGCGTGTTCCCCCTGGCCCCCAGCAGCAAGAGCACCAGCGGCGGCACAGC
CGCCCTGGGCTGCCTGGTGAAGGACTACTTCCCCGAACCGGTGACCGTGTCCTGGAACAGCGGAGCCCTGAC
CAGCGGCGTGCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACACCAAGGTGGA
CAAGAAGGTGGAGCCCAAGAGCTGTGACAAGACCCACACCTGCCCCCCCTGCCCTGCCCCCGAGCTGCTGGG
AGGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAG

**SEQ ID 49- CA8 J3 Humanised heavy chain**

QVQLVQSGAEVKKPGSSVKVSCKGSGYTFTNYWMHWVRQAPGQGLEWMGATYRGHSDTYYNQKFKGRVTITA
DTSTSTAYMELSSLRSEDTAVYYCTRGAIYNGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 50** - **CA8 J3 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGG
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACACGAGCACCAGCACCGCCTACATGGAACTGAGCAGCCTCAGGAGCGAGGACACCGCTGTGTATTACTG
CACCAGGGGCGCCATCTACAACGGCTACGACGTGCTGGACAACTGGGGCCAGGGCACACTAGTGACCGTGTC
CAGCGCCAGCACCAAGGGCCCCAGCGTGTTCCCCCTGGCCCCCAGCAGCAAGAGCACCAGCGGCGGCACAGC
CGCCCTGGGCTGCCTGGTGAAGGACTACTTCCCCGAACCGGTGACCGTGTCCTGGAACAGCGGAGCCCTGAC
CAGCGGCGTGCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACACCAAGGTGGA
CAAGAAGGTGGAGCCCAAGAGCTGTGACAAGACCCACACCTGCCCCCCCTGCCCTGCCCCCGAGCTGCTGGG
AGGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAG

**SEQ ID 51 - CA8 J4 Humanised heavy chain**

QVQLVQSGAEVKKPGSSVKVSCKGSGYTFTNYWMHWVRQAPGQGLEWIGATYRGHSDTYYNQKFKGRATLTA
DTSTSTAYMELSSLRSEDTAVYYCTRGAIYNGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 52 - CA8 J4 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGG
CAGCGGCTACACCTTCACCAACTACTGGATGCACTGGGTGAGGCAGGCCCCCGGACAGGGCCTGGAGTGGAT
CGGCGCCACCTACAGGGGCCACAGCGACACCTACTACAACCAGAAGTTCAAGGGCCGGGCGACCCTCACCGC
CGACACGAGCACCAGCACCGCCTACATGGAACTGAGCAGCCTCAGGAGCGAGGACACCGCTGTGTATTACTG
CACCAGGGGCGCCATCTACAACGGCTACGACGTGCTGGACAACTGGGGCCAGGGCACACTAGTGACCGTGTC
CAGCGCCAGCACCAAGGGCCCCAGCGTGTTCCCCCTGGCCCCCAGCAGCAAGAGCACCAGCGGCGGCACAGC
CGCCCTGGGCTGCCTGGTGAAGGACTACTTCCCCGAACCGGTGACCGTGTCCTGGAACAGCGGAGCCCTGAC
CAGCGGCGTGCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACACCAAGGTGGA
CAAGAAGGTGGAGCCCAAGAGCTGTGACAAGACCCACACCTGCCCCCCCTGCCCTGCCCCCGAGCTGCTGGG
AGGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAG

**SEQ ID 53 - CA8 J5 Humanised heavy chain**

QVQLVQSGAEVKKPGSSVKVSCKGSGYTFTNYWMHWVRQAPGQGLEWMGATYRGHSDTYYNQKFKGRVTITA
DTSTSTAYMELSSLRSEDTAVYYCTRGAIYDGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 54 - CA8 J5 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGG
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACACGAGCACCAGCACCGCCTACATGGAACTGAGCAGCCTCAGGAGCGAGGACACCGCTGTGTATTACTG
CACCAGGGGCGCCATCTACGACGGCTACGACGTGCTGGACAACTGGGGCCAGGGCACACTAGTGACCGTGTC
CAGCGCCAGCACCAAGGGCCCCAGCGTGTTCCCCCTGGCCCCCAGCAGCAAGAGCACCAGCGGCGGCACAGC

CGCCCTGGGCTGCCTGGTGAAGGACTACTTCCCCGAACCGGTGACCGTGTCCTGGAACAGCGGAGCCCTGAC
CAGCGGCGTGCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACACCAAGGTGGA
CAAGAAGGTGGAGCCCAAGAGCTGTGACAAGACCCACACCTGCCCCCCCTGCCCTGCCCCCGAGCTGCTGGG
AGGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAG

**SEQ ID 55 - CA8 J6 Humanised heavy chain**

QVQLVQSGAEVKKPGSSVKVSCKASGGTFSNYWMHWVRQAPGQGLEWMGATYRGHSDTYYNQKFKGRVTITA
DKSTSTAYMELSSLRSEDTAVYYCARGAIYDGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 56 - CA8 J6 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCGGCACCTTCAGCAACTACTGGATGCACTGGGTGAGGCAGGCCCCCGGACAGGGCCTGGAGTGGA
TGGGCGCCACCTACAGGGGCCACAGCGACACCTACTACAACCAGAAGTTCAAGGGCCGGGTGACCATCACCG
CCGACAAGAGCACCAGCACCGCCTACATGGAACTGAGCAGCCTCAGGAGCGAGGACACCGCTGTGTATTACT
GCGCCAGGGGCGCCATCTACGACGGCTACGACGTGCTGGACAACTGGGGCCAGGGCACACTAGTGACCGTG
TCCAGCGCCAGCACCAAGGGCCCCAGCGTGTTCCCCCTGGCCCCCAGCAGCAAGAGCACCAGCGGCGGCACA
GCCGCCCTGGGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGCGTGGTGACC
GTGCCCAGCAGCAGCCTGGGCACCCAGACCTACATCTGTAACGTGAACCACAAGCCCAGCAACACCAAGGTG
GACAAGAAGGTGGAGCCCAAGAGCTGTGACAAGACCCACACCTGCCCCCCCTGCCCTGCCCCCGAGCTGCTG
GGAGGCCCCAGCGTGTTCCTGTTCCCCCCCAAGCCTAAGGACACCCTGATGATCAGCAGAACCCCCGAGGTGA
CCTGTGTGGTGGTGGATGTGAGCCACGAGGACCCTGAGGTGAAGTTCAACTGGTACGTGGACGGCGTGGAG
GTGCACAATGCCAAGACCAAGCCCAGGGAGGAGCAGTACAACAGCACCTACCGGGTGGTGTCCGTGCTGACC

GTGCTGCACCAGGATTGGCTGAACGGCAAGGAGTACAAGTGTAAGGTGTCCAACAAGGCCCTGCCTGCCCCT
ATCGAGAAAACCATCAGCAAGGCCAAGGGCCAGCCCAGAGAGCCCCAGGTGTACACCCTGCCCCCTAGCAGA
GATGAGCTGACCAAGAACCAGGTGTCCCTGACCTGCCTGGTGAAGGGCTTCTACCCCAGCGACATCGCCGTG
GAGTGGGAGAGCAACGGCCAGCCCGAGAACAACTACAAGACCACCCCCCCTGTGCTGGACAGCGATGGCAG
CTTCTTCCTGTACAGCAAGCTGACCGTGGACAAGAGCAGATGGCAGCAGGGCAACGTGTTCAGCTGCTCCGT
GATGCACGAGGCCCTGCACAATCACTACACCCAGAAGAGCCTGAGCCTGTCCCCTGGCAAG

**SEQ ID 57 - CA8 J7 Humanised heavy chain**

QVQLVQSGAEVKKPGSSVKVSCKASGYTFTNYWMHWVRQAPGQGLEWMGATYRGHSDTYYNQKFKGRVTITA
DKSTSTAYMELSSLRSEDTAVYYCARGAIYDGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 58 - CA8 J7 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACAAGAGCACCAGCACCGCCTACATGGAACTGAGCAGCCTCAGGAGCGAGGACACCGCTGTGTATTACTG
CGCCAGGGGCGCCATCTACGACGGCTACGACGTGCTGGACAACTGGGGCCAGGGCACACTAGTGACCGTGTC
CAGCGCCAGCACCAAGGGCCCCAGCGTGTTCCCCCTGGCCCCCAGCAGCAAGAGCACCAGCGGCGGCACAGC
CGCCCTGGGCTGCCTGGTGAAGGACTACTTCCCCGAACCGGTGACCGTGTCCTGGAACAGCGGAGCCCTGAC
CAGCGGCGTGCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACACCAAGGTGGA
CAAGAAGGTGGAGCCCAAGAGCTGTGACAAGACCCACACCTGCCCCCCCTGCCCTGCCCCCGAGCTGCTGGG
AGGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAG

**SEQ ID 59 - CA8 J8 Humanised heavy chain**

QVQLVQSGAEVKKPGSSVKVSCKASGYTFTNYWMHWVRQAPGQGLEWMGATYRGHSDTYYNQKFKGRVTITA
DKSTSTAYMELSSLRSEDTAVYYCTRGAIYDGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 60 - CA8 J8 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGC
CAGCGGCTACACCTTCACCAACTACTGGATGCACTGGGTGAGGCAGGCCCCCGGACAGGGCCTGGAGTGGAT
GGGCGCCACCTACAGGGGCCACAGCGACACCTACTACAACCAGAAGTTCAAGGGCCGGGTGACCATCACCGC
CGACAAGAGCACCAGCACCGCCTACATGGAACTGAGCAGCCTCAGGAGCGAGGACACCGCTGTGTATTACTG
CACCAGGGGCGCCATCTACGACGGCTACGACGTGCTGGACAACTGGGGCCAGGGCACACTAGTGACCGTGTC
CAGCGCCAGCACCAAGGGCCCCAGCGTGTTCCCCCTGGCCCCCAGCAGCAAGAGCACCAGCGGCGGCACAGC
CGCCCTGGGCTGCCTGGTGAAGGACTACTTCCCCGAACCGGTGACCGTGTCCTGGAACAGCGGAGCCCTGAC
CAGCGGCGTGCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACACCAAGGTGGA
CAAGAAGGTGGAGCCCAAGAGCTGTGACAAGACCCACACCTGCCCCCCCTGCCCTGCCCCCGAGCTGCTGGG
AGGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAG

**SEQ ID 61 - CA8 J9 Humanised heavy chain**

QVQLVQSGAEVKKPGSSVKVSCKGSGYTFTNYWMHWVRQAPGQGLEWIGATYRGHSDTYYNQKFKGRATLTA
DTSTSTAYMELSSLRSEDTAVYYCTRGAIYDGYDVLDNWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ ID 62 - CA8 J9 Humanised heavy chain (Polynucleotide)**

CAGGTGCAGCTGGTCCAGAGCGGCGCCGAAGTGAAGAAGCCCGGCAGCTCCGTGAAAGTGAGCTGCAAGGG
CAGCGGCTACACCTTCACCAACTACTGGATGCACTGGGTGAGGCAGGCCCCCGGACAGGGCCTGGAGTGGAT
CGGCGCCACCTACAGGGGCCACAGCGACACCTACTACAACCAGAAGTTCAAGGGCCGGGCGACCCTCACCGC
CGACACGAGCACCAGCACCGCCTACATGGAACTGAGCAGCCTCAGGAGCGAGGACACCGCTGTGTATTACTG
CACCAGGGGCGCCATCTACGACGGCTACGACGTGCTGGACAACTGGGGCCAGGGCACACTAGTGACCGTGTC
CAGCGCCAGCACCAAGGGCCCCAGCGTGTTCCCCCTGGCCCCCAGCAGCAAGAGCACCAGCGGCGGCACAGC
CGCCCTGGGCTGCCTGGTGAAGGACTACTTCCCCGAACCGGTGACCGTGTCCTGGAACAGCGGAGCCCTGAC
CAGCGGCGTGCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACACCAAGGTGGA
CAAGAAGGTGGAGCCCAAGAGCTGTGACAAGACCCACACCTGCCCCCCCTGCCCTGCCCCCGAGCTGCTGGG
AGGCCCCAGCGTGTTCCTGTTCCCCCCCAAGCCTAAGGACACCCTGATGATCAGCAGAACCCCCGAGGTGACC
TGTGTGGTGGTGGATGTGAGCCACGAGGACCCTGAGGTGAAGTTCAACTGGTACGTGGACGGCGTGGAGGT
GCACAATGCCAAGACCAAGCCCAGGGAGGAGCAGTACAACAGCACCTACCGGGTGGTGTCCGTGCTGACCGT
GCTGCACCAGGATTGGCTGAACGGCAAGGAGTACAAGTGTAAGGTGTCCAACAAGGCCCTGCCTGCCCCTAT
CGAGAAAACCATCAGCAAGGCCAAGGGCCAGCCCAGAGAGCCCCAGGTGTACACCCTGCCCCCTAGCAGAGA
TGAGCTGACCAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGATGGCAGCTT
CTTCCTGTACAGCAAGCTGACCGTGGACAAGAGCAGATGGCAGCAGGGCAACGTGTTCAGCTGCTCCGTGAT
GCACGAGGCCCTGCACAATCACTACACCCAGAAGAGCCTGAGCCTGTCCCCTGGCAAG

**SEQ ID 63 - CA8 M0 Humanised light chain**

DIQMTQSPSSLSASVGDRVTITCSASQDISNYLNWYQQKPGKAPKLLIYYTSNLHSGVPSRFSGSGSGTDFTLTISSL
QPEDFATYYCQQYRKLPWTFGQGTKLEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVTKSFNRGEC

**SEQ ID 64 - CA8 M0 Humanised light chain (Polynucleotide)**

GACATCCAGATGACCCAGAGCCCTAGCTCACTGAGCGCCAGCGTGGGCGACAGGGTGACCATTACCTGCTCC
GCCAGCCAGGACATCAGCAACTACCTGAACTGGTACCAGCAGAAGCCCGGCAAGGCCCCCAAGCTGCTGATC
TACTACACCTCCAACCTGCACTCCGGCGTGCCCAGCAGGTTCAGCGGAAGCGGCAGCGGCACCGATTTCACCC
TGACCATCTCCAGCCTGCAGCCCGAGGACTTCGCCACCTACTACTGCCAGCAGTACAGGAAGCTCCCCTGGAC
TTTCGGCCAGGGCACCAAACTGGAGATCAAGCGTACGGTGGCCGCCCCCAGCGTGTTCATCTTCCCCCCCAGC
GATGAGCAGCTGAAGAGCGGCACCGCCAGCGTGGTGTGTCTGCTGAACAACTTCTACCCCCGGGAGGCCAAG
GTGCAGTGGAAGGTGGACAATGCCCTGCAGAGCGGCAACAGCCAGGAGAGCGTGACCGAGCAGGACAGCA
AGGACTCCACCTACAGCCTGAGCAGCACCCTGACCCTGAGCAAGGCCGACTACGAGAAGCACAAGGTGTACG
CCTGTGAGGTGACCCACCAGGGCCTGTCCAGCCCCGTGACCAAGAGCTTCAACCGGGGCGAGTGC

**SEQ ID 65 - CA8 M1 Humanised light chain**

DIQMTQSPSSLSASVGDRVTITCSASQDISNYLNWYQQKPGKAPKLLIYYTSNLHSGVPSRFSGSGSGTDYTLTISSL
QPEDFATYYCQQYRKLPWTFGQGTKLEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVTKSFNRGEC

**SEQ ID 66 - CA8 M1 Humanised light chain (Polynucleotide)**

GACATCCAGATGACCCAGAGCCCTAGCTCACTGAGCGCCAGCGTGGGCGACAGGGTGACCATTACCTGCTCC
GCCAGCCAGGACATCAGCAACTACCTGAACTGGTACCAGCAGAAGCCCGGCAAGGCCCCCAAGCTGCTGATC
TACTACACCTCCAACCTGCACTCCGGCGTGCCCAGCAGGTTCAGCGGAAGCGGCAGCGGCACCGATTACACCC
TGACCATCTCCAGCCTGCAGCCCGAGGACTTCGCCACCTACTACTGCCAGCAGTACAGGAAGCTCCCCTGGAC
TTTCGGCCAGGGCACCAAACTGGAGATCAAGCGTACGGTGGCCGCCCCCAGCGTGTTCATCTTCCCCCCCAGC
GATGAGCAGCTGAAGAGCGGCACCGCCAGCGTGGTGTGTCTGCTGAACAACTTCTACCCCCGGGAGGCCAAG
GTGCAGTGGAAGGTGGACAATGCCCTGCAGAGCGGCAACAGCCAGGAGAGCGTGACCGAGCAGGACAGCA
AGGACTCCACCTACAGCCTGAGCAGCACCCTGACCCTGAGCAAGGCCGACTACGAGAAGCACAAGGTGTACG
CCTGTGAGGTGACCCACCAGGGCCTGTCCAGCCCCGTGACCAAGAGCTTCAACCGGGGCGAGTGC

**SEQ ID 67 - CA8 M2 Humanised light chain**

DIQLTQSPSSLSASVGDRVTITCSASQDISNYLNWYQQKPGKAPELVIYYTSNLHSGVPSRFSGSGSGTDYTLTISSLQ
PEDFATYYCQQYRKLPWTFGQGTKLEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKSFNRGEC

**SEQ ID 68 - CA8 M2 Humanised light chain (Polynucleotide)**

GACATCCAGCTGACCCAGAGCCCTAGCTCACTGAGCGCCAGCGTGGGCGACAGGGTGACCATTACCTGCTCC
GCCAGCCAGGACATCAGCAACTACCTGAACTGGTACCAGCAGAAGCCCGGCAAGGCCCCCGAGCTGGTGATC
TACTACACCTCCAACCTGCACTCCGGCGTGCCCAGCAGGTTCAGCGGAAGCGGCAGCGGCACCGATTACACCC
TGACCATCTCCAGCCTGCAGCCCGAGGACTTCGCCACCTACTACTGCCAGCAGTACAGGAAGCTCCCCTGGAC
TTTCGGCCAGGGCACCAAACTGGAGATCAAGCGTACGGTGGCCGCCCCCAGCGTGTTCATCTTCCCCCCCAGC
GATGAGCAGCTGAAGAGCGGCACCGCCAGCGTGGTGTGTCTGCTGAACAACTTCTACCCCCGGGAGGCCAAG
GTGCAGTGGAAGGTGGACAATGCCCTGCAGAGCGGCAACAGCCAGGAGAGCGTGACCGAGCAGGACAGCA
AGGACTCCACCTACAGCCTGAGCAGCACCCTGACCCTGAGCAAGGCCGACTACGAGAAGCACAAGGTGTACG
CCTGTGAGGTGACCCACCAGGGCCTGTCCAGCCCCGTGACCAAGAGCTTCAACCGGGGCGAGTGC

**SEQ ID 69 - S307118G03 mouse variable heavy**

EVQLQQSGPELVKPGASVKISCKASGYTFTDYYMKWVKQSHGKSLEWIGEIYPNNGGITYNQKFKGKATLTVDKSS
STAYMELRSLTSEDSAVYYCANGYEFVYWGQGTLVTVSA

**SEQ ID 70 - S307118G03 mouse variable heavy (DNA sequence)**

GAGGTCCAGTTGCAACAATCTGGACCTGAGCTGGTGAAGCCTGGGGCTTCAGTGAAGATATCCTGTAAGGCT
TCTGGATACACATTCACTGACTACTACATGAAGTGGGTGAAGCAGAGCCATGGAAAGAGCCTTGAGTGGATT

GGAGAGATTTATCCTAATAATGGTGGTATTACCTACAACCAGAAGTTCAAGGGCAAGGCCACATTGACTGTAG
ACAAGTCCTCCAGCACAGCCTACATGGAGCTCCGCAGCCTGACATCTGAGGACTCTGCAGTCTATTACTGTGC
AAATGGTTACGAGTTTGTTTACTGGGGCCAAGGGACTCTGGTCACTGTCTCTGCA

**SEQ ID 71 - S307118G03 mouse variable light**

DIQMTQTASSLSASLGDRVTISCSASQGISNYLNWYQQKPDGTVKLLIYYTSSLHSGVPSRFSGSGSGTDYSLTISNLE
PEDIATYYCQQYSKLPWTFGGGTKLEIKR

**SEQ ID 72 - S307118G03 mouse variable light (DNA sequence)**

GATATCCAGATGACACAGACTGCATCCTCCCTGTCTGCCTCTCTGGGAGACAGAGTCACCATCAGTTGCAGTG
CAAGTCAGGGCATTAGCAATTATTTAAACTGGTATCAGCAGAAACCAGATGGAACTGTTAAACTCCTGATCTA
TTACACATCAAGTTTACACTCAGGAGTCCCATCAAGGTTCAGTGGCAGTGGGTCTGGGACAGATTATTCTCTCA
CCATCAGCAACCTGGAACCTGAAGATATTGCCACTTACTATTGTCAGCAGTATAGTAAGCTTCCGTGGACGTTC
GGTGGAGGCACCAAGCTGGAAATCAAACGG

**SEQ ID 73 - S307118G03 chimeric heavy chain**

EVQLQQSGPELVKPGASVKISCKASGYTFTDYYMKWVKQSHGKSLEWIGEIYPNNGGITYNQKFKGKATLTVDKSS
STAYMELRSLTSEDSAVYYCANGYEFVYWGQGTLVTVSAAKTTAPSVFPLAPSSKSTSGGTAALGCLVKDYFPEPVT
VSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSLSLSPGK

**SEQ ID 74 - S307118G03 chimeric heavy chain (DNA sequence)**

GAGGTCCAGTTGCAACAATCTGGACCTGAGCTGGTGAAGCCTGGGGCTTCAGTGAAGATATCCTGTAAGGCT
TCTGGATACACATTCACTGACTACTACATGAAGTGGGTGAAGCAGAGCCATGGAAAGAGCCTTGAGTGGATT
GGAGAGATTTATCCTAATAATGGTGGTATTACCTACAACCAGAAGTTCAAGGGCAAGGCCACATTGACTGTAG
ACAAGTCCTCCAGCACAGCCTACATGGAGCTCCGCAGCCTGACATCTGAGGACTCTGCAGTCTATTACTGTGC
AAATGGTTACGAGTTTGTTTACTGGGGCCAAGGGACTCTGGTCACTGTCTCTGCAGCCAAAACAACAGCCCCC
AGCGTGTTCCCCCTGGCCCCCAGCAGCAAGAGCACCAGCGGCGGCACAGCCGCCCTGGGCTGCCTGGTGAAG
GACTACTTCCCCGAACCGGTGACCGTGTCCTGGAACAGCGGAGCCCTGACCAGCGGCGTGCACACCTTCCCCG
CCGTGCTGCAGAGCAGCGGCCTGTACAGCCTGAGCAGCGTGGTGACCGTGCCCAGCAGCAGCCTGGGCACCC
AGACCTACATCTGTAACGTGAACCACAAGCCCAGCAACACCAAGGTGGACAAGAAGGTGGAGCCCAAGAGCT
GTGACAAGACCCACACCTGCCCCCCCTGCCCTGCCCCCGAGCTGCTGGGAGGCCCCAGCGTGTTCCTGTTCCC
CCCCAAGCCTAAGGACACCCTGATGATCAGCAGAACCCCCGAGGTGACCTGTGTGGTGGTGGATGTGAGCCA
CGAGGACCCTGAGGTGAAGTTCAACTGGTACGTGGACGGCGTGGAGGTGCACAATGCCAAGACCAAGCCCA
GGGAGGAGCAGTACAACAGCACCTACCGGGTGGTGTCCGTGCTGACCGTGCTGCACCAGGATTGGCTGAAC
GGCAAGGAGTACAAGTGTAAGGTGTCCAACAAGGCCCTGCCTGCCCCTATCGAGAAAACCATCAGCAAGGCC

AAGGGCCAGCCCAGAGAGCCCCAGGTGTACACCCTGCCCCCTAGCAGAGATGAGCTGACCAAGAACCAGGTG
TCCCTGACCTGCCTGGTGAAGGGCTTCTACCCCAGCGACATCGCCGTGGAGTGGGAGAGCAACGGCCAGCCC
GAGAACAACTACAAGACCACCCCCCCTGTGCTGGACAGCGATGGCAGCTTCTTCCTGTACAGCAAGCTGACCG
TGGACAAGAGCAGATGGCAGCAGGGCAACGTGTTCAGCTGCTCCGTGATGCACGAGGCCCTGCACAATCACT
ACACCCAGAAGAGCCTGAGCCTGTCCCCTGGCAAG

**SEQ ID 75 - S307118G03 chimeric light chain**

DIQMTQTASSLSASLGDRVTISCSASQGISNYLNWYQQKPDGTVKLLIYYTSSLHSGVPSRFSGSGSGTDYSLTISNLE
PEDIATYYCQQYSKLPWTFGGGTKLELKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKSFNRGEC

**SEQ ID 76 - S307118G03 chimeric light chain (DNA sequence)**

GATATCCAGATGACACAGACTGCATCCTCCCTGTCTGCCTCTCTGGGAGACAGAGTCACCATCAGTTGCAGTG
CAAGTCAGGGCATTAGCAATTATTTAAACTGGTATCAGCAGAAACCAGATGGAACTGTTAAACTCCTGATCTA
TTACACATCAAGTTTACACTCAGGAGTCCCATCAAGGTTCAGTGGCAGTGGGTCTGGGACAGATTATTCTCTCA
CCATCAGCAACCTGGAACCTGAAGATATTGCCACTTACTATTGTCAGCAGTATAGTAAGCTTCCGTGGACGTTC
GGTGGAGGCACCAAGCTGGAGCTGAAACGTACGGTGGCCGCCCCCAGCGTGTTCATCTTCCCCCCCAGCGAT
GAGCAGCTGAAGAGCGGCACCGCCAGCGTGGTGTGTCTGCTGAACAACTTCTACCCCCGGGAGGCCAAGGTG
CAGTGGAAGGTGGACAATGCCCTGCAGAGCGGCAACAGCCAGGAGAGCGTGACCGAGCAGGACAGCAAGG
ACTCCACCTACAGCCTGAGCAGCACCCTGACCCTGAGCAAGGCCGACTACGAGAAGCACAAGGTGTACGCCT
GTGAGGTGACCCACCAGGGCCTGTCCAGCCCCGTGACCAAGAGCTTCAACCGGGGCGAGTGC

**SEQ ID 77 - S307118G03 humanised H0 variable heavy**

QVQLVQSGAEVKKPGSSVKVSCKASGGTFSDYYMKWVRQAPGQGLEWMGEIYPNNGGITYNQKFKGRVTITAD
KSTSTAYMELSSLRSEDTAVYYCARGYEFVYWGQGTLVTVSS

**SEQ ID 78 - S307118G03 humanised H0 variable heavy (DNA sequence)**

CAGGTGCAGCTGGTGCAGAGCGGCGCCGAAGTGAAGAAGCCCGGCTCCAGCGTGAAGGTGAGCTGCAAGG
CTAGCGGCGGCACCTTCAGCGACTACTACATGAAGTGGGTGAGGCAGGCCCCCGGCCAGGGACTGGAGTGG
ATGGGCGAGATCTACCCCAACAACGGGGGCATCACCTACAACCAGAAGTTCAAGGGCAGGGTGACCATCACC
GCCGACAAAAGCACCAGCACCGCCTACATGGAACTGAGCAGCCTGAGGAGCGAGGACACCGCCGTGTACTAC
TGCGCCAGGGGCTACGAGTTCGTGTATTGGGGCCAGGGCACACTAGTGACCGTGTCCAGC

**SEQ ID 79 - S307118G03 humanised H1 variable heavy**

QVQLVQSGAEVKKPGSSVKVSCKASGYTFTDYYMKWVRQAPGQGLEWMGEIYPNNGGITYNQKFKGRVTITAD
KSTSTAYMELSSLRSEDTAVYYCARGYEFVYWGQGTLVTVSS

**SEQ ID 80 - S307118G03 humanised H1 variable heavy (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA

TGGGCGAGATCTACCCCAACAACGGGGGCATCACCTACAACCAGAAGTTCAAGGGCAGGGTGACCATCACCG
CCGACAAAAGCACCAGCACCGCCTACATGGAACTGAGCAGCCTGAGGAGCGAGGACACCGCCGTGTACTACT
GCGCCAGGGGCTACGAGTTCGTGTATTGGGGCCAGGGCACACTAGTGACCGTGTCCAGC

**SEQ ID 81 - S307118G03 humanised H2 variable heavy**

QVQLVQSGAEVKKPGSSVKVSCKASGYTFTDYYMKWVRQAPGQGLEWMGEIYPNNGGITYNQKFKGRVTITAD
KSTSTAYMELSSLRSEDTAVYYCANGYEFVYWGQGTLVTVSS

**SEQ ID 82 - S307118G03 humanised H2 variable heavy (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA
TGGGCGAGATCTACCCCAACAACGGGGGCATCACCTACAACCAGAAGTTCAAGGGCAGGGTGACCATCACCG
CCGACAAAAGCACCAGCACCGCCTACATGGAACTGAGCAGCCTGAGGAGCGAGGACACCGCCGTGTACTACT
GCGCCAACGGCTACGAGTTCGTGTATTGGGGCCAGGGCACACTAGTGACCGTGTCCAGC

**SEQ ID 83 - S307118G03 humanised H3 variable heavy**

QVQLVQSGAEVKKPGSSVKVSCKASGYTFTDYYMKWVRQAPGQGLEWIGEIYPNNGGITYNQKFKGRATLTVDK
STSTAYMELSSLRSEDTAVYYCANGYEFVYWGQGTLVTVSS

**SEQ ID 84 - S307118G03 humanised H3 variable heavy (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA
TAGGCGAGATCTACCCCAACAACGGGGGCATCACCTACAACCAGAAGTTCAAGGGCAGGGCGACCCTCACCG
TCGACAAAAGCACCAGCACCGCCTACATGGAACTGAGCAGCCTGAGGAGCGAGGACACCGCCGTGTACTACT
GCGCCAACGGCTACGAGTTCGTGTATTGGGGCCAGGGCACACTAGTGACCGTGTCCAGC

**SEQ ID 85 - S307118G03 humanised H4 variable heavy**

QVQLVQSGAEVKKPGSSVKVSCKASGYTFTDYYMKWVRQAPGQGLEWMGEIYPNNGGITYNQKFKGRVTITAD
KSTSTAYMELSSLRSEDTAVYYCADGYEFVYWGQGTLVTVSS

**SEQ ID 86 - S307118G03 humanised H4 variable heavy (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA
TGGGCGAGATCTACCCCAACAACGGGGGCATCACCTACAACCAGAAGTTCAAGGGCAGGGTGACCATCACCG
CCGACAAAAGCACCAGCACCGCCTACATGGAACTGAGCAGCCTGAGGAGCGAGGACACCGCCGTGTACTACT
GCGCCGACGGCTACGAGTTCGTGTATTGGGGCCAGGGCACACTAGTGACCGTGTCCAGC

**SEQ ID 87 - S307118G03 humanised H5 variable heavy**

QVQLVQSGAEVKKPGSSVKVSCKASGYTFTDYYMKWVRQAPGQGLEWIGEIYPNNGGITYNQKFKGRATLTVDK
STSTAYMELSSLRSEDTAVYYCANGYEFDYWGQGTLVTVSS

**SEQ ID 88 - S307118G03 humanised H5 variable heavy (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA
TAGGCGAGATCTACCCCAACAACGGGGGCATCACCTACAACCAGAAGTTCAAGGGCAGGGCGACCCTCACCG
TCGACAAAAGCACCAGCACCGCCTACATGGAACTGAGCAGCCTGAGGAGCGAGGACACCGCCGTGTACTACT
GCGCCAACGGCTACGAGTTCGACTATTGGGGCCAGGGCACACTAGTGACCGTGTCCAGC

**SEQ ID 89 - S307118G03 humanised L0 variable light**

DIQMTQSPSSLSASVGDRVTITCSASQGISNYLNWYQQKPGKAPKLLIYYTSSLHSGVPSRFSGSGSGTDFTLTISSLQ
PEDFATYYCQQYSKLPWTFGQGTKLEIKR

**SEQ ID 90 - S307118G03 humanised L0 variable light (DNA sequence)**

GACATCCAGATGACCCAGAGCCCCTCAAGCCTGAGCGCCAGCGTGGGCGACAGGGTGACTATCACCTGCAGC
GCCTCCCAGGGCATCAGCAACTACCTGAACTGGTACCAGCAGAAGCCCGGCAAGGCCCCTAAGCTGCTGATCT
ACTACACCAGCAGCCTGCACAGCGGCGTGCCCAGCAGGTTCTCCGGCAGCGGCAGCGGAACCGACTTCACCC
TGACCATTAGCAGCCTCCAGCCCGAGGACTTCGCCACCTACTACTGCCAGCAGTACAGCAAGCTGCCCTGGAC
CTTCGGCCAGGGCACCAAACTGGAGATCAAGCGT

**SEQ ID 91 - S307118G03 humanised L1 variable light**

DIQMTQSPSSLSASVGDRVTITCSASQGISNYLNWYQQKPGKAPKLLIYYTSSLHSGVPSRFSGSGSGTDYTLTISSLQ
PEDFATYYCQQYSKLPWTFGQGTKLEIKR

**SEQ ID 92 - S307118G03 humanised L1 variable light (DNA sequence)**

GACATCCAGATGACCCAGAGCCCCTCAAGCCTGAGCGCCAGCGTGGGCGACAGGGTGACTATCACCTGCAGC
GCCTCCCAGGGCATCAGCAACTACCTGAACTGGTACCAGCAGAAGCCCGGCAAGGCCCCTAAGCTGCTGATCT
ACTACACCAGCAGCCTGCACAGCGGCGTGCCCAGCAGGTTCTCCGGCAGCGGCAGCGGAACCGACTACACCC
TGACCATTAGCAGCCTCCAGCCCGAGGACTTCGCCACCTACTACTGCCAGCAGTACAGCAAGCTGCCCTGGAC
CTTCGGCCAGGGCACCAAACTGGAGATCAAGCGT

**SEQ ID 93 - S307118G03 CDRH1**
DYYMK

**SEQ ID 94 - S307118G03 CDRH2**
EIYPNNGGITYNQKFKG

**SEQ ID 95 - S307118G03 CDRH3**
GYEFVY

**SEQ ID 96 - S307118G03 CDRL1**
SASQGISNYLN

**SEQ ID 97 - S307118G03 CDRL2**
YTSSLHS

**SEQ ID 98 - S307118G03 CDRL3**
QQYSKLPWT

**SEQ ID 99 - S307118G03 humanised H5 CDRH3**
GYEFDY

**SEQ ID 100 - S307118G03 humanised H0 heavy chain**

QVQLVQSGAEVKKPGSSVKVSCKASGGTFSDYYMKWVRQAPGQGLEWMGEIYPNNGGITYNQKFKGRVTITAD
KSTSTAYMELSSLRSEDTAVYYCARGYEFVYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

**SEQ ID 101 - S307118G03 humanised H0 heavy chain (polynucleotide)**

CAGGTGCAGCTGGTGCAGAGCGGCGCCGAAGTGAAGAAGCCCGGCTCCAGCGTGAAGGTGAGCTGCAAGG
CTAGCGGCGGCACCTTCAGCGACTACTACATGAAGTGGGTGAGGCAGGCCCCCGGCCAGGGACTGGAGTGG
ATGGGCGAGATCTACCCCAACAACGGGGGCATCACCTACAACCAGAAGTTCAAGGGCAGGGTGACCATCACC
GCCGACAAAAGCACCAGCACCGCCTACATGGAACTGAGCAGCCTGAGGAGCGAGGACACCGCCGTGTACTAC
TGCGCCAGGGGCTACGAGTTCGTGTATTGGGGCCAGGGCACACTAGTGACCGTGTCCAGCGCCAGCACCAAG
GGCCCCAGCGTGTTCCCCCTGGCCCCCAGCAGCAAGAGCACCAGCGGCGGCACAGCCGCCCTGGGCTGCCTG
GTGAAGGACTACTTCCCCGAACCGGTGACCGTGTCCTGGAACAGCGGAGCCCTGACCAGCGGCGTGCACACC
TTCCCCGCCGTGCTGCAGAGCAGCGGCCTGTACAGCCTGAGCAGCGTGGTGACCGTGCCCAGCAGCAGCCTG
GGCACCCAGACCTACATCTGTAACGTGAACCACAAGCCCAGCAACACCAAGGTGGACAAGAAGGTGGAGCCC
AAGAGCTGTGACAAGACCCACACCTGCCCCCCCTGCCCTGCCCCCGAGCTGCTGGGAGGCCCCAGCGTGTTCC
TGTTCCCCCCCAAGCCTAAGGACACCCTGATGATCAGCAGAACCCCCGAGGTGACCTGTGTGGTGGTGGATGT
GAGCCACGAGGACCCTGAGGTGAAGTTCAACTGGTACGTGGACGGCGTGGAGGTGCACAATGCCAAGACCA
AGCCCAGGGAGGAGCAGTACAACAGCACCTACCGGGTGGTGTCCGTGCTGACCGTGCTGCACCAGGATTGGC
TGAACGGCAAGGAGTACAAGTGTAAGGTGTCCAACAAGGCCCTGCCTGCCCCTATCGAGAAAACCATCAGCA
AGGCCAAGGGCCAGCCCAGAGAGCCCCAGGTGTACACCCTGCCCCCTAGCAGAGATGAGCTGACCAAGAACC
AGGTGTCCCTGACCTGCCTGGTGAAGGGCTTCTACCCCAGCGACATCGCCGTGGAGTGGGAGAGCAACGGCC
AGCCCGAGAACAACTACAAGACCACCCCCCCTGTGCTGGACAGCGATGGCAGCTTCTTCCTGTACAGCAAGCT
GACCGTGGACAAGAGCAGATGGCAGCAGGGCAACGTGTTCAGCTGCTCCGTGATGCACGAGGCCCTGCACA
ATCACTACACCCAGAAGAGCCTGAGCCTGTCCCCTGGCAAG

**SEQ ID 102 - S307118G03 humanised H1 heavy chain**

QVQLVQSGAEVKKPGSSVKVSCKASGYTFTDYYMKWVRQAPGQGLEWMGEIYPNNGGITYNQKFKGRVTITAD
KSTSTAYMELSSLRSEDTAVYYCARGYEFVYWGQGTLVTVSSASTKGPSVFPLAPSSKSTSGGTAALGCLVKDYFPEP

VTVSWNSGALTSGVHTFPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

**SEQ ID 103 - S307118G03 humanised H1 heavy chain (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA
TGGGCGAGATCTACCCCAACAACGGGGGCATCACCTACAACCAGAAGTTCAAGGGCAGGGTGACCATCACCG
CCGACAAAAGCACCAGCACCGCCTACATGGAACTGAGCAGCCTGAGGAGCGAGGACACCGCCGTGTACTACT
GCGCCAGGGGCTACGAGTTCGTGTATTGGGGCCAGGGCACACTAGTGACCGTGTCCAGCGCCAGCACCAAG
GGCCCCAGCGTGTTCCCCCTGGCCCCCAGCAGCAAGAGCACCAGCGGCGGCACAGCCGCCCTGGGCTGCCTG
GTGAAGGACTACTTCCCCGAACCGGTGACCGTGTCCTGGAACAGCGGAGCCCTGACCAGCGGCGTGCACACC
TTCCCCGCCGTGCTGCAGAGCAGCGGCCTGTACAGCCTGAGCAGCGTGGTGACCGTGCCCAGCAGCAGCCTG
GGCACCCAGACCTACATCTGTAACGTGAACCACAAGCCCAGCAACACCAAGGTGGACAAGAAGGTGGAGCCC
AAGAGCTGTGACAAGACCCACACCTGCCCCCCCTGCCCTGCCCCCGAGCTGCTGGGAGGCCCCAGCGTGTTCC
TGTTCCCCCCCAAGCCTAAGGACACCCTGATGATCAGCAGAACCCCCGAGGTGACCTGTGTGGTGGTGGATGT
GAGCCACGAGGACCCTGAGGTGAAGTTCAACTGGTACGTGGACGGCGTGGAGGTGCACAATGCCAAGACCA
AGCCCAGGGAGGAGCAGTACAACAGCACCTACCGGGTGGTGTCCGTGCTGACCGTGCTGCACCAGGATTGGC
TGAACGGCAAGGAGTACAAGTGTAAGGTGTCCAACAAGGCCCTGCCTGCCCCTATCGAGAAAACCATCAGCA
AGGCCAAGGGCCAGCCCAGAGAGCCCCAGGTGTACACCCTGCCCCCTAGCAGAGATGAGCTGACCAAGAACC
AGGTGTCCCTGACCTGCCTGGTGAAGGGCTTCTACCCCAGCGACATCGCCGTGGAGTGGGAGAGCAACGGCC
AGCCCGAGAACAACTACAAGACCACCCCCCCTGTGCTGGACAGCGATGGCAGCTTCTTCCTGTACAGCAAGCT
GACCGTGGACAAGAGCAGATGGCAGCAGGGCAACGTGTTCAGCTGCTCCGTGATGCACGAGGCCCTGCACA
ATCACTACACCCAGAAGAGCCTGAGCCTGTCCCCTGGCAAG

**SEQ ID 104 - S307118G03 humanised H2 heavy chain**

QVQLVQSGAEVKKPGSSVKVSCKASGYTFTDYYMKWVRQAPGQGLEWMGEIYPNNGGITYNQKFKGRVTITAD
KSTSTAYMELSSLRSEDTAVYYCANGYEFVYWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPGK

**SEQ ID 105 - S307118G03 humanised H2 heavy chain (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA

TGGGCGAGATCTACCCCAACAACGGGGGCATCACCTACAACCAGAAGTTCAAGGGCAGGGTGACCATCACCG
CCGACAAAAGCACCAGCACCGCCTACATGGAACTGAGCAGCCTGAGGAGCGAGGACACCGCCGTGTACTACT
GCGCCAACGGCTACGAGTTCGTGTATTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTGGGCTGCCTGG
TGAAGGACTACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCAGCAGCCTGG
GCACCCAGACCTACATCTGTAACGTGAACCACAAGCCCAGCAACACCAAGGTGGACAAGAAGGTGGAGCCCA
AGAGCTGTGACAAGACCCACACCTGCCCCCCCTGCCCTGCCCCCGAGCTGCTGGGAGGCCCCAGCGTGTTCCT
GTTCCCCCCCAAGCCTAAGGACACCCTGATGATCAGCAGAACCCCCGAGGTGACCTGTGTGGTGGTGGATGT
GAGCCACGAGGACCCTGAGGTGAAGTTCAACTGGTACGTGGACGGCGTGGAGGTGCACAATGCCAAGACCA
AGCCCAGGGAGGAGCAGTACAACAGCACCTACCGGGTGGTGTCCGTGCTGACCGTGCTGCACCAGGATTGGC
TGAACGGCAAGGAGTACAAGTGTAAGGTGTCCAACAAGGCCCTGCCTGCCCCTATCGAGAAAACCATCAGCA
AGGCCAAGGGCCAGCCCAGAGAGCCCCAGGTGTACACCCTGCCCCCTAGCAGAGATGAGCTGACCAAGAACC
AGGTGTCCCTGACCTGCCTGGTGAAGGGCTTCTACCCCAGCGACATCGCCGTGGAGTGGGAGAGCAACGGCC
AGCCCGAGAACAACTACAAGACCACCCCCCCTGTGCTGGACAGCGATGGCAGCTTCTTCCTGTACAGCAAGCT
GACCGTGGACAAGAGCAGATGGCAGCAGGGCAACGTGTTCAGCTGCTCCGTGATGCACGAGGCCCTGCACA
ATCACTACACCCAGAAGAGCCTGAGCCTGTCCCCTGGCAAG

**SEQ ID 106 - S307118G03 humanised H3 heavy chain**

QVQLVQSGAEVKKPGSSVKVSCKASGYTFTDYYMKWVRQAPGQGLEWIGEIYPNNGGITYNQKFKGRATLTVDK
STSTAYMELSSLRSEDTAVYYCANGYEFVYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

**SEQ ID 107 - S307118G03 humanised H3 heavy chain (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA
TAGGCGAGATCTACCCCAACAACGGGGGCATCACCTACAACCAGAAGTTCAAGGGCAGGGCGACCCTCACCG
TCGACAAAAGCACCAGCACCGCCTACATGGAACTGAGCAGCCTGAGGAGCGAGGACACCGCCGTGTACTACT
GCGCCAACGGCTACGAGTTCGTGTATTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTGGGCTGCCTGG
TGAAGGACTACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCAGCAGCCTGG
GCACCCAGACCTACATCTGTAACGTGAACCACAAGCCCAGCAACACCAAGGTGGACAAGAAGGTGGAGCCCA

AGAGCTGTGACAAGACCCACACCTGCCCCCCCTGCCCTGCCCCCGAGCTGCTGGGAGGCCCCAGCGTGTTCCT
GTTCCCCCCCAAGCCTAAGGACACCCTGATGATCAGCAGAACCCCCGAGGTGACCTGTGTGGTGGTGGATGT
GAGCCACGAGGACCCTGAGGTGAAGTTCAACTGGTACGTGGACGGCGTGGAGGTGCACAATGCCAAGACCA
AGCCCAGGGAGGAGCAGTACAACAGCACCTACCGGGTGGTGTCCGTGCTGACCGTGCTGCACCAGGATTGGC
TGAACGGCAAGGAGTACAAGTGTAAGGTGTCCAACAAGGCCCTGCCTGCCCCTATCGAGAAAACCATCAGCA
AGGCCAAGGGCCAGCCCAGAGAGCCCCAGGTGTACACCCTGCCCCCTAGCAGAGATGAGCTGACCAAGAACC
AGGTGTCCCTGACCTGCCTGGTGAAGGGCTTCTACCCCAGCGACATCGCCGTGGAGTGGGAGAGCAACGGCC
AGCCCGAGAACAACTACAAGACCACCCCCCCTGTGCTGGACAGCGATGGCAGCTTCTTCCTGTACAGCAAGCT
GACCGTGGACAAGAGCAGATGGCAGCAGGGCAACGTGTTCAGCTGCTCCGTGATGCACGAGGCCCTGCACA
ATCACTACACCCAGAAGAGCCTGAGCCTGTCCCCTGGCAAG

**SEQ ID 108 - S307118G03 humanised H4 heavy chain**

QVQLVQSGAEVKKPGSSVKVSCKASGYTFTDYYMKWVRQAPGQGLEWMGEIYPNNGGITYNQKFKGRVTITAD
KSTSTAYMELSSLRSEDTAVYYCADGYEFVYWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPGK

**SEQ ID 109 - S307118G03 humanised H4 heavy chain (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA
TGGGCGAGATCTACCCCAACAACGGGGGCATCACCTACAACCAGAAGTTCAAGGGCAGGGTGACCATCACCG
CCGACAAAAGCACCAGCACCGCCTACATGGAACTGAGCAGCCTGAGGAGCGAGGACACCGCCGTGTACTACT
GCGCCGACGGCTACGAGTTCGTGTATTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTGGGCTGCCTGG
TGAAGGACTACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCAGCAGCCTGG
GCACCCAGACCTACATCTGTAACGTGAACCACAAGCCCAGCAACACCAAGGTGGACAAGAAGGTGGAGCCCA
AGAGCTGTGACAAGACCCACACCTGCCCCCCCTGCCCTGCCCCCGAGCTGCTGGGAGGCCCCAGCGTGTTCCT
GTTCCCCCCCAAGCCTAAGGACACCCTGATGATCAGCAGAACCCCCGAGGTGACCTGTGTGGTGGTGGATGT
GAGCCACGAGGACCCTGAGGTGAAGTTCAACTGGTACGTGGACGGCGTGGAGGTGCACAATGCCAAGACCA
AGCCCAGGGAGGAGCAGTACAACAGCACCTACCGGGTGGTGTCCGTGCTGACCGTGCTGCACCAGGATTGGC
TGAACGGCAAGGAGTACAAGTGTAAGGTGTCCAACAAGGCCCTGCCTGCCCCTATCGAGAAAACCATCAGCA
AGGCCAAGGGCCAGCCCAGAGAGCCCCAGGTGTACACCCTGCCCCCTAGCAGAGATGAGCTGACCAAGAACC
AGGTGTCCCTGACCTGCCTGGTGAAGGGCTTCTACCCCAGCGACATCGCCGTGGAGTGGGAGAGCAACGGCC

AGCCCGAGAACAACTACAAGACCACCCCCCCTGTGCTGGACAGCGATGGCAGCTTCTTCCTGTACAGCAAGCT
GACCGTGGACAAGAGCAGATGGCAGCAGGGCAACGTGTTCAGCTGCTCCGTGATGCACGAGGCCCTGCACA
ATCACTACACCCAGAAGAGCCTGAGCCTGTCCCCTGGCAAG

**SEQ ID 110 - S307118G03 humanised H5 heavy chain**

QVQLVQSGAEVKKPGSSVKVSCKASGYTFTDYYMKWVRQAPGQGLEWIGEIYPNNGGITYNQKFKGRATLTVDK
STSTAYMELSSLRSEDTAVYYCANGYEFDYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

**SEQ ID 111 - S307118G03 humanised H5 heavy chain (DNA sequence)**

CAGGTGCAGCTGGTGCAGAGCGGCGCCGAAGTGAAGAAGCCCGGCTCCAGCGTGAAGGTGAGCTGCAAGG
CTAGCGGCTACACCTTCACCGACTACTACATGAAGTGGGTGAGGCAGGCCCCCGGCCAGGGACTGGAGTGGA
TAGGCGAGATCTACCCCAACAACGGGGGCATCACCTACAACCAGAAGTTCAAGGGCAGGGCGACCCTCACCG
TCGACAAAAGCACCAGCACCGCCTACATGGAACTGAGCAGCCTGAGGAGCGAGGACACCGCCGTGTACTACT
GCGCCAACGGCTACGAGTTCGACTATTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTGGGCTGCCTGG
TGAAGGACTACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCAGCAGCCTGG
GCACCCAGACCTACATCTGTAACGTGAACCACAAGCCCAGCAACACCAAGGTGGACAAGAAGGTGGAGCCCA
AGAGCTGTGACAAGACCCACACCTGCCCCCCCTGCCCTGCCCCCGAGCTGCTGGGAGGCCCCAGCGTGTTCCT
GTTCCCCCCCAAGCCTAAGGACACCCTGATGATCAGCAGAACCCCCGAGGTGACCTGTGTGGTGGTGGATGT
GAGCCACGAGGACCCTGAGGTGAAGTTCAACTGGTACGTGGACGGCGTGGAGGTGCACAATGCCAAGACCA
AGCCCAGGGAGGAGCAGTACAACAGCACCTACCGGGTGGTGTCCGTGCTGACCGTGCTGCACCAGGATTGGC
TGAACGGCAAGGAGTACAAGTGTAAGGTGTCCAACAAGGCCCTGCCTGCCCCTATCGAGAAAACCATCAGCA
AGGCCAAGGGCCAGCCCAGAGAGCCCCAGGTGTACACCCTGCCCCCTAGCAGAGATGAGCTGACCAAGAACC
AGGTGTCCCTGACCTGCCTGGTGAAGGGCTTCTACCCCAGCGACATCGCCGTGGAGTGGGAGAGCAACGGCC
AGCCCGAGAACAACTACAAGACCACCCCCCCTGTGCTGGACAGCGATGGCAGCTTCTTCCTGTACAGCAAGCT
GACCGTGGACAAGAGCAGATGGCAGCAGGGCAACGTGTTCAGCTGCTCCGTGATGCACGAGGCCCTGCACA
ATCACTACACCCAGAAGAGCCTGAGCCTGTCCCCTGGCAAG

**SEQ ID 112 - S307118G03 humanised L0 light chain**

DIQMTQSPSSLSASVGDRVTITCSASQGISNYLNWYQQKPGKAPKLLIYYTSSLHSGVPSRFSGSGSGTDFTLTISSLQ
PEDFATYYCQQYSKLPWTFGQGTKLEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKSFNRGEC

**SEQ ID 113 - S307118G03 humanised L0 light chain (DNA sequence)**

GACATCCAGATGACCCAGAGCCCCTCAAGCCTGAGCGCCAGCGTGGGCGACAGGGTGACTATCACCTGCAGC
GCCTCCCAGGGCATCAGCAACTACCTGAACTGGTACCAGCAGAAGCCCGGCAAGGCCCCTAAGCTGCTGATCT
ACTACACCAGCAGCCTGCACAGCGGCGTGCCCAGCAGGTTCTCCGGCAGCGGCAGCGGAACCGACTTCACCC
TGACCATTAGCAGCCTCCAGCCCGAGGACTTCGCCACCTACTACTGCCAGCAGTACAGCAAGCTGCCCTGGAC
CTTCGGCCAGGGCACCAAACTGGAGATCAAGCGTACGGTGGCCGCCCCCAGCGTGTTCATCTTCCCCCCCAGC
GATGAGCAGCTGAAGAGCGGCACCGCCAGCGTGGTGTGTCTGCTGAACAACTTCTACCCCCGGGAGGCCAAG
GTGCAGTGGAAGGTGGACAATGCCCTGCAGAGCGGCAACAGCCAGGAGAGCGTGACCGAGCAGGACAGCA
AGGACTCCACCTACAGCCTGAGCAGCACCCTGACCCTGAGCAAGGCCGACTACGAGAAGCACAAGGTGTACG
CCTGTGAGGTGACCCACCAGGGCCTGTCCAGCCCCGTGACCAAGAGCTTCAACCGGGGCGAGTGC

**SEQ ID 114 - S307118G03 humanised L1 light chain**

DIQMTQSPSSLSASVGDRVTITCSASQGISNYLNWYQQKPGKAPKLLIYYTSSLHSGVPSRFSGSGSGTDYTLTISSLQ
PEDFATYYCQQYSKLPWTFGQGTKLEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKSFNRGEC

**SEQ ID 115 - S307118G03 humanised L1 light chain (DNA sequence)**

GACATCCAGATGACCCAGAGCCCCTCAAGCCTGAGCGCCAGCGTGGGCGACAGGGTGACTATCACCTGCAGC
GCCTCCCAGGGCATCAGCAACTACCTGAACTGGTACCAGCAGAAGCCCGGCAAGGCCCCTAAGCTGCTGATCT
ACTACACCAGCAGCCTGCACAGCGGCGTGCCCAGCAGGTTCTCCGGCAGCGGCAGCGGAACCGACTACACCC
TGACCATTAGCAGCCTCCAGCCCGAGGACTTCGCCACCTACTACTGCCAGCAGTACAGCAAGCTGCCCTGGAC
CTTCGGCCAGGGCACCAAACTGGAGATCAAGCGTACGGTGGCCGCCCCCAGCGTGTTCATCTTCCCCCCCAGC
GATGAGCAGCTGAAGAGCGGCACCGCCAGCGTGGTGTGTCTGCTGAACAACTTCTACCCCCGGGAGGCCAAG
GTGCAGTGGAAGGTGGACAATGCCCTGCAGAGCGGCAACAGCCAGGAGAGCGTGACCGAGCAGGACAGCA
AGGACTCCACCTACAGCCTGAGCAGCACCCTGACCCTGAGCAAGGCCGACTACGAGAAGCACAAGGTGTACG
CCTGTGAGGTGACCCACCAGGGCCTGTCCAGCCCCGTGACCAAGAGCTTCAACCGGGGCGAGTGC

**SEQ ID 116 - S332121F02 murine variable heavy chain**

EVQLQQSGPVLVKPGASVKMSCEASGYTFTDYYMNWVKQSHGKTLEWIGVINPYNGGTDYNQKFKGKATLTVD
KSSSTAYMELNSLTSEDSAVYYCARSVYDYPFDYWGQGTLVTVSS

**SEQ ID 117 S332121F02 murine variable heavy chain (DNA sequence)**

GAGGTGCAGCTGCAGCAGAGCGGCCCCGTGCTGGTGAAGCCTGGAGCCAGCGTGAAAATGAGCTGCAAGC
CAGCGGCTACACCTTCACCGACTACTACATGAACTGGGTGAAGCAGAGCCACGGCAAGACCCTGGAGTGGAT
CGGCGTGATCAACCCCTACAACGGGGGCACCGACTACAACCAGAAGTTCAAGGGCAAGGCCACTCTGACCGT
GGACAAGAGCTCCAGCACCGCCTACATGGAACTGAACAGCCTCACCTCTGAGGACAGCGCCGTCTATTACTGC
GCCAGGAGCGTGTACGACTACCCCTTCGACTACTGGGGCCAGGGCACACTAGTGACCGTGTCCAGC

**SEQ ID 118 - S332121F02 chimeric heavy chain**

EVQLQQSGPVLVKPGASVKMSCEASGYTFTDYYMNWVKQSHGKTLEWIGVINPYNGGTDYNQKFKGKATLTVD
KSSSTAYMELNSLTSEDSAVYYCARSVYDYPFDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLSPGK

**SEQ ID 119 - S332121F02 chimeric heavy chain (DNA sequence)**

GAGGTGCAGCTGCAGCAGAGCGGCCCCGTGCTGGTGAAGCCTGGAGCCAGCGTGAAAATGAGCTGCAAGC
CAGCGGCTACACCTTCACCGACTACTACATGAACTGGGTGAAGCAGAGCCACGGCAAGACCCTGGAGTGGAT
CGGCGTGATCAACCCCTACAACGGGGGCACCGACTACAACCAGAAGTTCAAGGGCAAGGCCACTCTGACCGT
GGACAAGAGCTCCAGCACCGCCTACATGGAACTGAACAGCCTCACCTCTGAGGACAGCGCCGTCTATTACTGC
GCCAGGAGCGTGTACGACTACCCCTTCGACTACTGGGGCCAGGGCACACTAGTGACCGTGTCCAGCGCCAGC
ACCAAGGGCCCCAGCGTGTTCCCCCTGGCCCCCAGCAGCAAGAGCACCAGCGGCGGCACAGCCGCCCTGGGC
TGCCTGGTGAAGGACTACTTCCCCGAACCGGTGACCGTGTCCTGGAACAGCGGAGCCCTGACCAGCGGCGTG
CACACCTTCCCCGCCGTGCTGCAGAGCAGCGGCCTGTACAGCCTGAGCAGCGTGGTGACCGTGCCCAGCAGC
AGCCTGGGCACCCAGACCTACATCTGTAACGTGAACCACAAGCCCAGCAACACCAAGGTGGACAAGAAGGTG
GAGCCCAAGAGCTGTGACAAGACCCACACCTGCCCCCCCTGCCCTGCCCCCGAGCTGCTGGGAGGCCCCAGC
GTGTTCCTGTTCCCCCCCAAGCCTAAGGACACCCTGATGATCAGCAGAACCCCCGAGGTGACCTGTGTGGTGG
TGGATGTGAGCCACGAGGACCCTGAGGTGAAGTTCAACTGGTACGTGGACGGCGTGGAGGTGCACAATGCC
AAGACCAAGCCCAGGGAGGAGCAGTACAACAGCACCTACCGGGTGGTGTCCGTGCTGACCGTGCTGCACCA
GGATTGGCTGAACGGCAAGGAGTACAAGTGTAAGGTGTCCAACAAGGCCCTGCCTGCCCCTATCGAGAAAAC
CATCAGCAAGGCCAAGGGCCAGCCCAGAGAGCCCCAGGTGTACACCCTGCCCCCTAGCAGAGATGAGCTGAC
CAAGAACCAGGTGTCCCTGACCTGCCTGGTGAAGGGCTTCTACCCCAGCGACATCGCCGTGGAGTGGGAGAG
CAACGGCCAGCCCGAGAACAACTACAAGACCACCCCCCCTGTGCTGGACAGCGATGGCAGCTTCTTCCTGTAC
AGCAAGCTGACCGTGGACAAGAGCAGATGGCAGCAGGGCAACGTGTTCAGCTGCTCCGTGATGCACGAGGC
CCTGCACAATCACTACACCCAGAAGAGCCTGAGCCTGTCCCCTGGCAAG

**SEQ ID 120 - S332121F02 murine variable light chain**

DIVLTQSPASLAVSLGQRATISCRASESVSIHGTHLMHWYQQKPGQPPKLLIYAASNLESGVPARFSGSGSETDFTL
NIHPVEEEDAATYFCQQSIEDPRTFGGGTKLEIK

**SEQ ID 121 - S332121F02 murine variable light chain (DNA sequence)**

GACATCGTCCTGACCCAGAGCCCCGCCAGCCTGGCCGTGAGCCTGGGCCAGAGGGCCACAATCAGCTGCAGG
GCCTCTGAGTCCGTGAGCATCCACGGCACCCACCTGATGCACTGGTATCAGCAGAAGCCCGGCCAGCCTCCCA
AGCTGCTGATCTACGCCGCCAGCAACCTGGAGAGCGGCGTGCCCGCTAGGTTCAGCGGAAGCGGCAGCGAG

ACCGACTTCACCCTGAACATCCACCCCGTGGAGGAGGAAGACGCCGCCACCTACTTCTGCCAGCAGAGCATCG
AGGACCCCAGGACCTTCGGCGGGGGCACCAAGCTCGAGATTAAGCGT

**SEQ ID 122 - S332121F02 chimeric light chain**

MGWSCIILFLVATATGVHSDIVLTQSPASLAVSLGQRATISCRASESVSIHGTHLMHWYQQKPGQPPKLLIYAASNL
ESGVPARFSGSGSETDFTLNIHPVEEEDAATYFCQQSIEDPRTFGGGTKLEIKRTVAAPSVFIFPPSDEQLKSGTASVV
CLLNNFYPREAKVQWKVDNALQSGNSQESVTEQDSKDSTYSLSSTLTLSKADYEKHKVYACEVTHQGLSSPVTKSF
NRGEC

**SEQ ID 123 - S332121F02 chimeric light chain (DNA sequence)**

ATGGGCTGGTCCTGCATCATCCTGTTTCTGGTGGCCACCGCCACCGGCGTGCACAGCGACATCGTCCTGACCC
AGAGCCCCGCCAGCCTGGCCGTGAGCCTGGGCCAGAGGGCCACAATCAGCTGCAGGGCCTCTGAGTCCGTGA
GCATCCACGGCACCCACCTGATGCACTGGTATCAGCAGAAGCCCGGCCAGCCTCCCAAGCTGCTGATCTACGC
CGCCAGCAACCTGGAGAGCGGCGTGCCCGCTAGGTTCAGCGGAAGCGGCAGCGAGACCGACTTCACCCTGA
ACATCCACCCCGTGGAGGAGGAAGACGCCGCCACCTACTTCTGCCAGCAGAGCATCGAGGACCCCAGGACCT
TCGGCGGGGGCACCAAGCTCGAGATTAAGCGTACGGTGGCCGCCCCCAGCGTGTTCATCTTCCCCCCCAGCG
ATGAGCAGCTGAAGAGCGGCACCGCCAGCGTGGTGTGTCTGCTGAACAACTTCTACCCCCGGGAGGCCAAGG
TGCAGTGGAAGGTGGACAATGCCCTGCAGAGCGGCAACAGCCAGGAGAGCGTGACCGAGCAGGACAGCAA
GGACTCCACCTACAGCCTGAGCAGCACCCTGACCCTGAGCAAGGCCGACTACGAGAAGCACAAGGTGTACGC
CTGTGAGGTGACCCACCAGGGCCTGTCCAGCCCCGTGACCAAGAGCTTCAACCGGGGCGAGTGC

**SEQ ID 124 - S322110D07 murine variable heavy chain**

EVQLQQSGPELVKPGTSVKIPCKTSGYIFTDYSIDWVKQSHGKSLEWIGDIDPNYGDPIYNHKFKGKATLTVDRSSST
AYMELRSLTSEDTAVYFCARRATGTDWFAFWGQGTLVTVSS

**SEQ ID 125 - S322110D07 murine variable heavy chain (DNA sequence)**

GAGGTGCAGCTGCAGCAGAGCGGCCCCGAGCTGGTGAAACCCGGCACCAGCGTGAAGATCCCCTGCAAGAC
CTCTGGCTACATCTTCACCGACTACAGCATCGACTGGGTGAAGCAGAGCCACGGCAAGTCTCTGGAGTGGATT
GGGGACATCGACCCCAACTACGGCGACCCCATCTACAACCACAAGTTCAAGGGCAAGGCCACCCTGACCGTG
GACAGGAGCAGCAGCACCGCCTACATGGAACTCAGGAGCCTGACCAGCGAGGACACCGCCGTGTATTTTTGC
GCCAGGAGGGCCACCGGCACTGATTGGTTCGCCTTCTGGGGCCAGGGCACACTAGTGACCGTGTCCAGC

**SEQ ID 126 - S322110D07 chimeric heavy chain**

EVQLQQSGPELVKPGTSVKIPCKTSGYIFTDYSIDWVKQSHGKSLEWIGDIDPNYGDPIYNHKFKGKATLTVDRSSST
AYMELRSLTSEDTAVYFCARRATGTDWFAFWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPGK

**SEQ ID 127 - S322110D07 chimeric heavy chain (DNA sequence)**

GAGGTGCAGCTGCAGCAGAGCGGCCCCGAGCTGGTGAAACCCGGCACCAGCGTGAAGATCCCCTGCAAGAC
CTCTGGCTACATCTTCACCGACTACAGCATCGACTGGGTGAAGCAGAGCCACGGCAAGTCTCTGGAGTGGATT
GGGGACATCGACCCCAACTACGGCGACCCCATCTACAACCACAAGTTCAAGGGCAAGGCCACCCTGACCGTG
GACAGGAGCAGCAGCACCGCCTACATGGAACTCAGGAGCCTGACCAGCGAGGACACCGCCGTGTATTTTTGC
GCCAGGAGGGCCACCGGCACTGATTGGTTCGCCTTCTGGGGCCAGGGCACACTAGTGACCGTGTCCAGCGCC
AGCACCAAGGGCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTGACCAGCGGC
GTGCACACCTTCCCCGCCGTGCTGCAGAGCAGCGGCCTGTACAGCCTGAGCAGCGTGGTGACCGTGCCCAGC
AGCAGCCTGGGCACCCAGACCTACATCTGTAACGTGAACCACAAGCCCAGCAACACCAAGGTGGACAAGAAG
GTGGAGCCCAAGAGCTGTGACAAGACCCACACCTGCCCCCCCTGCCCTGCCCCCGAGCTGCTGGGAGGCCCC
AGCGTGTTCCTGTTCCCCCCCAAGCCTAAGGACACCCTGATGATCAGCAGAACCCCCGAGGTGACCTGTGTGG
TGGTGGATGTGAGCCACGAGGACCCTGAGGTGAAGTTCAACTGGTACGTGGACGGCGTGGAGGTGCACAAT
GCCAAGACCAAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTATCGAGAAA
ACCATCAGCAAGGCCAAGGGCCAGCCCAGAGAGCCCCAGGTGTACACCCTGCCCCCTAGCAGAGATGAGCTG
ACCAAGAACCAGGTGTCCCTGACCTGCCTGGTGAAGGGCTTCTACCCCAGCGACATCGCCGTGGAGTGGGAG
AGCAACGGCCAGCCCGAGAACAACTACAAGACCACCCCCCCTGTGCTGGACAGCGATGGCAGCTTCTTCCTGT
ACAGCAAGCTGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAG

**SEQ ID 128 - S322110D07 murine variable light chain**

DIQMTQSPASLSVSVGETVTITCRASENIYNNLAWYQQKQGKSPQLLVYAATILADGVPSRFSGSGSGTQYSLKINS
LQSGDFGTYYCQHFWGTPLTFGAGTKLELKR

**SEQ ID 129 - S322110D07 murine variable light chain (DNA sequence)**

GACATCCAGATGACCCAGAGCCCCGCTAGCCTCAGCGTGTCCGTCGGCGAGACCGTGACCATCACCTGCAGG
GCCAGCGAGAACATCTACAACAACCTGGCCTGGTATCAGCAGAAGCAGGGCAAAAGCCCCCAGCTGCTGGTG
TACGCCGCCACCATTCTGGCCGACGGCGTGCCCAGCAGGTTCTCTGGAAGCGGCAGCGGCACCCAGTACAGC
CTGAAGATCAACAGCCTGCAGAGCGGGGACTTCGGCACCTACTACTGCCAGCACTTCTGGGGCACTCCCCTGA
CCTTCGGAGCCGGCACCAAGCTGGAGCTGAAGCGT

**SEQ ID 130 - S322110D07 chimeric light chain**

DIQMTQSPASLSVSVGETVTITCRASENIYNNLAWYQQKQGKSPQLLVYAATILADGVPSRFSGSGSGTQYSLKINS
LQSGDFGTYYCQHFWGTPLTFGAGTKLELKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKSFNRGEC

**SEQ ID 131 - S322110D07 chimeric light chain (DNA sequence)**

GACATCCAGATGACCCAGAGCCCCGCTAGCCTCAGCGTGTCCGTCGGCGAGACCGTGACCATCACCTGCAGG
GCCAGCGAGAACATCTACAACAACCTGGCCTGGTATCAGCAGAAGCAGGGCAAAGCCCCCAGCTGCTGGTG
TACGCCGCCACCATTCTGGCCGACGGCGTGCCCAGCAGGTTCTCTGGAAGCGGCAGCGGCACCCAGTACAGC
CTGAAGATCAACAGCCTGCAGAGCGGGGACTTCGGCACCTACTACTGCCAGCACTTCTGGGGCACTCCCCTGA
CCTTCGGAGCCGGCACCAAGCTGGAGCTGAAGCGTACGGTGGCCGCCCCCAGCGTGTTCATCTTCCCCCCCAG
CGATGAGCAGCTGAAGAGCGGCACCGCCAGCGTGGTGTGTCTGCTGAACAACTTCTACCCCCGGGAGGCCAA
GGTGCAGTGGAAGGTGGACAATGCCCTGCAGAGCGGCAACAGCCAGGAGAGCGTGACCGAGCAGGACAGC
AAGGACTCCACCTACAGCCTGAGCAGCACCCTGACCCTGAGCAAGGCCGACTACGAGAAGCACAAGGTGTAC
GCCTGTGAGGTGACCCACCAGGGCCTGTCCAGCCCCGTGACCAAGAGCTTCAACCGGGGCGAGTGC

**SEQ ID 132 - S332126E04 murine variable heavy chain**

QVQLQQPGAELVKPGASVKLSCKASGYTFTNYWMHWVKQRPGQGLEWIGIIHPNSGSTNYNEKFKSKATLTVDK
SSSTAYMQLSSLTSEDSAVYYCARGIYDYPFAYWGQGTLVTVSS

**SEQ ID 133 - S332126E04 murine variable heavy chain (DNA sequence)**

CAGGTGCAGCTCCAGCAGCCCGGAGCCGAACTGGTGAAGCCCGGAGCCAGCGTCAAACTGTCCTGCAAGGCC
AGCGGCTACACCTTCACCAACTACTGGATGCACTGGGTGAAGCAGAGGCCCGGCCAGGGCCTGGAGTGGATC
GGCATCATCCACCCCAACAGCGGGAGCACCAACTACAACGAGAAGTTCAAGAGCAAGGCCACCCTGACCGTG
GACAAGAGCAGCAGCACTGCCTACATGCAGCTGAGCAGCCTGACCAGCGAGGACAGCGCTGTGTACTACTGC
GCCAGGGGCATCTACGACTACCCCTTCGCCTATTGGGGCCAGGGCACACTAGTGACCGTGTCCAGC

**SEQ ID 134 - S332126E04 Chimeric heavy chain**

QVQLQQPGAELVKPGASVKLSCKASGYTFTNYWMHWVKQRPGQGLEWIGIIHPNSGSTNYNEKFKSKATLTVDK
SSSTAYMQLSSLTSEDSAVYYCARGIYDYPFAYWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLTCLVKGFYPSDIAVEW
ESNGQPENNYKTTPPVLDSDGSFFLYSKLTVDKSRWQQGNVFSCSVMHEALHNHYTQKSLSLSPGK

**SEQ ID 135 - S332126E04 Chimeric heavy chain (DNA sequence)**

CAGGTGCAGCTCCAGCAGCCCGGAGCCGAACTGGTGAAGCCCGGAGCCAGCGTCAAACTGTCCTGCAAGGCC
AGCGGCTACACCTTCACCAACTACTGGATGCACTGGGTGAAGCAGAGGCCCGGCCAGGGCCTGGAGTGGATC
GGCATCATCCACCCCAACAGCGGGAGCACCAACTACAACGAGAAGTTCAAGAGCAAGGCCACCCTGACCGTG
GACAAGAGCAGCAGCACTGCCTACATGCAGCTGAGCAGCCTGACCAGCGAGGACAGCGCTGTGTACTACTGC
GCCAGGGGCATCTACGACTACCCCTTCGCCTATTGGGGCCAGGGCACACTAGTGACCGTGTCCAGCGCCAGC
ACCAAGGGCCCCAGCGTGTTCCCCCTGGCCCCCAGCAGCAAGAGCACCAGCGGCGGCACAGCCGCCCTGGGC
TGCCTGGTGAAGGACTACTTCCCCGAACCGGTGACCGTGTCCTGGAACAGCGGAGCCCTGACCAGCGGCGTG

CACACCTTCCCCGCCGTGCTGCAGAGCAGCGGCCTGTACAGCCTGAGCAGCGTGGTGACCGTGCCCAGCAGC
AGCCTGGGCACCCAGACCTACATCTGTAACGTGAACCACAAGCCCAGCAACACCAAGGTGGACAAGAAGGTG
GAGCCCAAGAGCTGTGACAAGACCCACACCTGCCCCCCCTGCCCTGCCCCCGAGCTGCTGGGAGGCCCCAGC
GTGTTCCTGTTCCCCCCCAAGCCTAAGGACACCCTGATGATCAGCAGAACCCCCGAGGTGACCTGTGTGGTGG
TGGATGTGAGCCACGAGGACCCTGAGGTGAAGTTCAACTGGTACGTGGACGGCGTGGAGGTGCACAATGCC
AAGACCAAGCCCAGGGAGGAGCAGTACAACAGCACCTACCGGGTGGTGTCCGTGCTGACCGTGCTGCACCA
GGATTGGCTGAACGGCAAGGAGTACAAGTGTAAGGTGTCCAACAAGGCCCTGCCTGCCCCTATCGAGAAAAC
CATCAGCAAGGCCAAGGGCCAGCCCAGAGAGCCCCAGGTGTACACCCTGCCCCCTAGCAGAGATGAGCTGAC
CAAGAACCAGGTGTCCCTGACCTGCCTGGTGAAGGGCTTCTACCCCAGCGACATCGCCGTGGAGTGGGAGAG
CAACGGCCAGCCCGAGAACAACTACAAGACCACCCCCCCTGTGCTGGACAGCGATGGCAGCTTCTTCCTGTAC
AGCAAGCTGACCGTGGACAAGAGCAGATGGCAGCAGGGCAACGTGTTCAGCTGCTCCGTGATGCACGAGGC
CCTGCACAATCACTACACCCAGAAGAGCCTGAGCCTGTCCCCTGGCAAG

**SEQ ID 136 - S332126E04 murine variable light chain**

DIVLTQSPASLAVSLGQRATISCRASESVSIHGTHLMHWYQQKPGQPPKLLIYAASNLESGVPARFSGSGSETDFTL
NIHPVEEEDAATYFCQQSIEDPYTFGGGTKLEIKR

**SEQ ID 137 - S332126E04 murine variable light chain (DNA sequence)**

GACATCGTGCTGACCCAGTCTCCCGCTAGCCTGGCCGTGTCTCTGGGCCAGAGGGCCACAATCAGCTGCAGG
GCCAGCGAGAGCGTCAGCATTCACGGCACCCACCTGATGCACTGGTACCAGCAGAAGCCCGGCCAGCCTCCC
AAGCTCCTGATCTACGCCGCCAGCAACCTGGAAAGCGGAGTGCCCGCCAGGTTCAGCGGCAGCGGCTCCGAG
ACCGACTTCACCCTGAACATCCACCCCGTGGAGGAGGAGGACGCCGCCACCTACTTCTGCCAGCAGAGCATCG
AGGACCCCTACACCTTCGGCGGCGGCACCAAGCTGGAGATCAAGCGT

**SEQ ID 138 - S332126E04 Chimeric light chain**

DIVLTQSPASLAVSLGQRATISCRASESVSIHGTHLMHWYQQKPGQPPKLLIYAASNLESGVPARFSGSGSETDFTL
NIHPVEEEDAATYFCQQSIEDPYTFGGGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKSFNRGEC

**SEQ ID 139 - S332126E04 Chimeric light chain (DNA sequence)**

GACATCGTGCTGACCCAGTCTCCCGCTAGCCTGGCCGTGTCTCTGGGCCAGAGGGCCACAATCAGCTGCAGG
GCCAGCGAGAGCGTCAGCATTCACGGCACCCACCTGATGCACTGGTACCAGCAGAAGCCCGGCCAGCCTCCC
AAGCTCCTGATCTACGCCGCCAGCAACCTGGAAAGCGGAGTGCCCGCCAGGTTCAGCGGCAGCGGCTCCGAG
ACCGACTTCACCCTGAACATCCACCCCGTGGAGGAGGAGGACGCCGCCACCTACTTCTGCCAGCAGAGCATCG
AGGACCCCTACACCTTCGGCGGCGGCACCAAGCTGGAGATCAAGCGTACGGTGGCCGCCCCCAGCGTGTTCA
TCTTCCCCCCCAGCGATGAGCAGCTGAAGAGCGGCACCGCCAGCGTGGTGTGTCTGCTGAACAACTTCTACCC
CCGGGAGGCCAAGGTGCAGTGGAAGGTGGACAATGCCCTGCAGAGCGGCAACAGCCAGGAGAGCGTGACC
GAGCAGGACAGCAAGGACTCCACCTACAGCCTGAGCAGCACCCTGACCCTGAGCAAGGCCGACTACGAGAA

GCACAAGGTGTACGCCTGTGAGGTGACCCACCAGGGCCTGTCCAGCCCCGTGACCAAGAGCTTCAACCGGGG
CGAGTGC

**SEQ ID 140 - S336105A07 murine variable heavy chain**

EVKLLQSGGGLVQPGGSLKLSCAASGIDFSRYWMSWVRRAPGKGLEWIGEINPDRSTINYAPSLKDKFIISRDNAK
NTLYLQMSKVRSEDTALYYCAVFYYDYEGAMDYWGQGTSVTVSS

**SEQ ID 141 - S336105A07 murine variable heavy chain (DNA sequence)**

GAGGTGAAGCTTCTCCAGTCTGGAGGTGGCCTGGTGCAGCCTGGAGGATCCCTGAAACTCTCCTGTGCAGCCT
CAGGAATCGATTTTAGTAGATACTGGATGAGTTGGGTTCGGCGGGCTCCAGGGAAAGGACTAGAATGGATTG
GAGAAATTAATCCAGATAGGAGTACAATCAACTATGCACCATCTCTAAAGGATAAATTCATCATCTCCAGAGA
CAACGCCAAAAATACGCTGTACCTGCAAATGAGCAAAGTGAGATCTGAGGACACAGCCCTTTATTACTGTGCA
GTTTTCTACTATGATTACGAGGGTGCTATGGACTACTGGGGTCAAGGAACCTCAGTCACCGTCTCCTCA

**SEQ ID 142 - S336105A07 Chimeric heavy chain**

EVKLLQSGGGLVQPGGSLKLSCAASGIDFSRYWMSWVRRAPGKGLEWIGEINPDRSTINYAPSLKDKFIISRDNAK
NTLYLQMSKVRSEDTALYYCAVFYYDYEGAMDYWGQGTSVTVSSAKTTAPSVFPLAPSSKSTSGGTAALGCLVKDY
FPEPVTVSWNSGALTSGVHTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLSPGK

**SEQ ID 143 - S336105A07 Chimeric heavy chain (DNA sequence)**

GAGGTGAAGCTTCTCCAGTCTGGAGGTGGCCTGGTGCAGCCTGGAGGATCCCTGAAACTCTCCTGTGCAGCCT
CAGGAATCGATTTTAGTAGATACTGGATGAGTTGGGTTCGGCGGGCTCCAGGGAAAGGACTAGAATGGATTG
GAGAAATTAATCCAGATAGGAGTACAATCAACTATGCACCATCTCTAAAGGATAAATTCATCATCTCCAGAGA
CAACGCCAAAAATACGCTGTACCTGCAAATGAGCAAAGTGAGATCTGAGGACACAGCCCTTTATTACTGTGCA
GTTTTCTACTATGATTACGAGGGTGCTATGGACTACTGGGGTCAAGGAACCTCAGTCACCGTCTCCTCAGCCA
AAACAACAGCCCCCAGCGTGTTCCCCCTGGCCCCCAGCAGCAAGAGCACCAGCGGCGGCACAGCCGCCCTGG
GCTGCCTGGTGAAGGACTACTTCCCCGAACCGGTGACCGTGTCCTGGAACAGCGGAGCCCTGACCAGCGGCG
TGCACACCTTCCCCGCCGTGCTGCAGAGCAGCGGCCTGTACAGCCTGAGCAGCGTGGTGACCGTGCCCAGCA
GCAGCCTGGGCACCCAGACCTACATCTGTAACGTGAACCACAAGCCCAGCAACACCAAGGTGGACAAGAAGG
TGGAGCCCAAGAGCTGTGACAAGACCCACACCTGCCCCCCCTGCCCTGCCCCCGAGCTGCTGGGAGGCCCCA
GCGTGTTCCTGTTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGGTGCACAATG
CCAAGACCAAGCCCAGGGAGGAGCAGTACAACAGCACCTACCGGGTGGTGTCCGTGCTGACCGTGCTGCACC
AGGATTGGCTGAACGGCAAGGAGTACAAGTGTAAGGTGTCCAACAAGGCCCTGCCTGCCCCTATCGAGAAAA
CCATCAGCAAGGCCAAGGGCCAGCCCAGAGAGCCCCAGGTGTACACCCTGCCCCCTAGCAGAGATGAGCTGA

CCAAGAACCAGGTGTCCCTGACCTGCCTGGTGAAGGGCTTCTACCCCAGCGACATCGCCGTGGAGTGGGAGA
GCAACGGCCAGCCCGAGAACAACTACAAGACCACCCCCCCTGTGCTGGACAGCGATGGCAGCTTCTTCCTGTA
CAGCAAGCTGACCGTGGACAAGAGCAGATGGCAGCAGGGCAACGTGTTCAGCTGCTCCGTGATGCACGAGG
CCCTGCACAATCACTACACCCAGAAGAGCCTGAGCCTGTCCCCTGGCAAG

**SEQ ID 144 - S336105A07 murine varaible light chain**

DIVMTQSQKFMSTSVGDRVSVTCKASQNVDTNVAWYQQKPGQSPKALIYSASYRFSGVPDRFTGSGSGTDFTLTI
SNVQSEDLAEYFCQQYNSFPFTFGSGTKLEIKR

**SEQ ID 145 - S336105A07 murine variable light chain (DNA sequence)**

GACATTGTGATGACCCAGTCTCAAAAATTCATGTCCACATCAGTAGGAGACAGGGTCAGCGTCACCTGCAAGG
CCAGTCAGAATGTGGATACTAATGTAGCCTGGTATCAACAAAAACCAGGGCAATCTCCTAAAGCACTGATTTA
CTCGGCATCCTACCGGTTCAGTGGAGTCCCTGATCGCTTCACAGGCAGTGGATCTGGGACAGATTTCACTCTC
ACCATCAGCAATGTGCAGTCTGAAGACTTGGCAGAGTATTTCTGTCAGCAATATAACAGCTTTCCATTCACGTT
CGGCTCGGGGACAAAGTTGGAAATAAAACGT

**SEQ ID 146 - S336105A07 chimeric light chain**

DIVMTQSQKFMSTSVGDRVSVTCKASQNVDTNVAWYQQKPGQSPKALIYSASYRFSGVPDRFTGSGSGTDFTLTI
SNVQSEDLAEYFCQQYNSFPFTFGSGTKLEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPVTKSFNRGEC

**SEQ ID 147 - S336105A07 chimeric light chain (DNA sequence)**

GACATTGTGATGACCCAGTCTCAAAAATTCATGTCCACATCAGTAGGAGACAGGGTCAGCGTCACCTGCAAGG
CCAGTCAGAATGTGGATACTAATGTAGCCTGGTATCAACAAAAACCAGGGCAATCTCCTAAAGCACTGATTTA
CTCGGCATCCTACCGGTTCAGTGGAGTCCCTGATCGCTTCACAGGCAGTGGATCTGGGACAGATTTCACTCTC
ACCATCAGCAATGTGCAGTCTGAAGACTTGGCAGAGTATTTCTGTCAGCAATATAACAGCTTTCCATTCACGTT
CGGCTCGGGGACAAAGTTGGAAATAAAACGTACGGTGGCCGCCCCCAGCGTGTTCATCTTCCCCCCCAGCGAT
GAGCAGCTGAAGAGCGGCACCGCCAGCGTGGTGTGTCTGCTGAACAACTTCTACCCCCGGGAGGCCAAGGTG
CAGTGGAAGGTGGACAATGCCCTGCAGAGCGGCAACAGCCAGGAGAGCGTGACCGAGCAGGACAGCAAGG
ACTCCACCTACAGCCTGAGCAGCACCCTGACCCTGAGCAAGGCCGACTACGAGAAGCACAAGGTGTACGCCT
GTGAGGTGACCCACCAGGGCCTGTCCAGCCCCGTGACCAAGAGCTTCAACCGGGGCGAGTGC

**SEQ ID 148 - S335115G01 murine variable heavy chain**

PVQLQQPGTELVRPGTSVKLSCKASGYTFTSYWMHWVKQRPGQGLEWIGVIDPSDSYTNYNQKFKGKATLTVDT
SSSTAYMQLSSLTSEDSAVYYCARQVFDYPMDYWGQGTSVTVSS

**SEQID 149 - S335115G01 murine variable heavy chain (DNA sequence)**

CCGGTCCAACTGCAGCAGCCTGGGACTGAGCTGGTGAGGCCTGGGACTTCAGTGAAGTTGTCCTGCAAGGCT
TCTGGCTACACCTTCACCAGCTACTGGATGCACTGGGTAAAGCAGAGGCCTGGACAAGGCCTTGAGTGGATC
GGAGTGATTGATCCTTCTGATAGTTATACTAACTACAATCAAAAGTTCAAGGGCAAGGCCACATTGACTGTAG

ACACATCCTCCAGCACAGCCTACATGCAGCTCAGCAGCCTGACATCTGAGGACTCTGCGGTCTATTACTGTGCA
AGACAGGTGTTTGACTATCCTATGGACTACTGGGGTCAAGGAACCTCAGTCACCGTCTCCTCA

**SEQ ID 150 - S335115G01 Chimeric heavy chain**

PVQLQQPGTELVRPGTSVKLSCKASGYTFTSHWMHWVKQRPGQGLEWIGVIDPSDSYTNYNQKFKGKATLTVDT
SSSTAYMQLSSLTSEDSAVYYCARQVFDYPMDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLSPGK

**SEQ ID 151 - S335115G01 Chimeric heavy chain (DNA sequence)**

CCGGTCCAACTGCAGCAGCCTGGGACTGAGCTGGTGAGGCCTGGGACTTCAGTGAAGTTGTCCTGCAAGGCT
TCTGGCTACACCTTCACCAGCCACTGGATGCACTGGGTAAAGCAGAGGCCTGGACAAGGCCTTGAGTGGATC
GGAGTGATTGATCCTTCTGATAGTTATACTAACTACAATCAAAAGTTCAAGGGCAAGGCCACATTGACTGTAG
ACACATCCTCCAGCACAGCCTACATGCAGCTCAGCAGCCTGACATCTGAGGACTCTGCGGTCTATTACTGTGCA
AGACAGGTGTTTGACTATCCTATGGACTACTGGGGTCAAGGAACACTAGTGACCGTGTCCAGCGCCAGCACC
AAGGGCCCCAGCGTGTTCCCCCTGGCCCCCAGCAGCAAGAGCACCAGCGGCGGCACAGCCGCCCTGGGCTGC
CTGGTGAAGGACTACTTCCCCGAACCGGTGACCGTGTCCTGGAACAGCGGAGCCCTGACCAGCGGCGTGCAC
ACCTTCCCCGCCGTGCTGCAGAGCAGCGGCCTGTACAGCCTGAGCAGCGTGGTGACCGTGCCCAGCAGCAGC
CTGGGCACCCAGACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGAGGCCCCAGCGTGT
TCCTGTTCCCCCCCAAGCCTAAGGACACCCTGATGATCAGCAGAACCCCCGAGGTGACCTGTGTGGTGGTGGA
TGTGAGCCACGAGGACCCTGAGGTGAAGTTCAACTGGTACGTGGACGGCGTGGAGGTGCACAATGCCAAGA
CCAAGCCCAGGGAGGAGCAGTACAACAGCACCTACCGGGTGGTGTCCGTGCTGACCGTGCTGCACCAGGATT
GGCTGAACGGCAAGGAGTACAAGTGTAAGGTGTCCAACAAGGCCCTGCCTGCCCCTATCGAGAAAACCATCA
GCAAGGCCAAGGGCCAGCCCAGAGAGCCCCAGGTGTACACCCTGCCCCCTAGCAGAGATGAGCTGACCAAG
AACCAGGTGTCCCTGACCTGCCTGGTGAAGGGCTTCTACCCCAGCGACATCGCCGTGGAGTGGGAGAGCAAC
GGCCAGCCCGAGAACAACTACAAGACCACCCCCCCTGTGCTGGACAGCGATGGCAGCTTCTTCCTGTACAGCA
AGCTGACCGTGGACAAGAGCAGATGGCAGCAGGGCAACGTGTTCAGCTGCTCCGTGATGCACGAGGCCCTG
CACAATCACTACACCCAGAAGAGCCTGAGCCTGTCCCCTGGCAAG

**SEQ ID 152 - S335115G01 murine variable light chain**

DIVLTQSPASLAVSLGQRATISCRASESVSIHGTHLMHWYQQKPGQPPKLLIYAASNLESGVPARFSGSGSETDFTL
NIHPVEEEDAATYFCQQSIEDPWTFGGGTKLEIKR

**SEQ ID 153 - S335115G01 murine variable light chain (DNA sequence)**

GACATTGTGCTGACCCAATCTCCAGCTTCTTTGGCTGTGTCTCTAGGGCAGAGGGCCACCATCTCCTGCAGAGC
CAGTGAAAGTGTCAGTATTCATGGTACTCATTTAATGCACTGGTACCAACAGAAACCAGGACAGCCACCCAAA
CTCCTCATCTATGCTGCATCCAACCTAGAATCTGGAGTCCCTGCCAGGTTCAGTGGCAGTGGGTCTGAGACAG
ACTTCACCCTCAACATCCATCCTGTGGAGGAGGAGGATGCTGCAACCTATTTCTGTCAGCAAAGTATTGAGGA
TCCGTGGACGTTCGGTGGAGGCACCAAGCTGGAAATCAAACGT

**SEQ ID 154 - S335115G01 Chimeric light chain**

DIVLTQSPASLAVSLGQRATISCRASESVSIHGTHLMHWYQQKPGQPPKLLIYAASNLESGVPARFSGSGSETDFTL
NIHPVEEEDAATYFCQQSIEDPWTFGGGTKLEINRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEC

**SEQ ID 155 - S335115G01 Chimeric light chain (DNA sequence)**

GACATTGTGCTGACCCAATCTCCAGCTTCTTTGGCTGTGTCTCTAGGGCAGAGGGCCACCATCTCCTGCAGAGC
CAGTGAAAGTGTCAGTATTCATGGTACTCATTTAATGCACTGGTACCAACAGAAACCAGGACAGCCACCCAAA
CTCCTCATCTATGCTGCATCCAACCTAGAATCTGGAGTCCCTGCCAGGTTCAGTGGCAGTGGGTCTGAGACAG
ACTTCACCCTCAACATCCATCCTGTGGAGGAGGAGGATGCTGCAACCTATTTCTGTCAGCAAAGTATTGAGGA
TCCGTGGACGTTCGGTGGAGGCACCAAGCTGGAAATCAATCGTACGGTGGCCGCCCCCAGCGTGTTCATCTTC
CCCCCCAGCGATGAGCAGCTGAAGAGCGGCACCGCCAGCGTGGTGTGTCTGCTGAACAACTTCTACCCCCGG
GAGGCCAAGGTGCAGTGGAAGGTGGACAATGCCCTGCAGAGCGGCAACAGCCAGGAGAGCGTGACCGAGC
AGGACAGCAAGGACTCCACCTACAGCCTGAGCAGCACCCTGACCCTGAGCAAGGCCGACTACGAGAAGCACA
AGGTGTACGCCTGTGAGGTGACCCACCAGGGCCTGTCCAGCCCCGTGACCAAGAGCTTCAACCGGGGCGAGT
GC

**SEQ ID 156 - S335122F05 murine variable heavy chain**

QVQLQQSGAELVRPGASVTLSCKASGYTFTDYEMHWVKQTPVHGLEWIGAIDPETGGTAYNQKFKGKAILTADKS
SSTAYMELRSLTSEDSAVYYCTRSIYDYYFDYWGQGTTLTVSS

**SEQ ID 157 - S335122F05 murine variable heavy chain (DNA sequence)**

CAGGTTCAACTGCAGCAGTCTGGGGCTGAGCTGGTGAGGCCTGGGGCTTCAGTGACGCTGTCCTGCAAGGCT
TCGGGCTACACATTTACTGACTATGAAATGCACTGGGTGAAGCAGACACCTGTGCATGGCCTGGAATGGATTG
GAGCTATTGATCCTGAAACTGGTGGTACTGCCTACAATCAGAAGTTCAAGGGCAAGGCCATACTGACTGCAG
ACAAATCCTCCAGCACAGCCTACATGGAGCTCCGCAGCCTGACATCTGAGGACTCTGCCGTCTATTACTGTACA
AGATCGATTTATGATTACTACTTTGACTACTGGGGCCAAGGCACCACTCTCACAGTCTCCTCA

**SEQ ID 158 - S335122F05 Chimeric heavy chain**

QVQLQQSGAELVRPGASVTLSCKASGYTFTDYEMHWVKQTPVHGLEWIGAIDPETGGTAYNQKFKGKAILTADKS
SSTAYMELRSLTSEDSAVYYCTRSIYDYYFDYWGQGTTLTVSSAKTTPPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCP

APELLGGPSVFLFPPKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

**SEQ ID 159 - S335122F05 Chimeric heavy chain (DNA sequence)**

CAGGTTCAACTGCAGCAGTCTGGGGCTGAGCTGGTGAGGCCTGGGGCTTCAGTGACGCTGTCCTGCAAGGCT
TCGGGCTACACATTTACTGACTATGAAATGCACTGGGTGAAGCAGACACCTGTGCATGGCCTGGAATGGATTG
GAGCTATTGATCCTGAAACTGGTGGTACTGCCTACAATCAGAAGTTCAAGGGCAAGGCCATACTGACTGCAG
ACAAATCCTCCAGCACAGCCTACATGGAGCTCCGCAGCCTGACATCTGAGGACTCTGCCGTCTATTACTGTACA
AGATCGATTTATGATTACTACTTTGACTACTGGGGCCAAGGCACCACTCTCACAGTCTCCTCAGCCAAAACGAC
ACCCCCCAGCGTGTTCCCCCTGGCCCCCAGCAGCAAGAGCACCAGCGGCGGCACAGCCGCCCTGGGCTGCCT
GGTGAAGGACTACTTCCCCGAACCGGTGACCGTGTCCTGGAACAGCGGAGCCCTGACCAGCGGCGTGCACAC
CTTCCCCGCCGTGCTGCAGAGCAGCGGCCTGTACAGCCTGAGCAGCGTGGTGACCGTGCCCAGCAGCAGCCT
GGGCACCCAGACCTACATCTGTAACGTGAACCACAAGCCCAGCAACACCAAGGTGGACAAGAAGGTGGAGCC
CAAGAGCTGTGACAAGACCCACACCTGCCCCCCCTGCCCTGCCCCCGAGCTGCTGGGAGGCCCCAGCGTGTTC
CTGTTCCCCCCCAAGCCTAAGGACACCCTGATGATCAGCAGAACCCCCGAGGTGACCTGTGTGGTGGTGGATG
TGAGCCACGAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCACCAGGATTG
GCTGAACGGCAAGGAGTACAAGTGTAAGGTGTCCAACAAGGCCCTGCCTGCCCCTATCGAGAAAACCATCAG
CAAGGCCAAGGGCCAGCCCAGAGAGCCCCAGGTGTACACCCTGCCCCCTAGCAGAGATGAGCTGACCAAGAA
CCAGGTGTCCCTGACCTGCCTGGTGAAGGGCTTCTACCCCAGCGACATCGCCGTGGAGTGGGAGAGCAACGG
CCAGCCCGAGAACAACTACAAGACCACCCCCCCTGTGCTGGACAGCGATGGCAGCTTCTTCCTGTACAGCAAG
CTGACCGTGGACAAGAGCAGATGGCAGCAGGGCAACGTGTTCAGCTGCTCCGTGATGCACGAGGCCCTGCAC
AATCACTACACCCAGAAGAGCCTGAGCCTGTCCCCTGGCAAG

**SEQ ID 160 - S335122F05 murine variable light chain**

DIVLTQSPASLAVSLGQRATISCRASESVSIHGTHLMHWYQQKPGQPPKLLIYAASNLESGVPARFSGGGSETDFTL
NIHPVEEEDGATYFCQQSIEYPRTFGGGTKLEINR

**SEQ ID 161 - S335122F05 murine variable light chain (DNA sequence)**

GACATTGTGCTGACCCAATCTCCAGCTTCTTTGGCTGTGTCTCTAGGGCAGAGGGCCACCATCTCCTGCAGAGC
CAGTGAAAGTGTCAGTATTCATGGTACTCATTTAATGCACTGGTACCAACAGAAACCAGGACAGCCACCCAAA
CTCCTCATCTATGCTGCATCCAACCTAGAATCTGGAGTCCCTGCCAGGTTCAGTGGCGGTGGGTCTGAGACAG
ACTTCACCCTCAACATCCATCCTGTGGAGGAGGAGGATGGTGCAACCTATTTCTGTCAGCAAAGTATTGAGTA
TCCTCGGACGTTCGGTGGAGGCACCAAGCTGGAAATCAATCGT

**SEQ ID 162 - S335122F05 Chimeric light chain**

DIVLTQSPASLAVSLGQRATISCRASESVSIHGTHLMHWYQQKPGQPPKLLIYAASNLESGVPARFSGGGSETDFTL
NIHPVEEEDGATYFCQQSIEYPRTFGGGTKLEINRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEC

**SEQ ID 163 - S335122F05 Chimeric light chain (DNA sequence)**

GACATTGTGCTGACCCAATCTCCAGCTTCTTTGGCTGTGTCTCTAGGGCAGAGGGCCACCATCTCCTGCAGAGC
CAGTGAAAGTGTCAGTATTCATGGTACTCATTTAATGCACTGGTACCAACAGAAACCAGGACAGCCACCCAAA
CTCCTCATCTATGCTGCATCCAACCTAGAATCTGGAGTCCCTGCCAGGTTCAGTGGCGGTGGGTCTGAGACAG
ACTTCACCCTCAACATCCATCCTGTGGAGGAGGAGGATGGTGCAACCTATTTCTGTCAGCAAAGTATTGAGTA
TCCTCGGACGTTCGGTGGAGGCACCAAGCTGGAAATCAATCGTACGGTGGCCGCCCCCAGCGTGTTCATCTTC
CCCCCCAGCGATGAGCAGCTGAAGAGCGGCACCGCCAGCGTGGTGTGTCTGCTGAACAACTTCTACCCCCGG
GAGGCCAAGGTGCAGTGGAAGGTGGACAATGCCCTGCAGAGCGGCAACAGCCAGGAGAGCGTGACCGAGC
AGGACAGCAAGGACTCCACCTACAGCCTGAGCAGCACCCTGACCCTGAGCAAGGCCGACTACGAGAAGCACA
AGGTGTACGCCTGTGAGGTGACCCACCAGGGCCTGTCCAGCCCCGTGACCAAGAGCTTCAACCGGGGCGAGT
GC

**SEQ.I.D.NO: 164 - S332121F02 CDRH1**
DYYNM

**SEQ.I.D.NO: 165 - S332121F02 CDRH2**
VINPYNGGTDYNQKFG

**SEQ.I.D.NO: 166 - S332121F02 CDRH3**
SVYDYPFDY

**SEQ.I.D.NO: 167 - S332121F02 CDRL1**
RASESVSIHGTHLMH

**SEQ.I.D.NO: 168 - S332121F02 CDRL2**
AASNLES

**SEQ.I.D.NO: 169 - S332121F02 CDRL3**
QQSIEDPRT

**SEQ.I.D.NO: 170 - S322110D07 CDRH1**
DYSID

**SEQ.I.D.NO: 171 - S322110D07 CDRH2**
DIDPNYGDPIYNHKFKG

**SEQ.I.D.NO: 172** - **S322110D07 CDRH3**
RATGTDWFAF

**SEQ.I.D.NO: 173 - S322110D07CDRL1**
RASENIYNNLA

**SEQ.I.D.NO: 174 - S322110D07 CDRL2**
AATILAD

**SEQ.I.D.NO: 175 - S322110D07 CDRL3**
QHFWGTPLT

**SEQ.I.D.NO: 176 - S332126E04CDRH1**
NYWMH

**SEQ.I.D.NO: 177 - S332126E04 CDRH2**
IIHPNSGSTNYNEKFKS

**SEQ.I.D.NO: 178 - S332126E04 CDRH3**
GIYDYPFAY

**SEQ.I.D.NO: 179 - S332126E04 CDRL1**
RASESVSIHGTHLMH

**SEQ.I.D.NO: 180 - S332126E04 CDRL2**
AASNLES

**SEQ.I.D.NO: 181 - S332126E04 CDRL3**
QQSIEDPYT

**SEQ.I.D.NO: 182 - S336105A07 CDRH1**
RYWMS

**SEQ.I.D.NO: 183 - S336105A07 CDRH2**
EINPDRSTINYAPSLKD

**SEQ.I.D.NO: 184 - S336105A07 CDRH3**
FYYDYEGAMDY

**SEQ.I.D.NO: 185 - S336105A07 CDRL1**
KASQNVDTNVA

**SEQ.I.D.NO: 186 - S336105A07 CDRL2**
SASYRFS

**SEQ.I.D.NO: 187 - S336105A07 CDRL3**
QQYNSFPFT

**SEQ.I.D.NO: 188 - S335115G01 CDRH1**
SYWMH

**SEQ.I.D.NO: 189 - S335115G01 CDRH2**
VIDPSDSYTNYNQKFKG

**SEQ.I.D.NO: 190 - S335115G01 CDRH3**
QVFDYPMDY

**SEQ.I.D.NO: 191 - S335115G01 CDRL1**
RASESVSIHGTHLMH

**SEQ.I.D.NO: 192 - S335115G01 CDRL2**
AASNLES

**SEQ.I.D.NO: 193 - S335115G01 CDRL3**
QQSIEDPWT

**SEQ.I.D.NO: 194 - S335122F05 CDRH1**
DYEMH

**SEQ.I.D.NO: 195 - S335122F05 CDRH2**
AIDPETGGTAYNQKFKG

**SEQ.I.D.NO: 196 - S335122F05 CDRH3**
SIYDYYFDY

**SEQ.I.D.NO: 197 - S335122F05 CDRL1**
RASESVSIHGTHLMH

**SEQ.I.D.NO: 198 - S335122F05 CDRL2**
AASNLES

**SEQ.I.D.NO: 199 - S335122F05 CDRL3**
QQSIEYPRT

**SEQ.I.D.NO: 200 - CDRH3 variant N99D**
GAIYDGYDVLDN

**SEQ.I.D.NO: 201 - Pembrolizumab CDRH1**
NYYMY

**SEQ.I.D.NO: 202 - Pembrolizumab CDRH2**
GINPSNGGTNFNEKFKN

**SEQ.I.D.NO: 203 - Pembrolizumab CDRH3**
RDYRFDMGFDY

**SEQ.I.D.NO: 204 - Pembrolizumab CDRL1**
RASKGVSTSGYSYLH

SEQ.I.D.NO: 205 - Pembrolizumab CDRL2
LASYLES

**SEQ.I.D.NO: 206 - Pembrolizumab CDRL3**
QHSRDLPLT

**SEQ.I.D.NO: 207 - Heavy Chain Variable Region of pembrolizumab**

**SEQ.I.D.NO: 208 - Light Chain Variable Region of pembrolizumab**

**SEQ.I.D.NO: 209 - Heavy Chain of pembrolizumab**

QVQLVQSGVEVKKPGASVKVSCKASGYTFTNYYMYWVRQAPGQGLEWMGGINPSNGGTNFNEKFKNRVTLTT
DSSTTTAYMELKSLQFDDTAVYYCARRDYRFDMGFDYWGQGTTVTVSSASTKGPSVFPLAPCSRSTSESTAALGCL
VKDYFPEPVTVSWNSGALTSGVHTFPAVLQSSGLYSLSSVVTVPSSSLGTKTYTCNVDHKPSNTKVDKRVESKYGPP
CPPCPAPEFLGGPSVFLFPPKPKDTLMISRTPEVTCVVVDVSQEDPEVQFNWYVDGVEVHNAKTKPREEQFNSTYV
VSVLTVLHQDWLNGKEYKCKVSNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEALHNHYTQKSLSLSLGK

**SEQ.I.D.NO: 210 - Light Chain of pembrolizumab**

EIVLTQSPATLSLSPGERATLSCRASKGVSTSGYSYLHWYQQKPGQAPRLLIYLASYLESGVPARFSGSGSGTDFTLTIS
SLEPEDFAVYYCQHSRDLPLTFGGGTKVEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKSFNRGEC

**SEQ.I.D.NO: 211 - Nivolumab CDRH1**
NSGMH

**SEQ.I.D.NO: 212 - Nivolumab CDRH2**
VIWYDGSKRYYADSVKG

**SEQ.I.D.NO: 213 - Nivolumab CDRH3**
NDDY

**SEQ.I.D.NO: 214 - Nivolumab CDRL1**
RASQSVSSYLA

**SEQ.I.D.NO: 215 - Nivolumab CDRL2**
DASNRAT

**SEQ.I.D.NO: 216 - Nivolumab CDRL3**
QQSSNWPRT

**SEQ.I.D.NO: 217 - Heavy Chain Variable region of Nivolumab**

**SEQ.I.D.NO: 218 - Light Chain Variable region of Nivolumab**

**SEQ.I.D.NO: 219- 106-222 CDRH1**
DYSMH

**SEQ.I.D.NO: 220- 106-222 CDRH2**
WINTETGEPTYADDFKG

**SEQ.I.D.NO: 221- 106-222 CDRH3**
PYYDYVSYYAMDY

**SEQ.I.D.NO: 222- 106-222 CDRL1**
KASQDVSTAVA

**SEQ.I.D.NO: 223- 106-222 CDRL2**
SASYLYT

**SEQ.I.D.NO: 224 - 106-222 CDRL3**
QQHYSTPRT

**SEQ.I.D.NO: 225- 106-222 Variable heavy chain**

QIQLVQSGPELKKPGETVKISCKASGYTFTDYSMHWVKQAPGKGLKWMGWINTETGEPTYADDFKGRFAFSLETS
ASTAYLQINNLKNEDTATYFCANPYYDYVSYYAMDYWGHGTSVTVSS

**SEQ.I.D.NO: 226- 106-222 Variable heavy chain (polynucleotide)**

ACTAGTACCA CCATGGCTTG GGTGTGGACC TTGCTATTCC TGATGGCAGC TGCCCAAAGTATCCAAGCAC
AGGTTCAGTT GGTGCAGTCT GGATCTGAGC TGAAGAAGCC TGGAGCCTCAGTCAAGGTTTCTGCAAGGC
TTCTGGTTAT ACCTTCACAG ACTATTCAAT GCACTGGGTGCGACAGGCTC CAGGACAAGG TTTAAAGTGG
ATGGGCTGGA TAAACACTGA GACTGGTGAGCCAACATATG CAGATGACTTAGGGACGG TTTGTCTTCT
CTTTGGACAC CTCTGTCAGCACTGCCTATT TGCAGATCAG CAGCCTCAAA GCTGAGGACA CGGCTGTGTA
TTACTGTGCTAATCCCTACT ATGATTACGT CTCTTACTAT GCTATGGACT ACTGGGGTCA
GGGAACCACGGTCACCGTCT CCTCAGGTAA GAATGGCCTC TCAAGCTT

**SEQ.I.D.NO: 227- 106-222 Variable Light chain**

DIVMTQSHKFMSTSVRDRVSITCKASQDVSTAVAWYQQKPGQSPKLLIYSASYLYTGVPDRFTGSGSGTDFTFTISS
VQAEDLAVYYCQQHYSTPRTFGGGTKLEIK

**SEQ.I.D.NO: 228- 106-222 Variable Light chain (polynucleotide)**

GCTAGCACCA CCATGGAGTC ACAGATTCAG GTCTTTGTAT TCGTGTTTCT CTGGTTGTCTGGTGTTGACG

GAGACATTCA GATGACCCAG TCTCCATCCT CCCTGTCCGC ATCAGTGGGAGACAGGGTCA CCATCACCTG

CAAGGCCAGT CAGGATGTGA GTACTGCTGT AGCCTGGTATCAACAGAAAC CAGGAAAAGC CCCTAAACTA

CTGATTTACT CGGCATCCTA CCTCTACACT GGAGTCCCTT CACGCTTCAG TGGCAGTGGA TCTGGGACGG

ATTTCACTTT CACCATCAGC AGTCTGCAGC CTGAAGACAT TGCAACATAT TACTGTCAGC AACATTATAG
TACTCCTCGG ACGTTCGGTC AGGGCACCAA GCTGGAAATC AAACGTAAGT AGAATCCAAA GAATTC

**SEQ.I.D.NO: 229- 106-222 Variable Heavy chain**

QVQLVQSGSELKKPGASVKVSCKASGYTFTDYSMHWVRQAPGQGLKWMGWINTETGEPTYADDFKGRFVFSLD
TSVSTAYLQISSLKAEDTAVYYCANPYYDYVSYYAMDYWGQGTTVTVSS

**SEQ.I.D.NO: 230- 106-222 Variable Light chain**

DIQMTQSPSSLSASVGDRVTITCKASQDVSTAVAWYQQKPGKAPKLLIYSASYLYTGVPSRFSGSGSGTDFTFTISSL
QPEDIATYYCQQHYSTPRTFGQGTKLEIK

**SEQ.I.D.NO: 231- 119-222 CDRH1**
SHDMS

**SEQ.I.D.NO: 232- 119-222 CDRH2**
AINSDGGSTYYPDTME

**SEQ.I.D.NO: 233- 119-222 CDRH3**
HYDDYYAWFAY

**SEQ.I.D.NO: 234- 119-222 CDRHL1**
RASKSVSTSGYSYMH

**SEQ.I.D.NO: 235- 119-222 CDRL2**
LASNLES

**SEQ.I.D.NO: 236- 119-222 CDRL3**
QHSRELPLT

**SEQ.I.D.NO: 237- 119-222 Variable Heavy chain**

EVQLVESGGGLVQPGESLKLSCESNEYEFPSHDMSWVRKTPEKRLELVAAINSDGGSTYYPDTMERRFIISRDNTKK
TLYLQMSSLRSEDTALYYCARHYDDYYAWFAYWGQGTLVTVSA

**SEQ.I.D.NO: 238- 119-222 Variable Heavy chain (polynucleotide)**

ACTAGTACCA CCATGGACTT CGGGCTCAGC TTGGTTTTCC TTGTCCTTAT TTTAAAAGTGTACAGTGTG
AGGTGCAGCT GGTGGAGTCT GGGGGAGGCT TAGTGCAGCC TGGAGGGTCCCTGAGACTCT CTGTGCAGC
CTCTGAATAC GAGTTCCCTT CCCATGACAT GTCTTGGGTCCGCCAGGCTC CGGGGAAGGG GCTGGAGTTG
GTCGCAGCCA TTAATAGTGA TGGTGGTAGCACCTACTATC CAGACACCAT GGAGAGACGA TTCACCATCT
CCAGAGACAA TGCCAAGAACTCACTGTACC TGCAAATGAA CAGTCTGAGG GCCGAGGACA CAGCCGTGTA
TTACTGTGCAAGACACTATG ATGATTACTA CGCCTGGTTT GCTTACTGGG GCCAAGGGAC
TATGGTCACTGTCTCTTCAG GTGAGTCCTA ACTTCAAGCT T

**SEQ.I.D.NO: 239- 119-222 Variable Light chain**

DIVLTQSPASLAVSLGQRATISCRASKSVSTSGYSYMHWYQQKPGQPPKLLIYLASNLESGVPARFSGSGSGTDFTL
NIHPVEEEDAATYYCQHSRELPLTFGAGTKLELK

**SEQ.I.D.NO: 240- 119-222 Variable Light chain (polynucleotide)**

GCTAGCACCA CCATGGAGAC AGACACACTC CTGTTATGGG TACTGCTGCT CTGGGTTCCAGGTTCCACTG
GTGAAATTGT GCTGACACAG TCTCCTGCTA CCTTATCTTT GTCTCCAGGGGAAAGGGCCA CCCTCTCATG
CAGGGCCAGC AAAAGTGTCA GTACATCTGG CTATAGTTATATGCACTGGT ACCAACAGAA ACCAGGACAG
GCTCCCAGAC TCCTCATCTA TCTTGCATCCAACCTAGAAT CTGGGGTCCC TGCCAGGTTC AGTGGCAGTG
GGTCTGGGAC AGACTTCACCCTCACCATCA GCAGCCTAGA GCCTGAGGAT TTTGCAGTTT ATTACTGTCA
GCACAGTAGGGAGCTTCCGC TCACGTTCGG CGGAGGGACC AAGGTCGAGA TCAAACGTAA GTACACTTTT
CTGAATTC

**SEQ.I.D.NO: 241- 119-222 Variable Heavy chain**

EVQLVESGGGLVQPGGSLRLSCAASEYEFPSHDMSWVRQAPGKGLELVAAINSDGGSTYYPDTMERRFTISRDNA
KNSLYLQMNSLRAEDTAVYYCARHYDDYYAWFAYWGQGTMVTVSS

**SEQ.I.D.NO: 242- 119-222 Variable Light chain**

EIVLTQSPATLSLSPGERATLSCRASKSVSTSGYSYMHWYQQKPGQAPRLLIYLASNLESGVPARFSGSGSGTDFTLTI

SSLEPEDFAVYYCQHSRELPLTFGGGTKVEIK

**SEQ.I.D.NO: 243 - 106-222 Heavy Chain**

QVQLVQSGSELKKPGASVKVSCKASGYTFTDYSMHWVRQAPGQGLKWMGWINTETGEPTYADDFKGRFVFSLD
TSVSTAYLQISSLKAEDTAVYYCANPYYDYVSYYAMDYWGQGTTVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKSLSLSPGK

**SEQ. I.D. NO: 244 - 106-222: Light Chain**

DIQMTQSPSSLSASVGDRVTITCKASQDVSTAVAWYQQKPGKAPKLLIYSASYLYTGVPSRFSGSGSGTDFTFTISSL
QPEDIATYYCQQHYSTPRTFGQGTKLEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKSFNRGEC

## Claims

1. A combination comprising a therapeutically effective amount of an antigen binding protein that binds BCMA and a therapeutically effective amount of at least one antigen binding protein that binds an immunomodulatory target.

2. The combination of claim 1, wherein the immunomodulatory target is PD1 or OX40.

3. The combination of any preceding claim, wherein the antigen binding protein that binds BCMA is conjugated to a cytotoxic agent as an immunoconjugate, and wherein the cytotoxic agent is MMAE or MMAF.

4. The combination of claim 3, wherein the cytotic agent is conjugated to the antigen binding protein that binds BCMA via a linker, and wherein the linker is citruline-valine or maleimidocaproyl.

5. The combination of any preceding claim, wherein the antigen binding protein that binds BCMA comprises CDRH3 variant N99D of SEQ. ID. NO: 200.

6. The combination of claims 1-4, wherein the antigen binding protein that binds BCMA comprises CDRH3 of SEQ. ID. NO: 3, or a variant thereof.

7. The combination of claim 1, wherein the antigen binding protein that binds BCMA comprises CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof.

8. The combination of any preceding claim, wherein the antigen binding protein that binds BCMA comprises heavy chain variable region of SEQ. ID. NO: 23 and light chain variable region of SEQ. ID. NO: 31.

9. The combination of claim 2, wherein the antigen binding protein that binds PD-1 is pembrolizumab or nivolumab.

10. The combination of claim 2, wherein the antigen binding protein that binds OX40 comprises CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

11. The combination of claim 2 wherein the antigen binding protein that binds OX40 comprises heavy chain variable region of SEQ. ID. NO: 229 and light chain variable region of SEQ. ID. NO: 230.

12. A combination comprising:
i) an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID..NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) pembrolizumab or nivolumab.

13. A combination comprising:
i) an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID.
ii) NO: 200, CDRL1 of SEQ. ID. NO: 4, CDRL2 of SEQ. ID. NO: 5, CDRL3 of SEQ. ID. NO: 6 or variants thereof conjugated to MMAF; and
iii) an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

14. A pharmaceutical composition comprising the combination of any one of claims 1 to 13.

15. A method of treating cancer in a mammal in need thereof comprising administering a therapeutically effective amount of a combination comprising a therapeutically effective amount of an antigen binding protein that binds BCMA and a therapeutically effective amount of at least one antigen binding protein that binds an immunomodulatory target.

16. The method of claim 15, wherein the immunomodulatory target is PD1 or OX40.

17. The method of claim 15, wherein the mammal is human.

18. The method of claim 15, wherein the cancer is multiple myeloma or non-Hodgkins lymphoma.

19. A method of treating cancer in a human in need thereof comprising administering a therapeutically effective amount of a combination comprising:
i) a therapeutically effective amount an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of pembrolizumab or nivolumab.

20. A method of treating cancer in a human in need thereof comprising administering a therapeutically effective amount of a combination comprising:
i) a therapeutically effective amount of an antigen binding protein that binds BCMA, wherein the antigen binding protein that binds BCMA is an immunoconjugate comprising an anti-BCMA antibody comprising CDRH1 of SEQ. ID. NO: 1, CDRH2 of SEQ. ID. NO: 2, CDRH3 of SEQ. ID. NO: 200, CDRL1 of SEQ. ID. NO: 4, CDRL2 of SEQ. ID. NO: 5, CDRL3 of SEQ. ID. NO: 6 or variants thereof conjugated to MMAF; and
ii) a therapeutically effective amount of an antigen binding protein that binds OX40 comprising CDRH1 of SEQ. ID. NO: 219, CDRH2 of SEQ. ID. NO: 220, CDRH3 of SEQ. ID. NO: 221, CDRL1 of SEQ. ID. NO: 222, CDRL2 of SEQ. ID. NO: 223, CDRL3 of SEQ. ID. NO: 224, or variants thereof.

21. A combination according to claims 1-13 for use in the treatment of cancer.

22. Use of the combination according to claims 1-13 in the manufacture of a medicament for the treatment of cancer.
